(19) Europäisches Patentamt
European Patent Office
Office européen des brevets

(11) **EP 4 620 959 A1**

(12) **EUROPEAN PATENT APPLICATION**
published in accordance with Art. 153(4) EPC

(43) Date of publication:
**24.09.2025 Bulletin 2025/39**

(21) Application number: **23890903.0**

(22) Date of filing: **17.11.2023**

(51) International Patent Classification (IPC):
***C07D 487/16*** (2006.01) ***C07D 498/16*** (2006.01)
***C07D 487/22*** (2006.01) ***A61K 31/519*** (2006.01)
***A61K 31/53*** (2006.01) ***A61K 31/554*** (2006.01)
***A61P 35/00*** (2006.01) ***A61P 37/00*** (2006.01)
***A61P 29/00*** (2006.01) ***A61P 1/00*** (2006.01)
***A61P 17/00*** (2006.01) ***A61P 3/10*** (2006.01)
***A61P 27/02*** (2006.01) ***A61P 9/14*** (2006.01)
***A61P 25/16*** (2006.01)

(52) Cooperative Patent Classification (CPC):
**A61K 31/519; A61K 31/529; A61K 31/53; A61K 31/554; A61P 1/00; A61P 1/02; A61P 1/04; A61P 1/06; A61P 1/16; A61P 1/18; A61P 3/10; A61P 7/08; A61P 9/10; A61P 9/14; A61P 11/00;**
(Cont.)

(86) International application number:
**PCT/CN2023/132436**

(87) International publication number:
**WO 2024/104475 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.11.2022 CN 202211440715**

(71) Applicant: **Shanghai Institute of Organic Chemistry, Chinese Academy of Sciences**
**Shanghai 200032 (CN)**

(72) Inventors:
- **WANG, Zhaoyin**
  **Shanghai 200032 (CN)**
- **GUO, Weichen**
  **Shanghai 200032 (CN)**
- **YANG, Shuailong**
  **Shanghai 200032 (CN)**

(74) Representative: **Mewburn Ellis LLP**
**Aurora Building**
**Counterslip**
**Bristol BS1 6BX (GB)**

(54) **CYCLIC COMPOUNDS USED AS MULTI-TARGET KINASE INHIBITORS AND PREPARATION METHOD THEREFOR**

(57) The present invention discloses cyclic compounds as multi-target kinase inhibitors and preparation methods thereof. The multi-target kinase inhibitors of the present invention are as shown in general formula I, wherein $R^1$, $R^2$, $R^3$, $R^{3a}$, $L^1$, $L^2$, $L^3$, ring A, and ring B are as shown in the Specification and Claims. The present invention also discloses preparation methods of general formula I and its inhibitory activity against multiple kinases. The compounds of general formula I described in the present invention can be used for treating cancers and neurodegenerative diseases such as Parkinson's disease, etc.

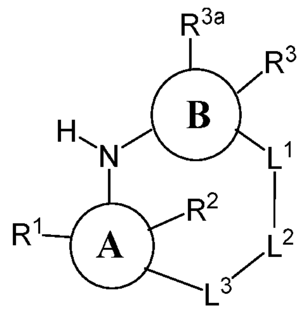

I

EP 4 620 959 A1

(52) Cooperative Patent Classification (CPC): (Cont.)
**A61P 11/06; A61P 13/12; A61P 17/00;
A61P 17/06; A61P 19/02; A61P 19/06;
A61P 19/08; A61P 25/00; A61P 25/14;
A61P 25/16; A61P 25/28; A61P 27/02;
A61P 29/00; A61P 31/04; A61P 31/06;
A61P 35/00; A61P 37/00; A61P 37/02;
A61P 37/06; A61P 37/08; C07D 487/16;
C07D 487/18; C07D 487/22; C07D 498/16;
C07D 498/18**

## Description

### TECHNICAL FIELD

The present invention provides a multi-target kinase inhibitor with a cyclic structure, and specifically relates to a preparation method thereof and its use in the treatment and prevention of kinase-mediated inflammatory diseases, cancers, and neurodegenerative diseases, such as cancers, Parkinson's disease, and Alzheimer's disease. The present invention also unexpectedly discovered that several cyclic compounds possess blood-brain barrier permeability.

### BACKGROUND

Leucine-rich repeat kinase 2 (LRRK2) is the most commonly mutated gene in familial Parkinson's disease (PD) (Monfrini, E. & Di Fonzo, A. Leucine-Rich Repeat Kinase (LRRK2) Genetics and Parkinson'sDisease. Adv. Neurobiol. 14, 3-30 (2017)). Mutations in the LRRK2 gene are also associated with idiopathic PD (Di Maio, R. et al. LRRK2 activation in idiopathic Parkinson's disease. Sci. Transl. Med. 10, (2018)), therefore, LRRK2 kinase inhibitors may be an effective therapeutic drug for PD. In addition, LRRK2 mutations are also associated to immune-related diseases, such as inflammatory bowel diseases (IBDs), and represent a potential target for treating such conditions (Wallings R.L., et al. LRRK2 at the Interface Between Peripheral and Central Immune Function in Parkinson's). Mutations in tyrosine kinases such as FGFR and VEGFR, etc., are clearly associated with the occurrence and abnormal development of a number of cancers. Therefore, inhibitors of these kinases may serve as effective therapeutic drugs for treating cancers, other neurodegenerative diseases such as Parkinson's disease, heteroplasia, and Alzheimer's disease, etc.

### SUMMARY

The present invention discloses a class of cyclic compounds exhibiting high-efficiency inhibition against multiple kinases.

Another objective of the present invention is to provide methods for preparing said compounds.

In a first aspect, the present invention provides a compound as shown in general formula I, or various isomers and pharmaceutically acceptable salts thereof:

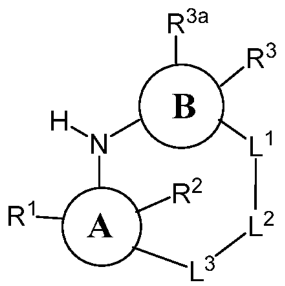

I

wherein:

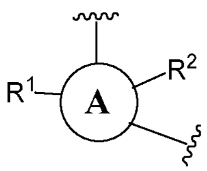

is selected from the following ring structures:

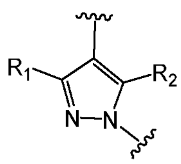 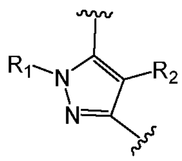 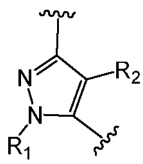 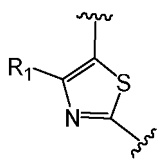

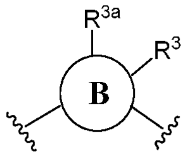

is selected from the following ring structures:

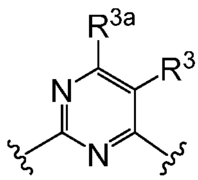
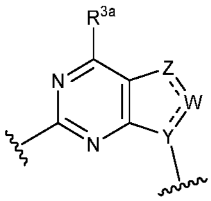
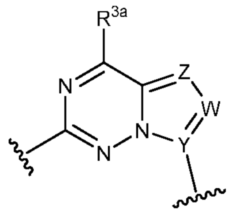
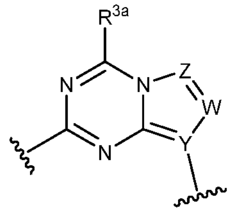

wherein:

$R^1$ is selected from hydrogen, halo, cyano, $SF_5$, $C_{1-12}$ alkyl, $C_{2-12}$ alkenyl, $C_{2-12}$ alkynyl, $C_{1-12}$ alkoxy, $C_{3-10}$ cycloalkyl, $-NR^4R^5$, 3-10 membered heterocyclyl, $C_{6-10}$ aryl, and 5-10 membered heteroaryl; wherein each alkyl, alkenyl, alkynyl, alkoxy, cycloalkyl, heterocyclyl, aryl, and heteroaryl of $R^1$ is optionally and independently substituted with one or more groups selected from halogen, hydroxy, amino, cyano, $C_{1-6}$ alkoxy, and oxo (=O);

$R^2$ is selected from hydrogen, halogen, cyano, $C_{1-12}$ alkyl, $C_{2-6}$ alkenyl, $C_{2-6}$ alkynyl, $C_{1-6}$ alkoxy, $C_{3-6}$ cycloalkyl, $C(O)R^4$, $C(O)OR^4$, $-NR^4R^5$, $C(O)NR^4R^5$, $SO_2R^4$, $SO_2NR^4R^5$, wherein each alkyl, alkenyl, alkynyl, alkoxy, and cycloalkyl of $R^2$ is optionally and independently substituted with one or more groups selected from halo, hydroxy, amino, and cyano;

$R^{3a}$ is selected from hydrogen, halo, cyano, $C_{1-12}$ alkyl, $C_{2-6}$ alkenyl, $C_{2-6}$ alkynyl, $C_{1-6}$ alkoxy, $C_{3-6}$ cycloalkyl, $C(O)R^4$, $C(O)OR^4$, $-NR^4R^5$, $C(O)NR^4R^5$, $SO_2R^4$, $SO_2NR^4R^5$, wherein each alkyl, alkenyl, alkynyl, alkoxy, and cycloalkyl of $R^{3a}$ is optionally and independently substituted with one or more groups selected from halo, hydroxy, amino, and cyano;

$R^3$ is independently selected from hydrogen, halo, cyano, $SF_5$, $C_{1-12}$ alkyl, $C_{1-12}$ haloalkyl, $C_{2-6}$ alkenyl, $C_{2-6}$ alkynyl, $C_{3-10}$ cycloalkyl, $C_{1-6}$ alkoxy, $-C(O)R^4$, $-C(O)OR^4$, $-NR^4R^5$, $-C(O)NR^4R^5$, $SO_2R^4$, $SO_2NR^4R^5$, wherein the alkyl of $R^3$ is optionally substituted with one or more halo or $C_{1-3}$ alkoxy; $R^3$ and $R^{3a}$ may join to form a 5-membered heterocycle or heteroaryl ring containing N, $NR^6$, $NC_{1-6}$ alkyl, O, or S; or two $R^3$ join together to form =O;

$R^4$ and $R^5$ are each independently selected from hydrogen, $C_{1-12}$ alkyl, $C_{2-12}$ alkenyl, $C_{2-12}$ alkynyl, $C_{1-12}$ alkoxy, $C_{3-10}$ cycloalkyl, 3-10 membered heterocyclyl, $C_{6-10}$ aryl, and 5-10 membered heteroaryl, wherein each alkyl, alkenyl, alkynyl, alkoxy, cycloalkyl, heterocyclyl, aryl, and heteroaryl of $R^4$ or $R^5$ is independently and optionally substituted with one or more groups selected from halo, hydroxy, amino, nitro, cyano, ($C_{1-6}$) alkoxy, and oxo (=O); or $R^4$ and $R^5$ together with the atoms to which they are attached form an optionally substituted heterocyclyl;

Y is a carbon atom or nitrogen atom, provided that the requirements for the necessary covalent bonds are satisfied;

Z and W are independently CH, $CH_2$, $(CR^3{}_2)_t$, $(CR^3)_t$, NH, O, S, or N; or Z and W together with the substituents to which they are attached form a 3-7 membered ring, comprising a cycloalkane ring,

cycloalkene ring, heterocyclic or heteroaryl ring containing 1-3 heteroatoms selected from N, O, or S; or when Z or W is $(CR^3{}_2)_t$, any two $R^3$ join to the carbon atom to form =O or a 3-6 membered ring, and the ring comprises a cycloalkane or cycloalkene ring;

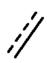 represents a single bond or a double bond; and

t is 1, 2, or 3.

Preferably, in the compound of general formula I,

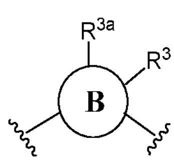

is selected from the following ring structures:

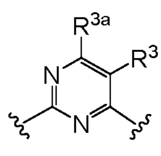
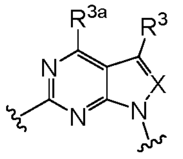
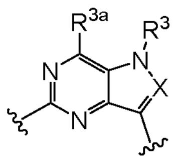
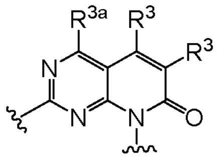
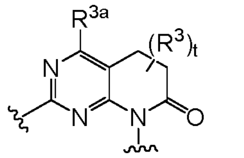
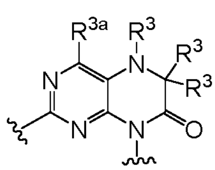
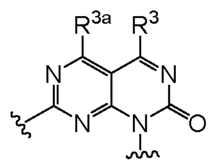
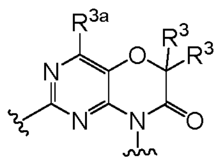
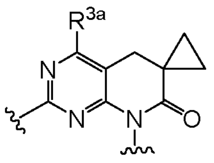
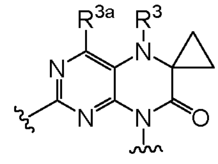
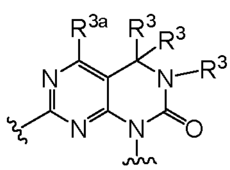
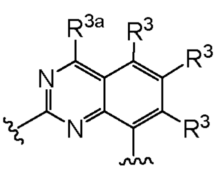
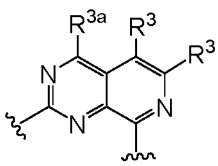
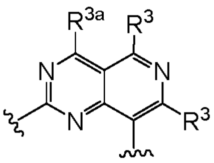
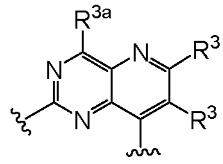
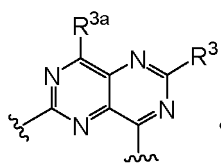
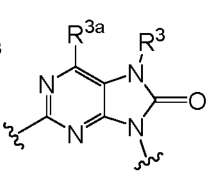
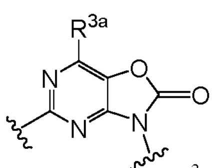
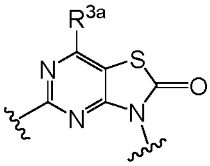
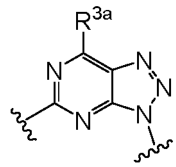
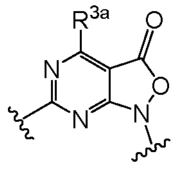
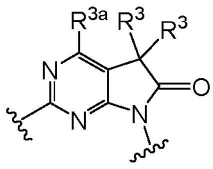
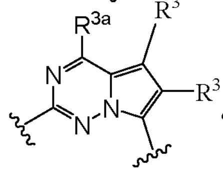
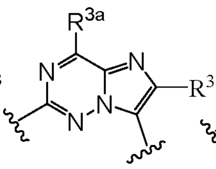
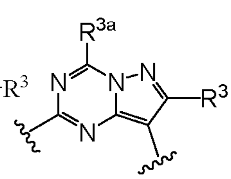
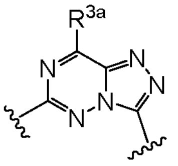
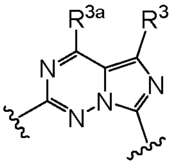
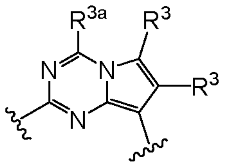
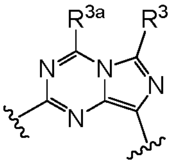
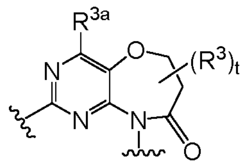
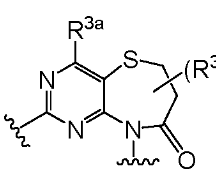
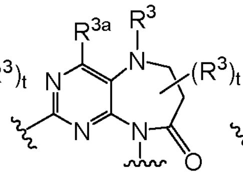
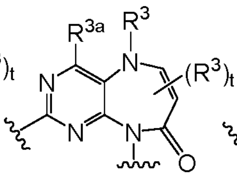
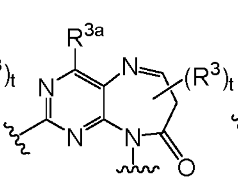
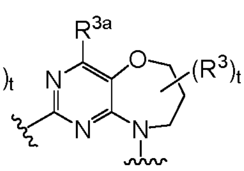
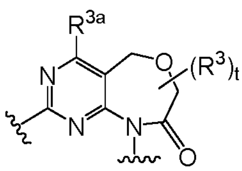
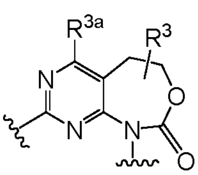
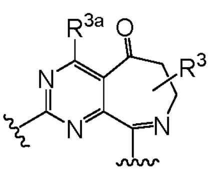
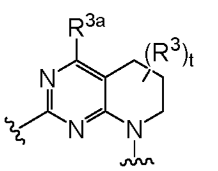
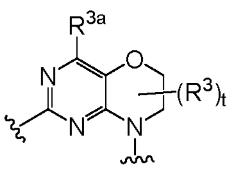

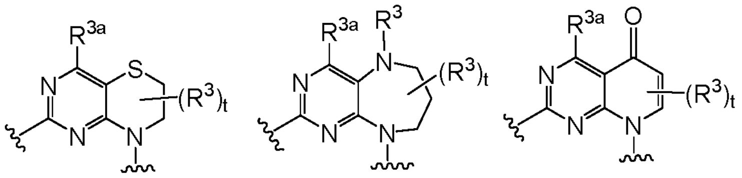

two $R^3$, together with the same carbon atom or different carbon atoms to which they are attached, form a 3- to 10-membered carbocycle or heterocycle, wherein the heteroatom is selected from N, NH, $NC_{1-6}$ alkyl, O, or S;

$L^1$ is selected from a chemical bond, $-(CR^aR^b)_nNR^6(CR^aR^b)_m-$, $-O(CR^aR^b)_nNR^6(CR^aR^b)_m-$, $-(CR^aR^b)_nNR^6C(O)-$, $-O(CR^aR^b)_nNR^6C(O)-$, $-(CR^aR^b)_nC(O)NR^6-$, $-O(CR^aR^b)_nC(O)NR^6-$, $-(CR^aR^b)_nNR^6(CR^aR^b)_mNR^6-$, $-(CR^aR^b)_nNR^6(CR^aR^b)_m-$, $-(CR^aR^b)_nO(CR^aR^b)_m-$, $-NR^6(CR^aR^b)_nO(CR^aR^b)_m-$, $-NR^6(CR^aR^b)_mNR^6-$, $-(CR^aR^b)_nO(CR^aR^b)_mNR^6-$, $-(CR^aR^b)_nO(CR^aR^b)_mO-$, $-(CR^aR^b)_nS(CR^aR^b)_m-$, $-(CR^aR^b)_nS(CR^aR^b)_mNR^6-$, $-(CR^aR^b)_m-$, $-NR^6(CR^aR^b)_m-$, $-(CR^aR^b)_mNR^6-$, $-O(CR^aR^b)_m-$, $-(CR^aR^b)_m,O-$, $-O(CR^aR^b)_mO-$, $-(CR^aR^b)_nNR^6C_{3-6}$ cycloalkyl-, $-(CR^aR^b)_nNR^6C_{3-6}$ cycloalkyl-$NR^6$-, $-O(R^aR^b)_qCH=CH(R^aR^b)_r-$, $-(CR^aR^b)_qCH=CH(CR^aR^b)_r-$, $-O(R^aR^b)_qCH=CH(R^aR^b)_rNR^6-$, $O(CR^aR^b)_qCH=CH(CR^aR^b)_rNR^6C(O)-$, $-O(CR^aR^b)_qCH=CH(CR^aR^b)_rC(O)NR^6-$, $-(CR^aR^b)_qCH=CH(CR^aR^b)_rNR^6-$, $-(CR^aR^b)_qCH=CH(CR^aR^b)_rNR^6CO-$, $-(CR^aR^b)_qCH=CH(CR^aR^b)_rC(O)NR^6-$, $-O(R^aR^b)_nCH=CH(R^aR^b)_mO-$, $-NR^6(R^aR^b)_nCH=CH(R^aR^b)_m-$, $-NR^6(R^aR^b)_nCH=CH(R^aR^b)_mO-$, $-NR^6(R^aR^b)_nCH=CH(R^aR^b)_mS-$, $-NR^6(R^aR^b)_nCH=-CH(R^aR^b)_mNR^6-$, $-S(R^aR^b)_nCH=CH(R^aR^b)_m-$, $S(R^aR^b)_nCH=CH(R^aR^b)_mNR^6-$, $-NR^6(R^aR^b)_nCH=CH(R^aR^b)_mS-$, $-(R^aR^b)_nCH=CH(R^aR^b)_mS-$, $-O(R^aR^b)_nCH=CH(R^aR^b)_mS-$, $-S(R^aR^b)_nCH=CH(R^aR^b)_mO-$, -phenyl-, -heterocyclyl-, -heteroaryl-, and $NR^6(CR^aR^b)_nC=C(CR^aR^b)_m-$;

n is selected from 0, 1, 2, 3, 4, 5, 6, 7, 8, 9, or 10;

m is selected from 1, 2, 3, 4, 5, 6, 7, 8, 9, or 10;

$L^2$ is selected from $-CR^aR^b-$, $-CR^{aa}=CR^{bb}-$, -C(O)-,-C(O)C(O)-, -C(S)-, $-S(O)_2-$, $-C_{3-6}$cycloalkyl-,-phenyl-, -5- or 6-membered heterocyclyl-, and -5- or 6-membered heteroaryl-;

$L^3$ is selected from a chemical bond, $-(CR^aR^b)_q-$,$-(CR^aR^b)_q-NR^6-$,$-NR^6(CR^aR^b)_q-$,$-O(CR^aR^b)_q-NR^6-$, $-NR^6(CR^aR^b)_qO-$,$-O(CR^aR^b)_q-$, $-(CR^aR^b)_qO-$, $-(CR^aR^b)_q-C_{3-6}$cycloalkyl-, 5- or 6-membered heterocyclyl, $-(CR^aR^b)_q-CR^{aa}=CR^{bb}-$, $-C_{3-6}$cycloalkyl-$NR^6$-, $-(CR^aR^b)_qNR^6-$, $-(CR^aR^b)_qNR^6C(O)-$, $-(CR^aR^b)_qNR^6C(O)O-$, $-(CR^aR^b)qC(O)NR^6-$, and $(CR^aR^b)_qOC(O)NR^6-$;

q is selected from 0, 1, 2, 3, 4, 5, or 6;

r is an integer from 1 to 4;

$R^6$ is each independently selected from hydrogen,$C_{1-6}$alkyl,$C_{3-6}$cycloalkyl,$-C(O)C_{1-6}$alkyl,$-C(O)C_{1-6}$aryl,$-C(O)C_{1-6}$heteroaryl, $-C(O)C_{3-6}$cycloalkyl,$-S(O)_2C_{1-6}$alkyl, $-S(O)_2C_{1-6}$aryl, $-S(O)_2C_{1-6}$heteroaryl, $-S(O)_2C_{3-6}$cycloalkyl,$-C(O)NR^7C_{1-6}$aryl, $-C(O)NR^7C_{1-6}$heteroaryl, $-C(O)NR^7C_{3-6}$cycloalkyl,$-SO_2NR^7C_{1-6}$alkyl, $-S(O)_2NR^7C_{1-6}$aryl, $-S(O)_2NR^7C_{1-6}$heteroaryl, $-S(O)_2NR^7C_{3-6}$cycloalkyl, $-C(O)C(O)NR^7C_{1-6}$aryl, $-C(O)C(O)NR^7C_{1-6}$heteroaryl, $-C(O)C(O)NR^7C_{3-6}$cycloalkyl; wherein each alkyl, alkenyl, alkynyl, alkoxy, cycloalkyl, heterocyclyl, aryl, and heteroaryl of $R^6$ is optionally and independently substituted with one or more groups selected from halo, hydroxy, amino, cyano, $(C_{1-6})$alkoxy, and oxo (=O);

$R^7$ is selected from hydrogen, $C_{1-6}$alkyl, $C_{3-6}$ cycloalkyl, heterocyclyl, aryl, and heteroaryl; wherein each alkyl, cycloalkyl,heterocyclyl, aryl, and heteroaryl of $R^7$ is optionally and independently substituted with one or more groups selected from halo, hydroxy, amino, cyano, $(C_{1-6})$alkoxy, and oxo (=O);

$R^a$ and $R^b$ are independently selected from hydrogen, halogen, cyano, $C_{1-6}$ alkyl, $C_{1-6}$haloalkyl, $C_{1-6}$cycloalkyl, $C_{1-6}$halocycloalkyl,$C_{1-6}$alkoxy,$C_{1-6}$haloalkoxy, hydroxy, $C(O)NH_2$, $C(O)NHC_{1-6}$alkyl, $C(O)N(C_{1-6}alkyl)_2$, $C_{1-6}$alkylsulfonyl, $S(O)_2NH_2$, $S(O)_2NHC_{1-6}$alkyl, $NHC(O)NH_2$, $NHC(O)NHC_{1-6}$alkyl, $NHC(O)OC_{1-6}$alkyl, $C(O)-C_{1-6}$alkyl, $C_{1-6}$ heteroalkyl, heterocyclyl, or heterocyclylalkyl; or $R^a$ and $R^b$ together with the carbon atom to which they are attached may form a 3- to 6-membered carbocycle or heterocycle, wherein the heteroatom in the heterocycle is selected from $NR^6$, O, or S; $R^{aa}$ and $R^{bb}$ are independently selected from hydrogen, halogen, cyano, $C_{1-6}$alkyl, $C_{1-6}$haloalkyl, $C_{1-6}$cycloalkyl, $C_{1-6}$halocycloalkyl; or $R^{aa}$ and $R^{bb}$ together with the carbon atom to which they are attached may form a 3- to 6-membered carbocycle or heterocycle, wherein the heteroatom in the heterocycle is selected from $NR^6$, O, or S.

In another preferred embodiment, $R^1$ and $R^2$ are each independently selected from hydrogen, halo, cyano, $SF_5$, $C_{1-6}$ alkyl, $C_{1-6}$ haloalkyl, $C_{2-6}$ alkenyl, $C_{2-6}$ alkynyl, $C_{1-6}$ alkoxy, $C_{3-6}$ cycloalkyl, heterocyclyl, aryl, and heteroaryl.

Preferably, the compound of general formula I is a compound as shown in formula II:

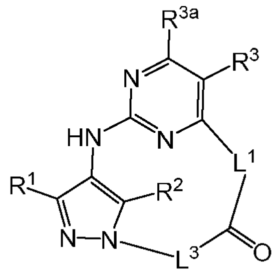

II

wherein $R^1$, $R^2$, $R^3$, $R^{3a}$, $L^1$, and $L^3$ are as defined in general formula I.

In another preferred embodiment, the compound of general formula I is a compound as shown in formula III:

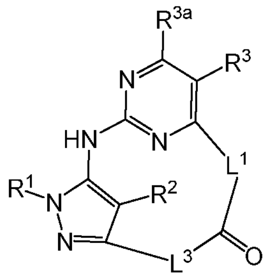

III

wherein $R^1$, $R^2$, $R^3$, $R^{3a}$, $L^1$, and $L^3$ are as defined in general formula I.

In another preferred embodiment, the compound of general formula I is a compound as shown in formula IV:

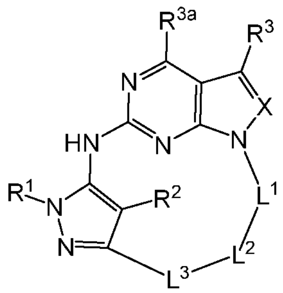

IV

wherein $R^1$, $R^2$, $R^3$, $R^{3a}$, $L^1$, $L^2$, and $L^3$ are as defined in formula I;
X is N or $CR^x$; $R^x$ is selected from the group consisting of H, halogen, CN, $C_{1-6}$ alkyl, $C_{1-6}$ haloalkyl, $C_{3-6}$ cycloalkyl, $C_{3-6}$ halocycloalkyl, wherein the alkyl of $R^X$ is optionally substituted with one or more halo or $C_{1-3}$ alkoxy;

In another preferred embodiment, the compound of general formula I is a compound as shown in formula V:

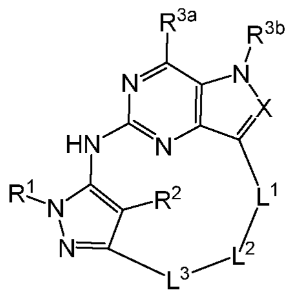

V

wherein $R^1$, $R^2$, $R^{3a}$, $L^1$, $L^2$, and $L^3$ are as defined in general formula I;
$R^{3b}$ is selected from hydrogen, $C_{1-12}$ alkyl, $C_{2-6}$ alkenyl, $C_{2-6}$ alkynyl, $C_{3-6}$ cycloalkyl, -C(O)$R^4$, - C(O)O$R^4$, and -C(O)N$R^4R^5$, wherein the alkyl and cycloalkyl of $R^{3b}$ are optionally substituted with one or more halo or $C_{1-3}$ alkoxy;
$R^4$ and $R^5$ are each independently selected from hydrogen, $C_{1-12}$ alkyl, $C_{2-12}$ alkenyl, $C_{2-12}$ alkynyl, $C_{1-12}$ alkoxy, $C_{3-10}$ cycloalkyl, heterocyclyl, aryl, and heteroaryl, wherein each alkyl, alkenyl, alkynyl, alkoxy, cycloalkyl, heterocyclyl, aryl, and heteroaryl of $R^4$ or $R^5$ is independently and optionally substituted with one or more groups selected from halo, hydroxy, amino, nitro, cyano, ($C_{1-6}$) alkoxy, and oxo (=O); or $R^4$ and $R^5$ together with the atom to which they are attached form an optionally substituted heterocyclyl;
X is N or C$R^x$; $R^x$ is selected from H, halogen, CN, $C_{1-6}$ alkyl, $C_{1-6}$ haloalkyl, $C_{3-6}$ cycloalkyl, or $C_{3-6}$ halocycloalkyl;

In another preferred embodiment, the compound of general formula I is a compound as shown in formula VI:

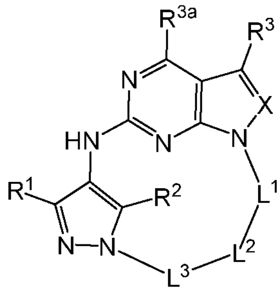

VI

wherein $R^1$, $R^2$, $R^3$, $R^{3a}$, $L^1$, $L^2$, and $L^3$ are as defined in formula I;
X is N or C$R^x$; $R^x$ is selected from H, halogen, CN, $C_{1-6}$ alkyl, $C_{1-6}$ haloalkyl, $C_{3-6}$ cycloalkyl, or $C_{3-6}$ halocycloalkyl.

In another preferred embodiment, the compound of general formula I is a compound as shown in formula VII:

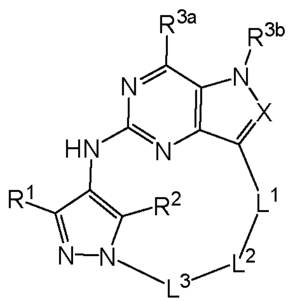

VII

wherein $R^1$, $R^2$, $R^{3a}$, $L^1$, $L^2$, and $L^3$ are as defined in general formula I;
$R^{3b}$ is selected from $C_{1-12}$ alkyl, $C_{1-12}$ haloalkyl, $C_{2-6}$ alkenyl, $C_{2-6}$ alkynyl, $C_{3-6}$ cycloalkyl, -C(O)$R^4$, -C(O)O$R^4$, and -C(O)N$R^4R^5$, wherein the alkyl of $R^{3b}$ is optionally substituted with one or more halo or $C_{1-3}$ alkoxy;
$R^4$ and $R^5$ are each independently selected from hydrogen, $C_{1-12}$ alkyl, $C_{2-12}$ alkenyl, $C_{2-12}$ alkynyl, $C_{1-12}$ alkoxy, $C_{3-10}$ cycloalkyl, heterocyclyl, aryl, and heteroaryl, wherein each alkyl, alkenyl, alkynyl, alkoxy, cycloalkyl, heterocyclyl, aryl, and heteroaryl of $R^4$ or $R^5$ is independently and optionally substituted with one or more groups selected from halo, hydroxy, amino, nitro, cyano, ($C_{1-6}$) alkoxy, and oxo (=O); or $R^4$ and $R^5$ together with the atom to which they are attached form an optionally substituted heterocyclyl;
X is N or C$R^x$; $R^x$ is selected from H, halogen, CN,$C_{1-6}$ alkyl, $C_{1-6}$ haloalkyl, $C_{3-6}$ cycloalkyl, or $C_{3-6}$ halocycloalkyl;

In another preferred embodiment, the compound of general formula I is a compound as shown in formula VIII:

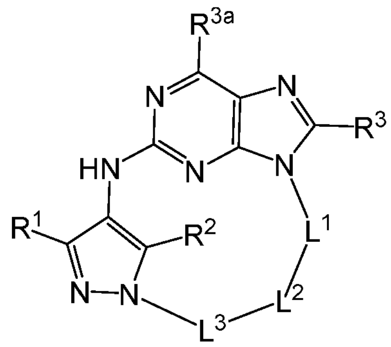

VIII

wherein $R^1$, $R^2$, $R^3$, $R^{3a}$, $L^1$, $L^2$, and $L^3$ are as defined in general formula I.

In another preferred embodiment, the compound of general formula I is a compound as shown in formula IX:

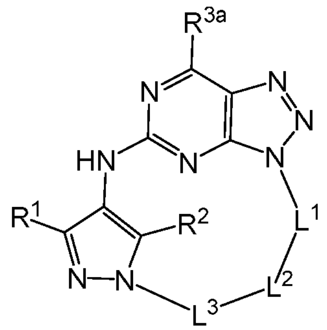

IX

wherein $R^1$, $R^2$, $R^{3a}$, $L^1$, $L^2$, and $L^3$ are as defined in general formula I.

In another preferred embodiment, the compound of general formula I is a compound as shown in formula X:

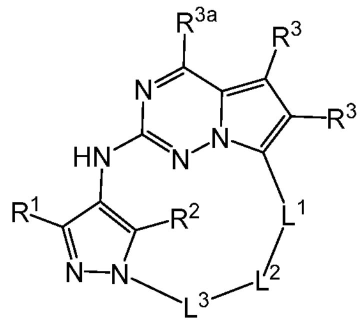

X

wherein $R^1$, $R^2$, $R^{3a}$, $L^1$, $L^2$, and $L^3$ are as defined in general formula I.

$R^3$ is independently selected from hydrogen, halo, cyano, $SF_5$, $C_{1-12}$ alkyl, $C_{1-12}$ haloalkyl, $C_{2-6}$ alkenyl, $C_{2-6}$ alkynyl, $C_{3-6}$ cycloalkyl, -C(O)$R^4$, -C(O)O$R^4$, and -C(O)N$R^4R^5$, wherein the alkyl of $R^3$ is optionally substituted with one or more halo or $C_{1-3}$ alkoxy;

$R^4$ and $R^5$ are each independently selected from hydrogen, $C_{1-12}$ alkyl, $C_{2-12}$ alkenyl, $C_{2-12}$ alkynyl, $C_{1-12}$ alkoxy, $C_{3-10}$ cycloalkyl, heterocyclyl, aryl, and heteroaryl, wherein each alkyl, alkenyl, alkynyl, alkoxy, cycloalkyl, heterocyclyl, aryl, and heteroaryl of $R^4$ or $R^5$ is independently and optionally substituted with one or more groups selected from halo, hydroxy, amino, nitro, cyano, ($C_{1-6}$) alkoxy, and oxo (=O); or $R^4$ and $R^5$ together with the atom to which they are attached form an optionally substituted heterocyclyl;

In another preferred embodiment, the compound of general formula I is a compound as shown in formula XI:

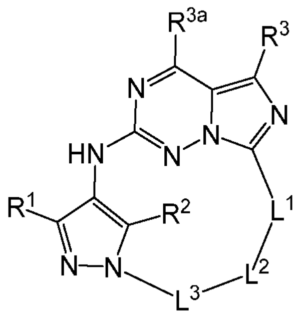

XI

wherein $R^1$, $R^2$, $R^3$, $R^{3a}$, $L^1$, $L^2$, and $L^3$ are as defined in general formula I.

In another preferred embodiment, the compound of general formula I is a compound as shown in formula XII:

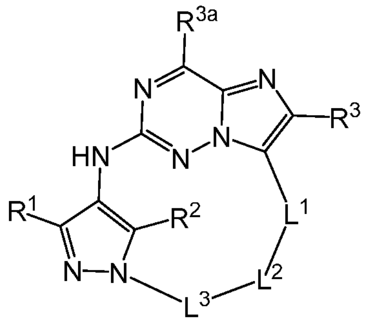

XII

wherein $R^1$, $R^2$, $R^3$, $R^{3a}$, $L^1$, $L^2$, and $L^3$ are as defined in general formula I.

In another preferred embodiment, the compound of general formula I is a compound as shown in formula XIII:

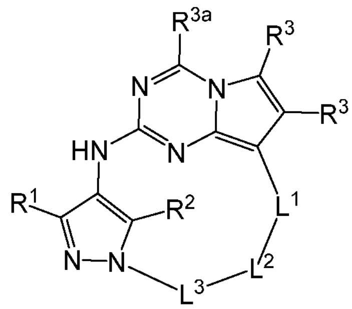

XIII

wherein $R^1$, $R^2$, $R^{3a}$, $L^1$, $L^2$, and $L^3$ are as defined in general formula I;

$R^3$ is independently selected from hydrogen, halo, cyano, $SF_5$, $C_{1-12}$ alkyl, $C_{1-12}$ haloalkyl, $C_{2-6}$ alkenyl, $C_{2-6}$ alkynyl, $C_{3-6}$ cycloalkyl, $-C(O)R^4$, $-C(O)OR^4$, and $-C(O)NR^4R^5$, wherein the alkyl of $R^3$ is optionally substituted with one or more halo or $C_{1-3}$ alkoxy;

$R^4$ and $R^5$ are each independently selected from hydrogen, $C_{1-12}$ alkyl, $C_{2-12}$ alkenyl, $C_{2-12}$ alkynyl, $C_{1-12}$ alkoxy, $C_{3-10}$ cycloalkyl, heterocyclyl, aryl, and heteroaryl, wherein each alkyl, alkenyl, alkynyl, alkoxy, cycloalkyl, heterocyclyl, aryl, and heteroaryl of $R^4$ or $R^5$ is independently and optionally substituted with one or more groups selected from halo, hydroxy, amino, nitro, cyano, $(C_{1-6})$alkoxy, and oxo (=O); or $R^4$ and $R^5$ together with the atom to which they are attached form an optionally substituted heterocyclyl.

In another preferred embodiment, the compound of general formula I is a compound as shown in formula XIV:

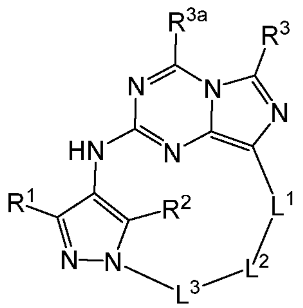

XIV

wherein $R^1$, $R^2$, $R^3$, $R^{3a}$, $L^1$, $L^2$, and $L^3$ are as defined in general formula I.

In another preferred embodiment, the compound of general formula I is a compound as shown in formula XV:

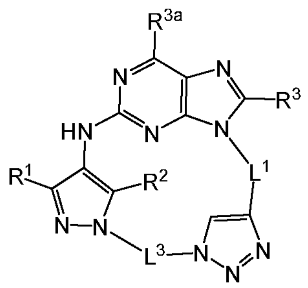

XV

wherein $R^1$, $R^2$, $R^3$, $R^{3a}$, $L^1$, and $L^3$ are as defined in general formula I.

In another preferred embodiment, the compound of general formula I is a compound as shown in formula XVI:

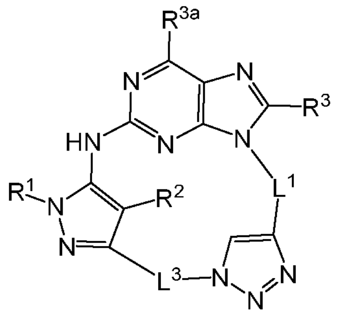

XVI

wherein $R^1$, $R^2$, $R^3$, $R^{3a}$, $L^1$, and $L^3$ are as defined in general formula I.

In another preferred embodiment, the compound of general formula I is a compound as shown in formula XVII:

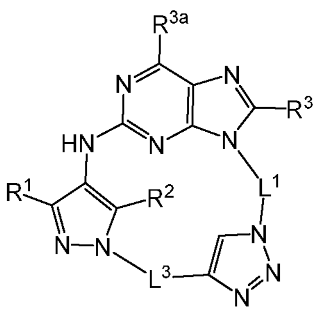

XVII

wherein $R^1$, $R^2$, $R^3$, $R^{3a}$, $L^1$, and $L^3$ are as defined in general formula I.

In another preferred embodiment, the compound of general formula I is a compound as shown in formula XVIII:

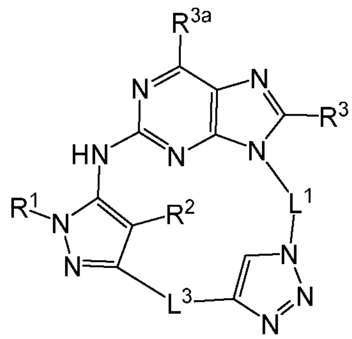

XVIII

wherein $R^1$, $R^2$, $R^3$, $R^{3a}$, $L^1$, and $L^3$ are as defined in general formula I.

In another preferred embodiment, the compound of general formula I is a compound as shown in formula XIX:

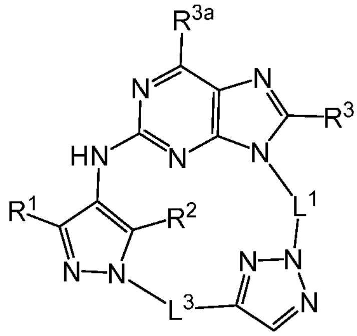

XIX

wherein $R^1$, $R^2$, $R^3$, $R^{3a}$, $L^1$, and $L^3$ are as defined in general formula I.

In another preferred embodiment, the compound of general formula I is a compound as shown in formula XX:

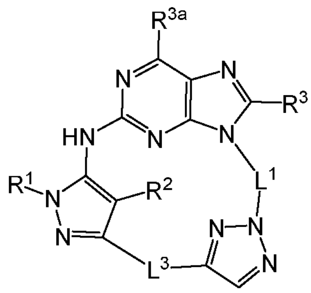

XX

wherein $R^1$, $R^2$, $R^3$, $R^{3a}$, $L^1$, and $L^3$ are as defined in general formula I.

In another preferred embodiment, the compound of general formula I is a compound as shown in formula XXI:

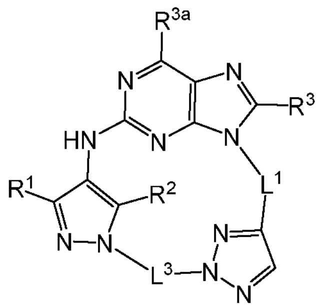

XXI

wherein $R^1$, $R^2$, $R^3$, $R^{3a}$, $L^1$, and $L^3$ are as defined in general formula I.

In another preferred embodiment, the compound of general formula I is a compound as shown in formula XXII:

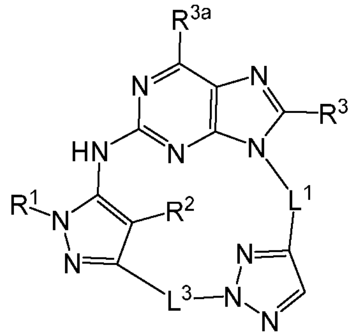

XXII

wherein $R^1$, $R^2$, $R^3$, $R^{3a}$, $L^1$, and $L^3$ are as defined in general formula I.

In another preferred embodiment, in all general formulas, $R^1$ and $R^2$ are independently selected from hydrogen, halogen, $C_{1-6}$alkyl, $C_{1-6}$ haloalkyl, or $C_{3-6}$ cycloalkyl;

$R^3$ is selected from hydrogen, halogen,$NR^4R^5$,CF3, $SF_5$, or CN;
$R^{3a}$ is selected from hydrogen, halogen, CF, CN, $NR^4R^5$, $SO_2Me$, $SO_2$ cyclopropane, or $SO_2NR^4R^5$
$L^3$ is selected from:

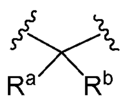 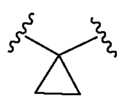 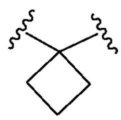 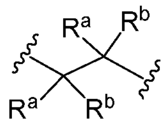 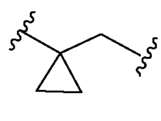 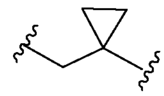

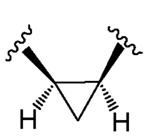 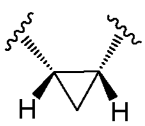 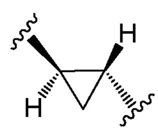 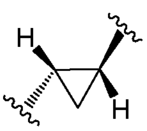 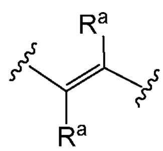 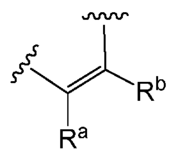

wherein $R^a$ and $R^b$ are independently selected from hydrogen, halogen, cyano, $C_{1-6}$ alkyl, $C_{1-6}$ haloalkyl, $C_{1-6}$ cycloalkyl, $C_{1-6}$ halocycloalkyl, $C_{1-6}$ alkoxy, $C_{1-6}$ haloalkoxy, or hydroxy; or $R^a$ and $R^b$ together with the carbon atom to which they are attached may form a 3- to 6-membered carbocycle or heterocycle, wherein the heteroatom in the heterocycle is selected from $NR^6$, O, or S; and $R^6$ is as defined above.

In another preferred embodiment, $L^2$ in the compound of general formula I is selected from -C(O)- or the following heterocyclic structures:

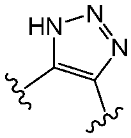 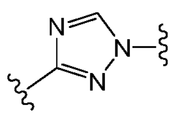 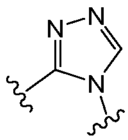 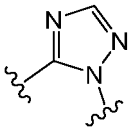 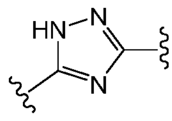 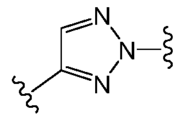

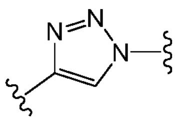 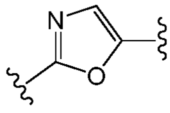 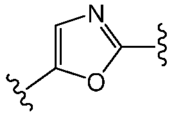 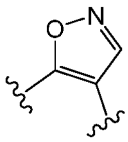 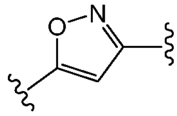 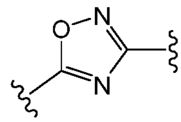

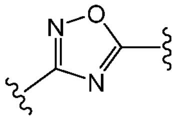 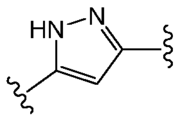 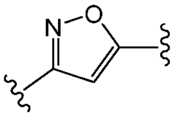 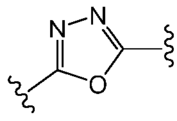 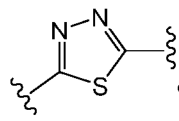 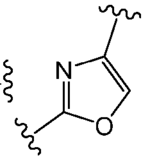

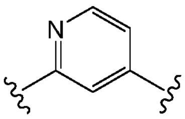 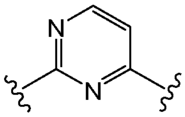 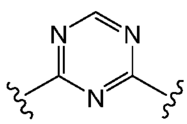 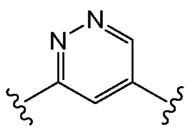 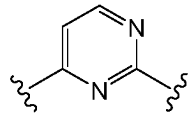

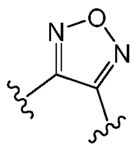 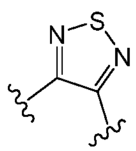 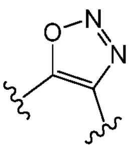 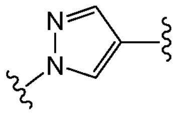 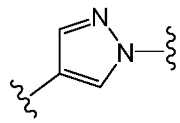

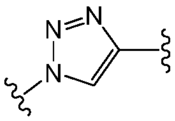 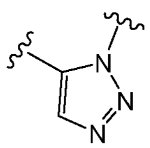 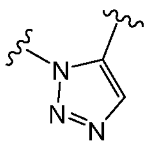

The aforementioned heterocycle may be substituted with one or two of halogen, -CN, $C_1$-$C_6$ alkyl, or $C_1$-$C_6$ haloalkyl;

In another preferred embodiment, the compounds of general formula I to general formulaXXII are:

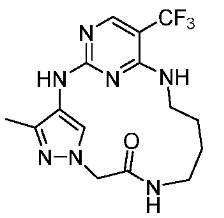 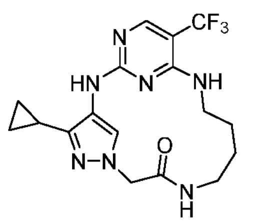 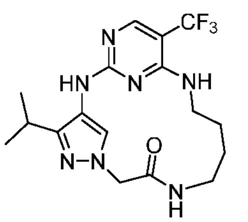 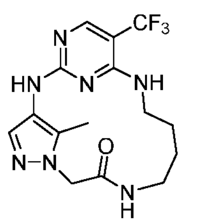

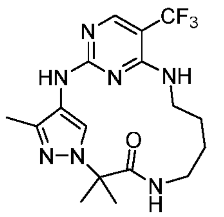 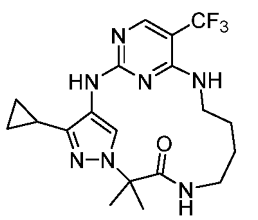 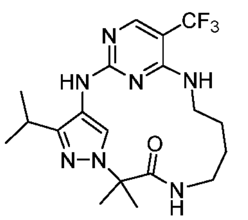 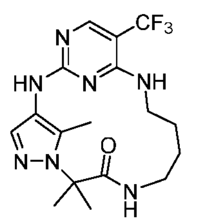

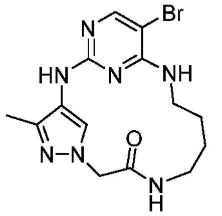
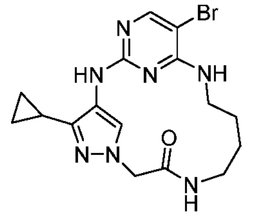
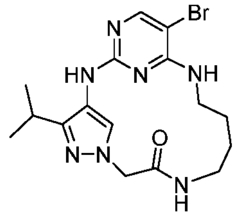
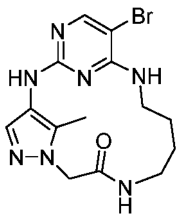

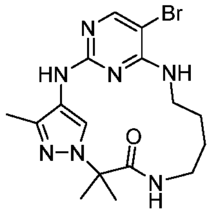
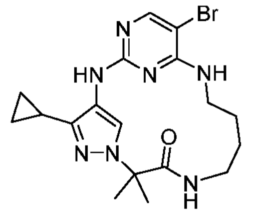
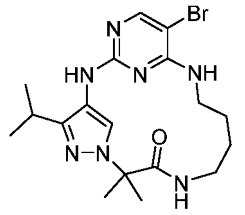
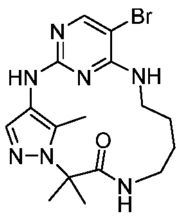

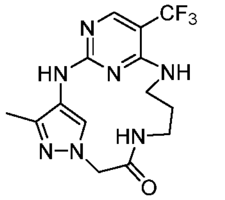
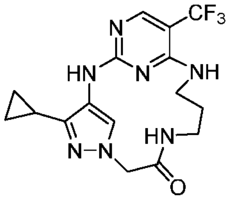
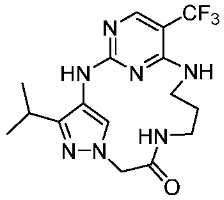
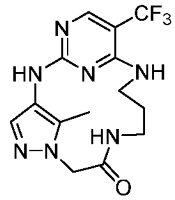

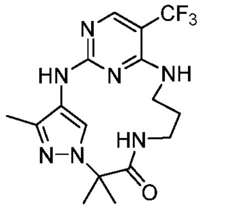
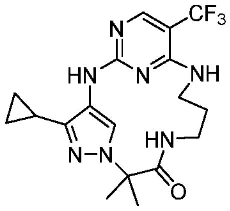
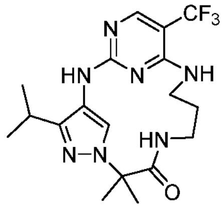
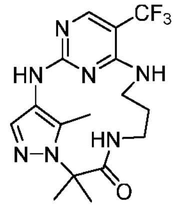

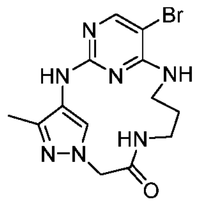
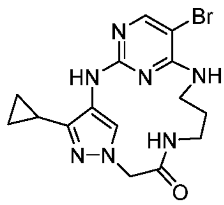
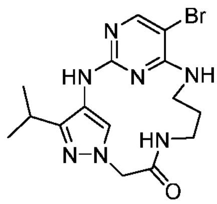
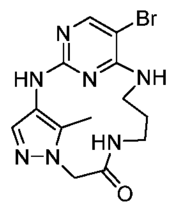

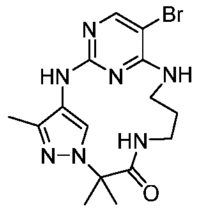
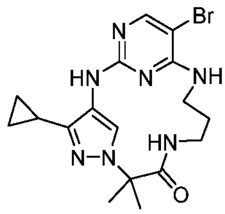
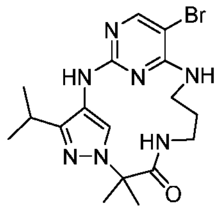
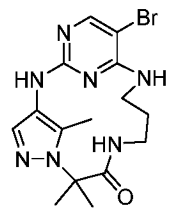

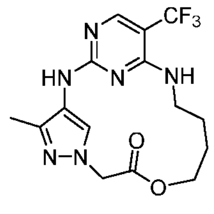
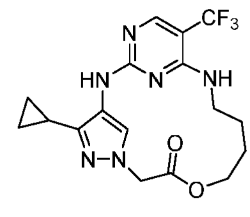
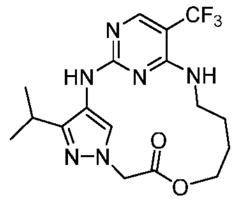
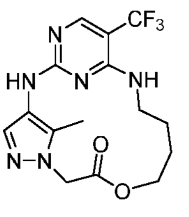

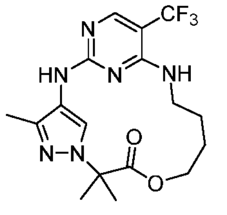
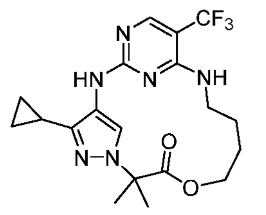
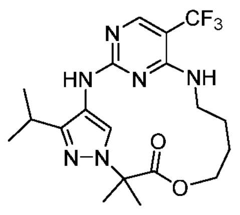
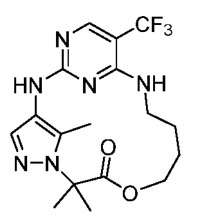

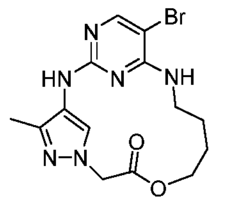
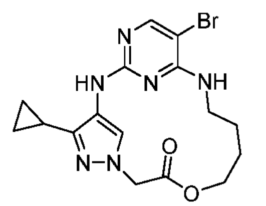
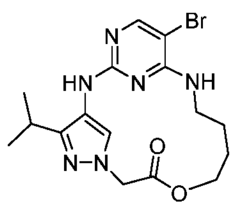
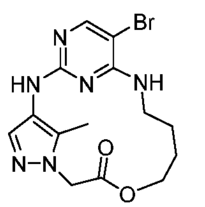

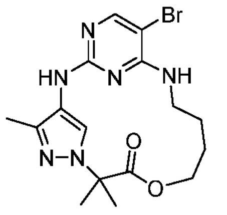
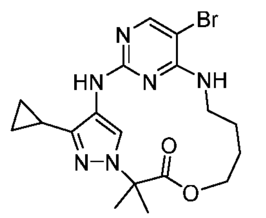
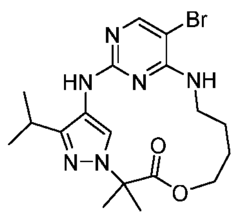
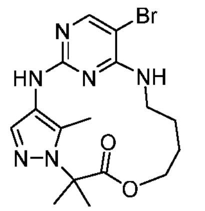

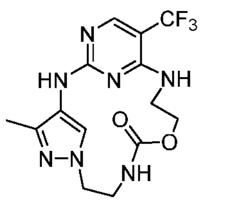
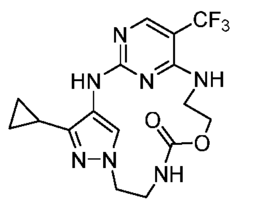
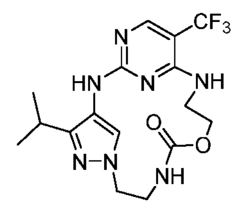
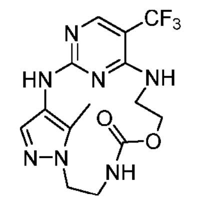

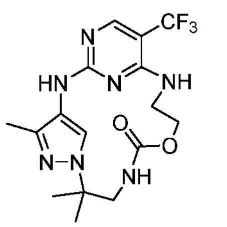
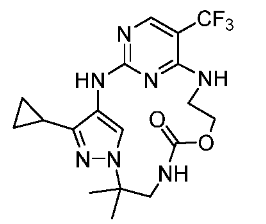
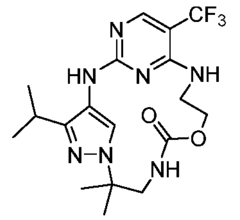
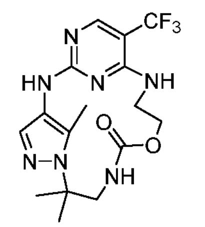

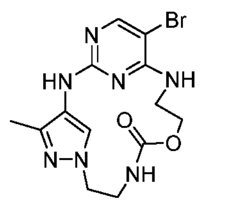
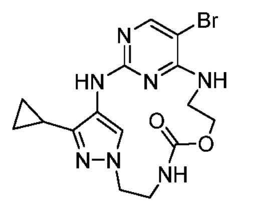
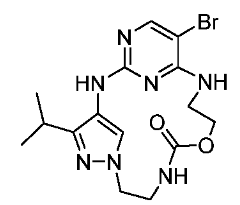
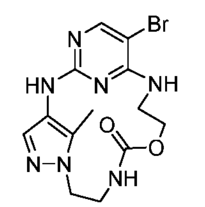

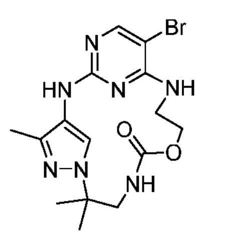
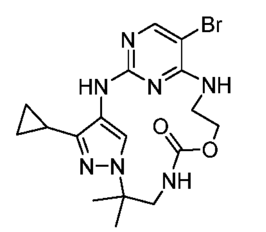
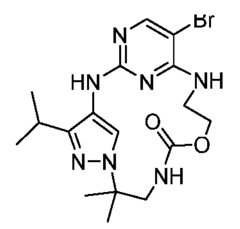
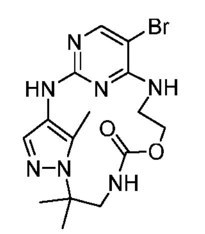

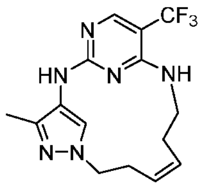
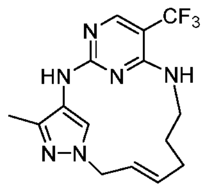
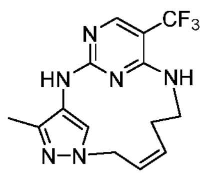
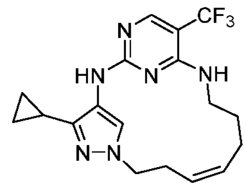

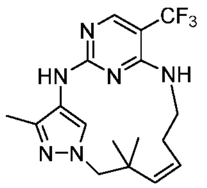
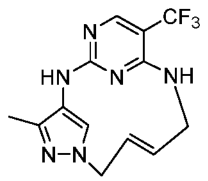
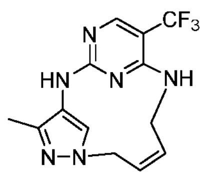
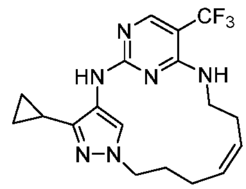

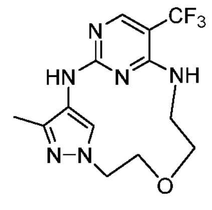
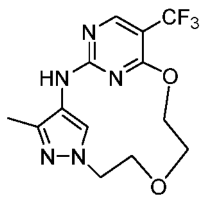
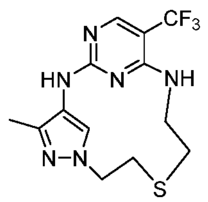
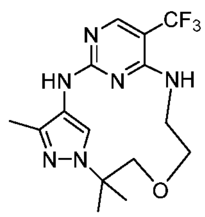

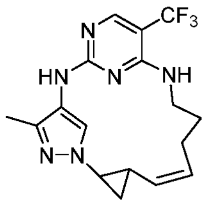
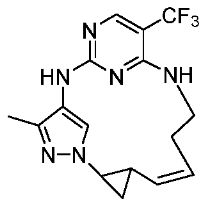
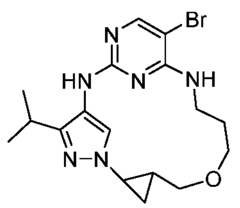
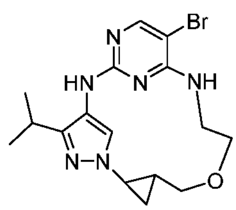

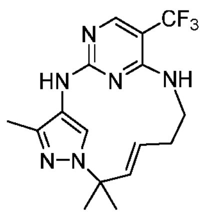
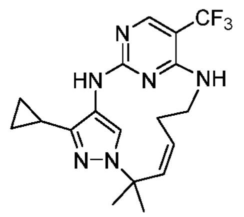
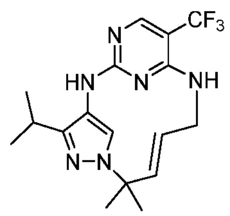
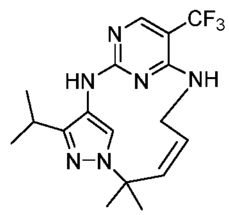

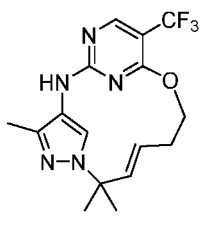
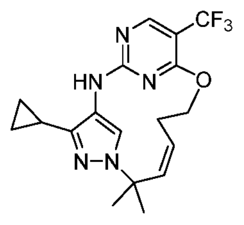
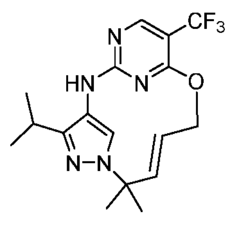
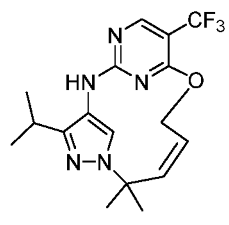

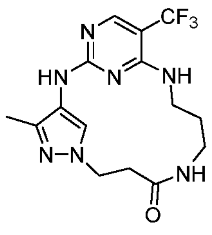
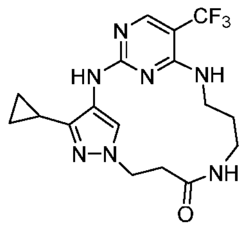
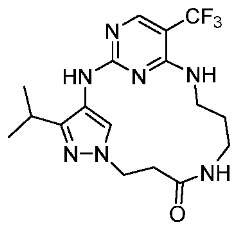
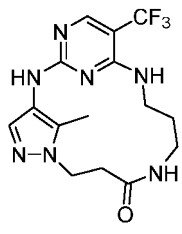

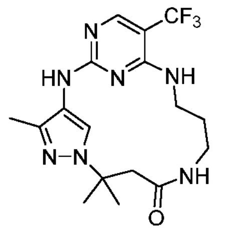 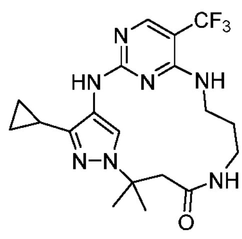 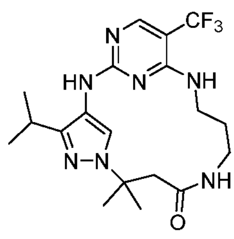 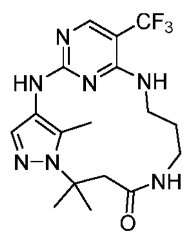

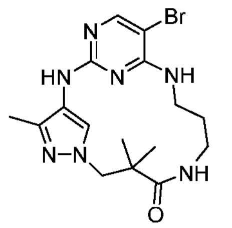 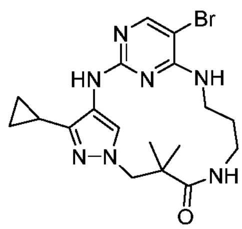 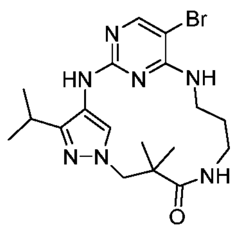 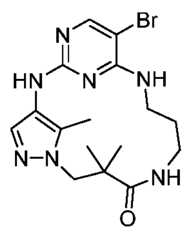

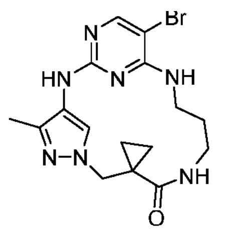 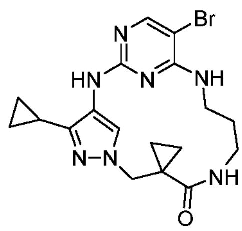 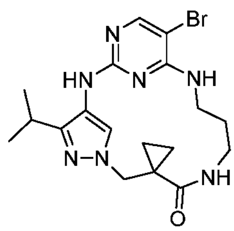 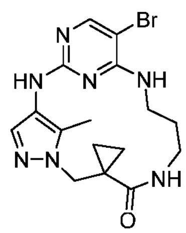

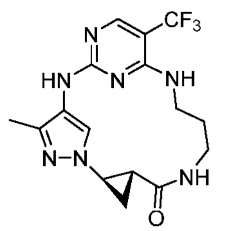 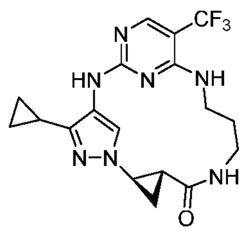 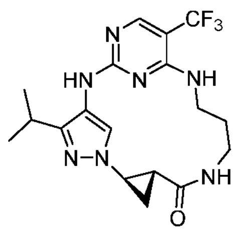 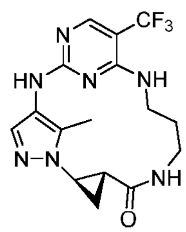

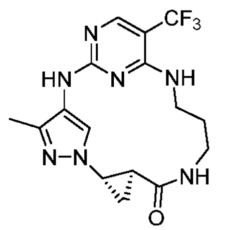 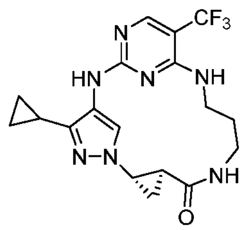 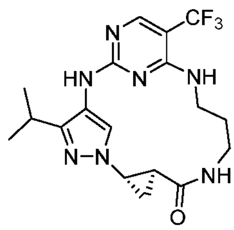 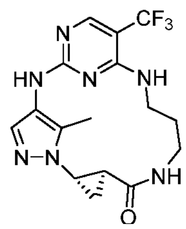

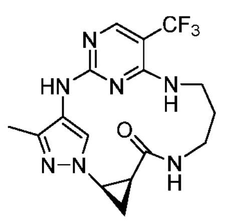 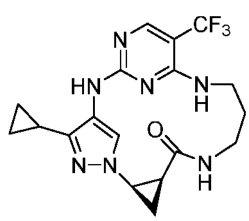 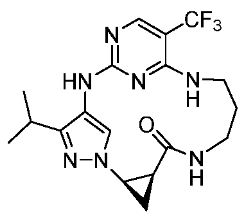 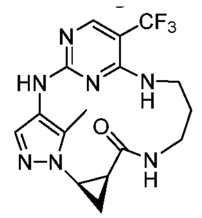

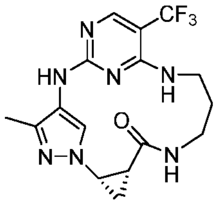 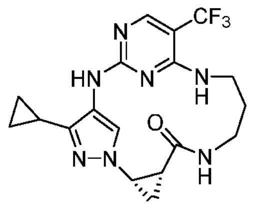 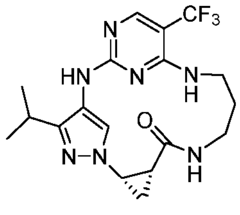 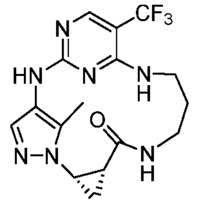

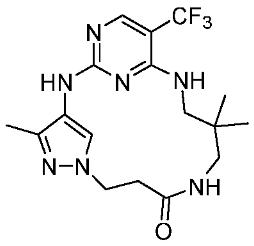 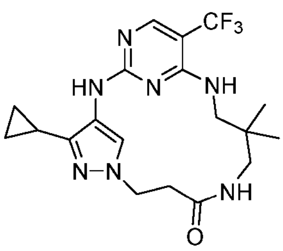 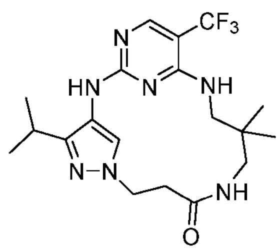 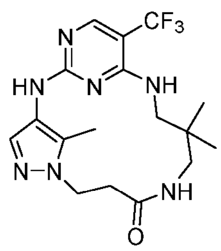

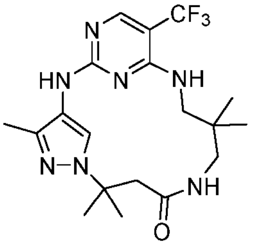 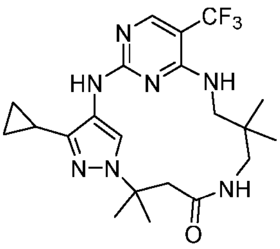 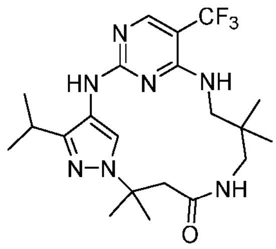 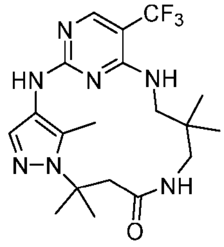

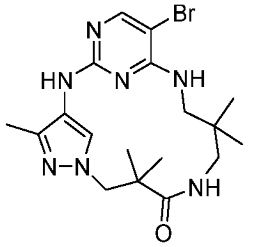 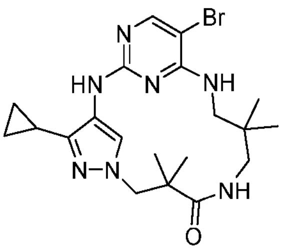 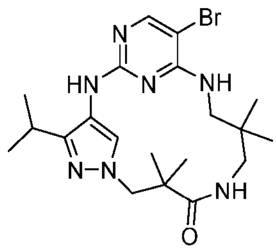 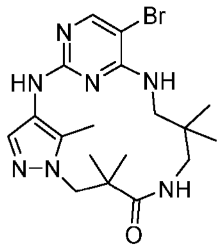

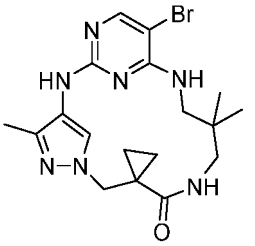 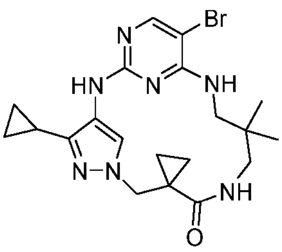 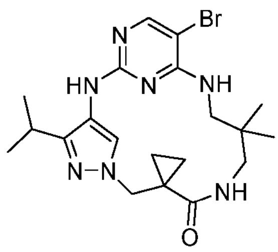 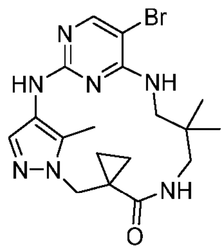

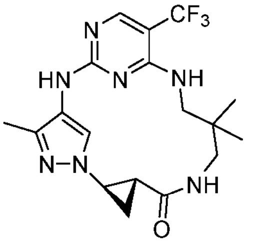 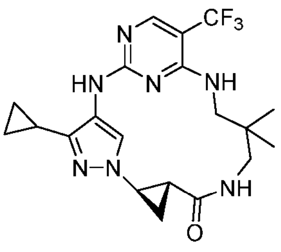 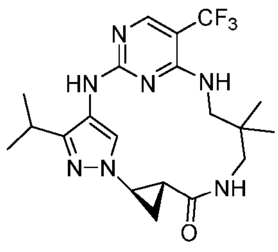 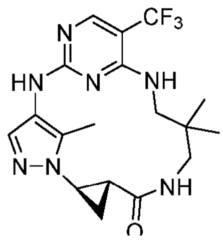

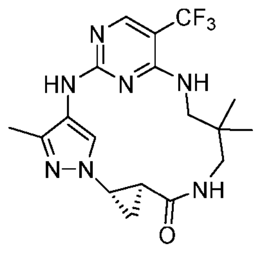
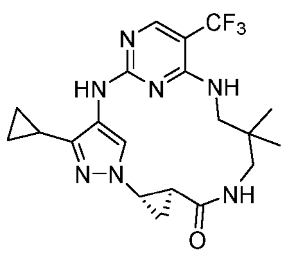
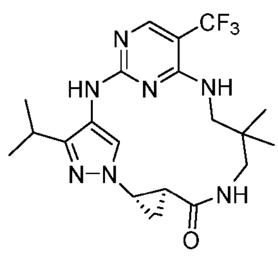
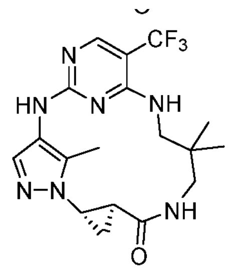
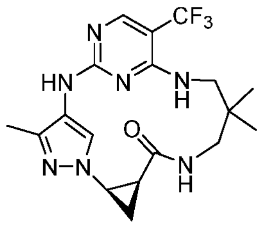
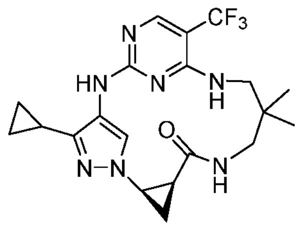
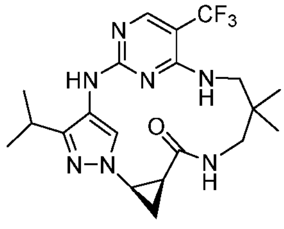
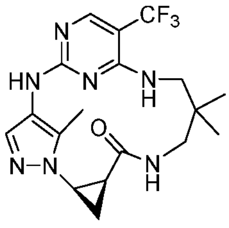
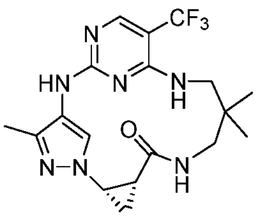
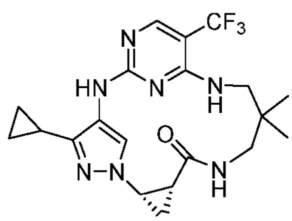
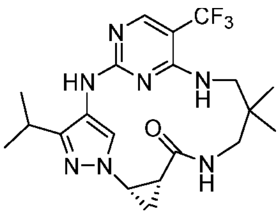
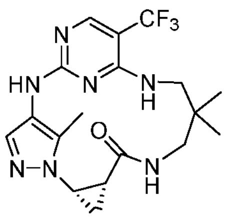
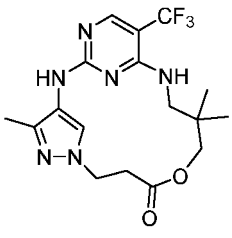
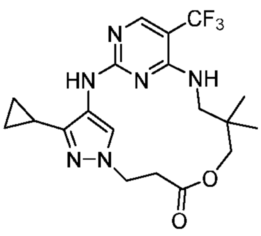
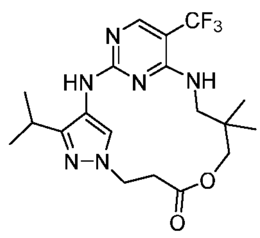
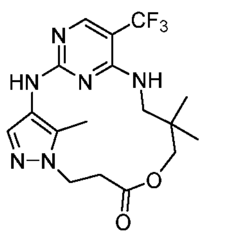
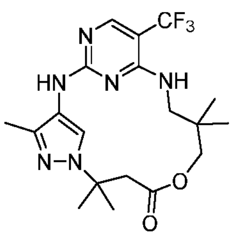
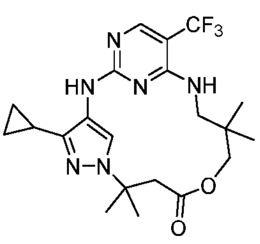
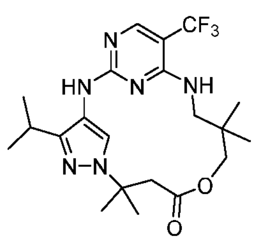
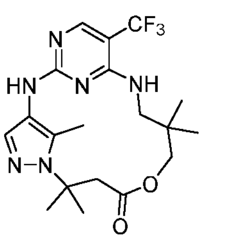
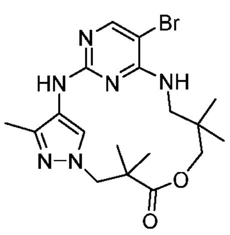
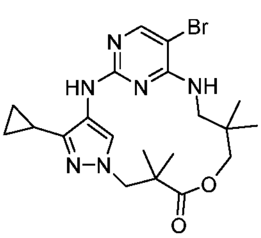
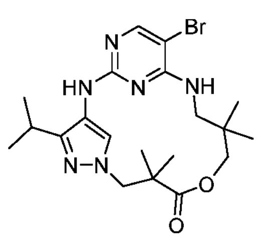
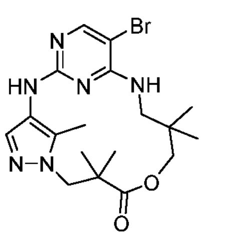

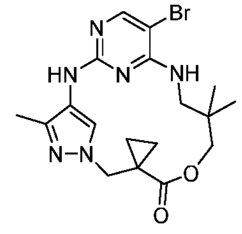 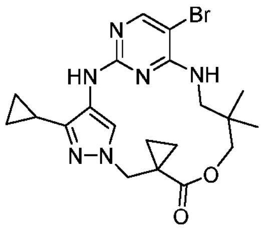 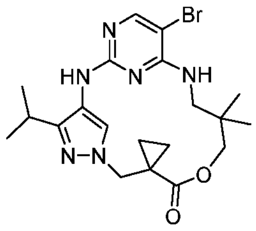 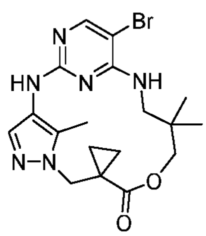

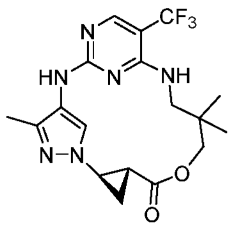 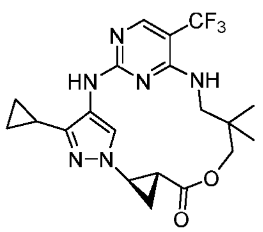 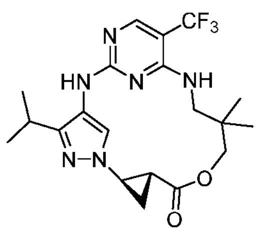 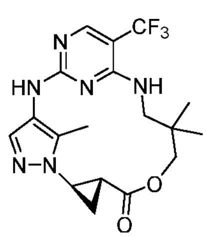

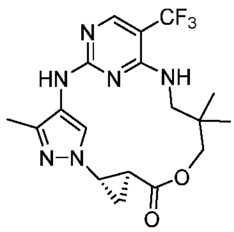 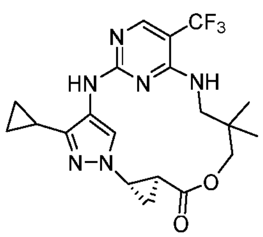 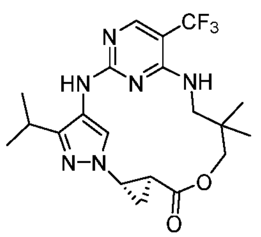 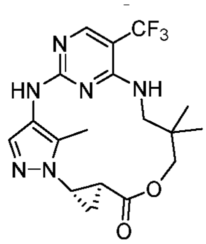

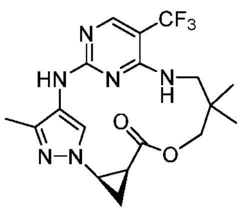 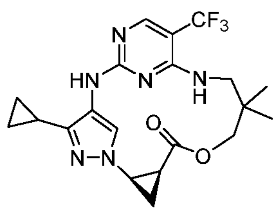 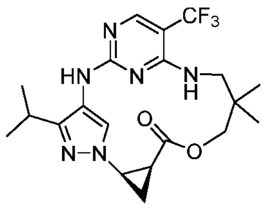 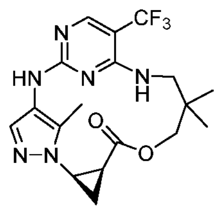

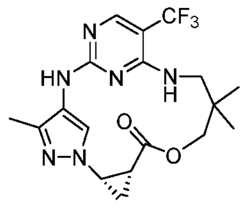 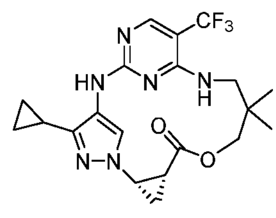 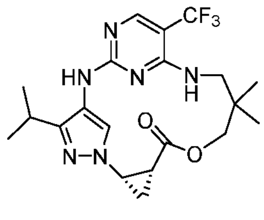 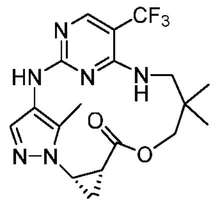

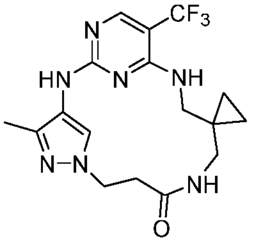 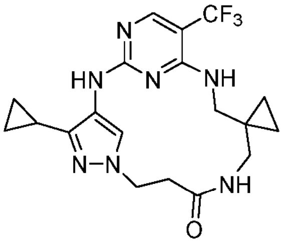 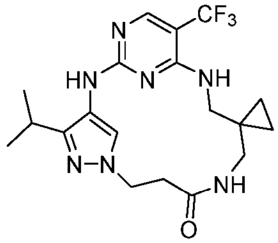 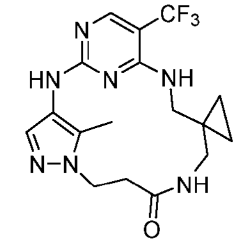

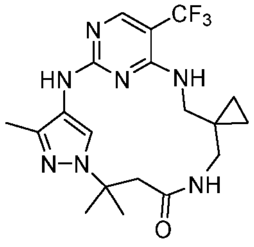 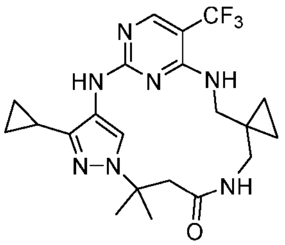 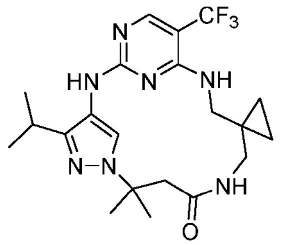 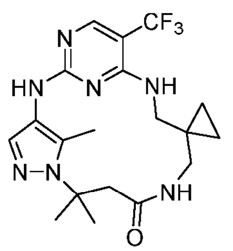

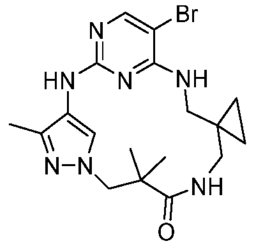 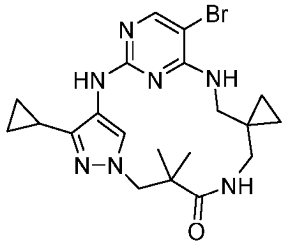 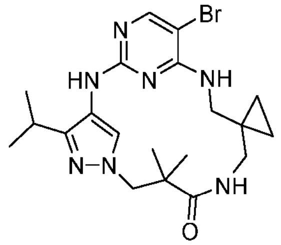 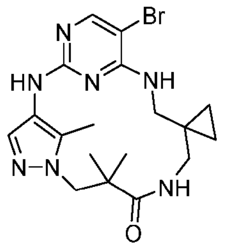

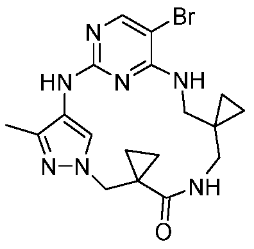 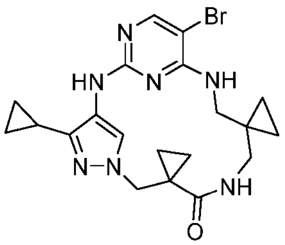 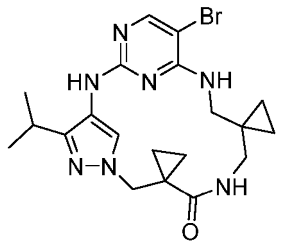 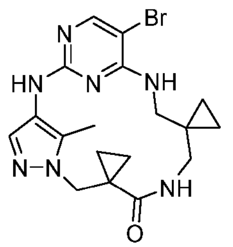

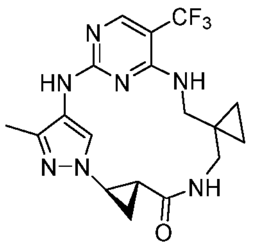 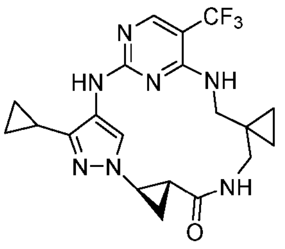 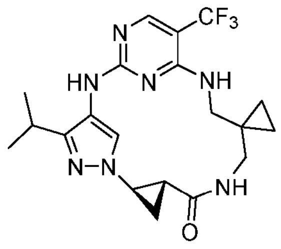 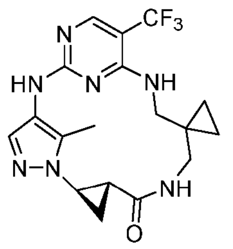

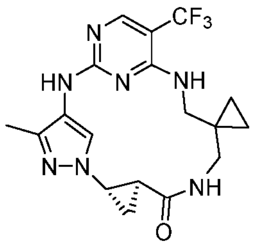 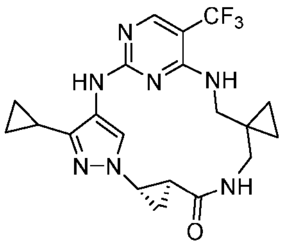 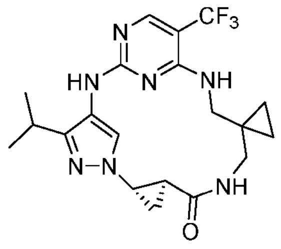 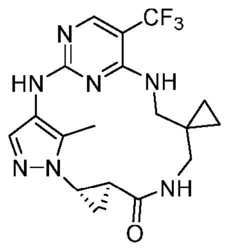

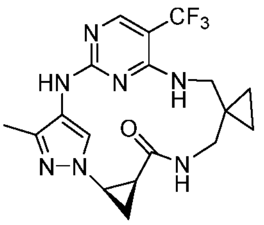 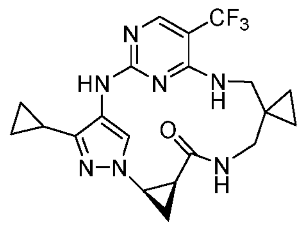 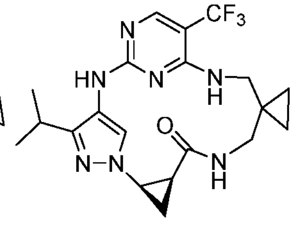 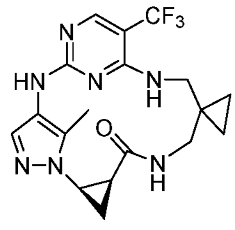

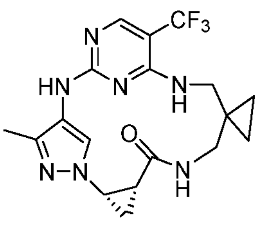 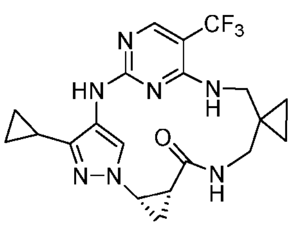 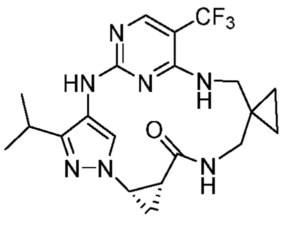 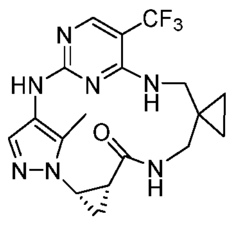

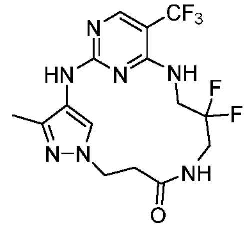 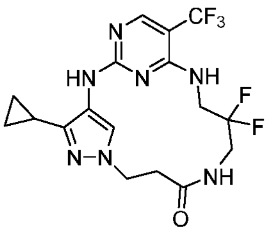 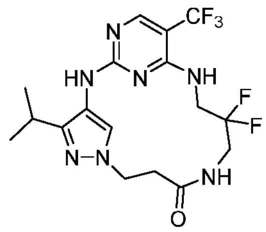 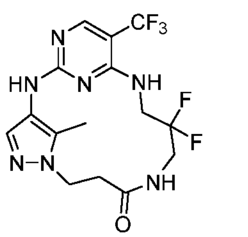

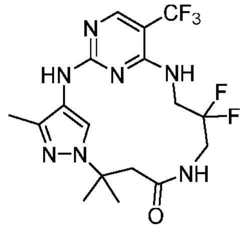 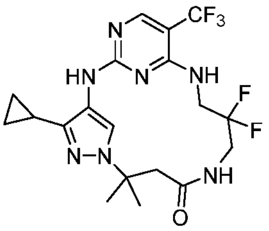 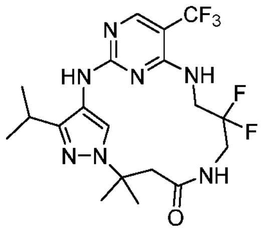 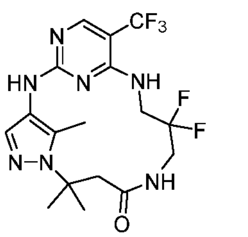

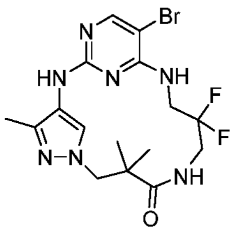 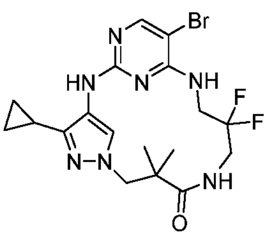 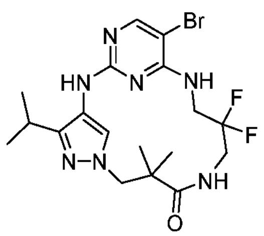 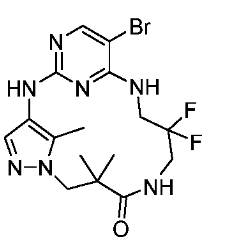

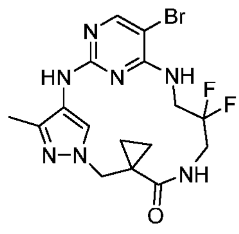 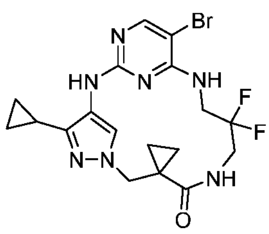 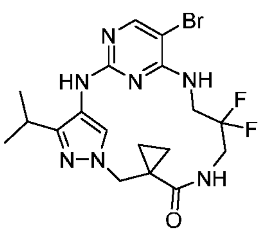 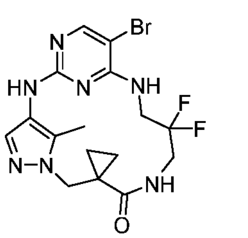

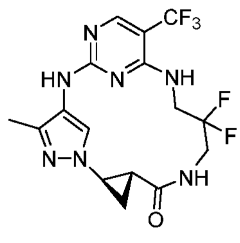 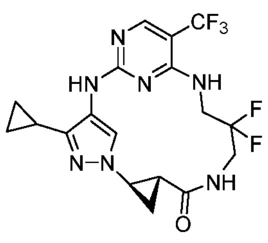 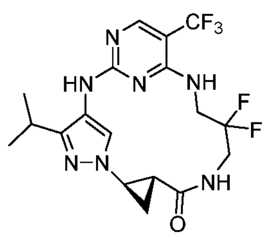 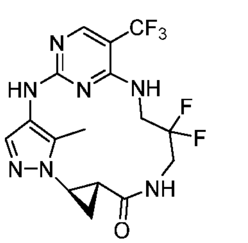

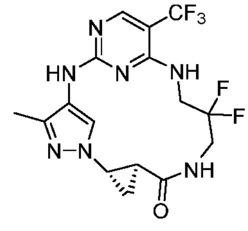
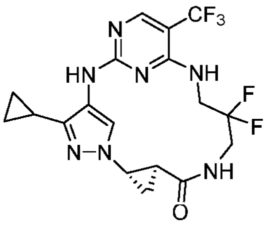
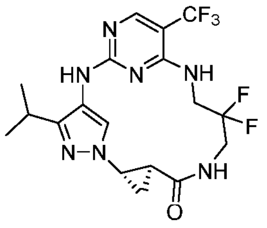
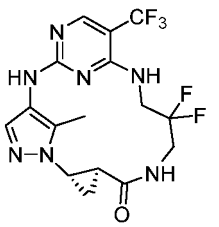

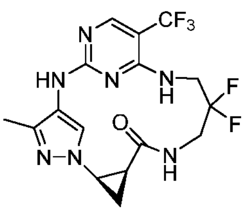
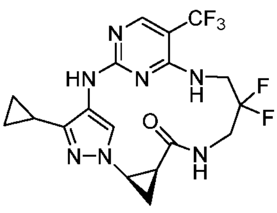
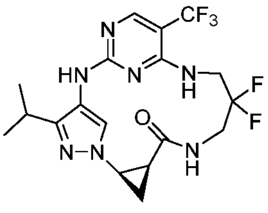
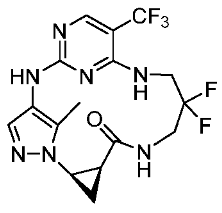

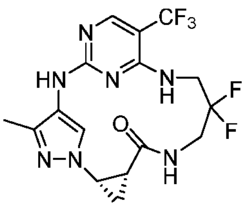
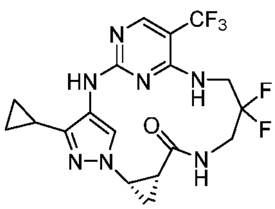
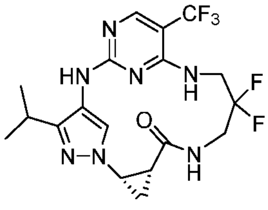
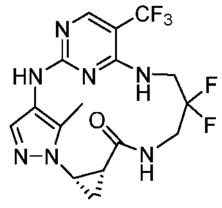

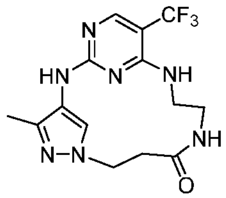
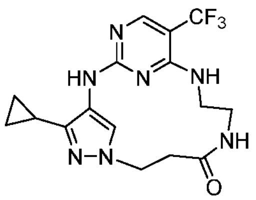
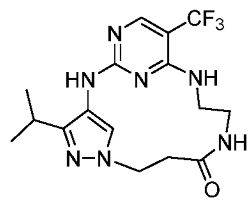
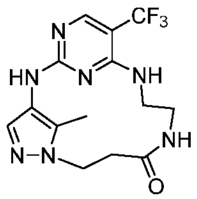

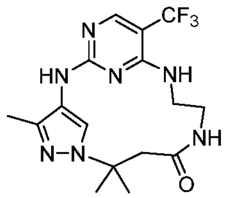
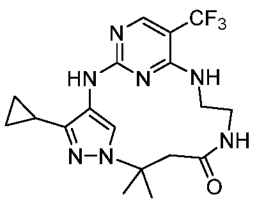
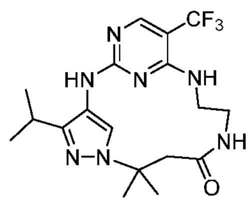
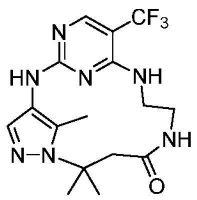

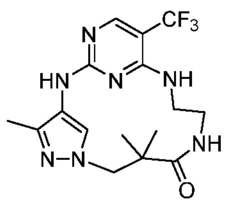
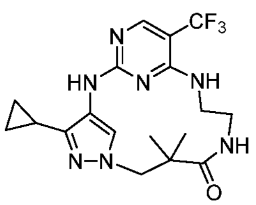
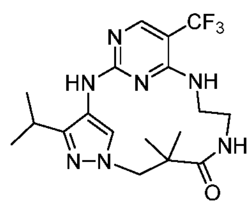
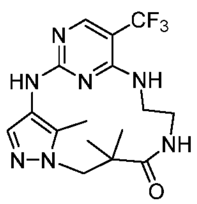

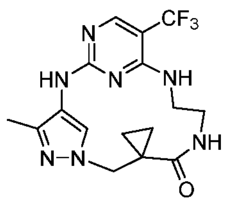
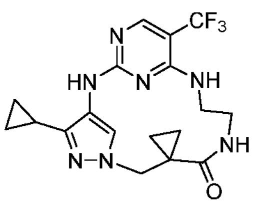
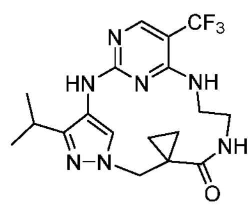
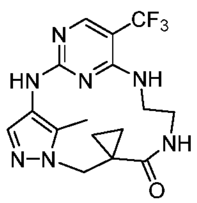

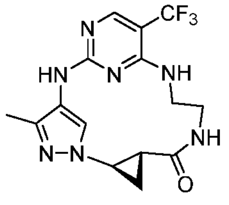 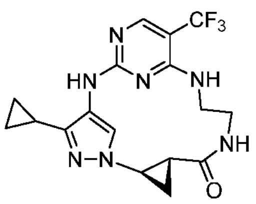 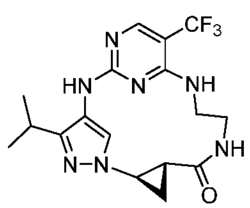 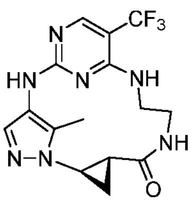

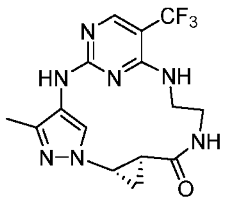 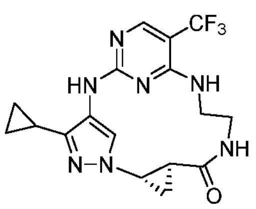 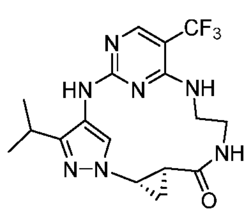 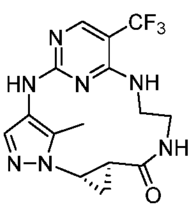

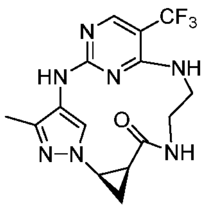 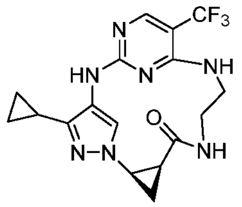 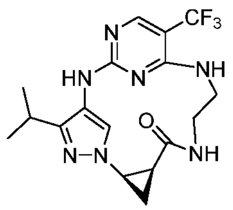 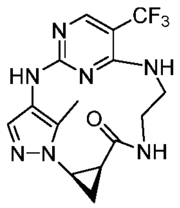

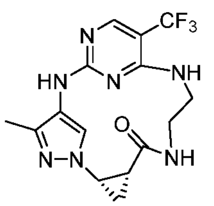 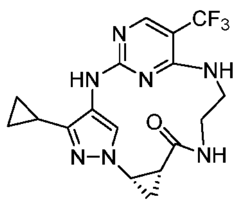 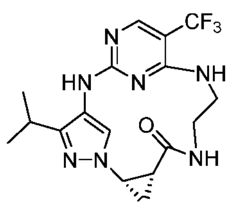 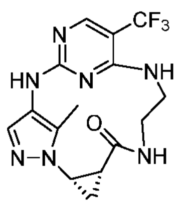

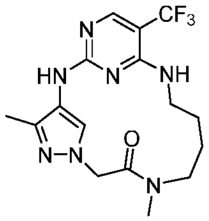 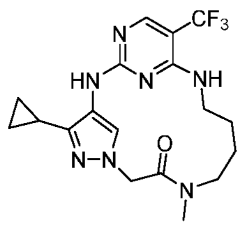 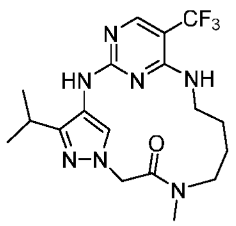 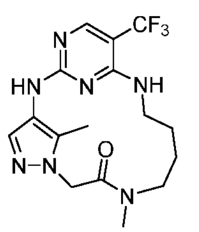

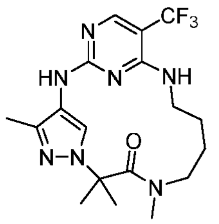 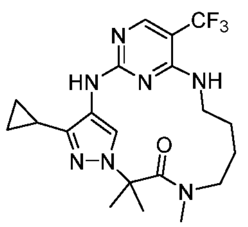 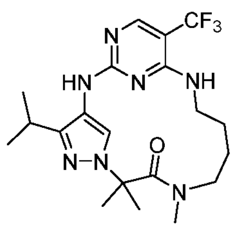 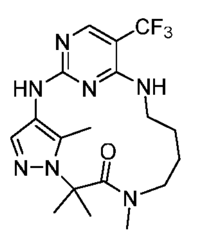

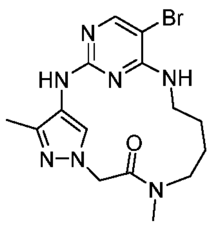 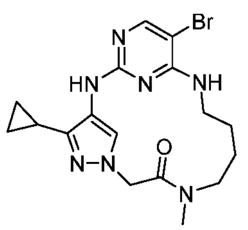 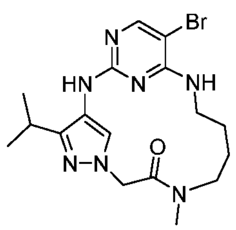 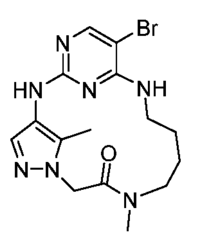

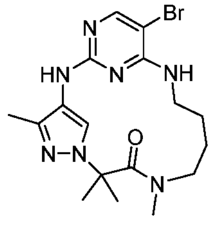
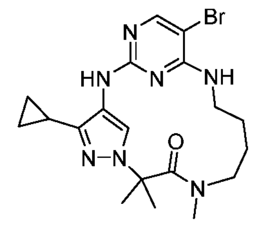
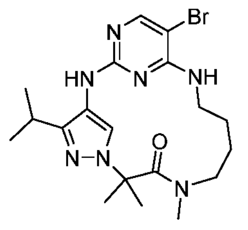
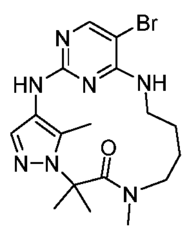

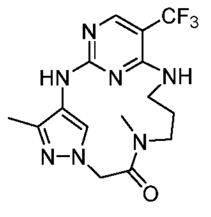
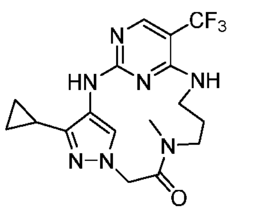
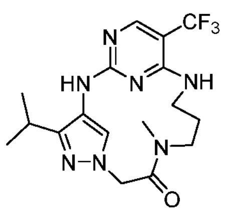
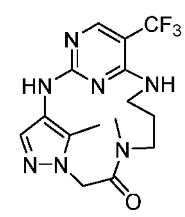

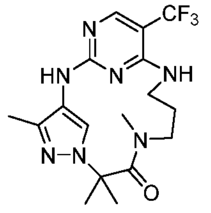
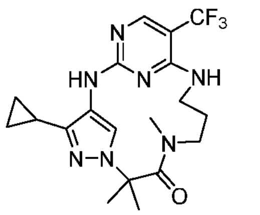
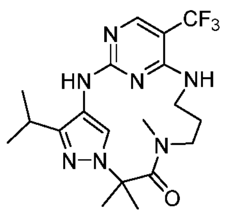
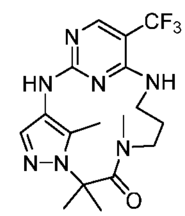

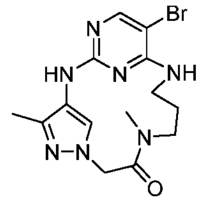
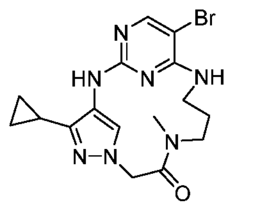
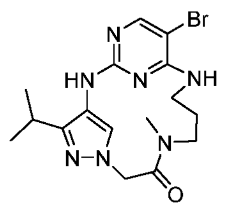
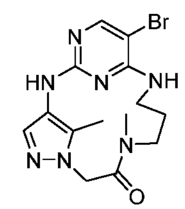

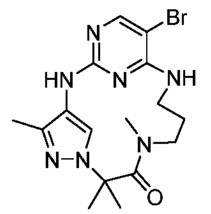
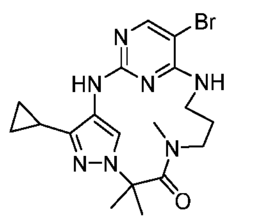
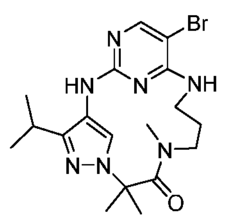
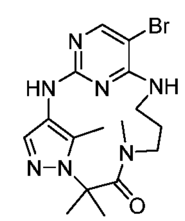

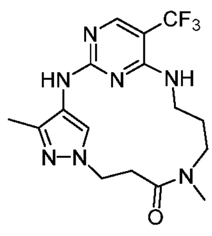
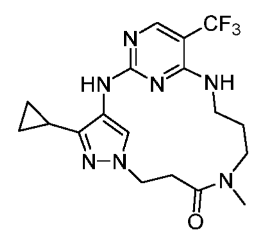
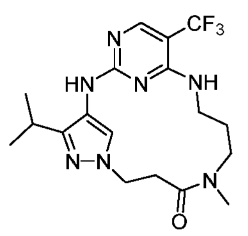
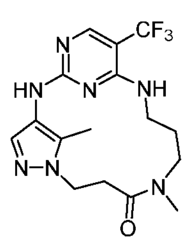

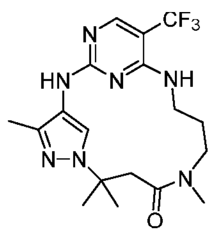
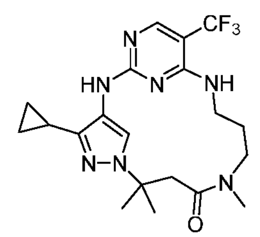
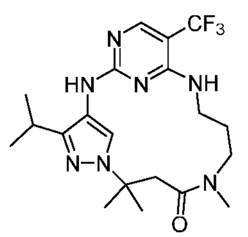
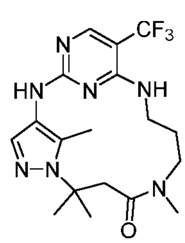

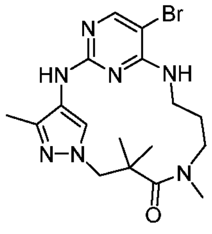 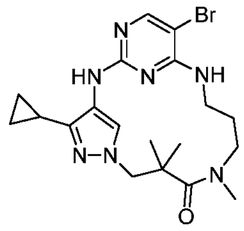 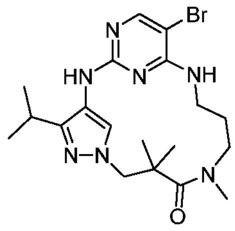 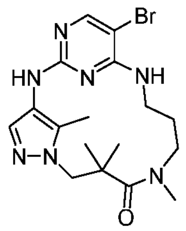

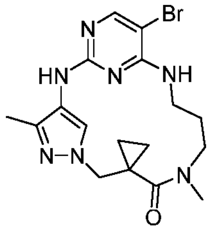 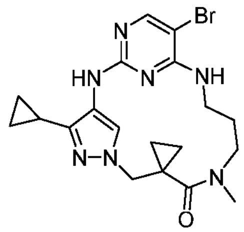 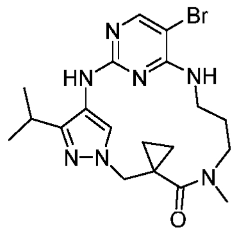 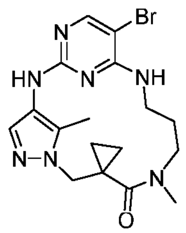

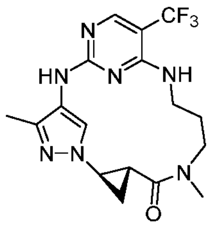 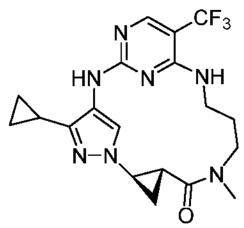 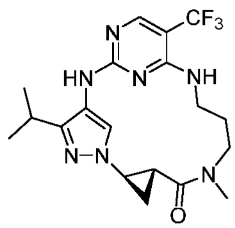 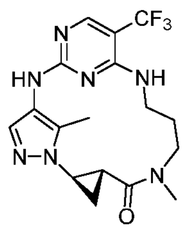

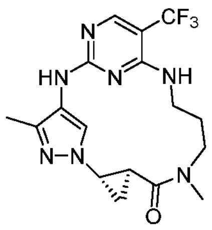 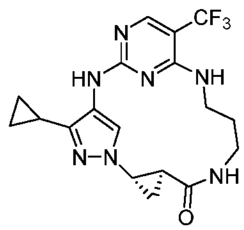 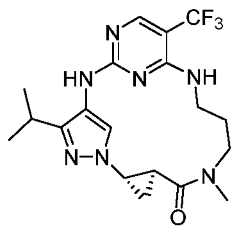 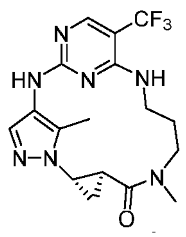

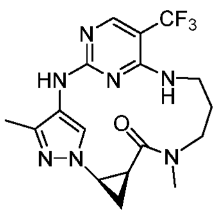 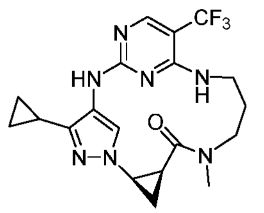 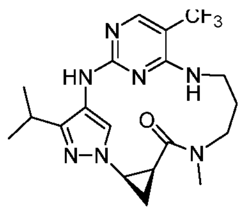 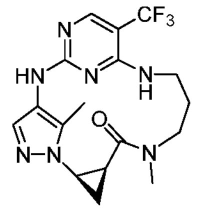

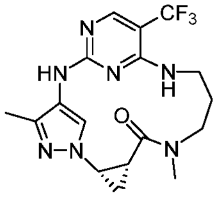 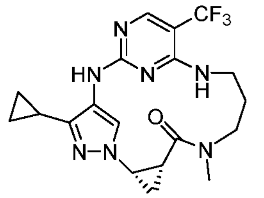 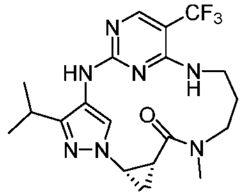 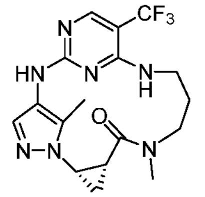

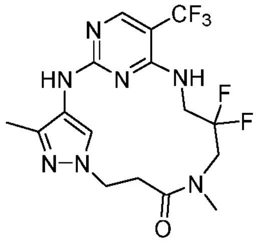
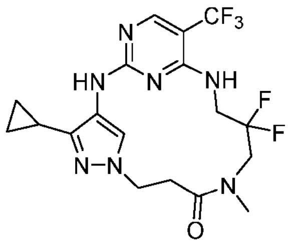
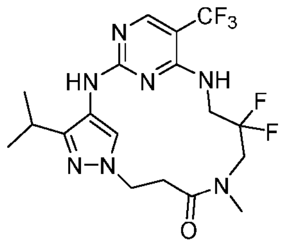
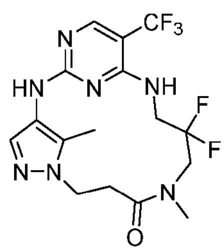

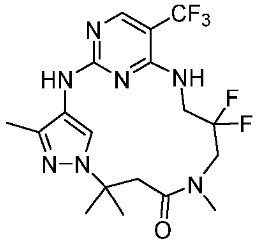
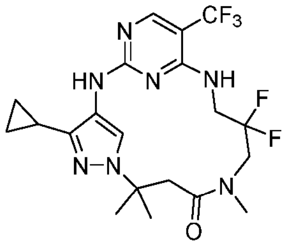
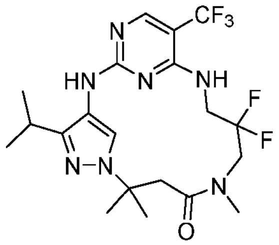
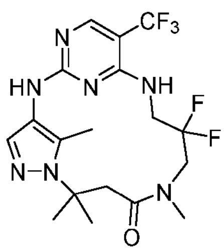

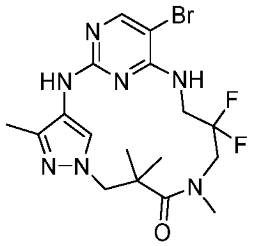
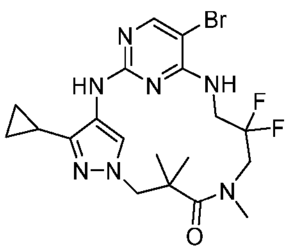
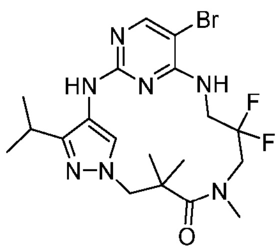
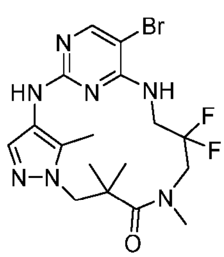

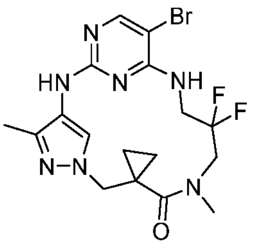
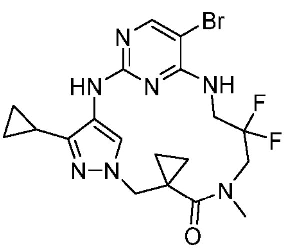
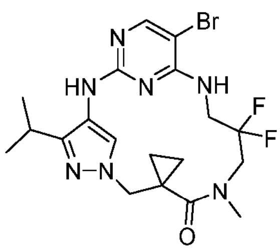
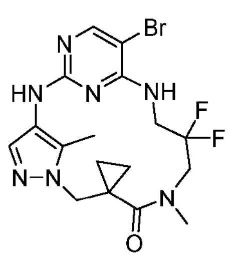

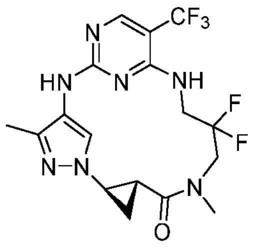
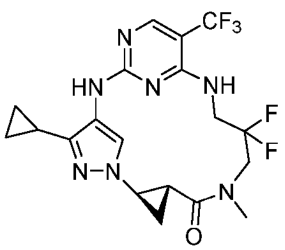
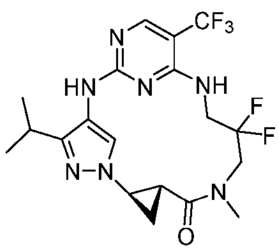
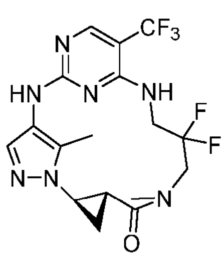

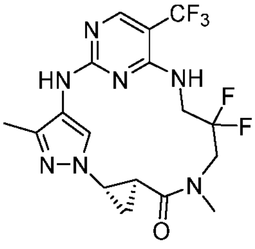
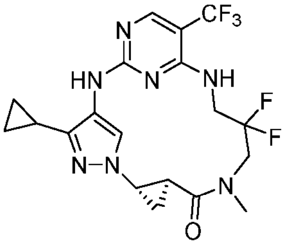
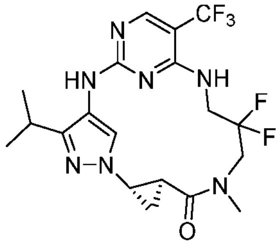
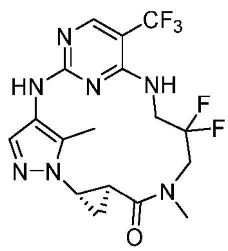

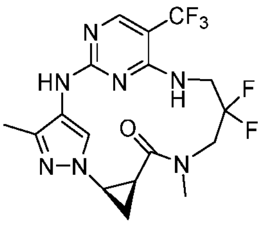
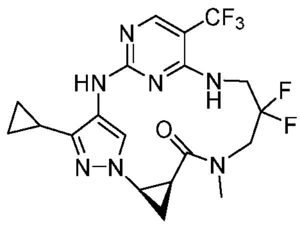
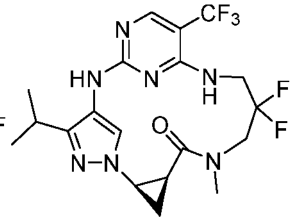
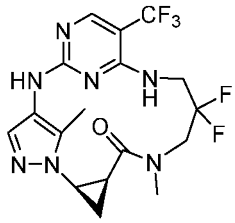

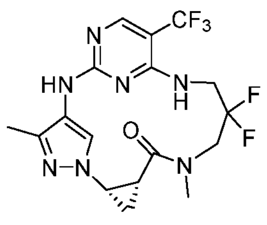
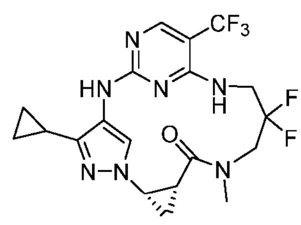
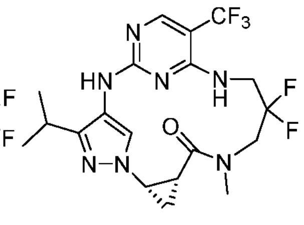
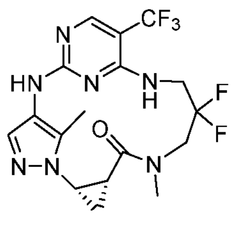

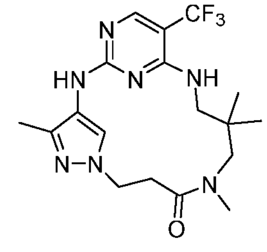
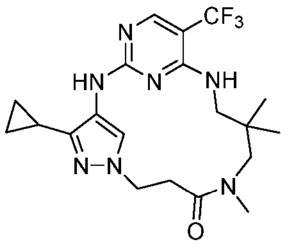
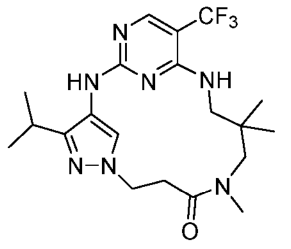
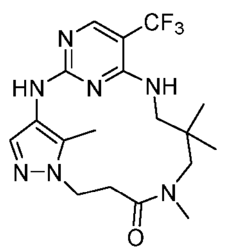

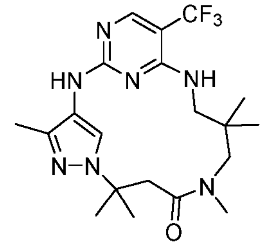
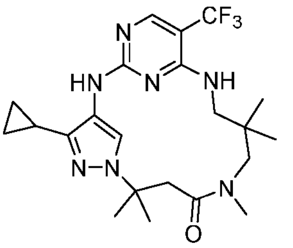
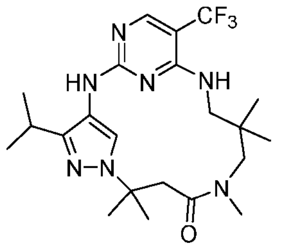
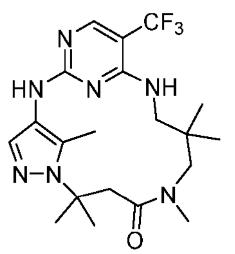

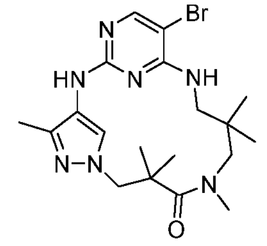
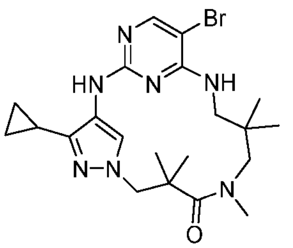
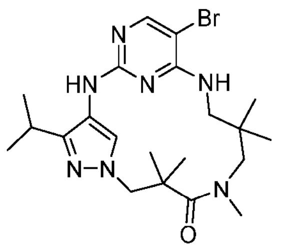
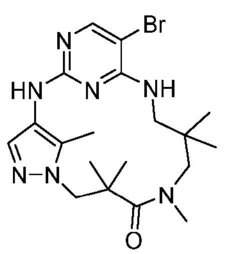

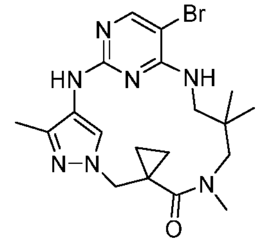
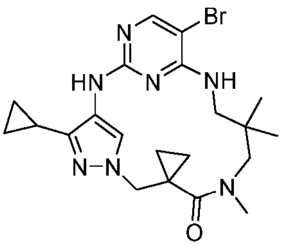
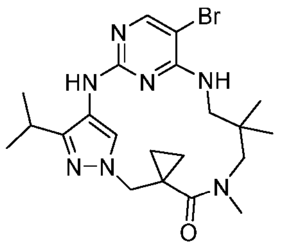
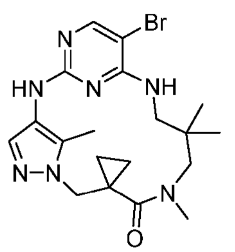

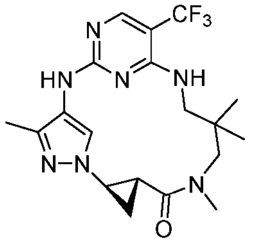
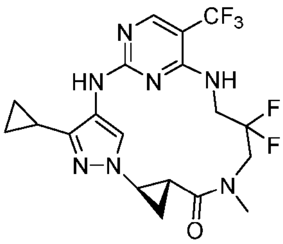
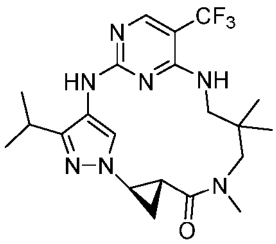
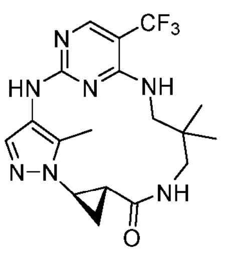
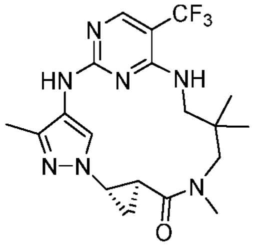
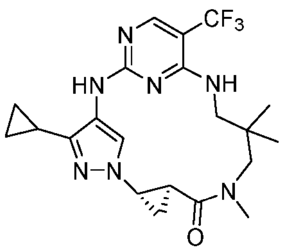
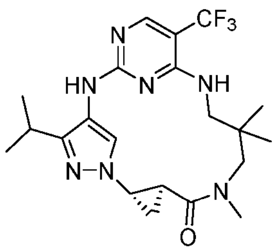
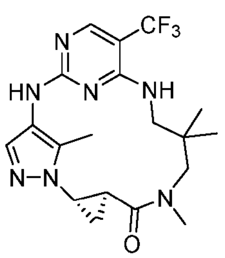
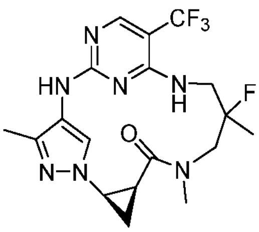
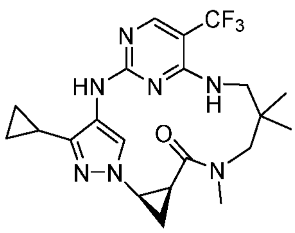
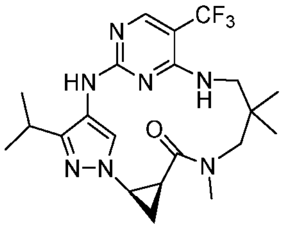
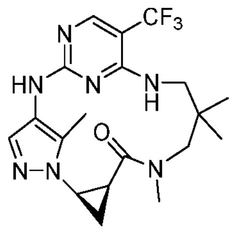
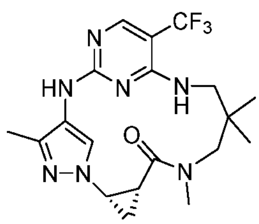
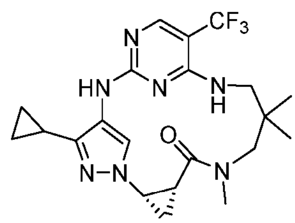
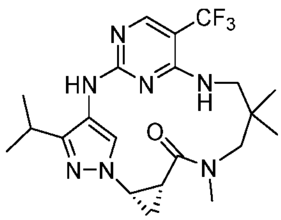
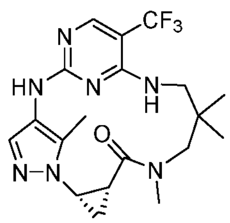
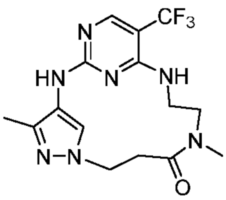
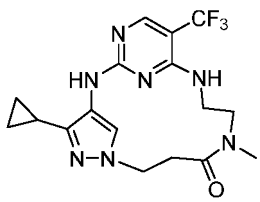
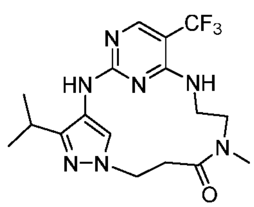
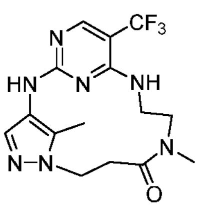
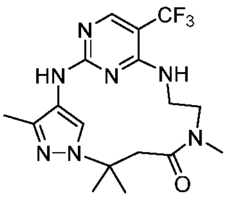
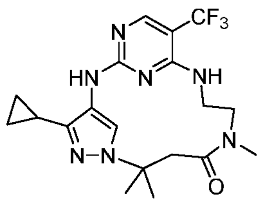
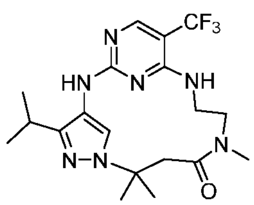
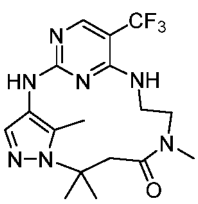

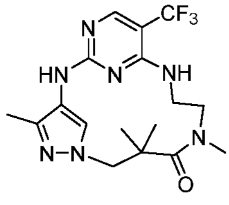 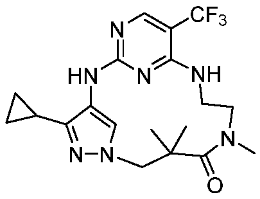 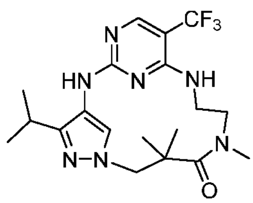 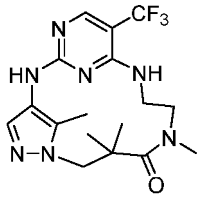

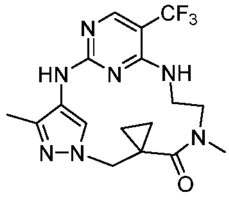 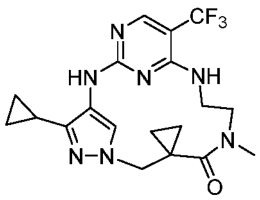 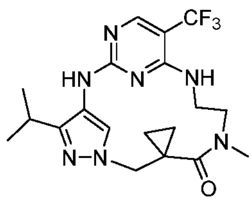 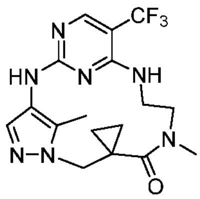

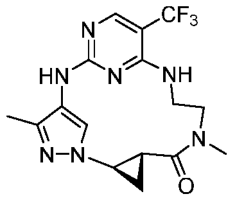 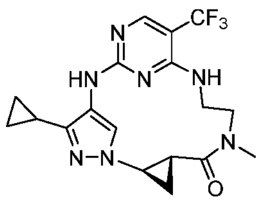 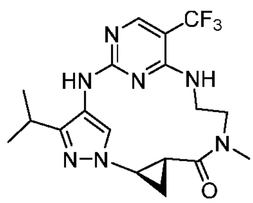 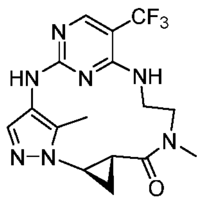

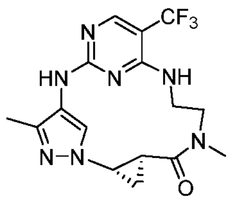 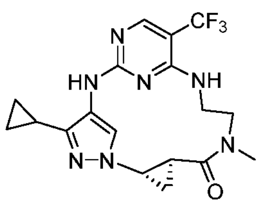 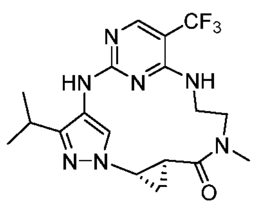 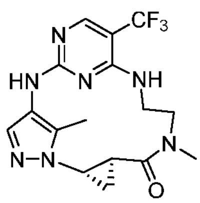

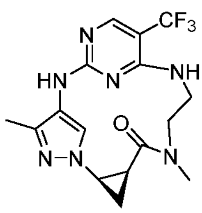 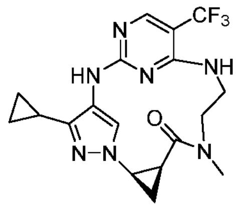 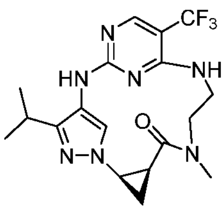 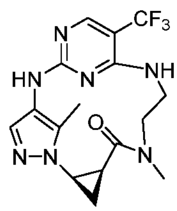

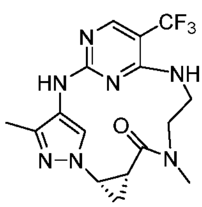 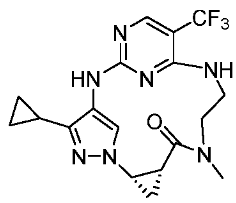 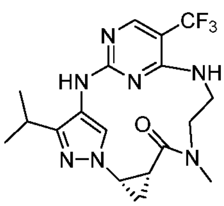 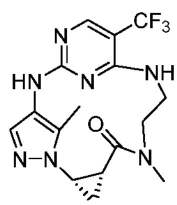

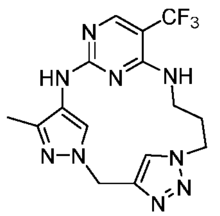 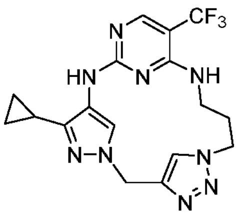 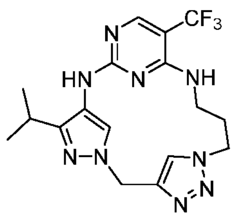 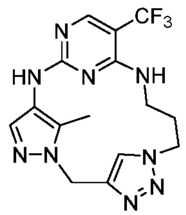

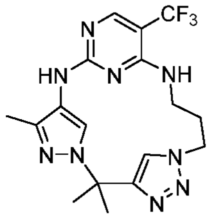
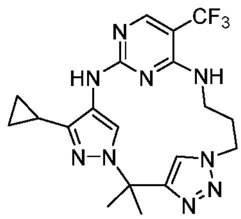
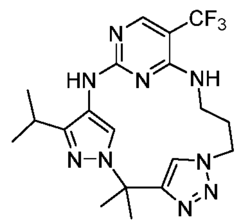
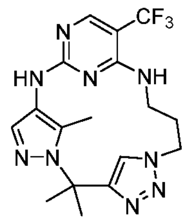

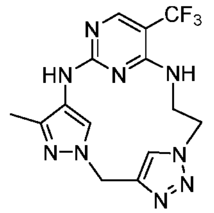
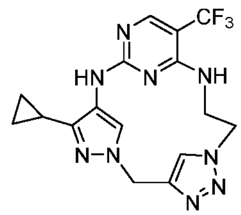
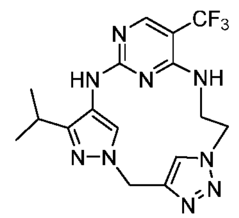
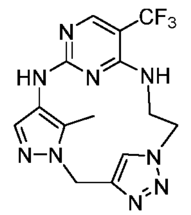

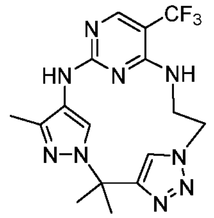
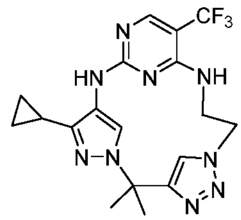
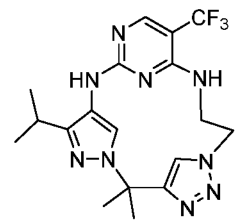
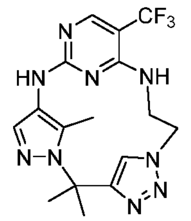

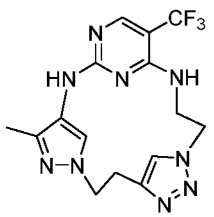
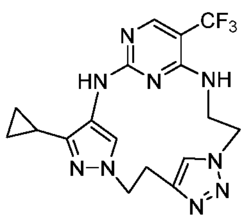
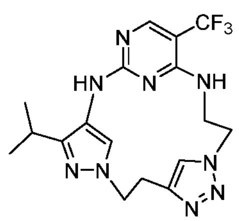
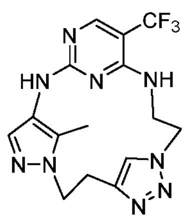

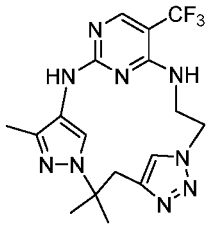
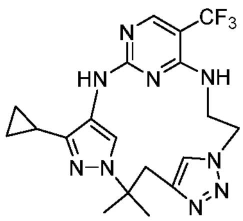
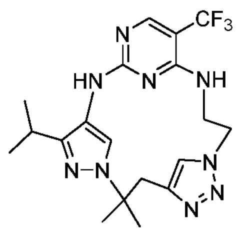
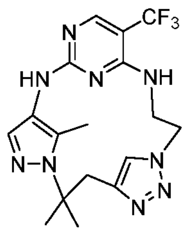

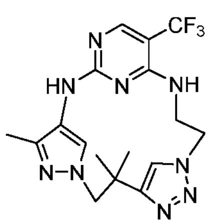
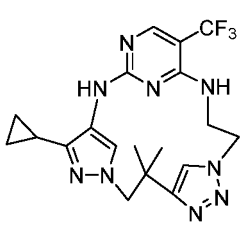
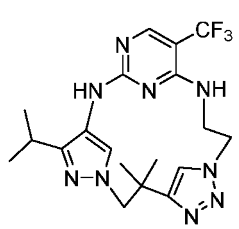
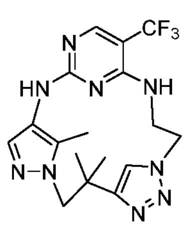

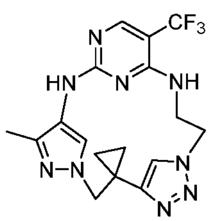
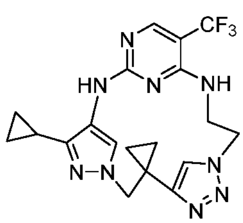
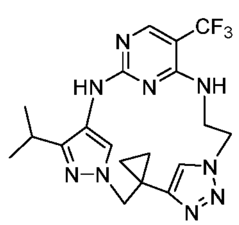
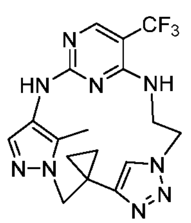

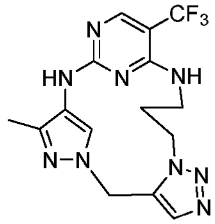
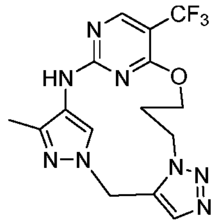
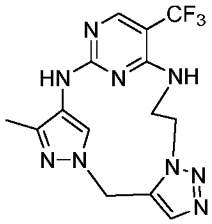
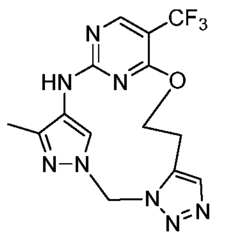

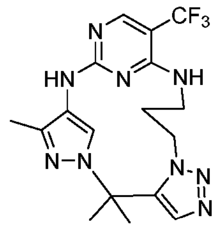
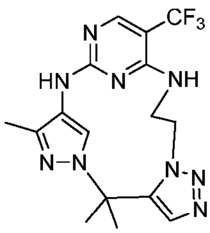
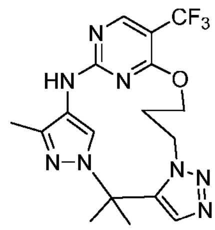
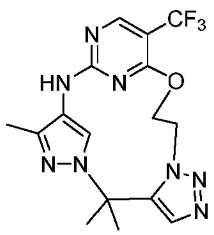

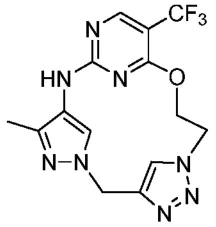
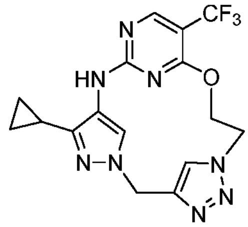
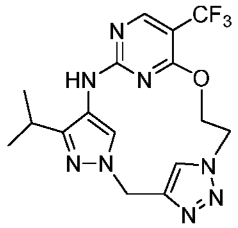
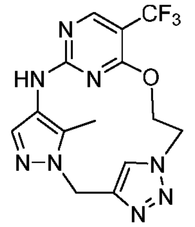

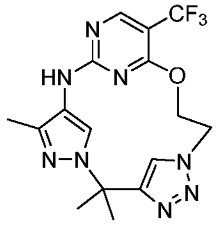
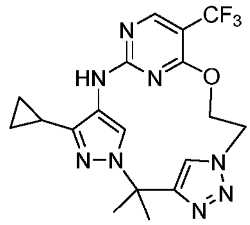
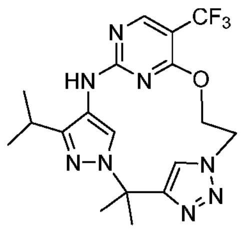
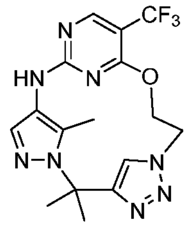

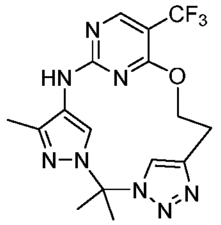
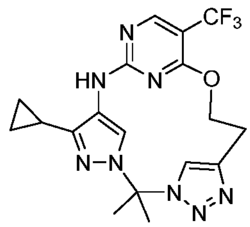
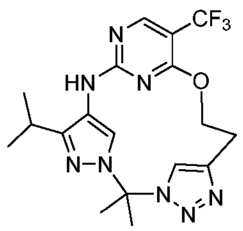
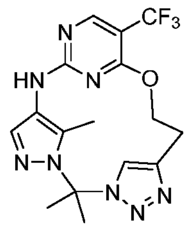

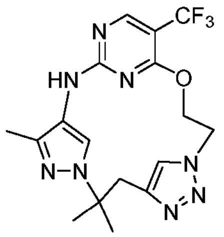
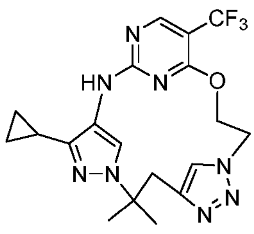
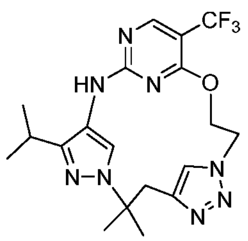
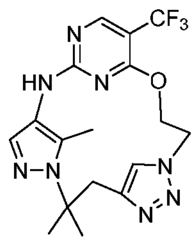

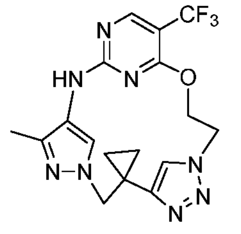
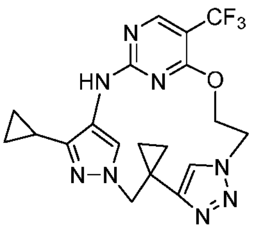
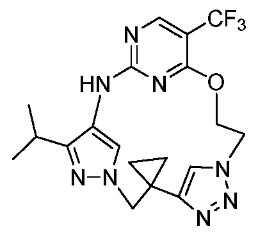
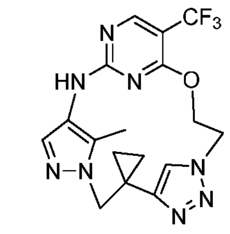

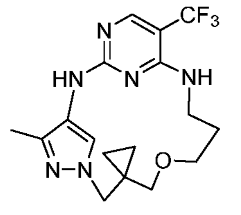
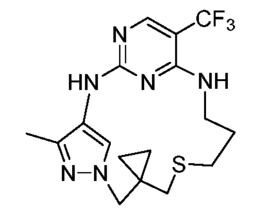
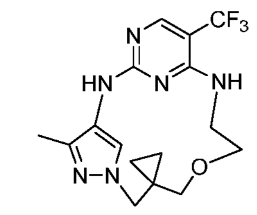
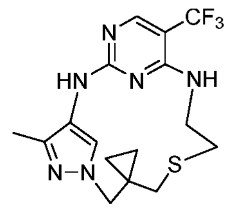

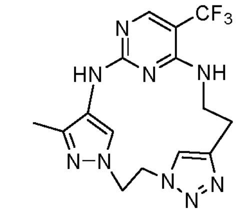
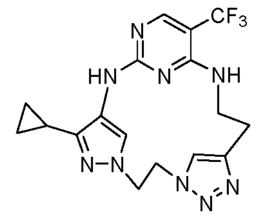
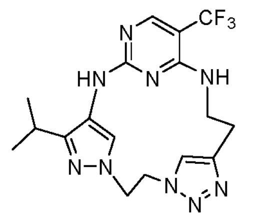
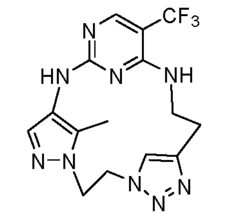

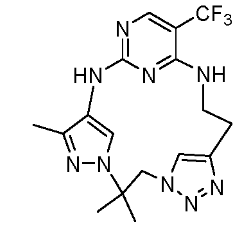
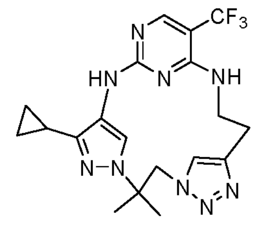
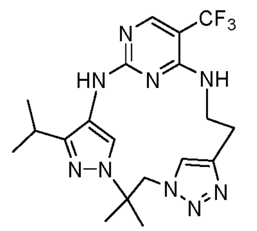
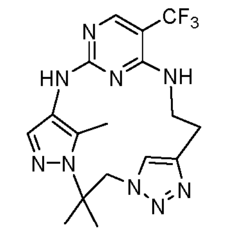

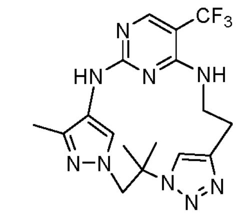
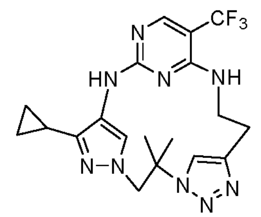
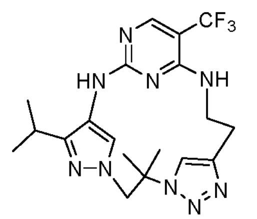
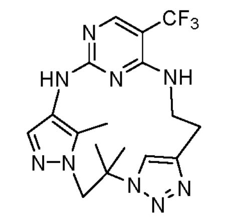

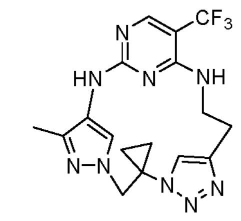
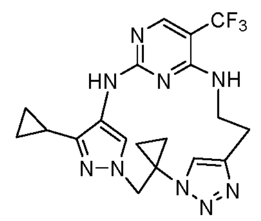
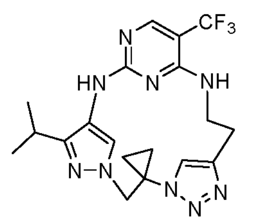
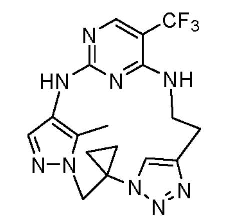

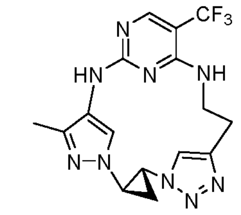
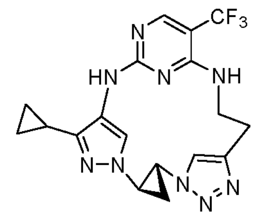
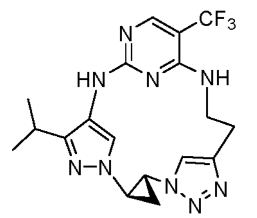
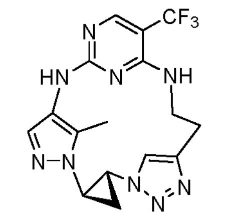

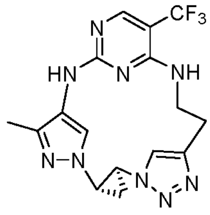
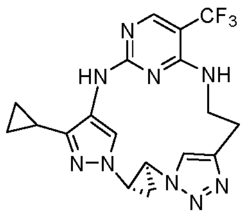
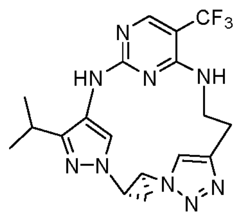
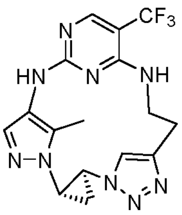

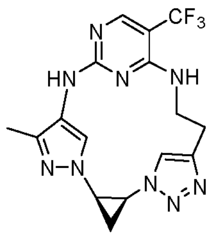
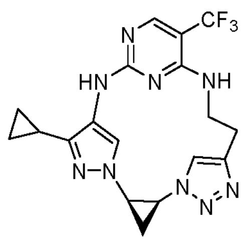
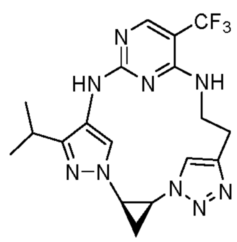
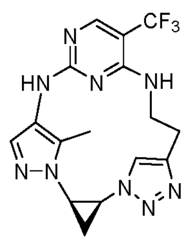

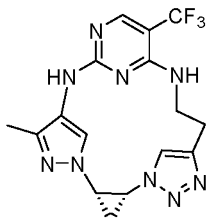
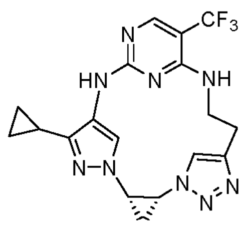
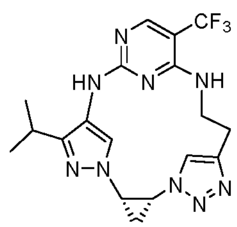
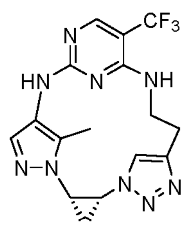

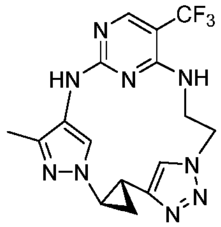
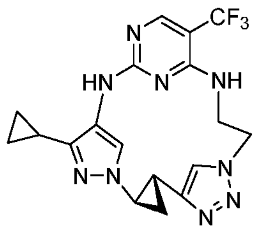
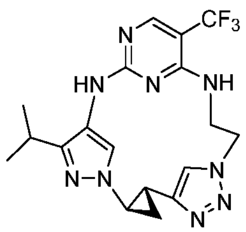
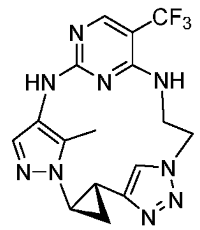

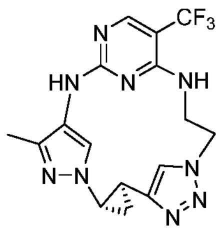
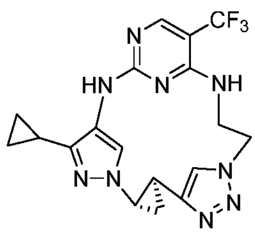
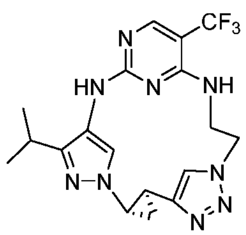
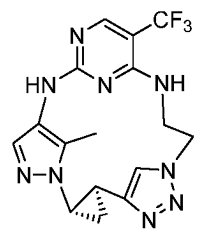

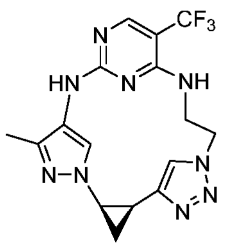
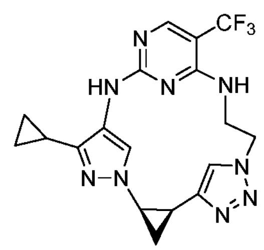
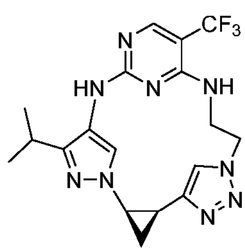
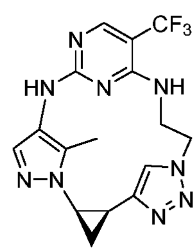

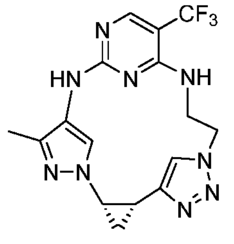
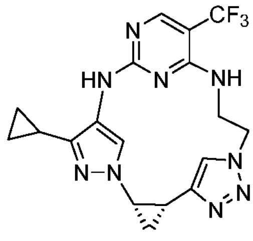
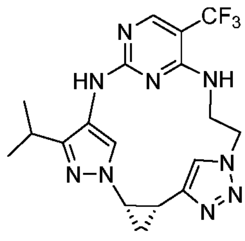
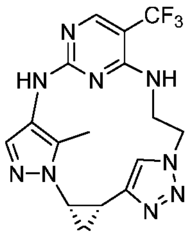
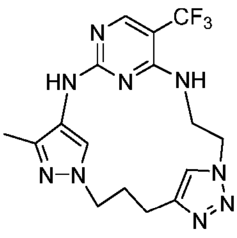
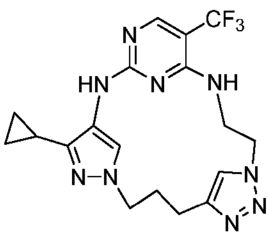
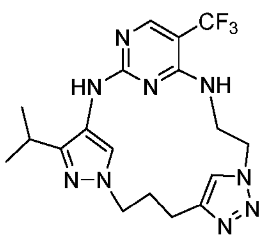
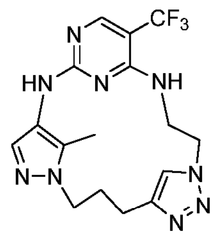
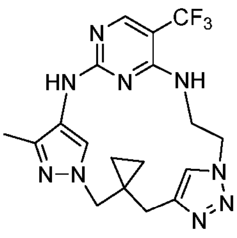
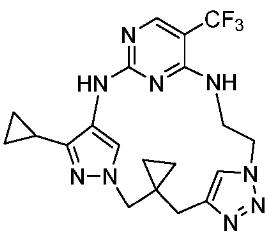
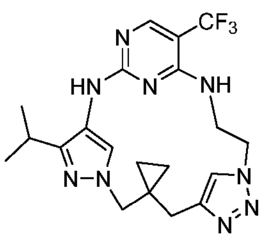
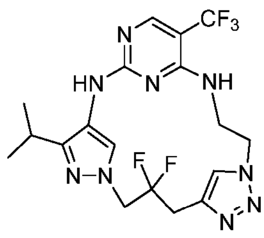
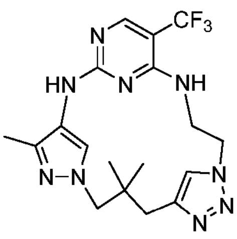
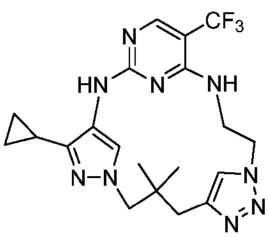
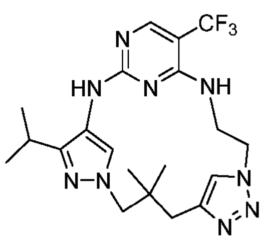
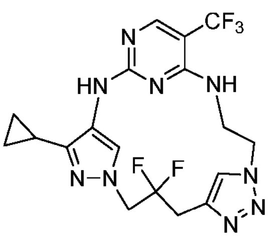
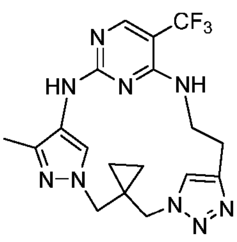
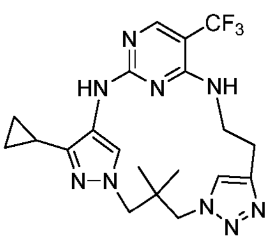
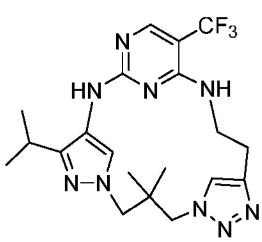
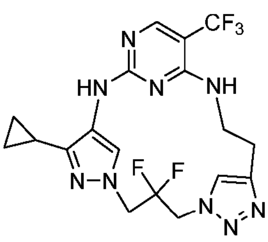
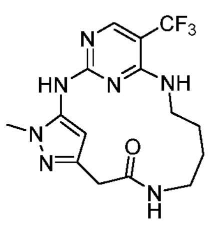
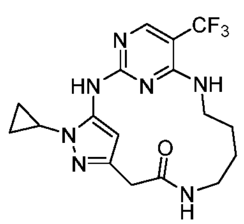
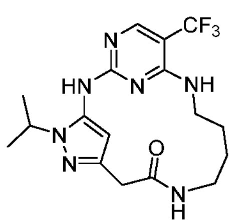
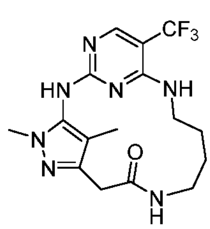

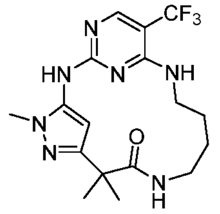
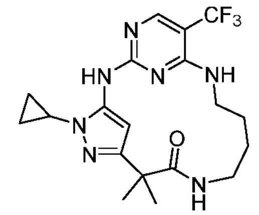
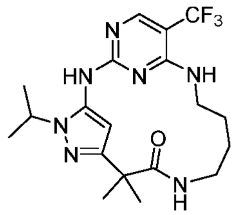
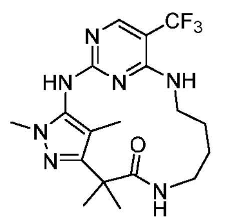
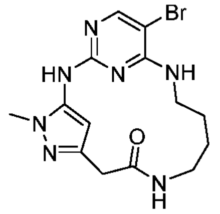
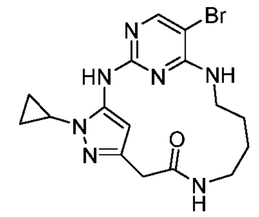
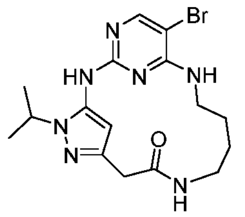
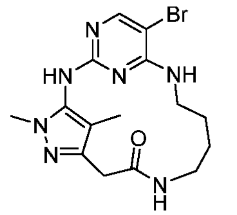
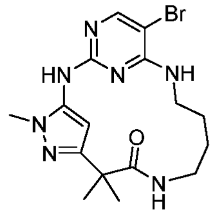
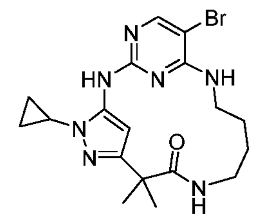
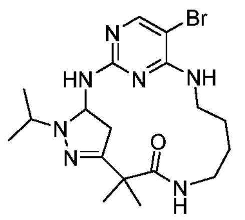
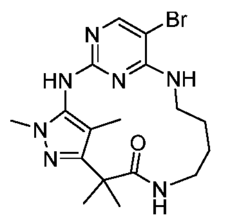
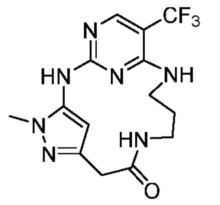
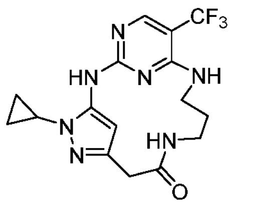
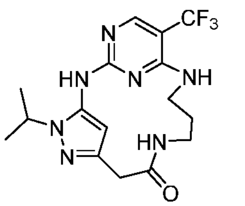
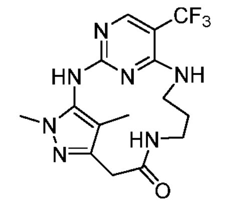
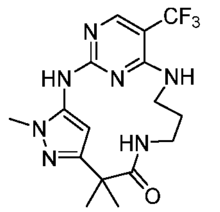
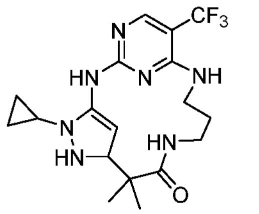
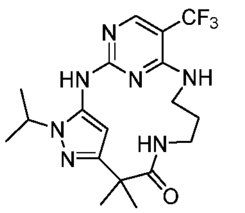
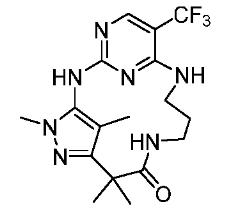
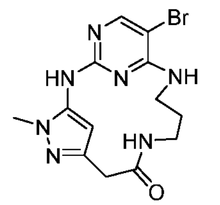
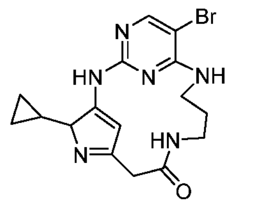
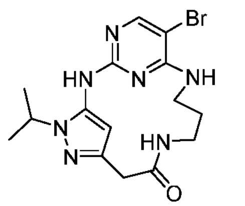
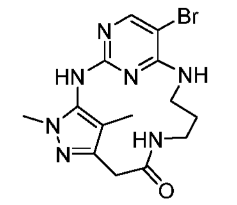
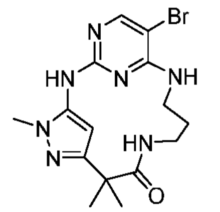
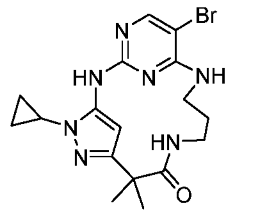
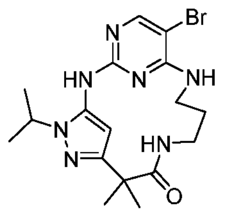
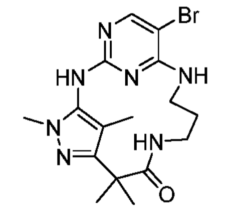

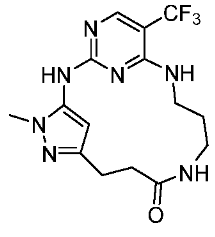
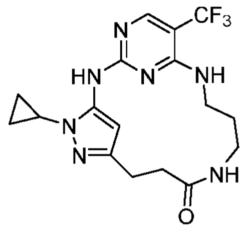
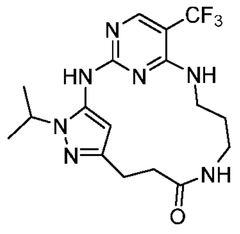
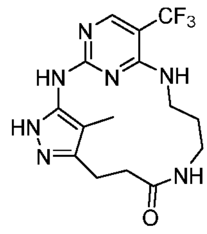

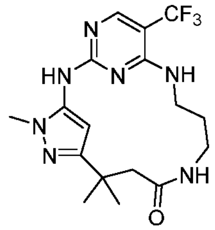
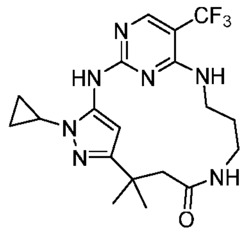
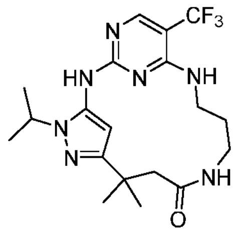
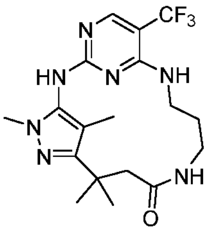

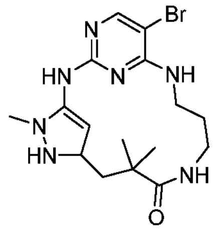
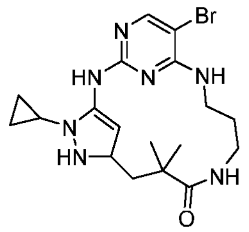
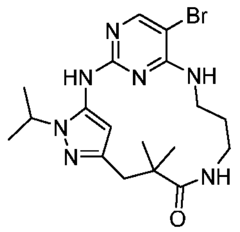
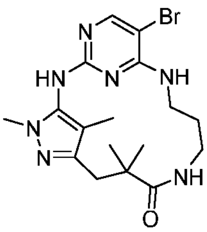

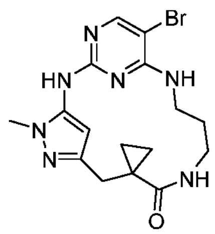
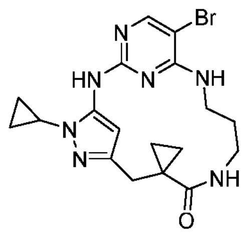
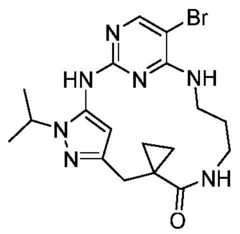
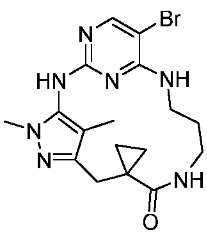

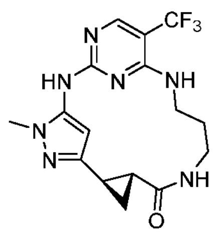
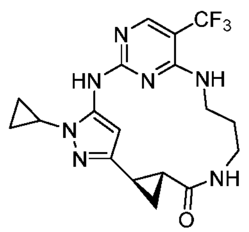
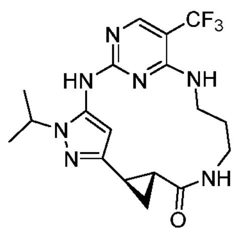
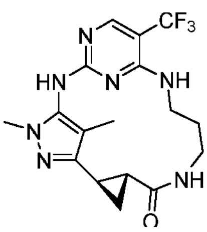

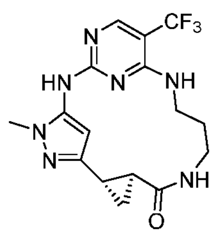
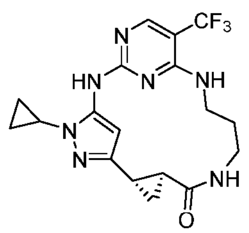
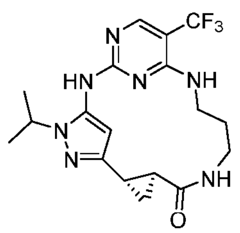
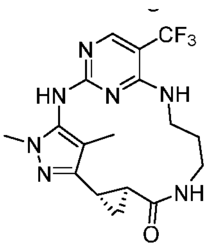

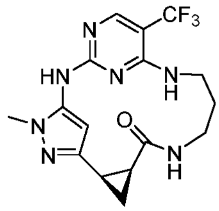
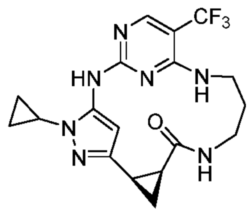
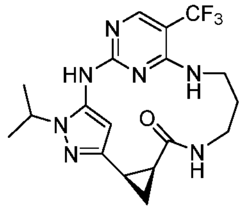
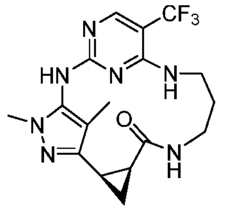
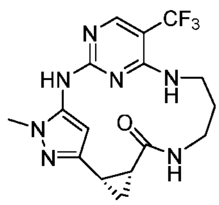
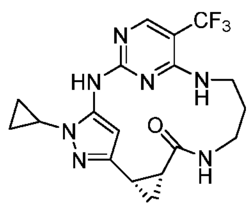
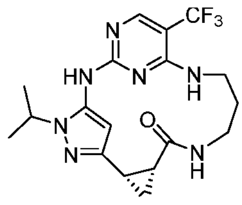
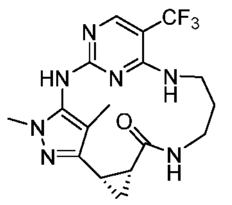
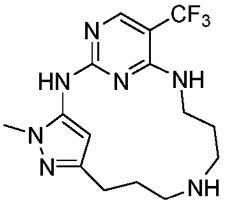
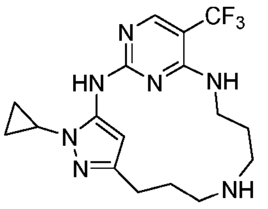
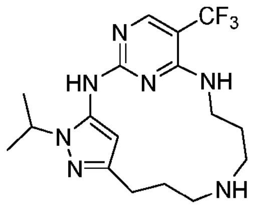
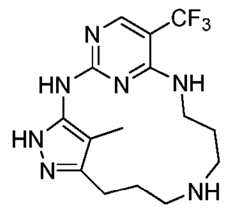
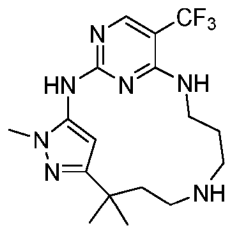
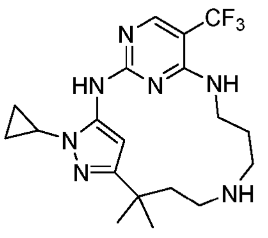
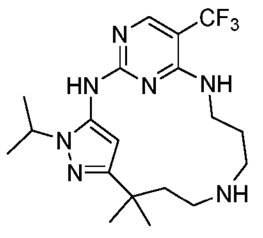
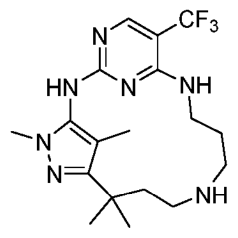
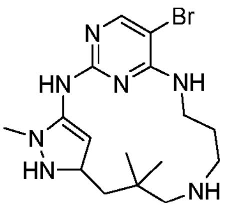
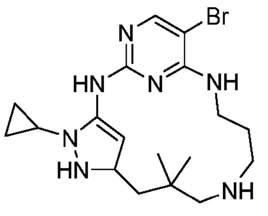
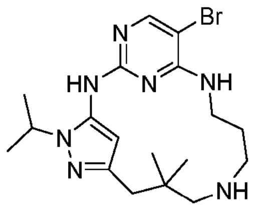
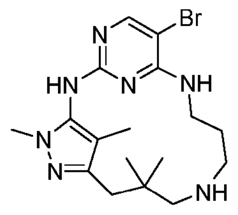
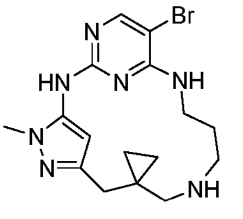
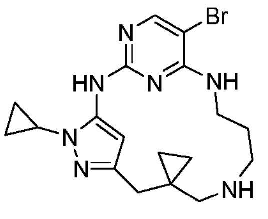
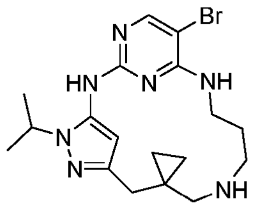
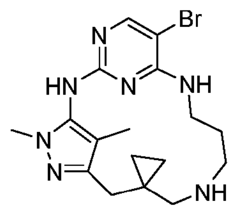
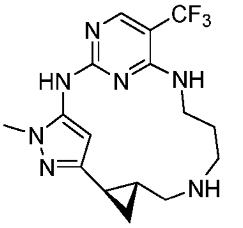
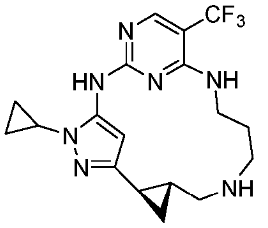
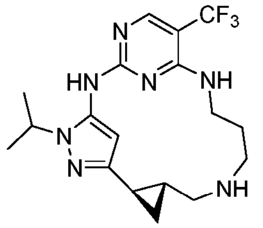
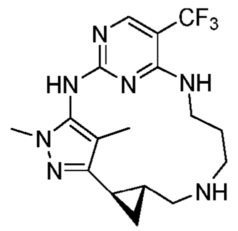

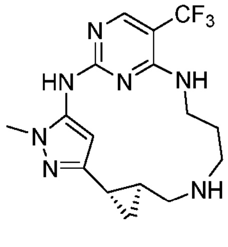
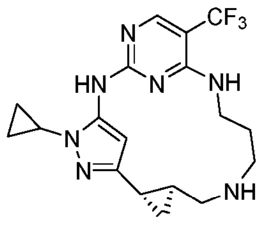
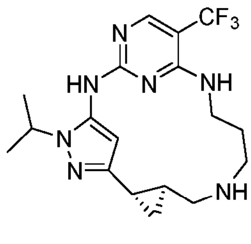
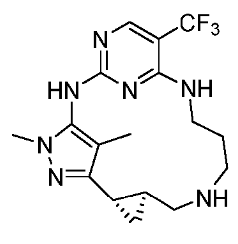

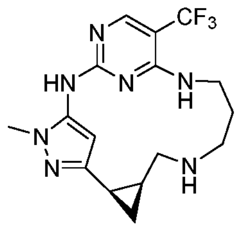
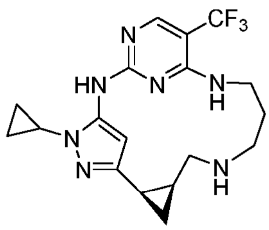
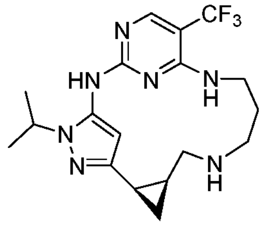
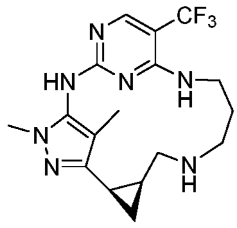

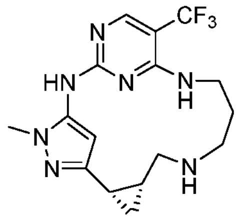
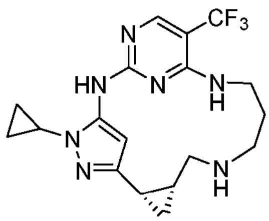
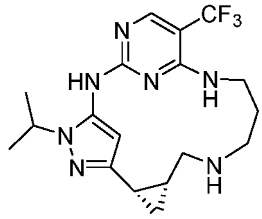
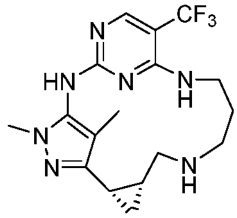

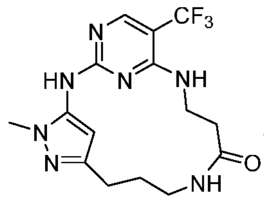
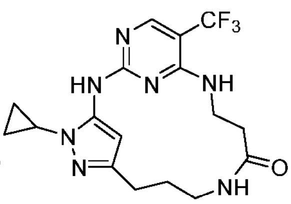
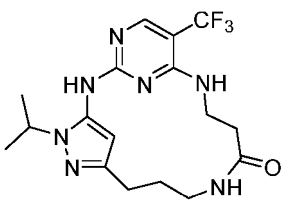
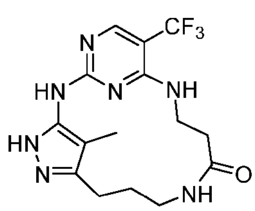

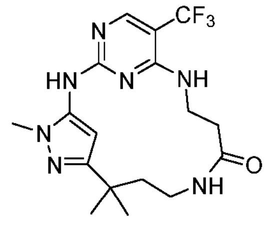
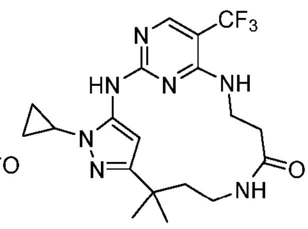
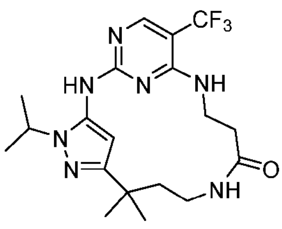
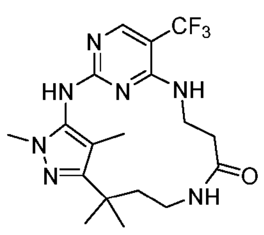

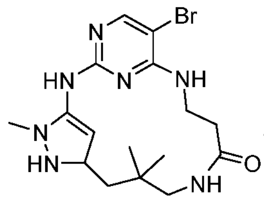
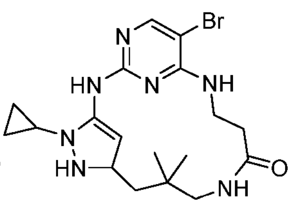
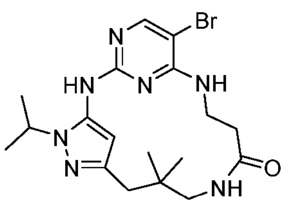
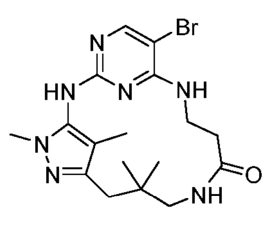

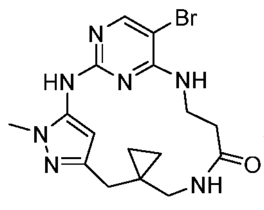
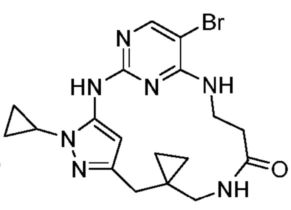
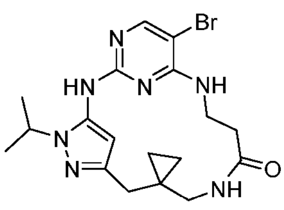
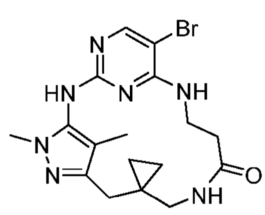

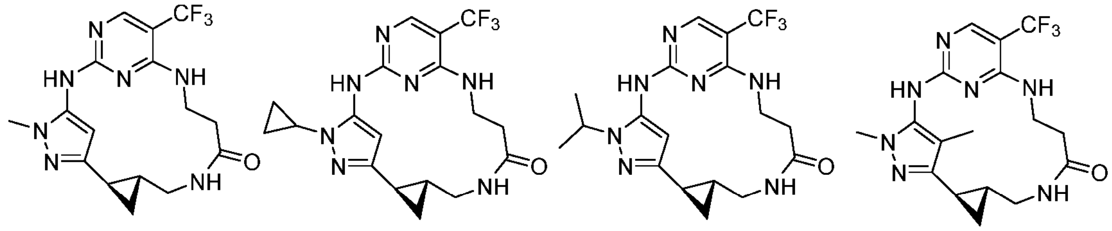

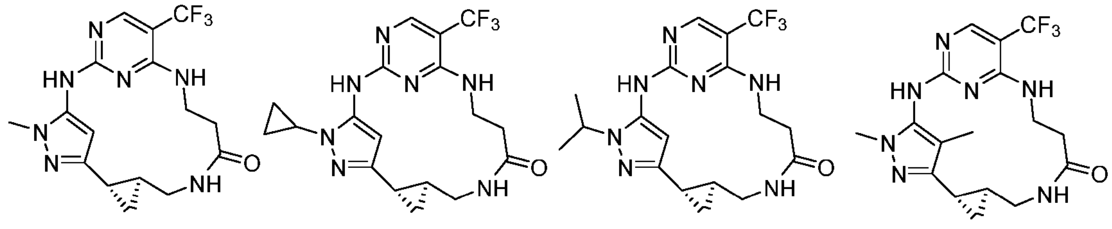

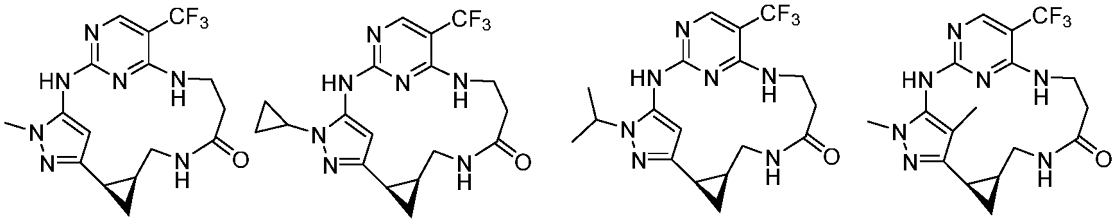

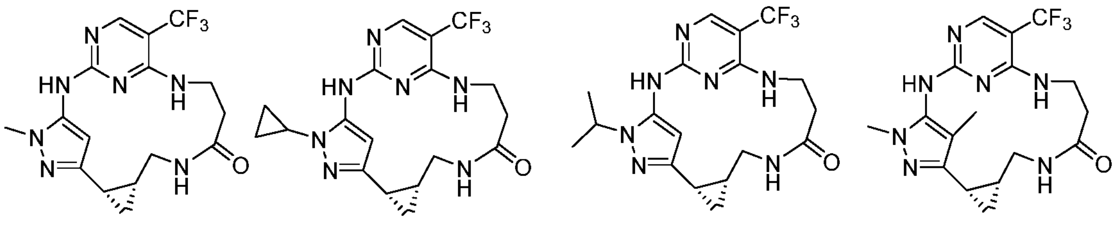

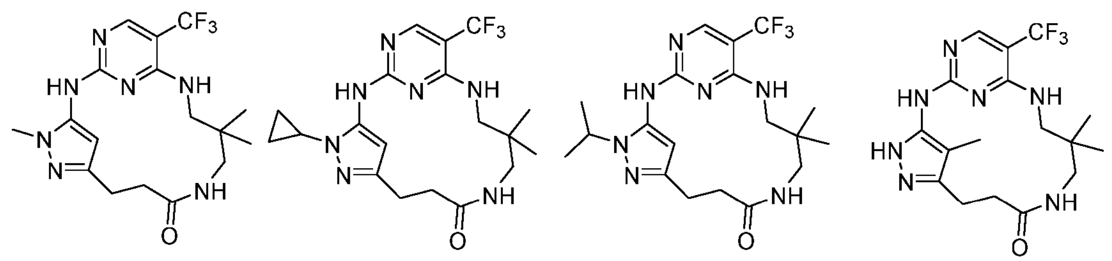

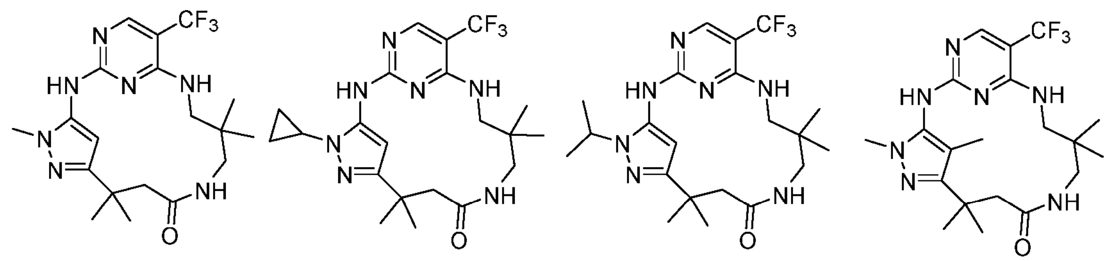

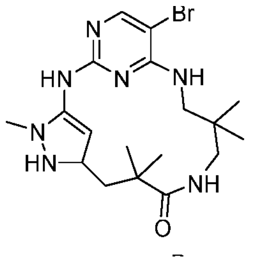
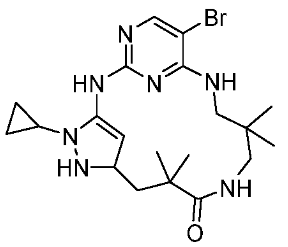
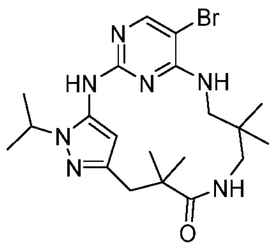
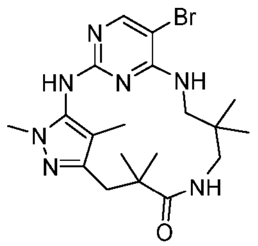

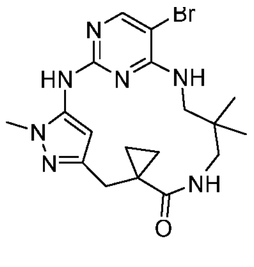
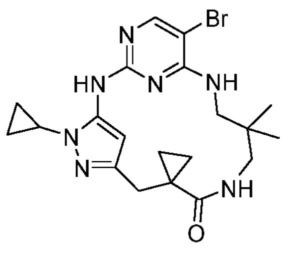
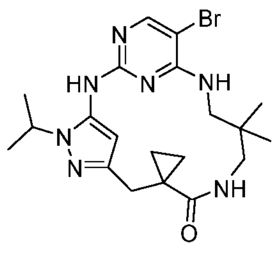
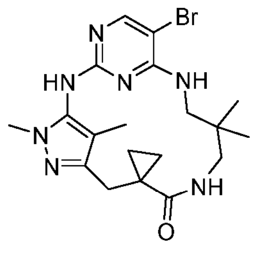

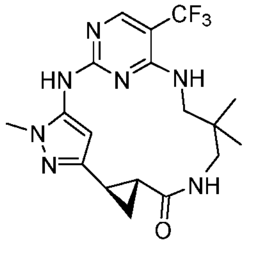
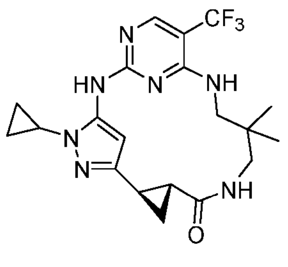
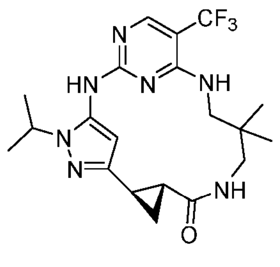
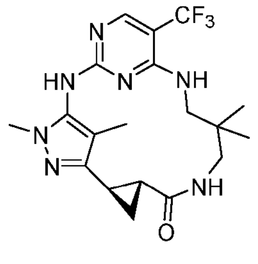

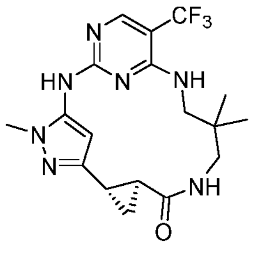
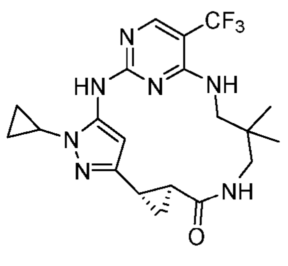
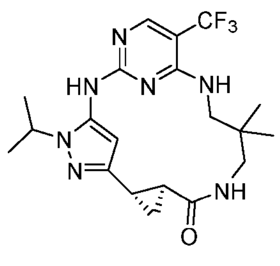
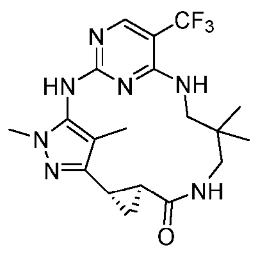

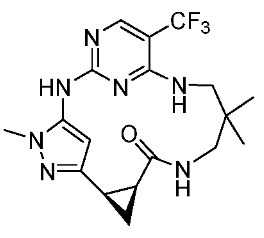
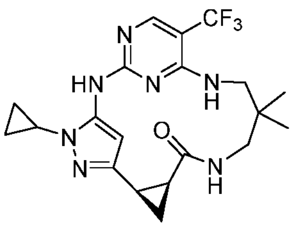
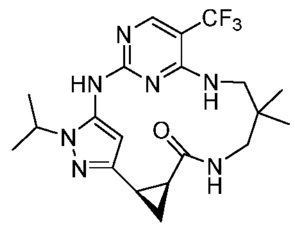
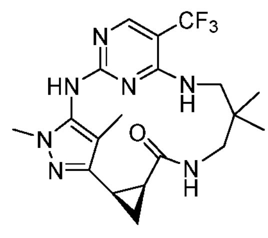

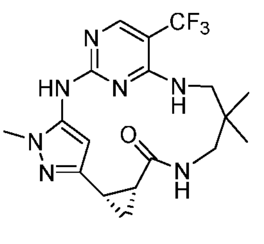
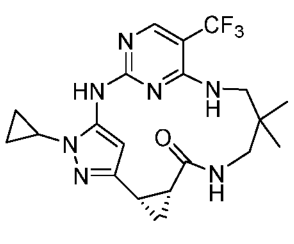
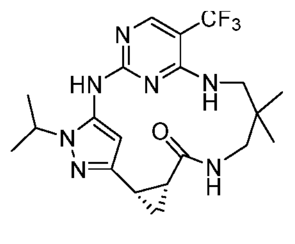
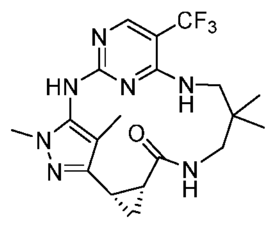

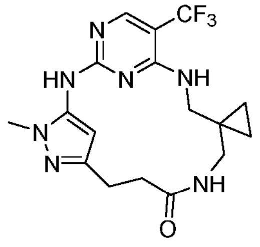
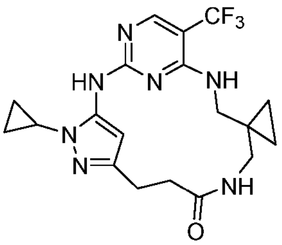
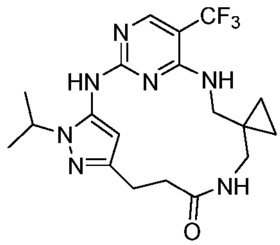
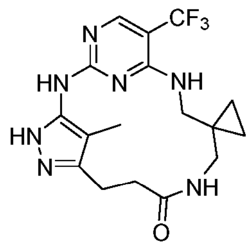

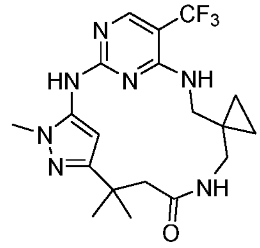
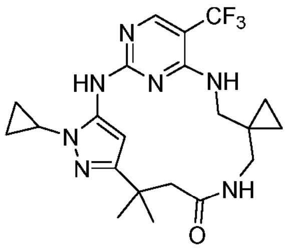
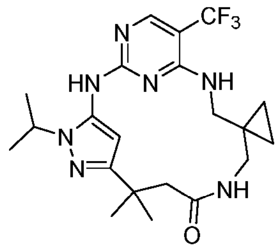
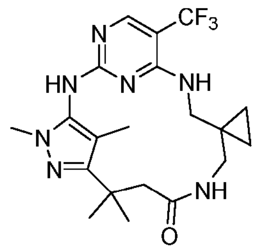

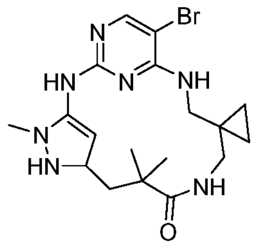
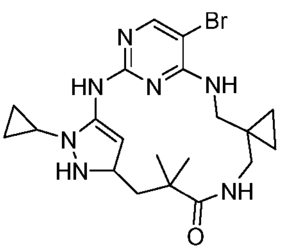
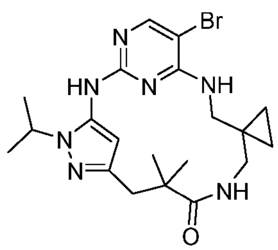
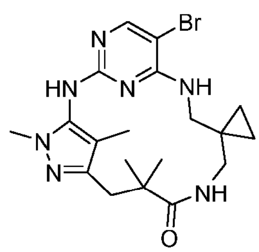

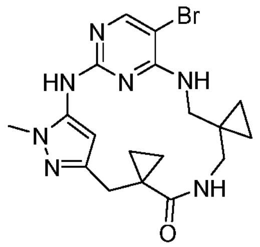
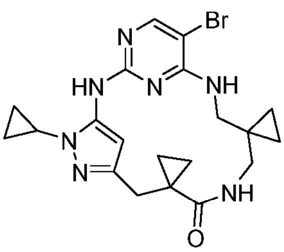
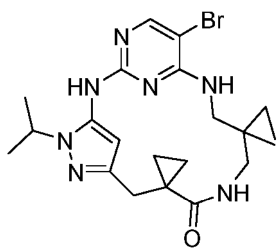
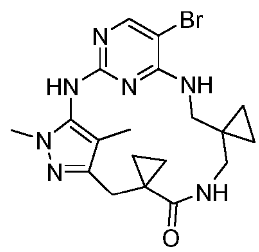

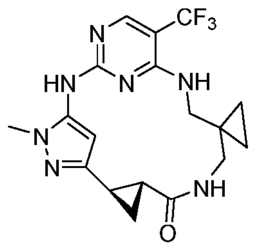
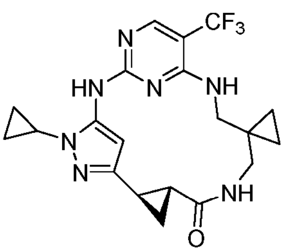
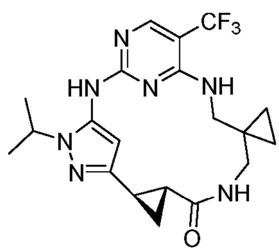
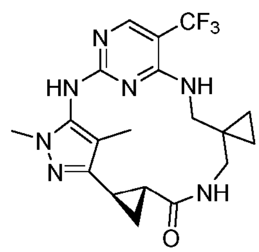

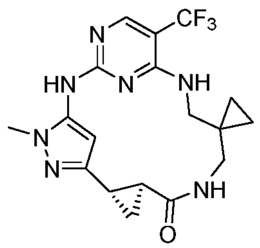
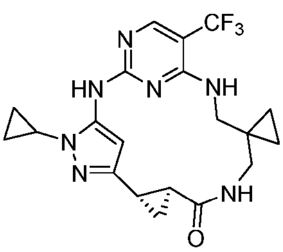
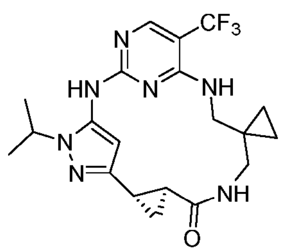
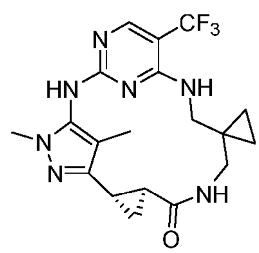

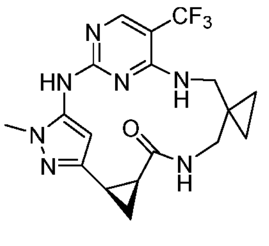
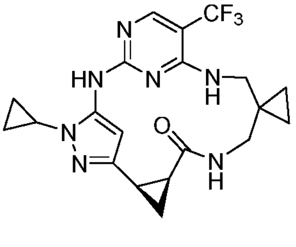
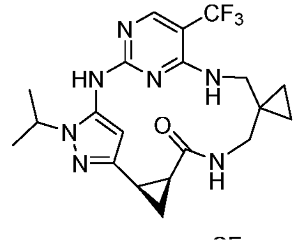
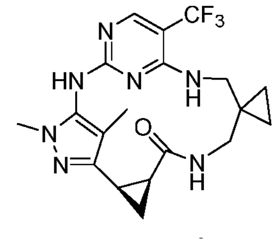

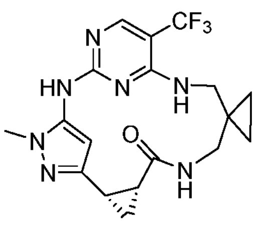
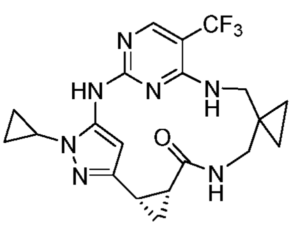
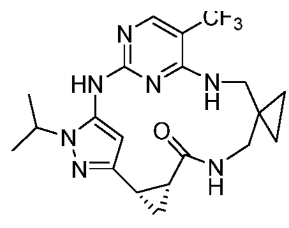
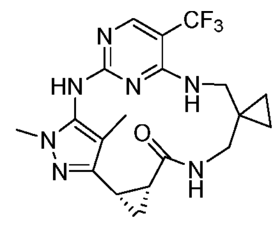

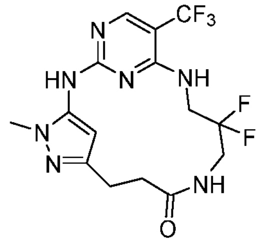
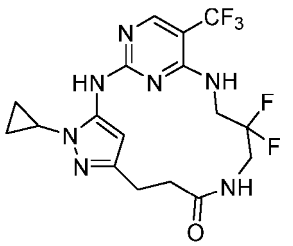
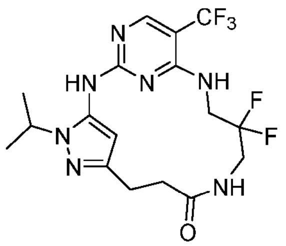
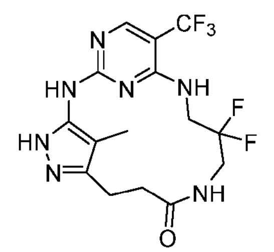

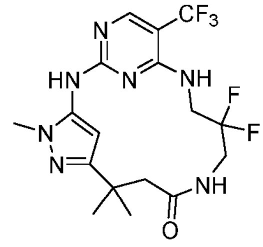
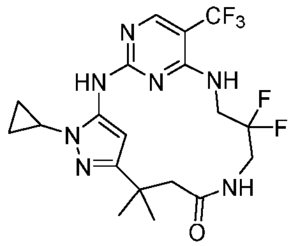
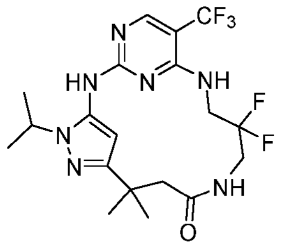
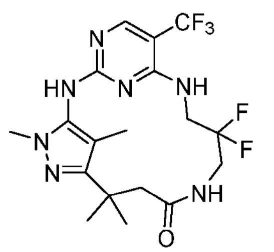

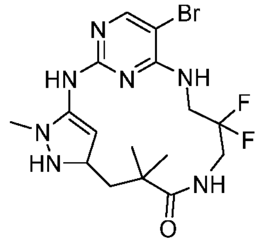
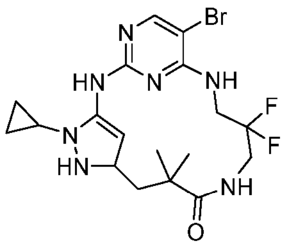
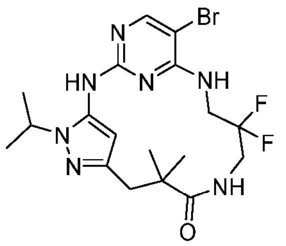
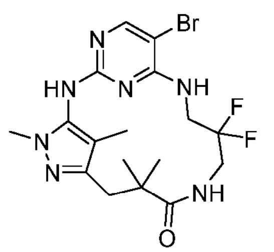

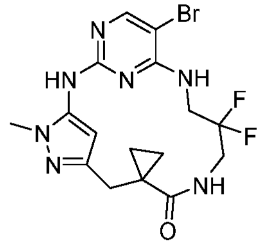
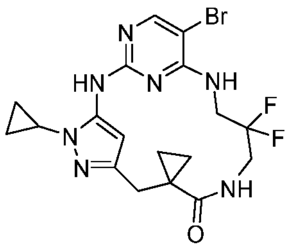
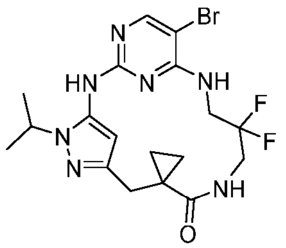
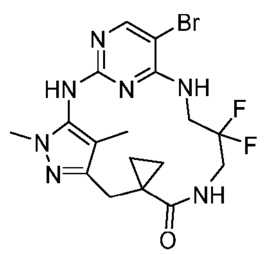

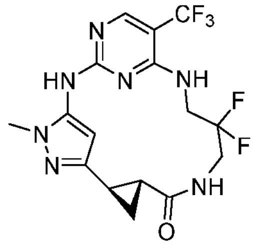
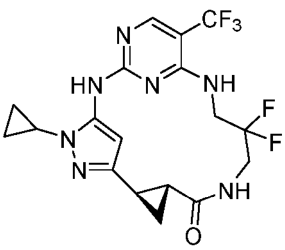
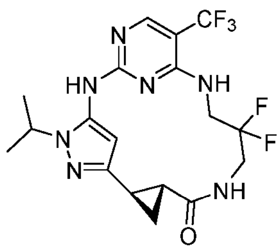
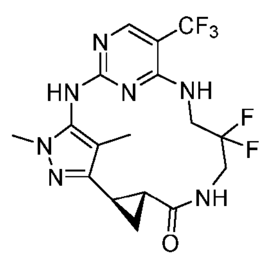
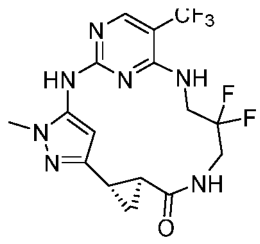
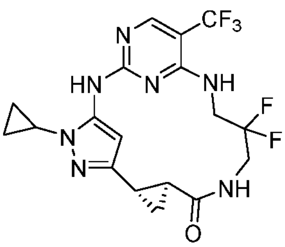
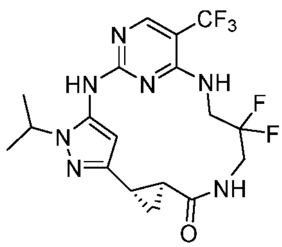
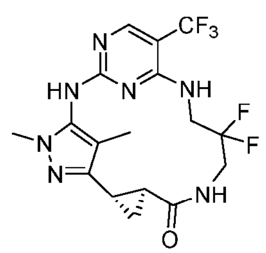
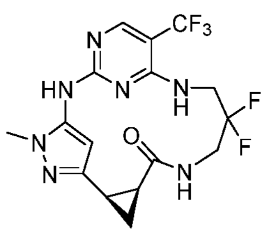
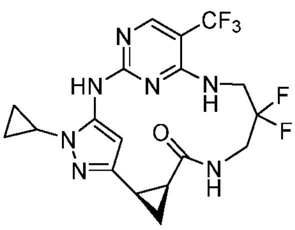
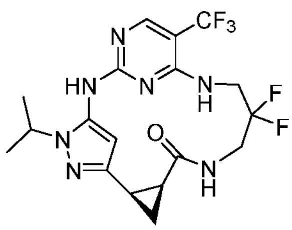
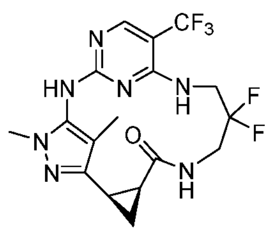
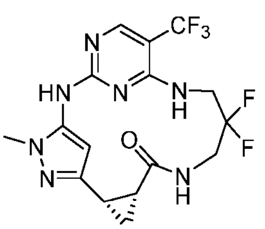
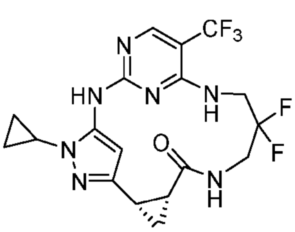
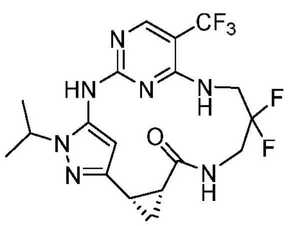
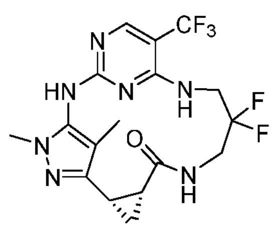
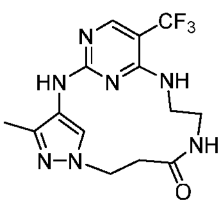
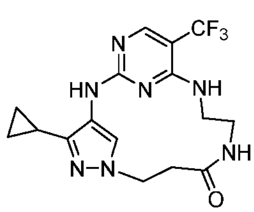
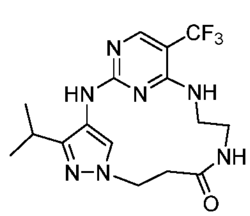
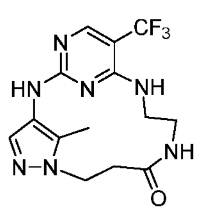
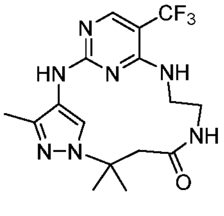
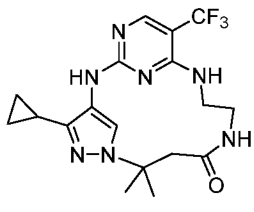
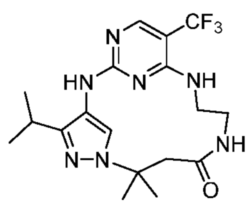
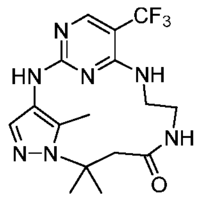

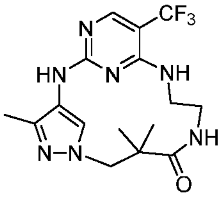
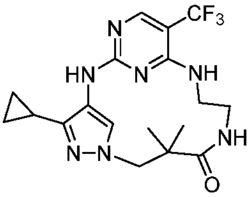
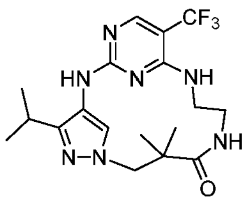
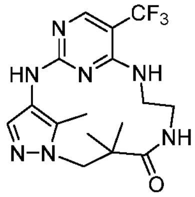

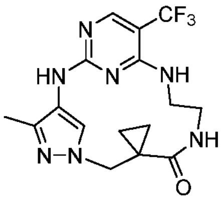
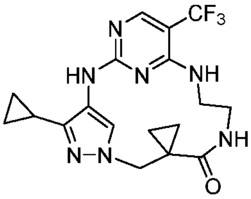
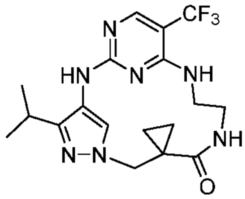
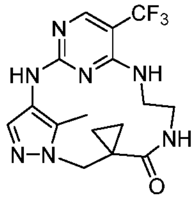

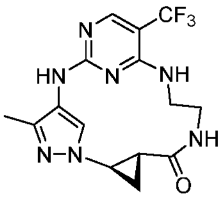
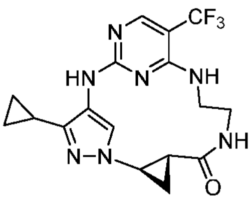
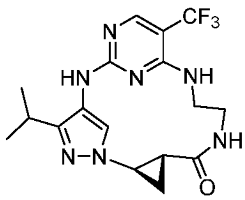
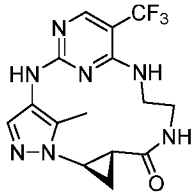

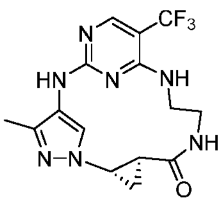
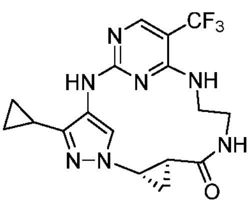
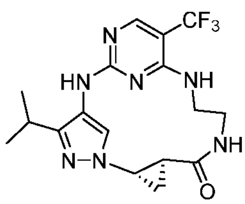
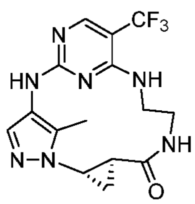

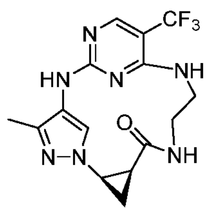
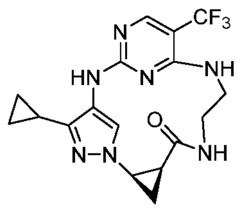
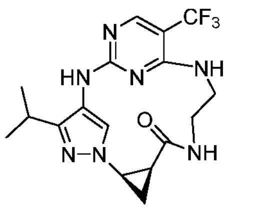
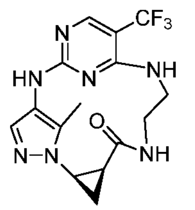

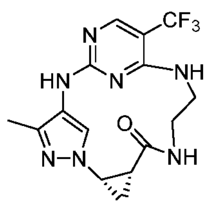
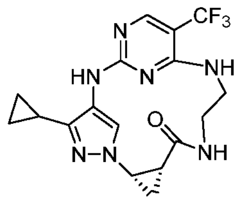
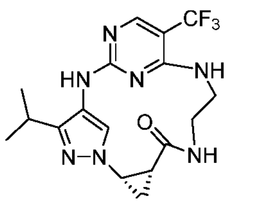
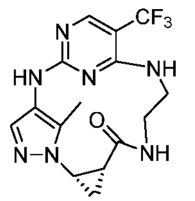

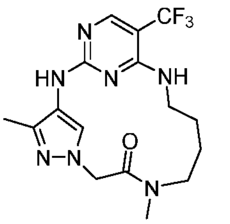
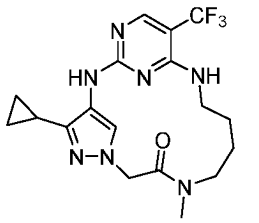
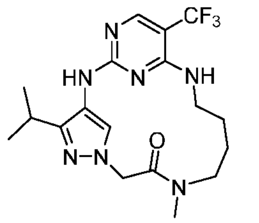
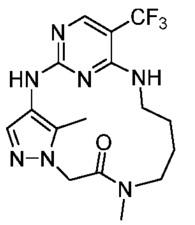

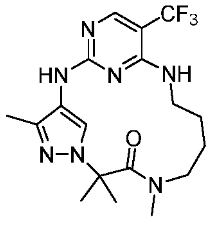
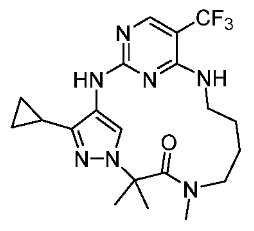
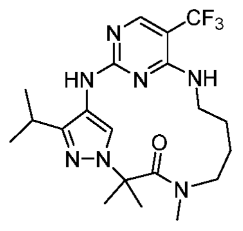
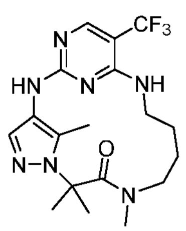

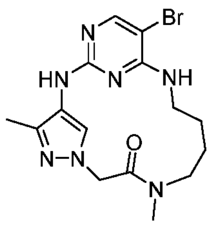
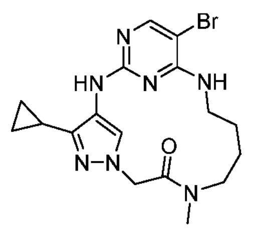
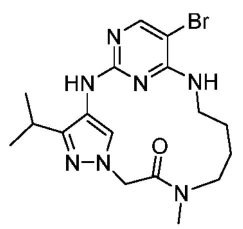
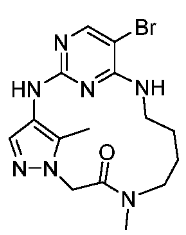

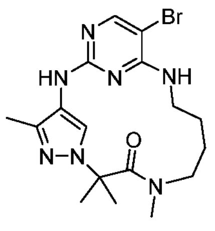
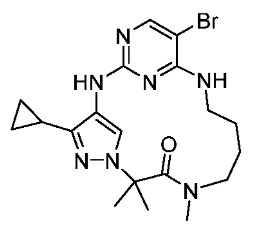
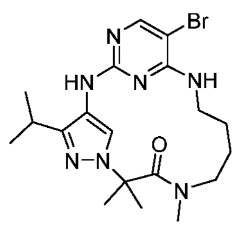
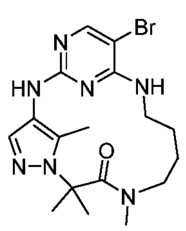

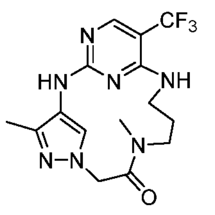
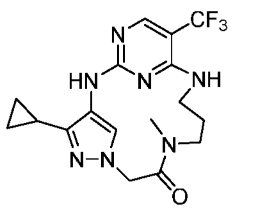
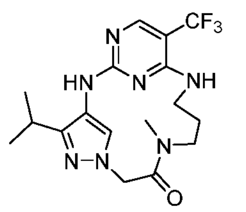
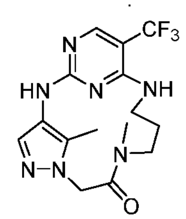

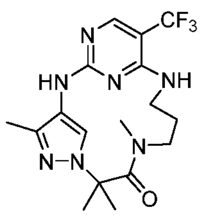
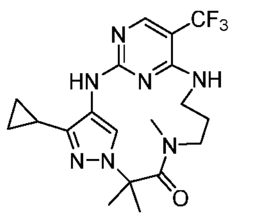
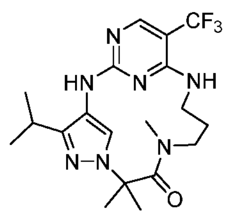
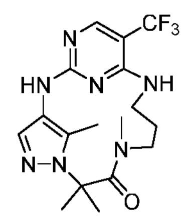

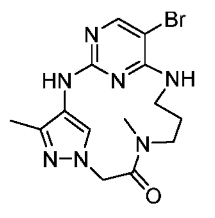
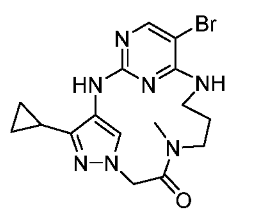
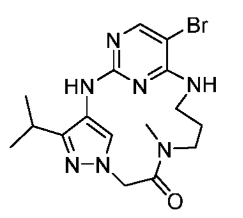
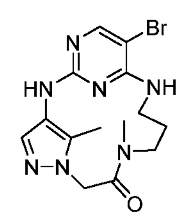

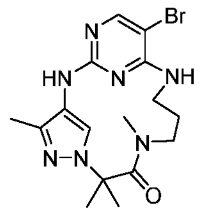
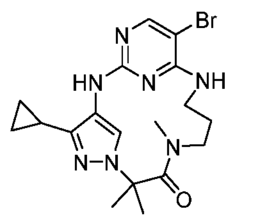
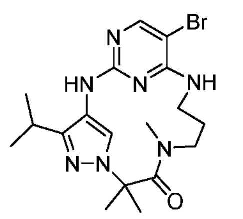
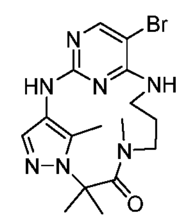

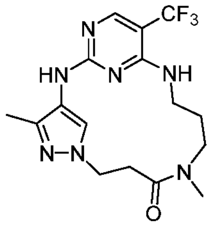
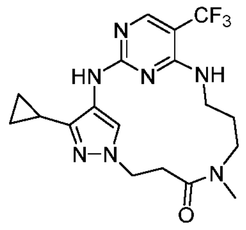
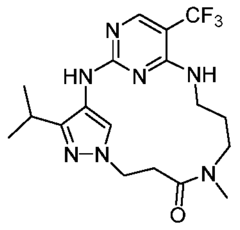
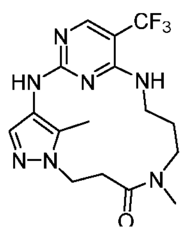

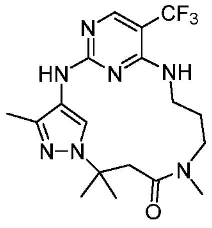
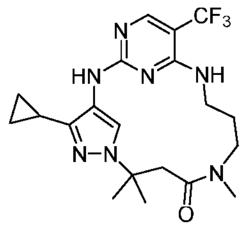
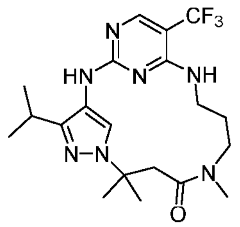
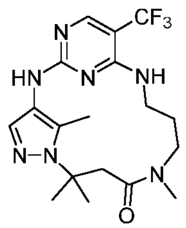

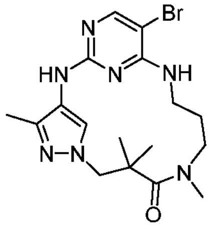
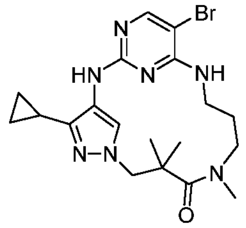
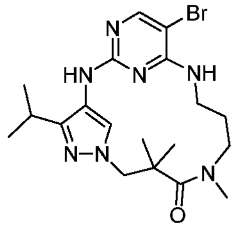
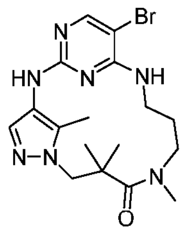

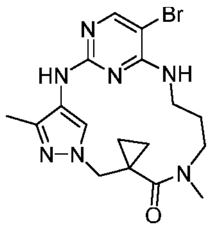
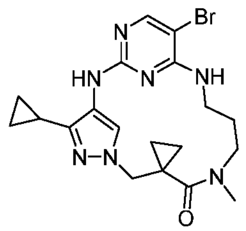
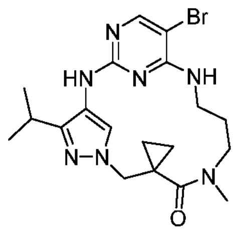
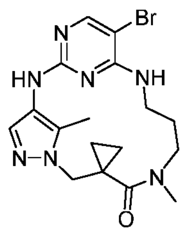

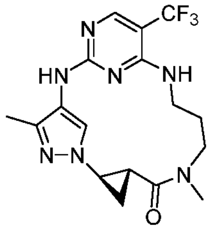
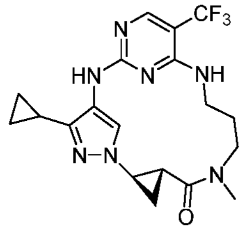
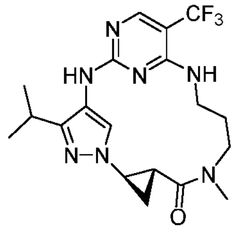
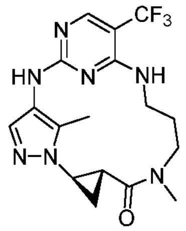

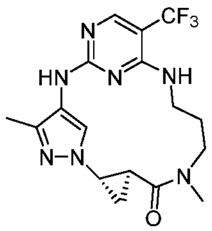
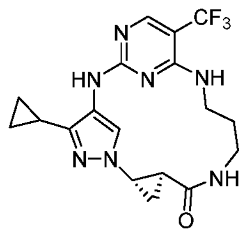
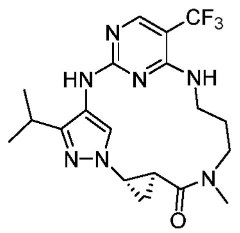
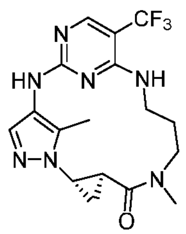

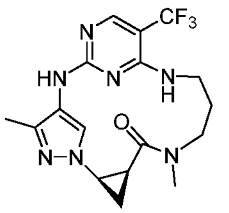
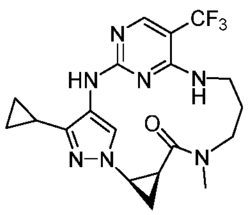
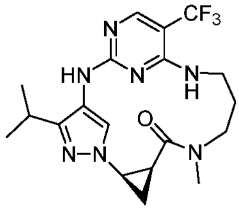
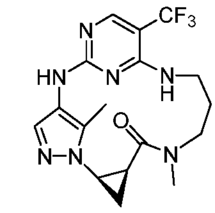

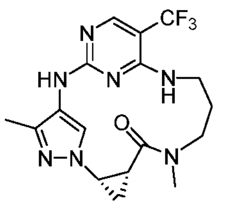
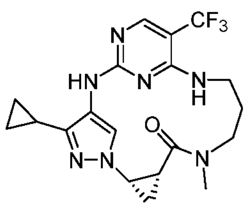
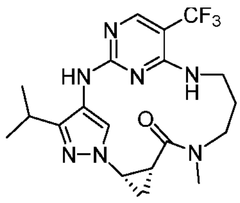
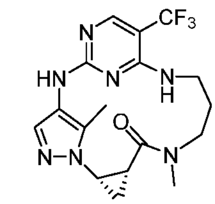

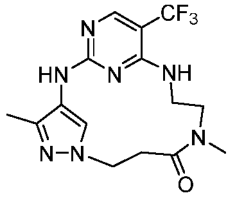
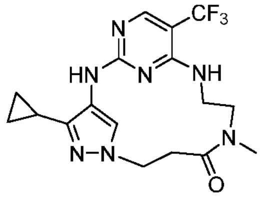
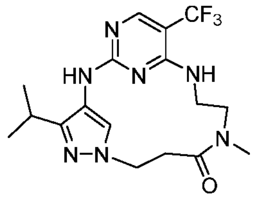
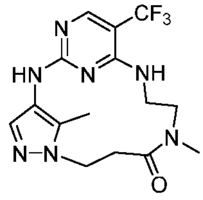

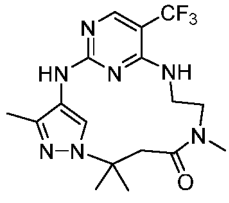
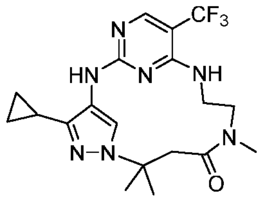
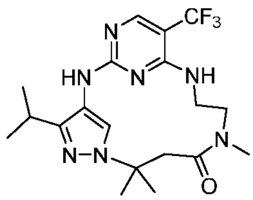
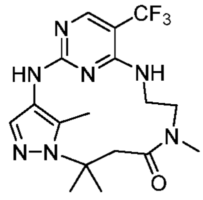

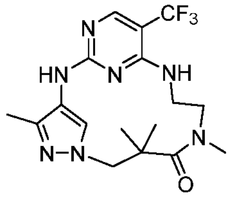
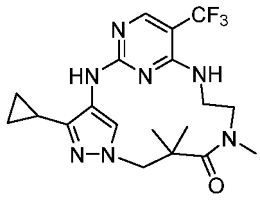
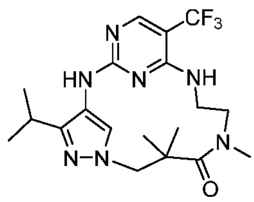
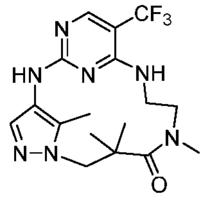

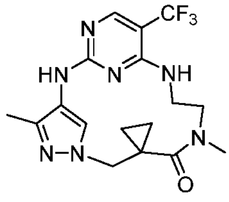
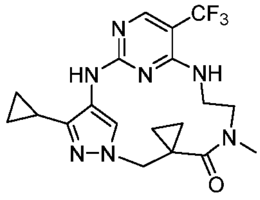
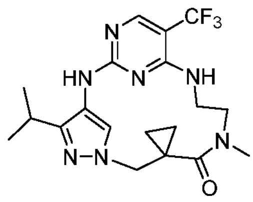
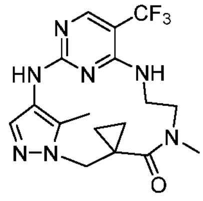

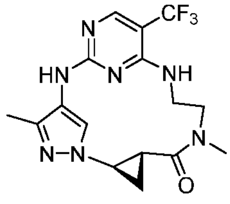
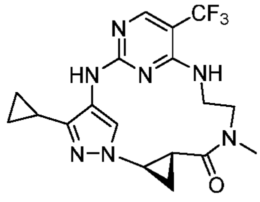
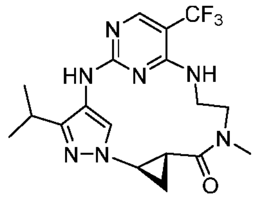
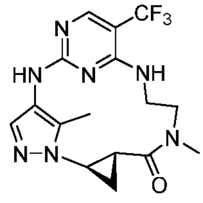

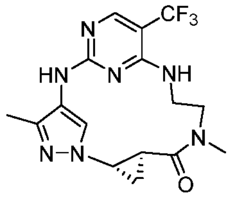 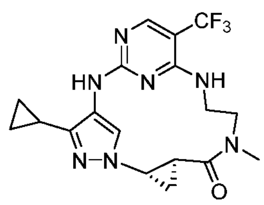 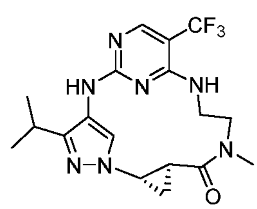 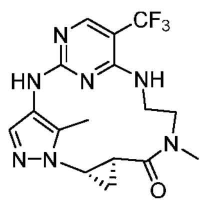

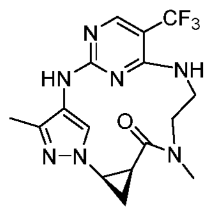 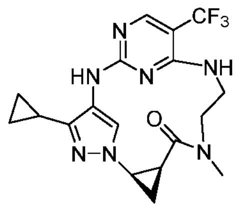 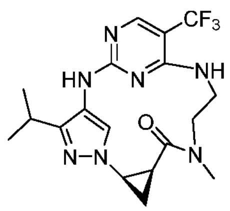 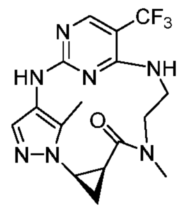

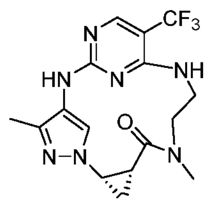 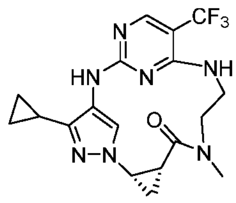 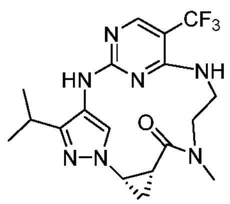 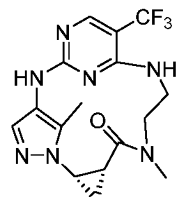

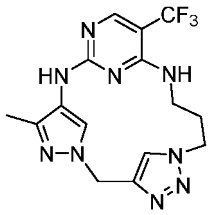 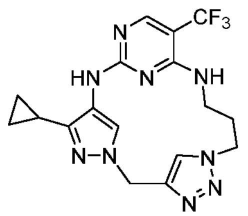 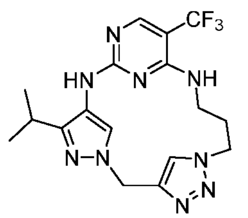 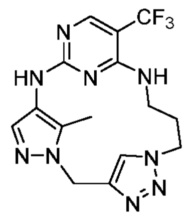

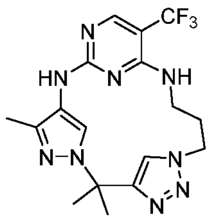 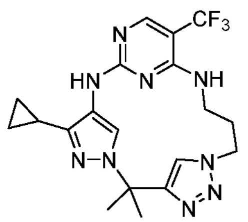 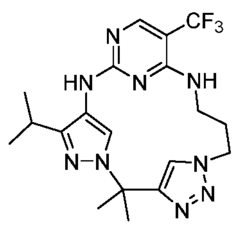 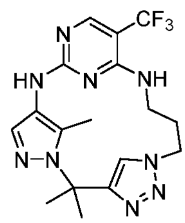

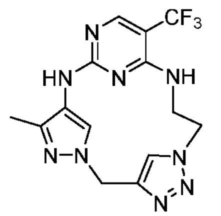 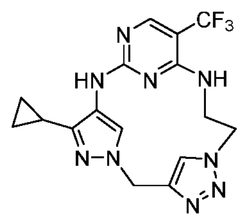 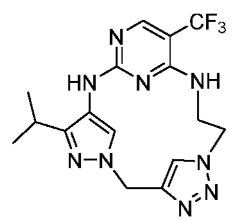 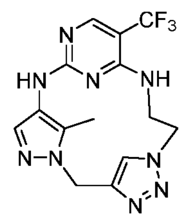

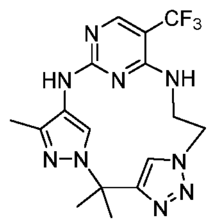 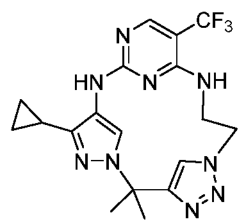 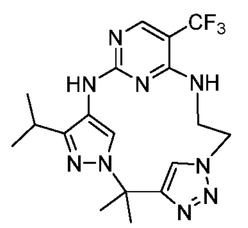 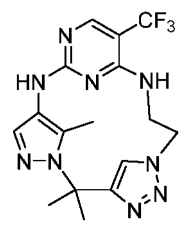

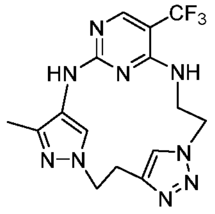
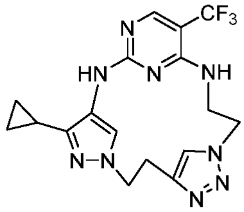
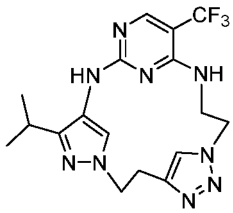
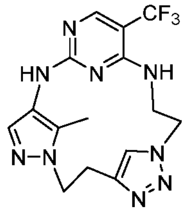

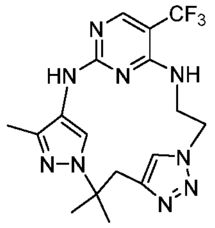
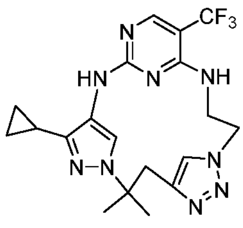
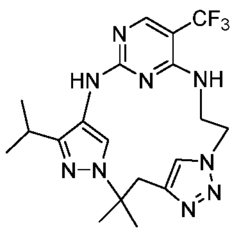
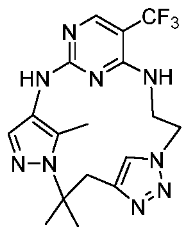

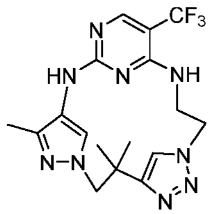
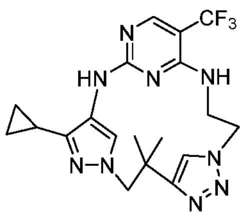
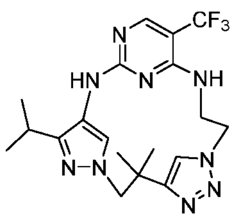
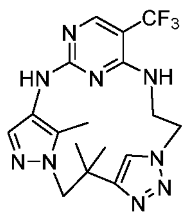

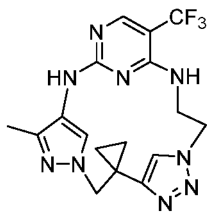
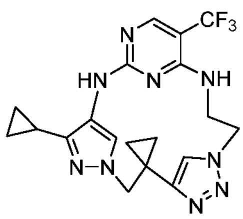
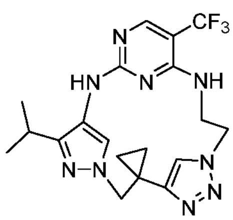
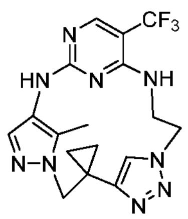

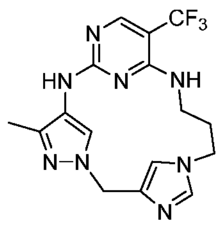
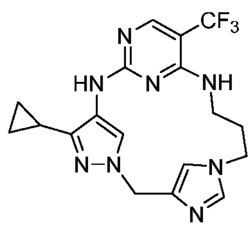
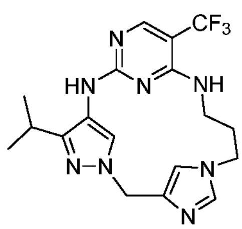
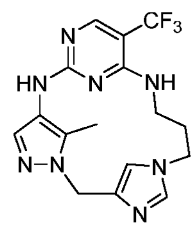

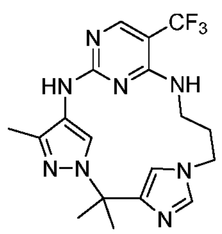
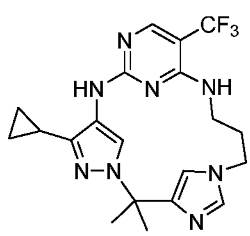
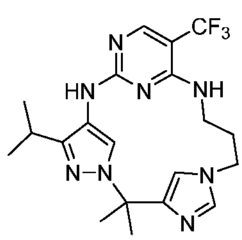
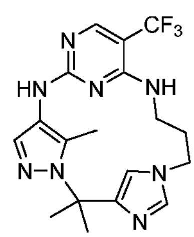

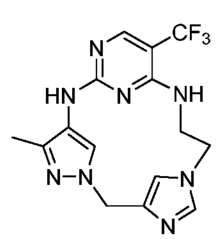
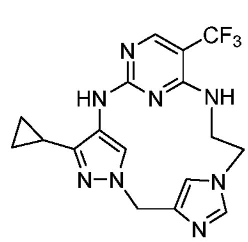
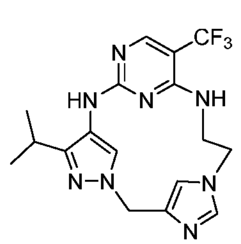
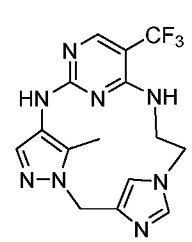

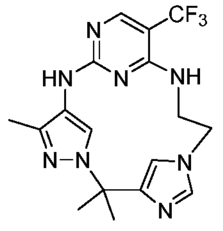
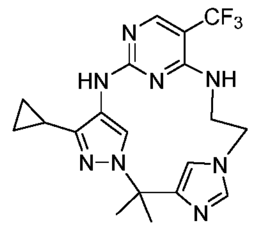
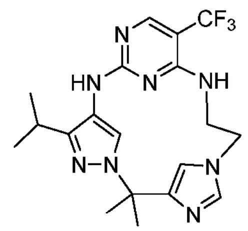
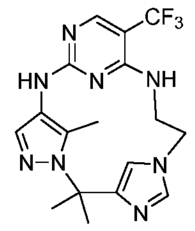

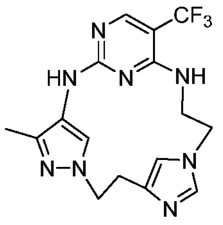
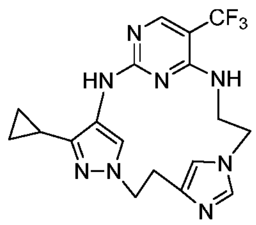
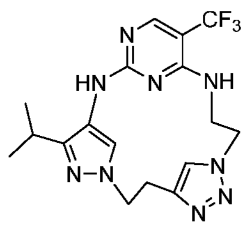
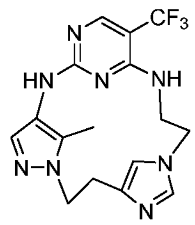

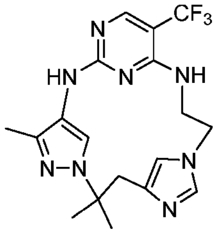
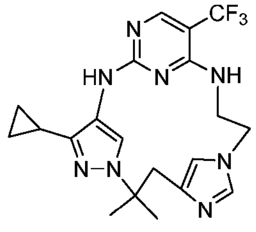
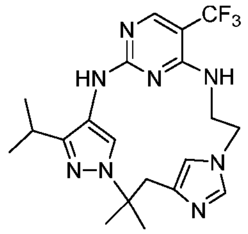
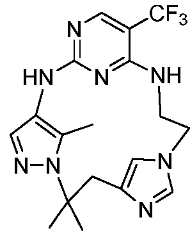

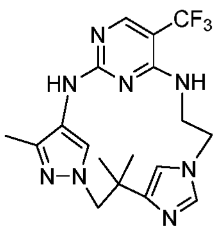
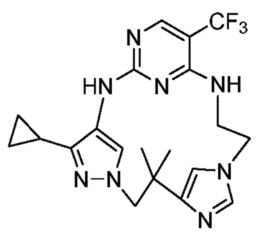
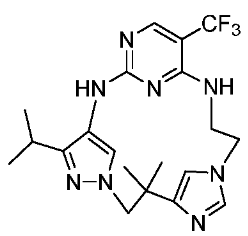
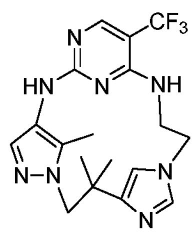

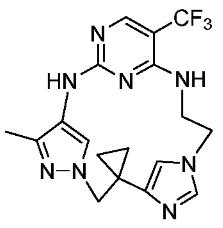
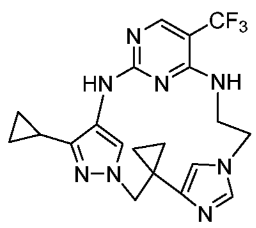
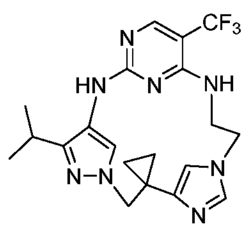
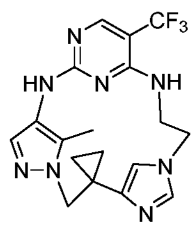

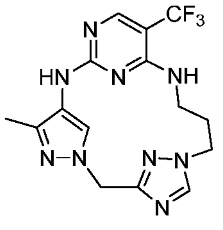
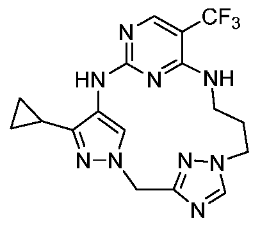
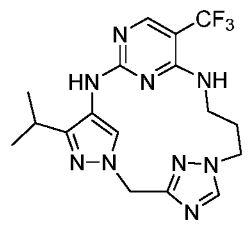
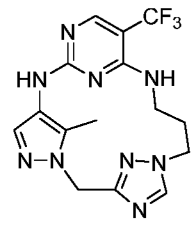

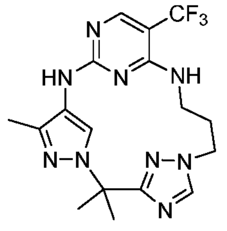
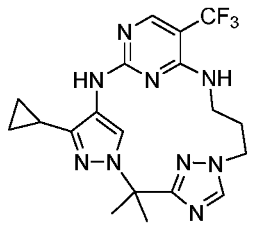
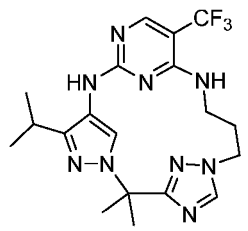
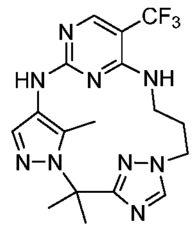

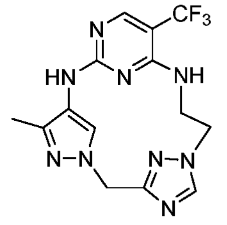
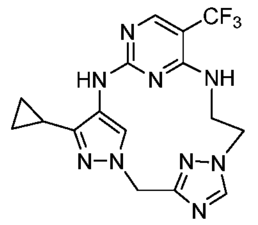
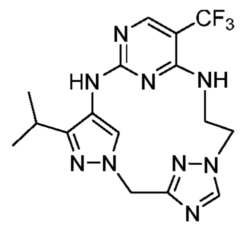
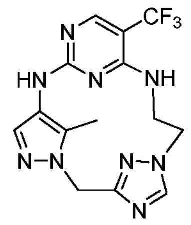

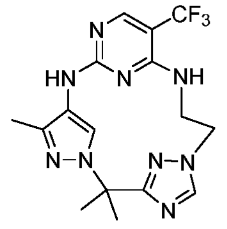
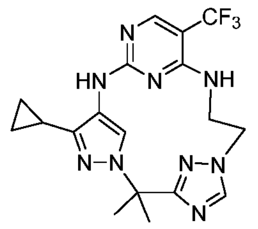
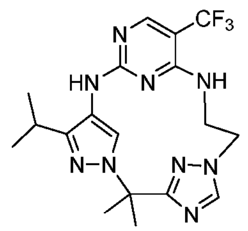
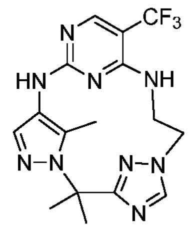

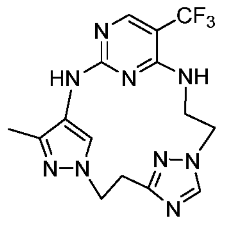
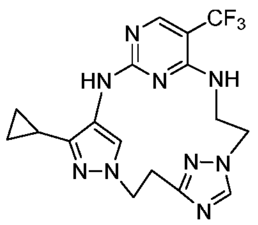
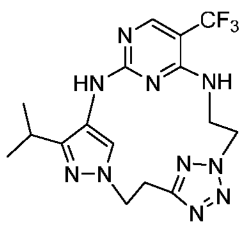
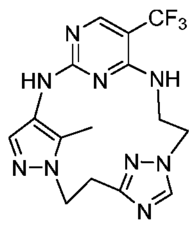

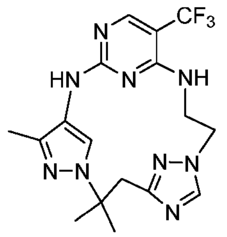
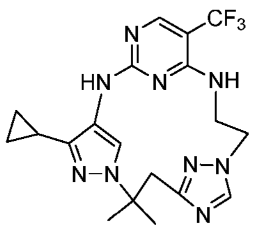
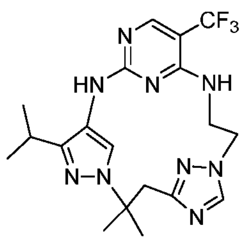
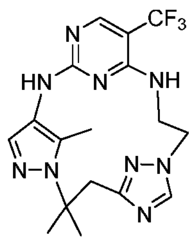

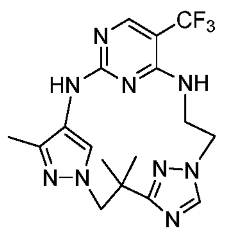
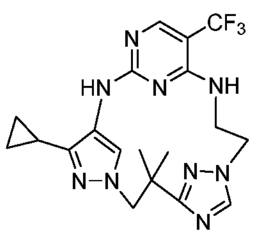
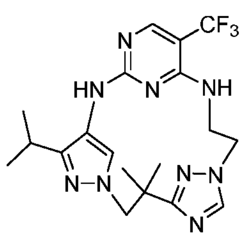
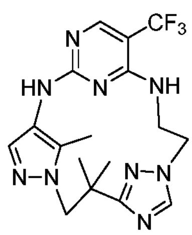

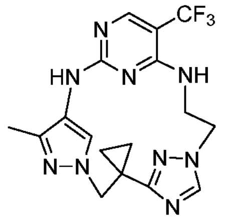 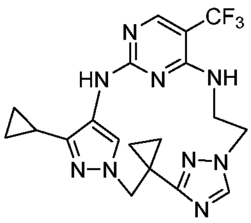 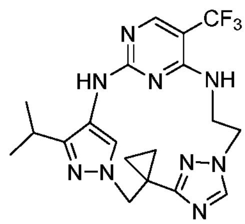 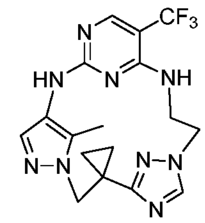

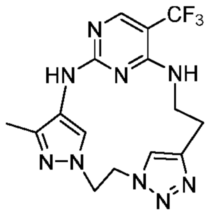 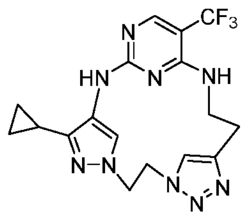 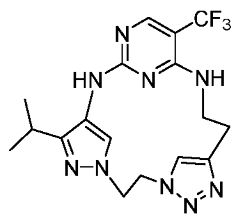 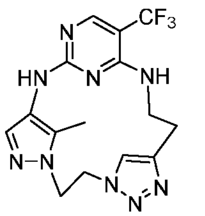

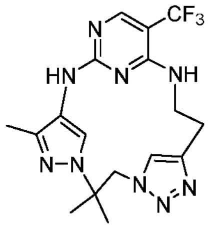 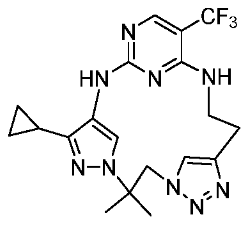 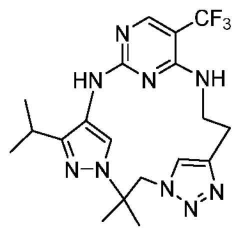 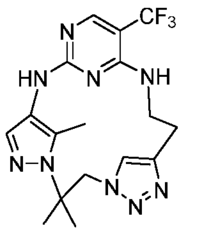

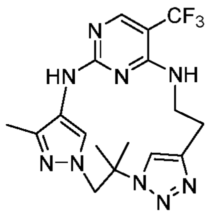 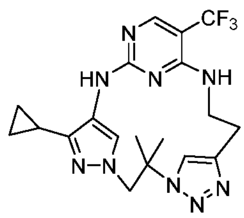 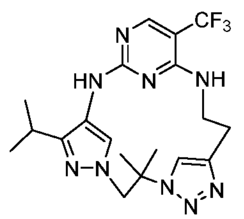 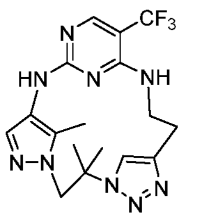

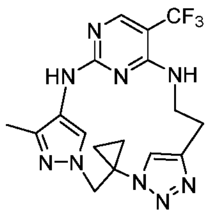 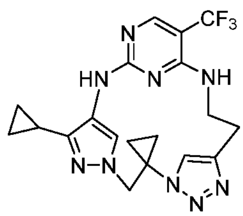 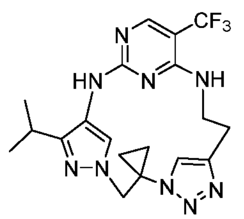 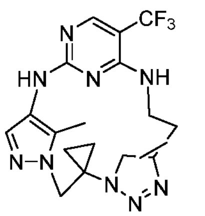

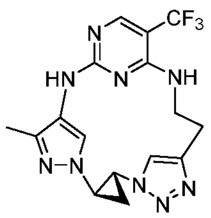 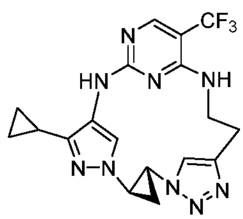 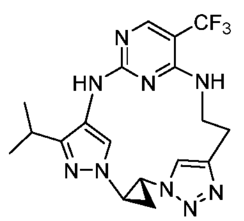 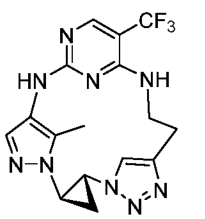

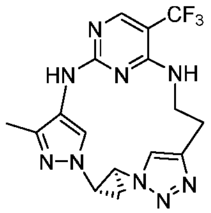 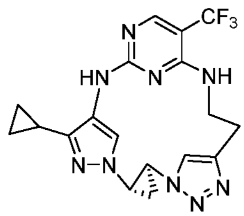 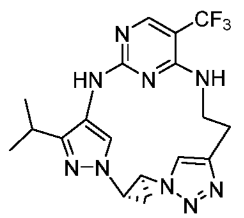 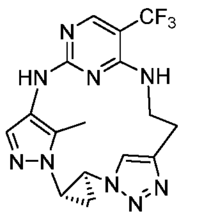

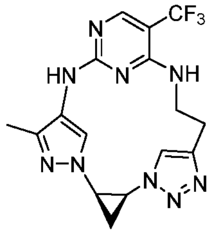
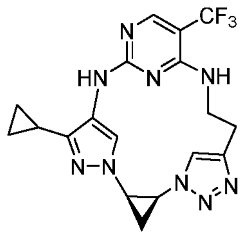
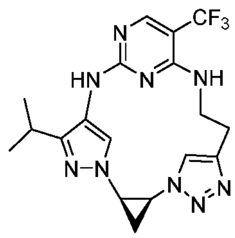
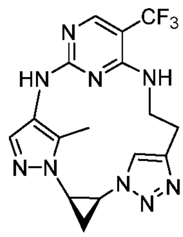
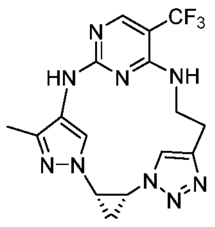
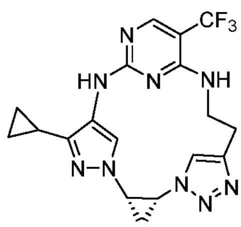
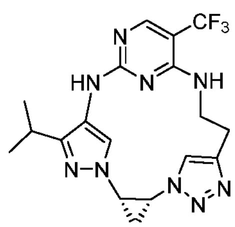
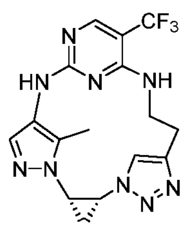
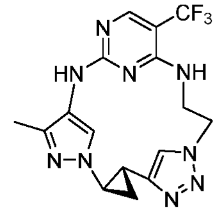
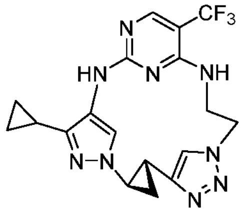
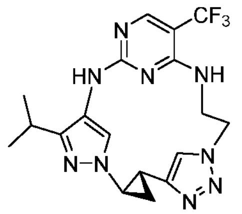
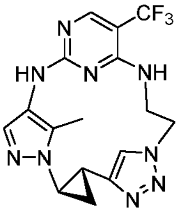
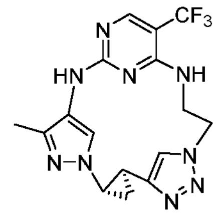
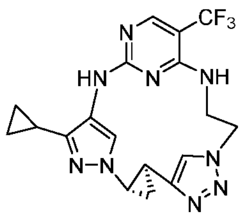
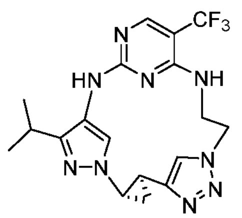
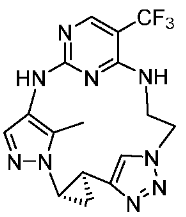
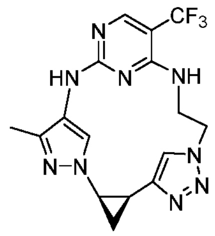
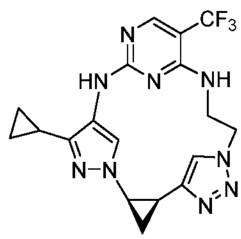
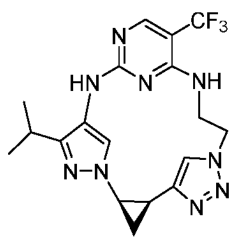
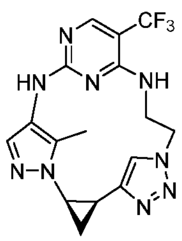
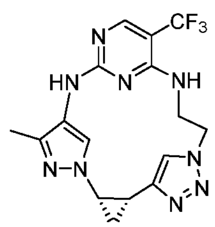
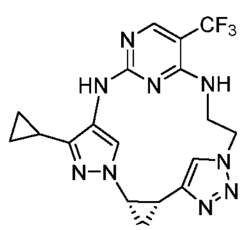
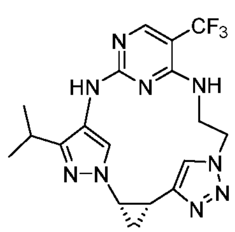
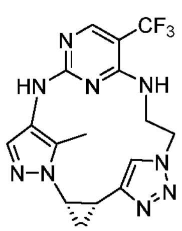

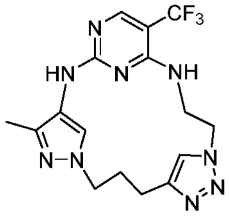 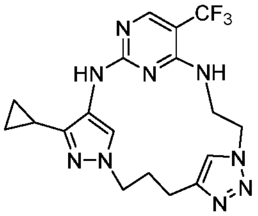 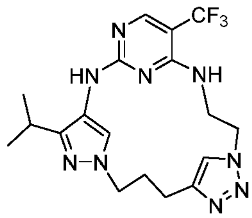 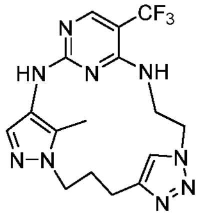

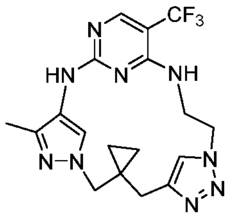 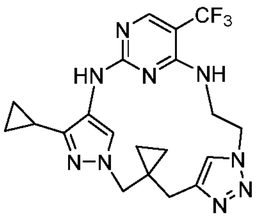 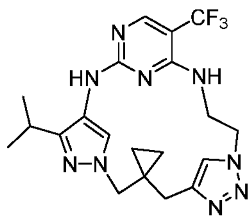 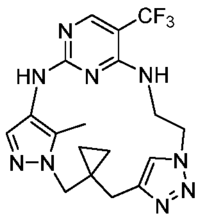

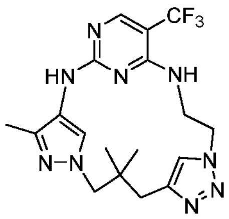 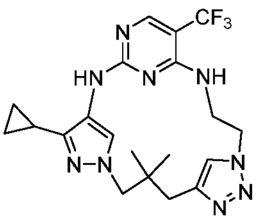 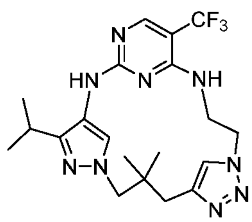 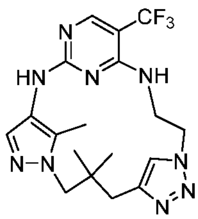

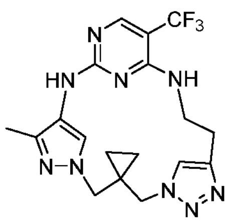 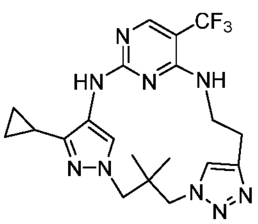 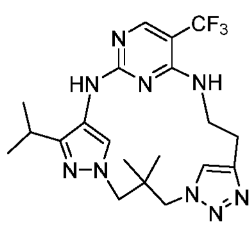 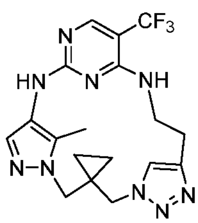

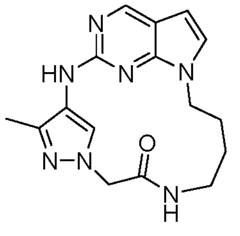 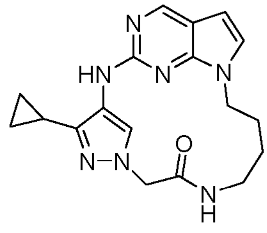 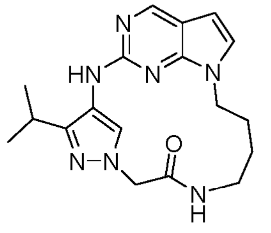 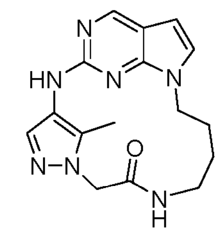

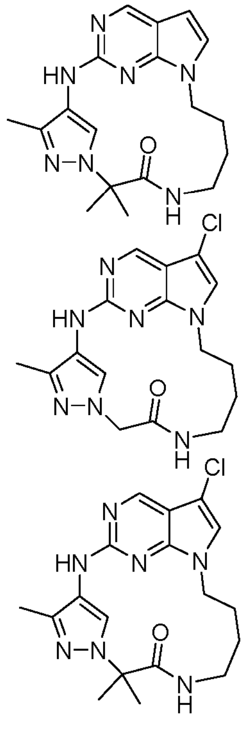
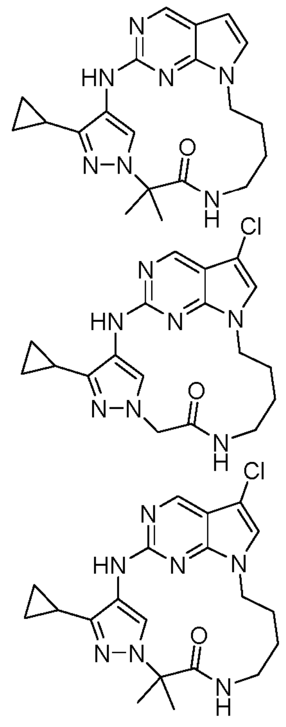
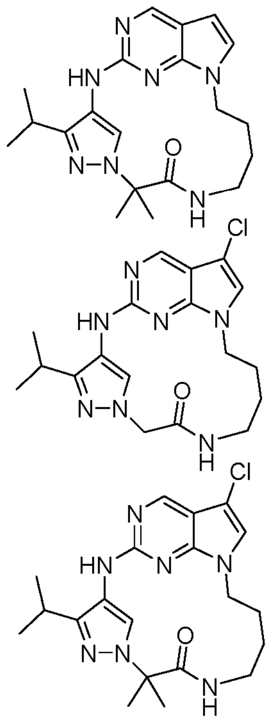
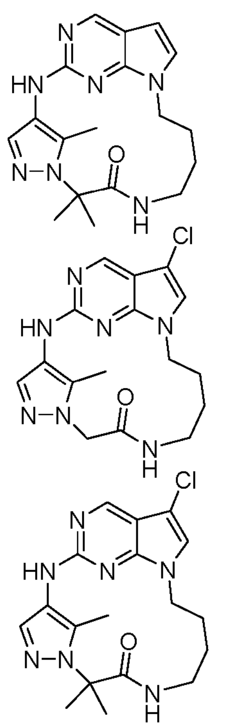

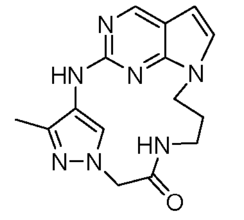
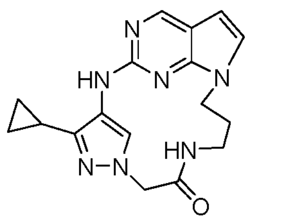
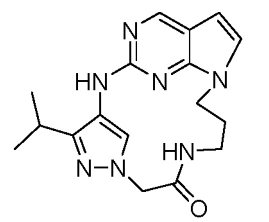
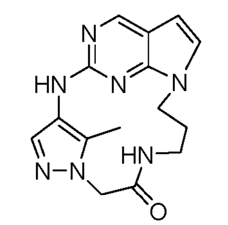

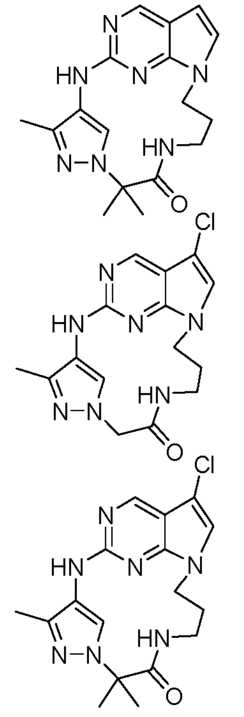
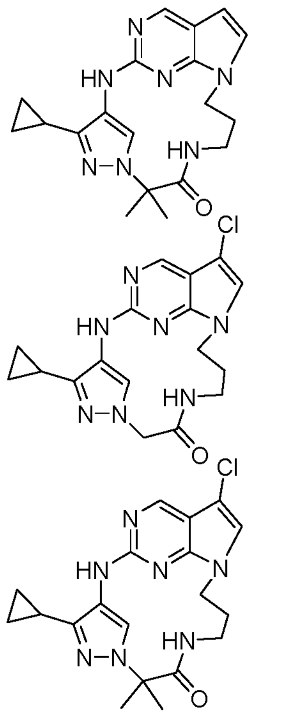
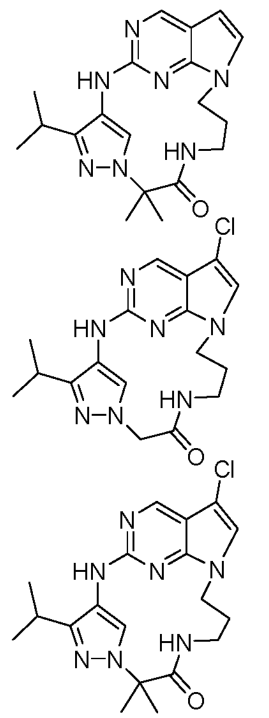
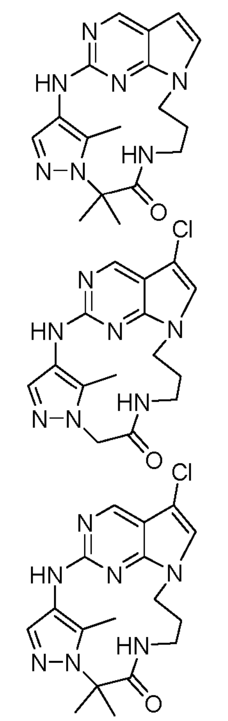

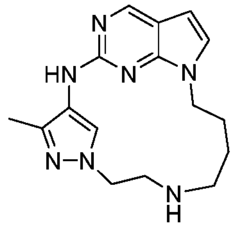 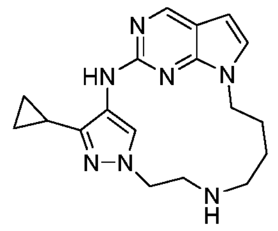 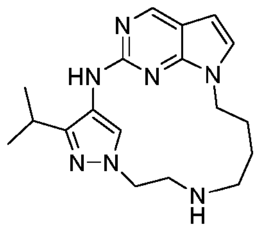 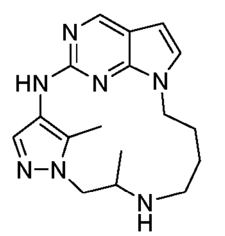

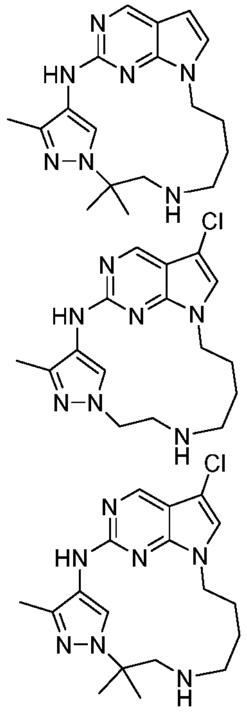 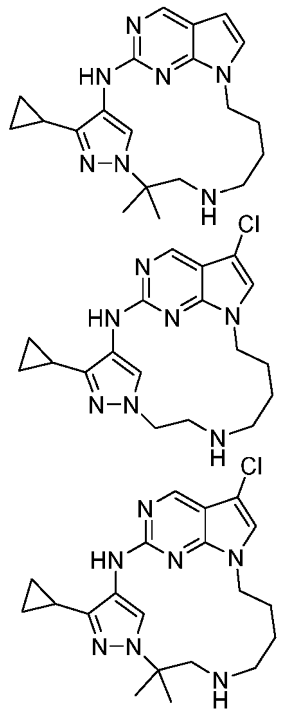 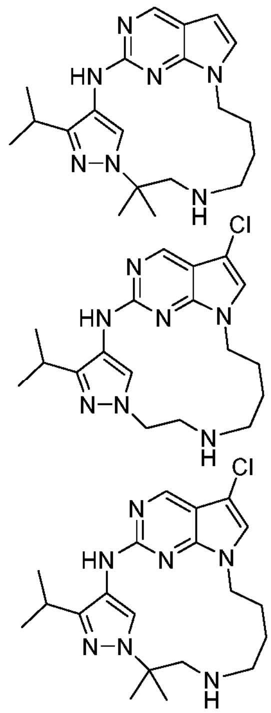 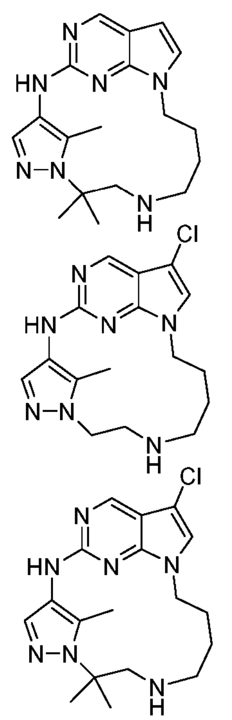

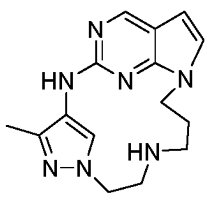 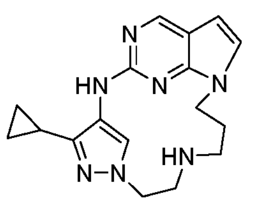 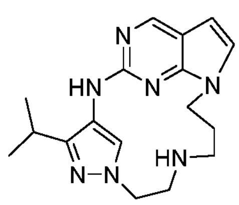 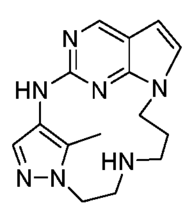

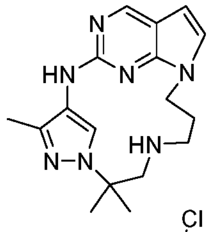 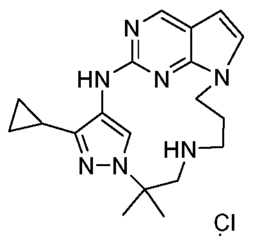 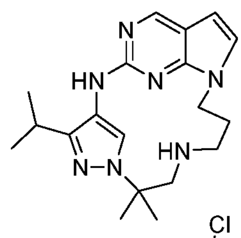 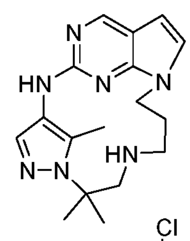

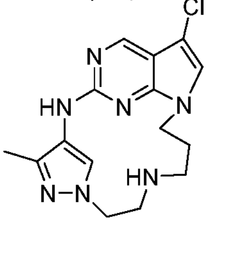 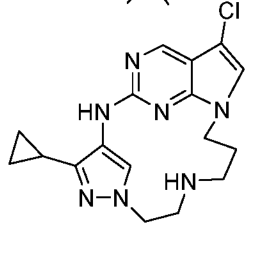 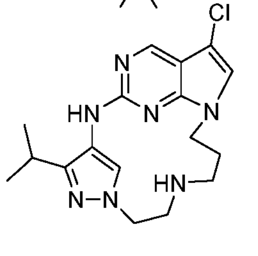 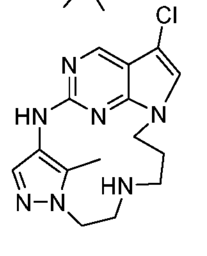

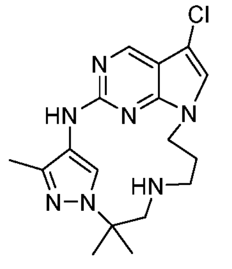
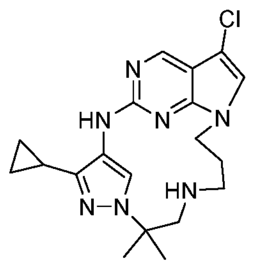
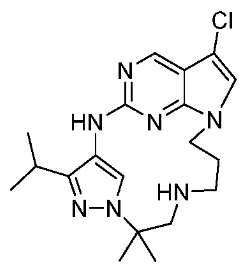
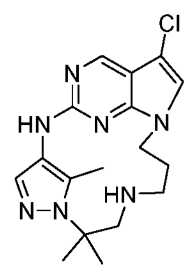

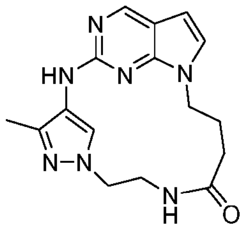
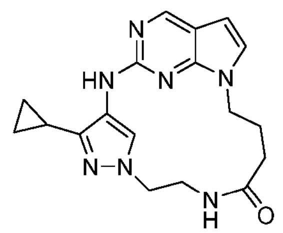
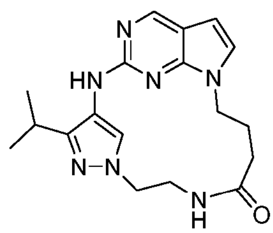
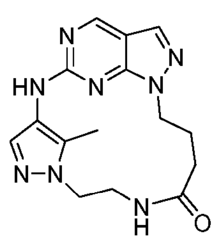

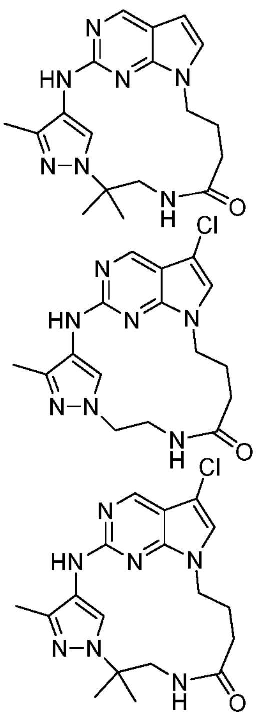
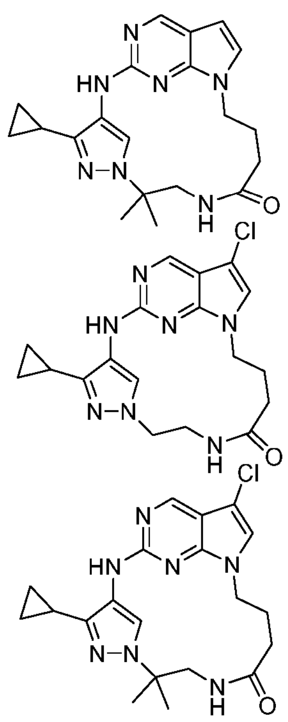
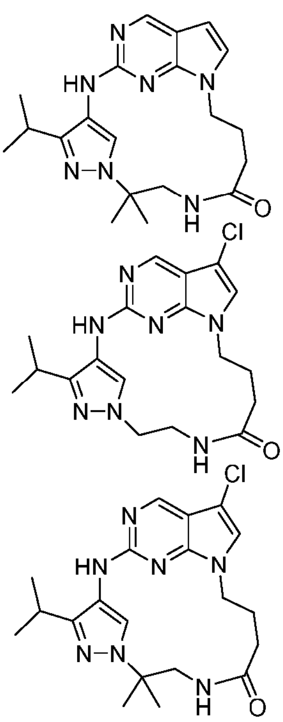
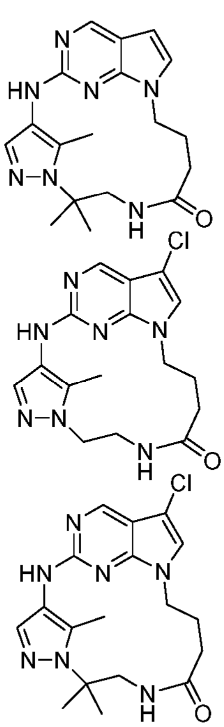

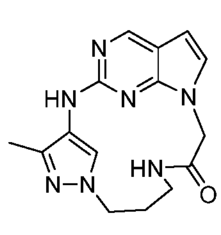
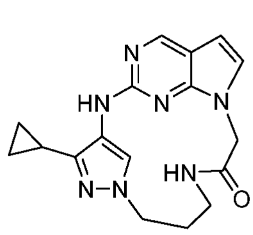
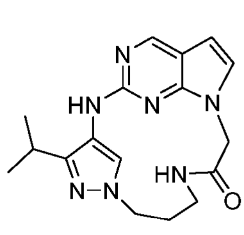
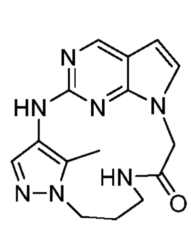

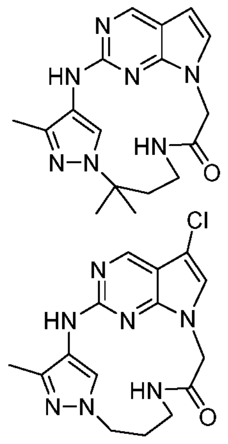
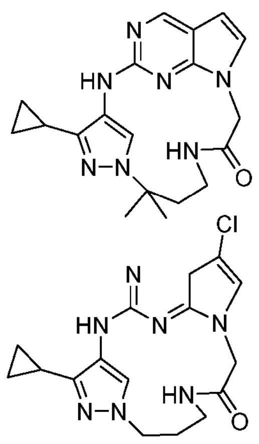
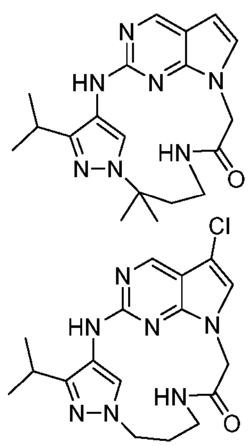
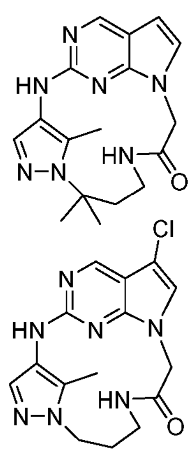

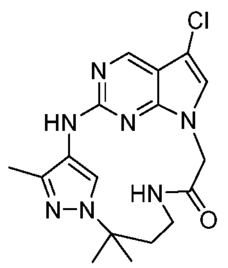
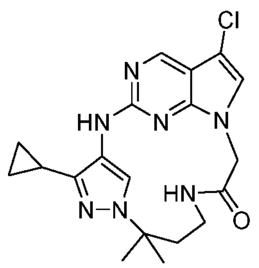
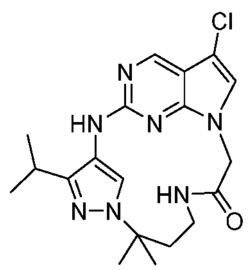
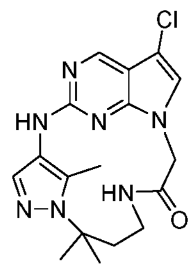

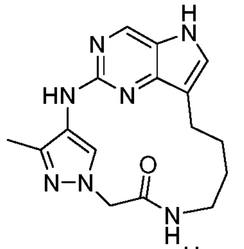
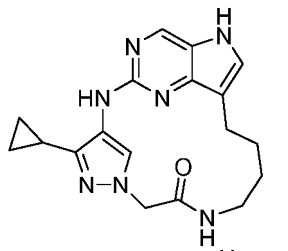
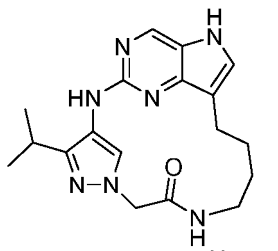
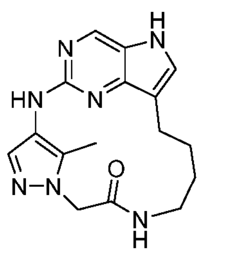
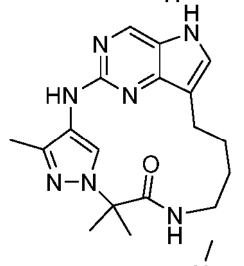
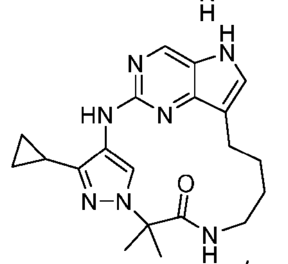
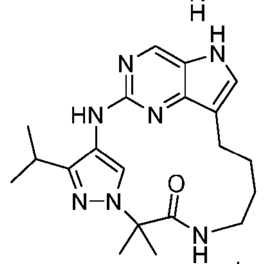
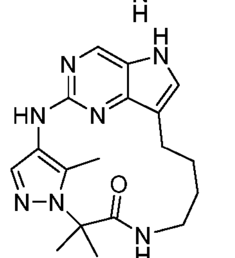
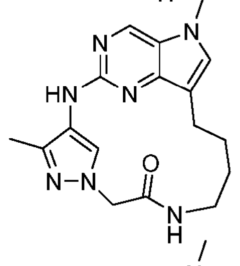
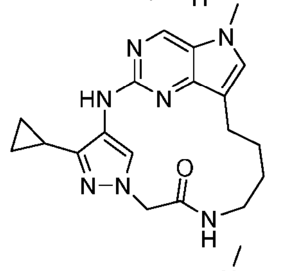
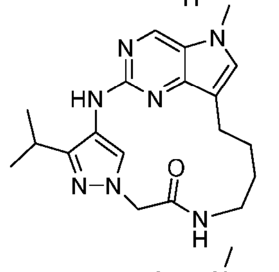
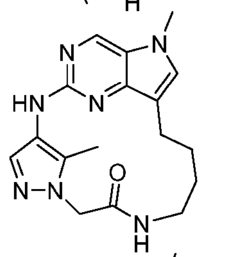
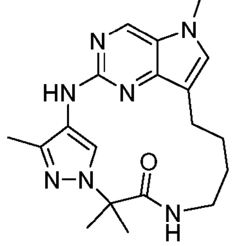
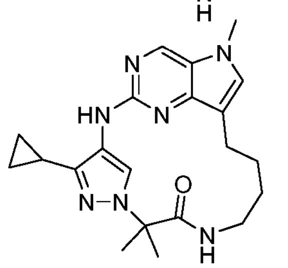
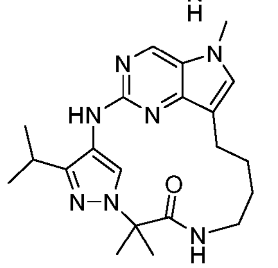
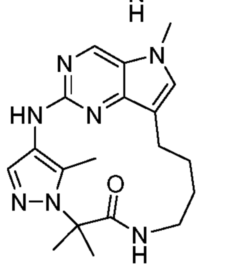
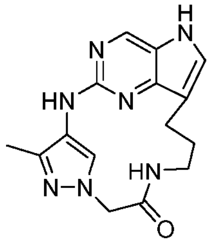
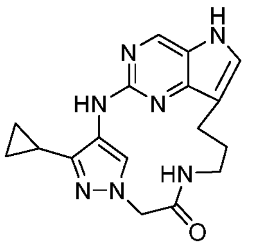
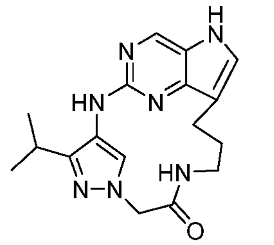
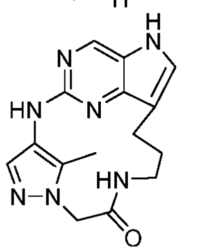

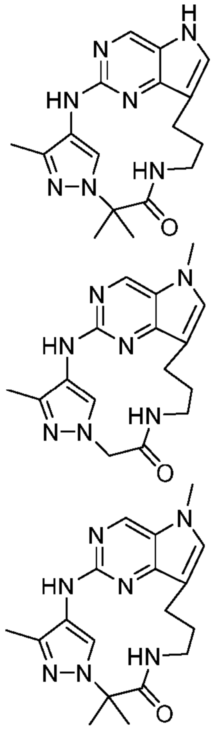 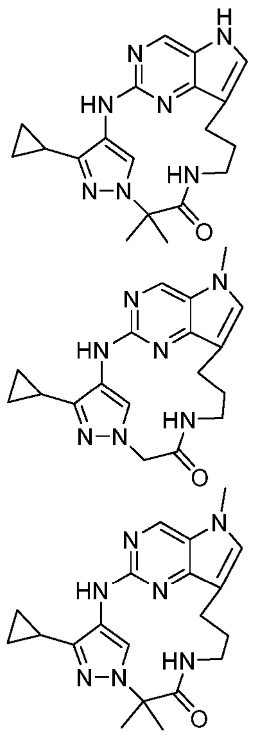 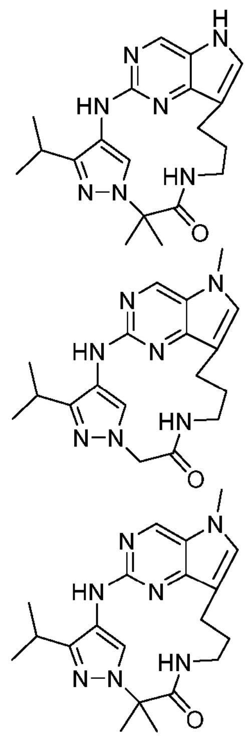 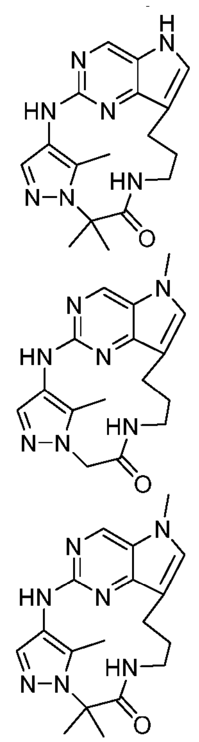

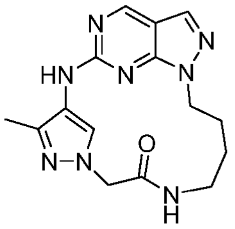 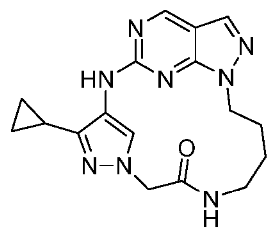 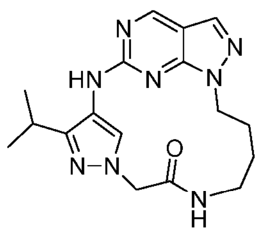 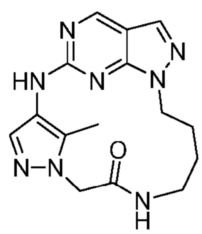

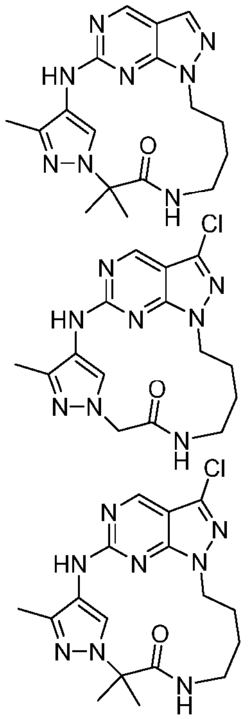 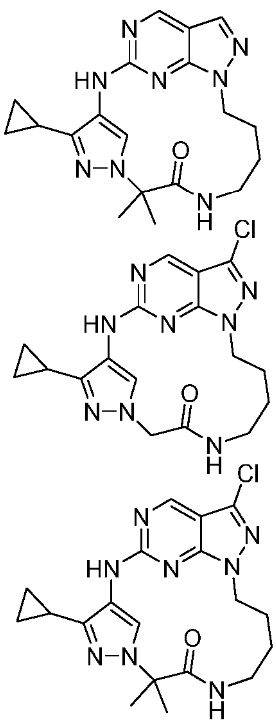 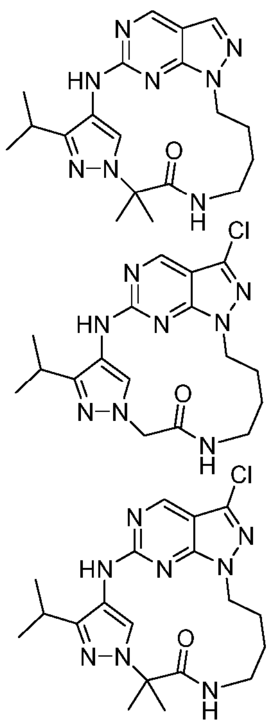 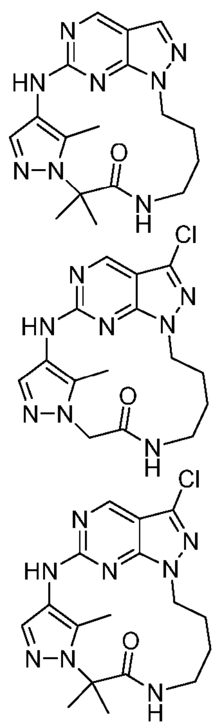

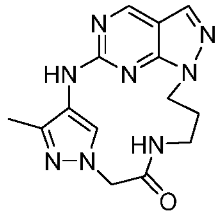
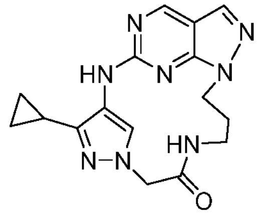
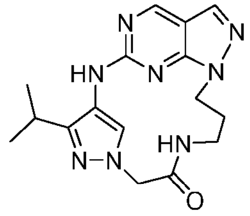
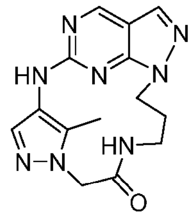
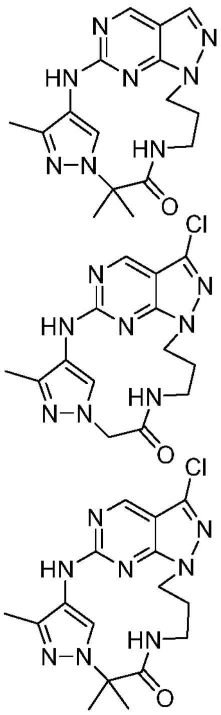
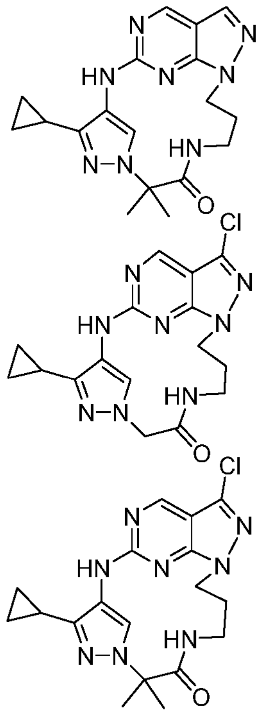
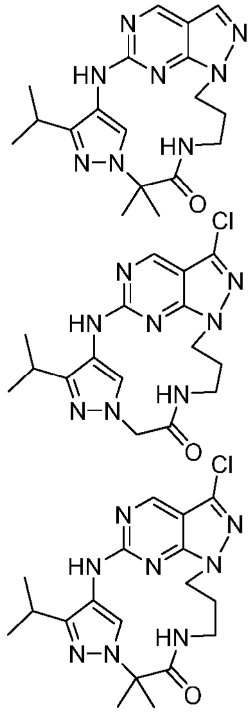
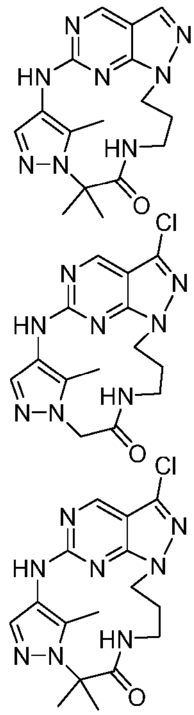

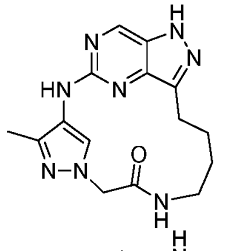
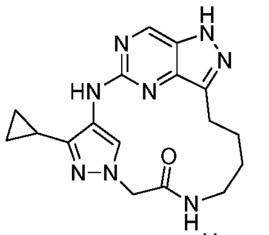
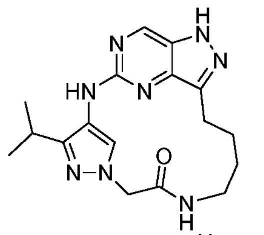
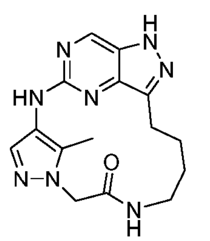
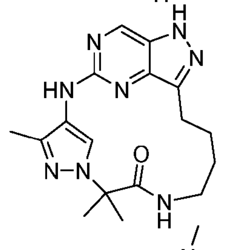
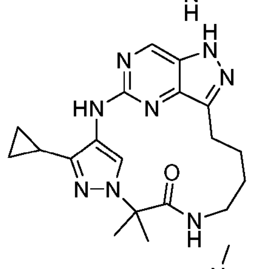
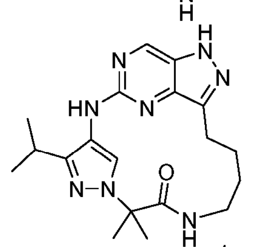
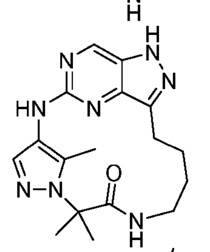
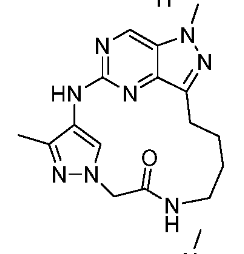
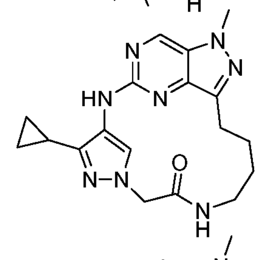
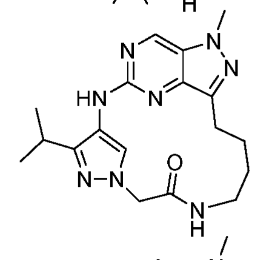
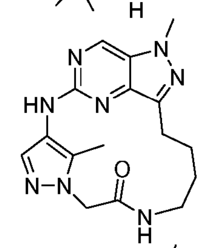
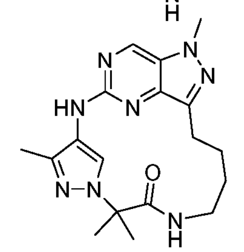
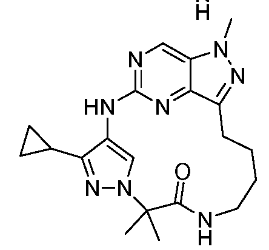
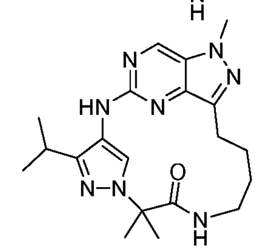
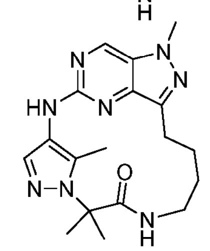
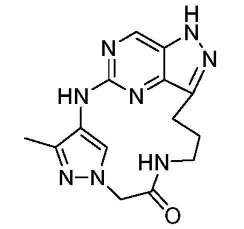
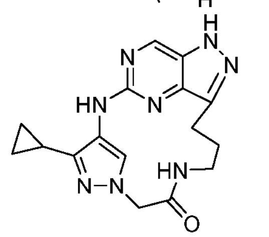
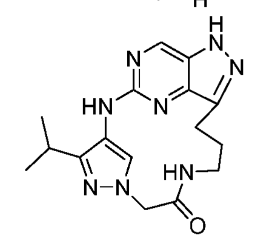
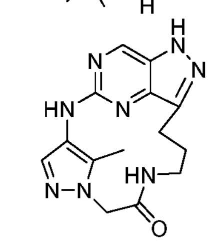
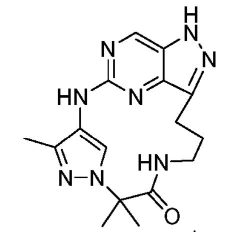
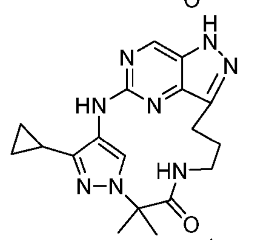
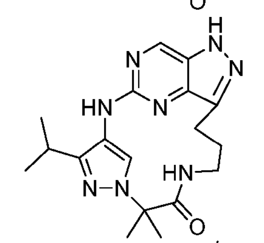
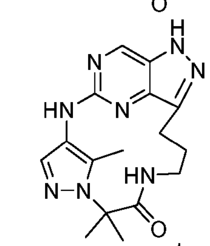
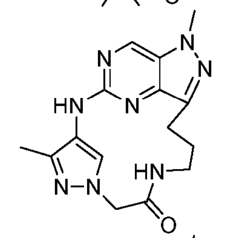
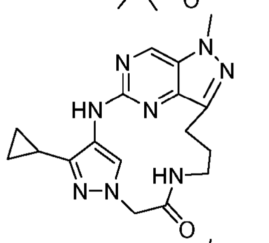
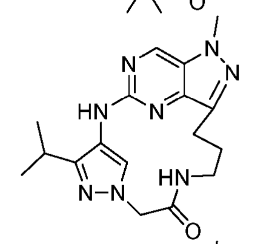
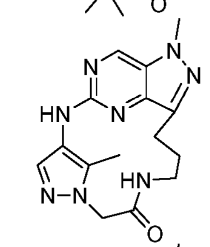
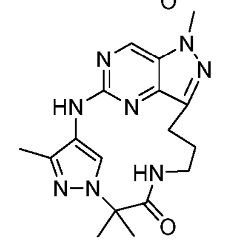
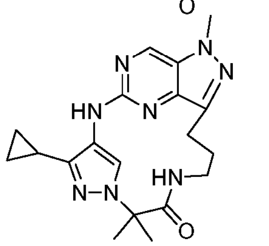
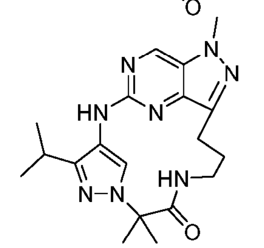
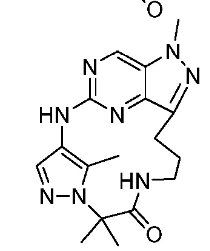

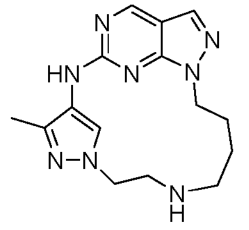
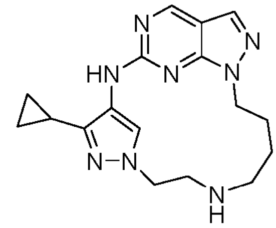
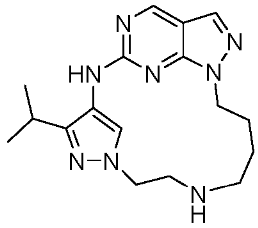
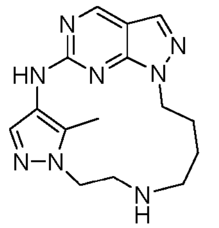
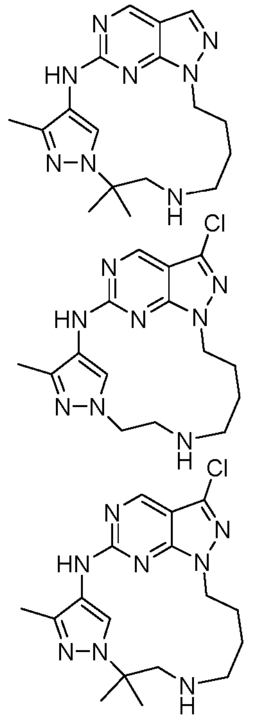
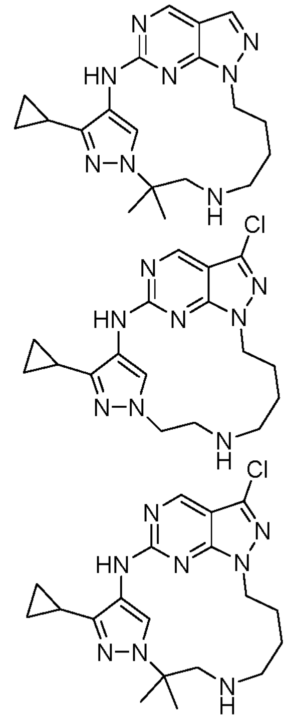
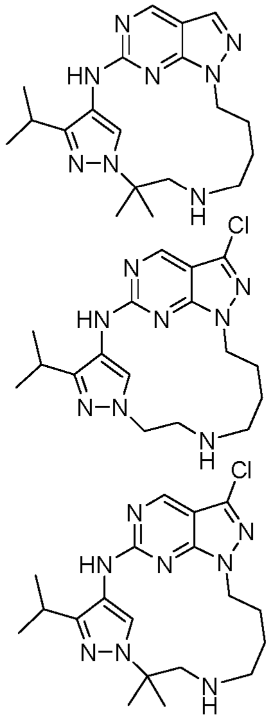
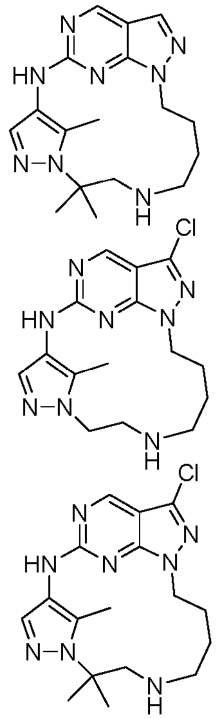
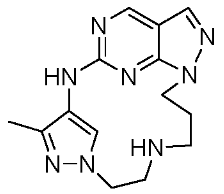
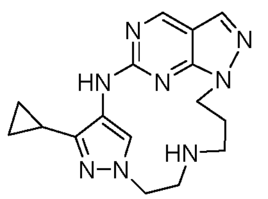
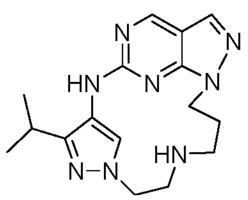
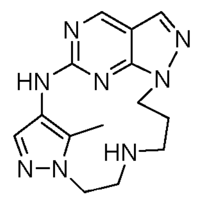
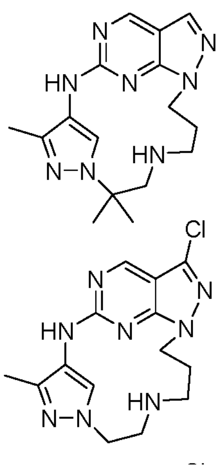
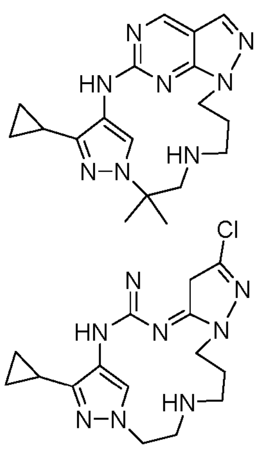
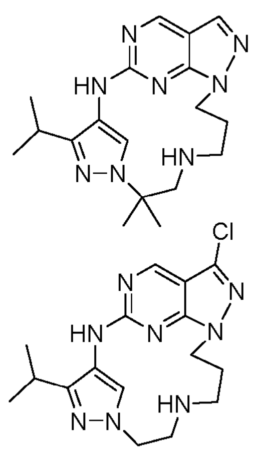
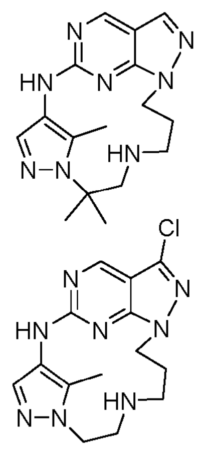

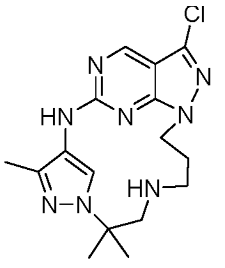
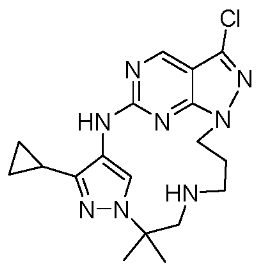
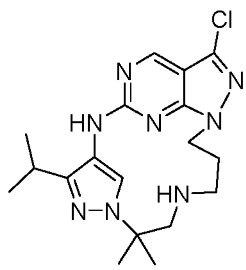
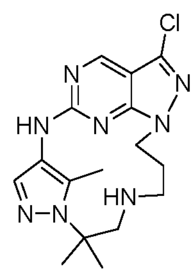

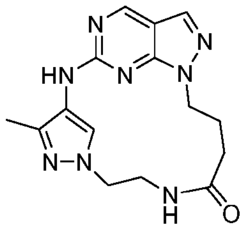
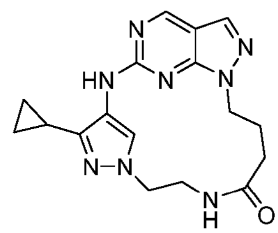
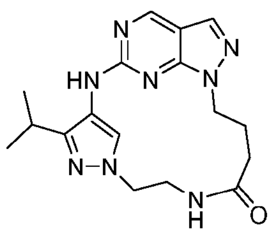
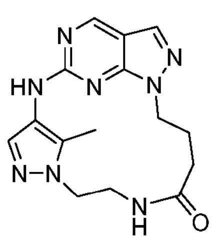

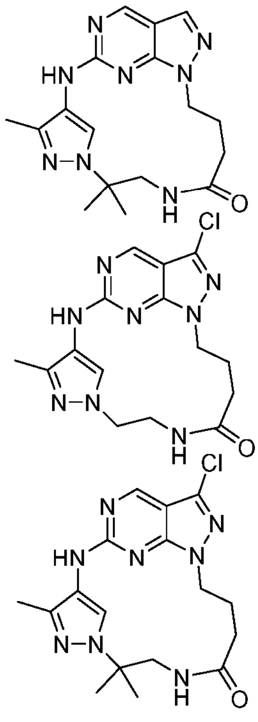
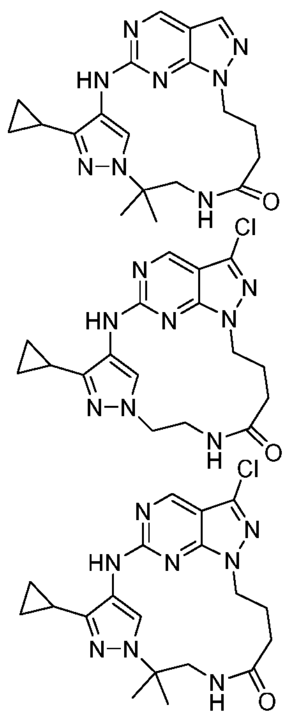
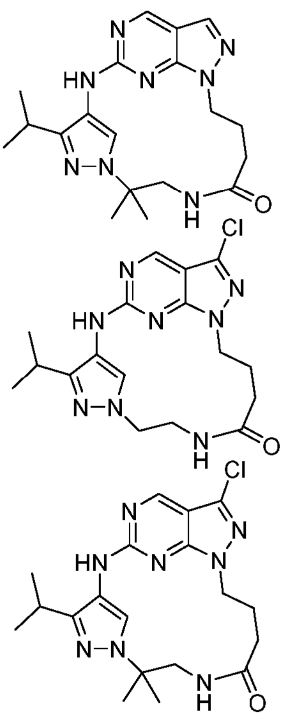
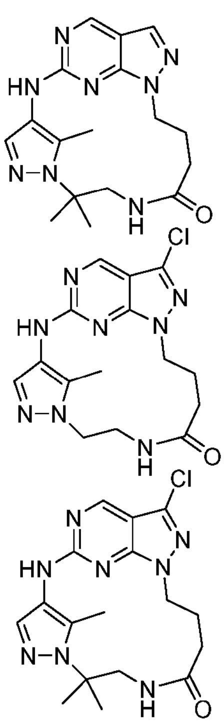

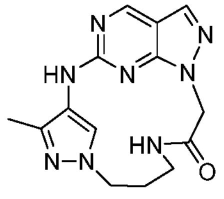
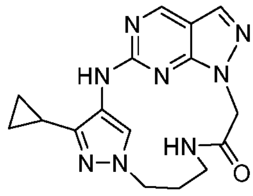
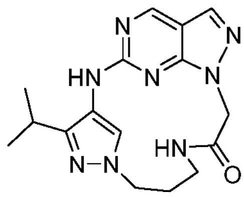
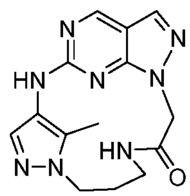

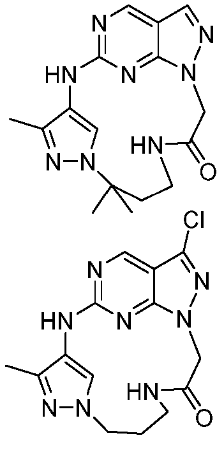 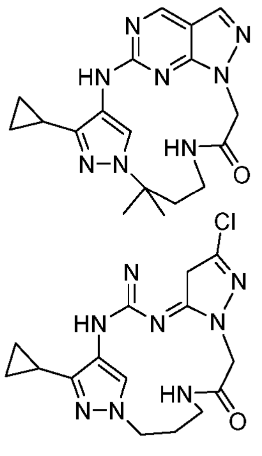 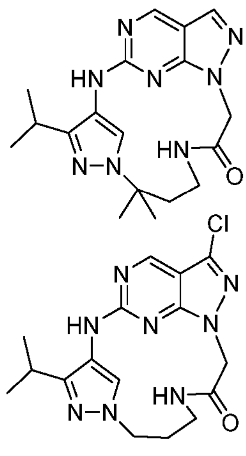 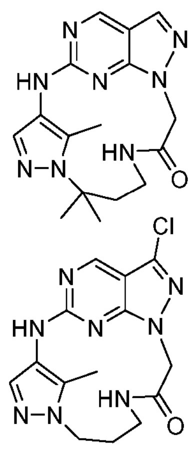

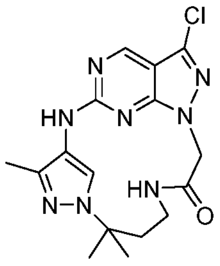 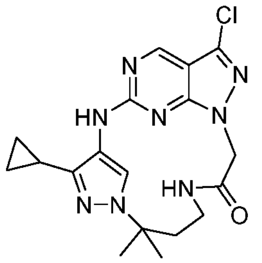 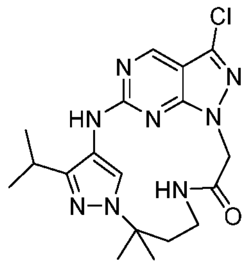 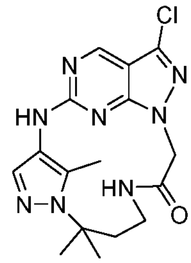

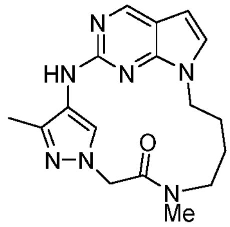 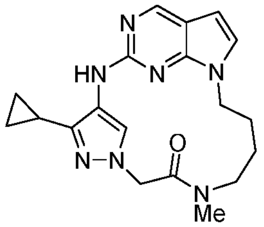 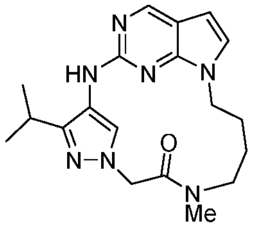 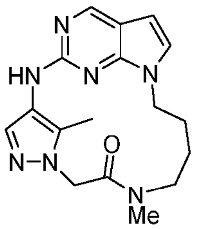

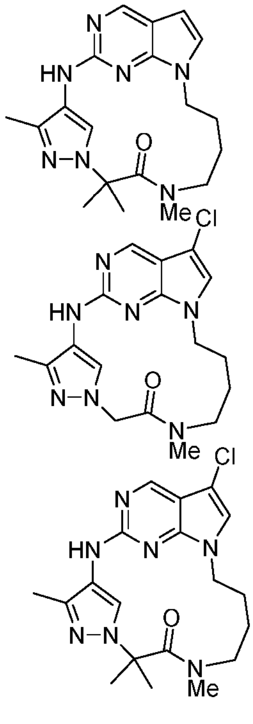
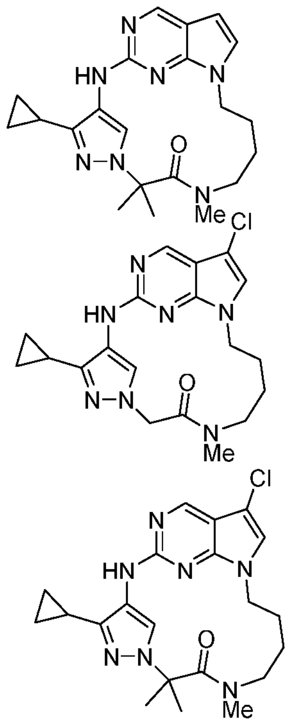
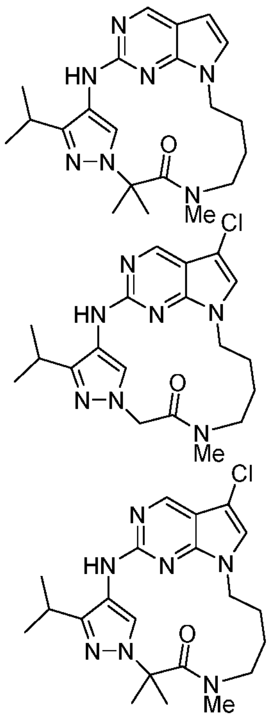
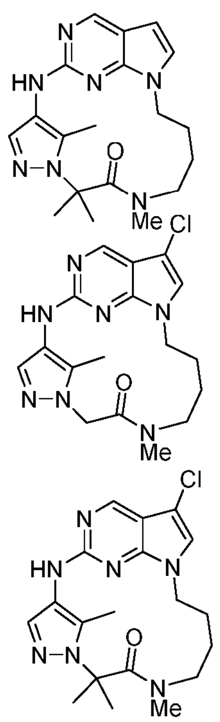

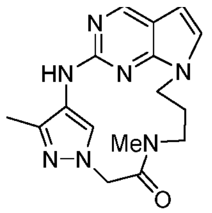
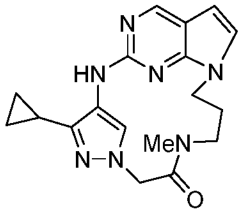
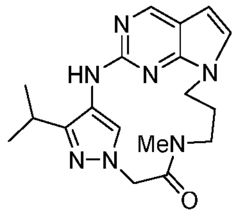
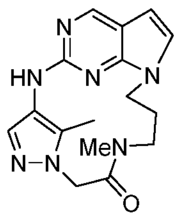

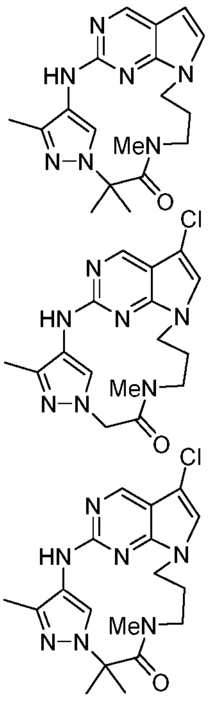
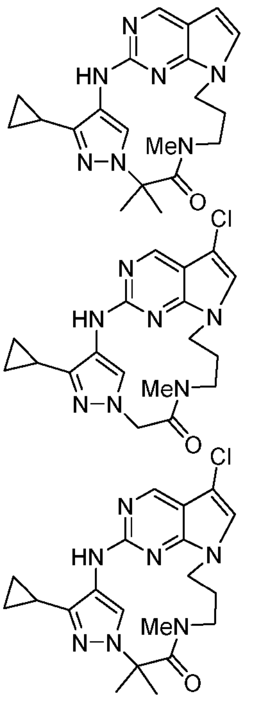
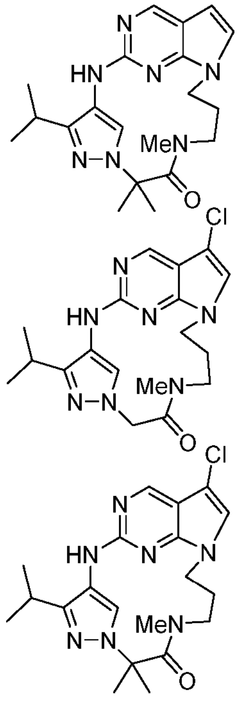
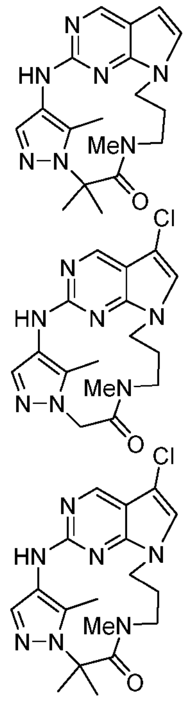

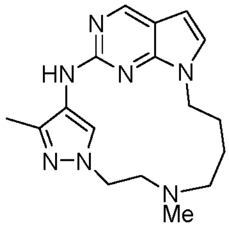
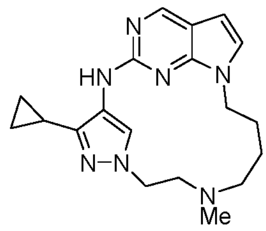
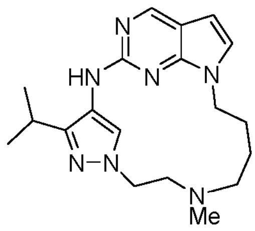
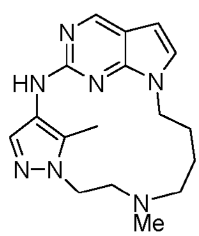

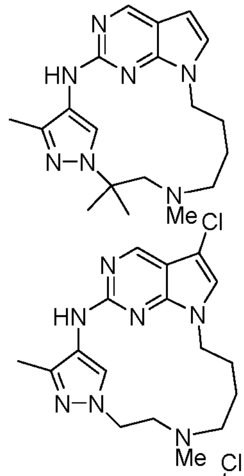
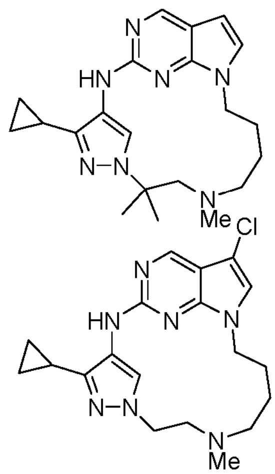
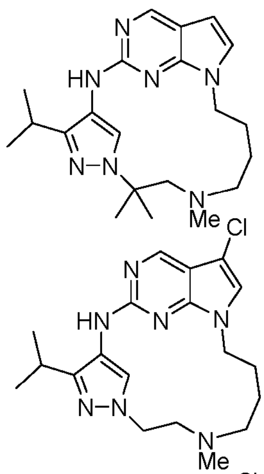
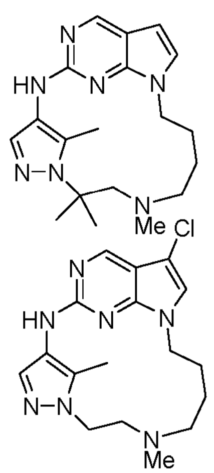

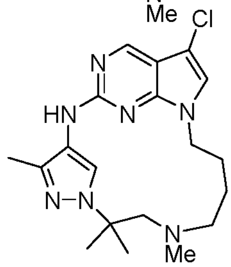
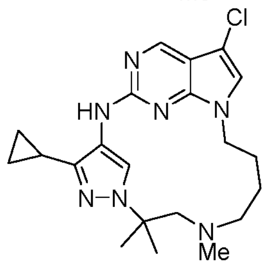
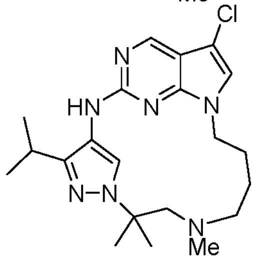
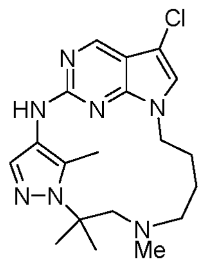

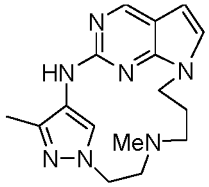
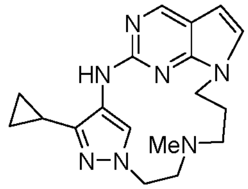
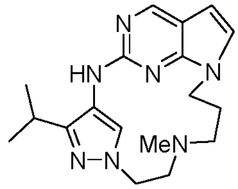
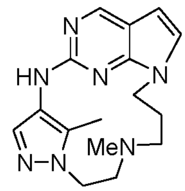

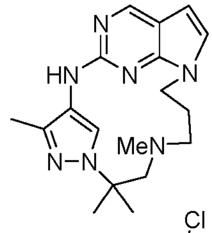
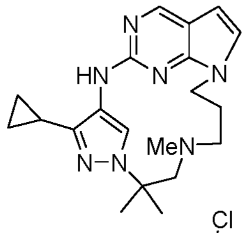
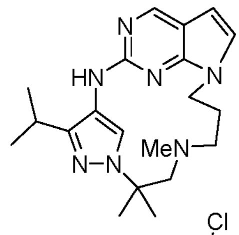
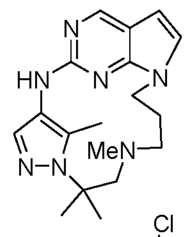

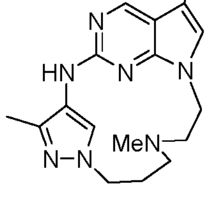
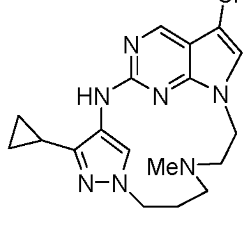
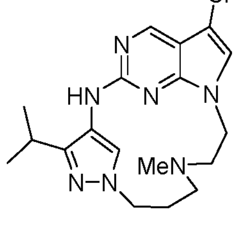
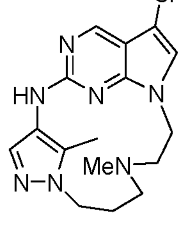

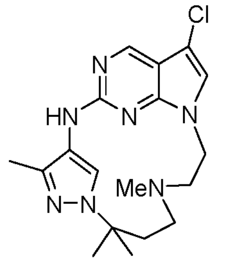
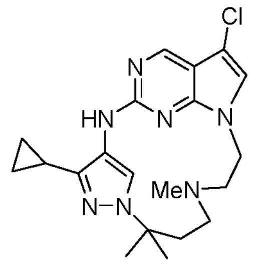
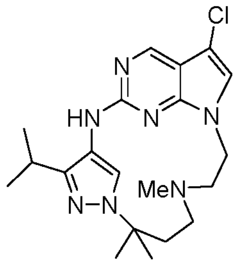
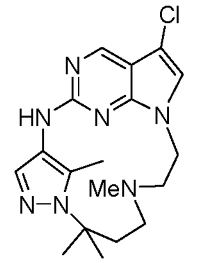

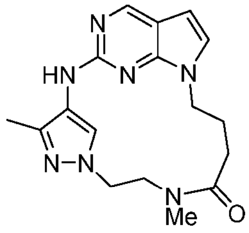
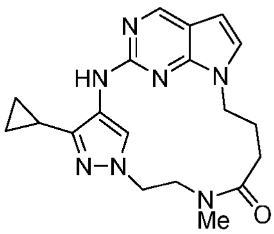
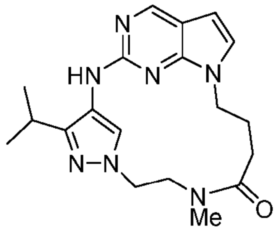
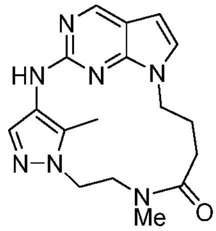

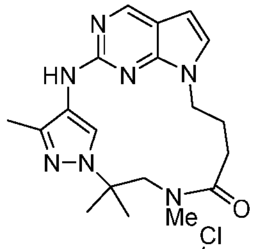
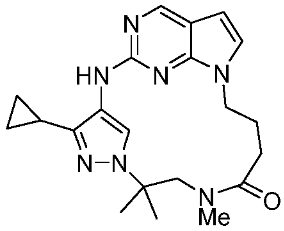
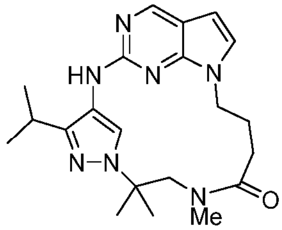
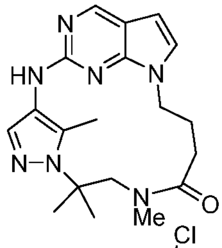

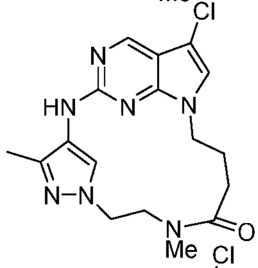
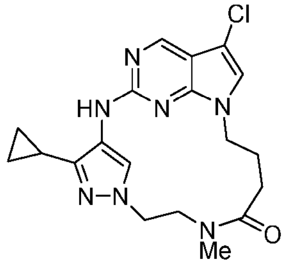
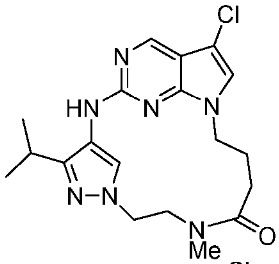
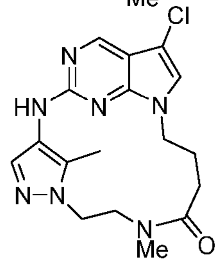

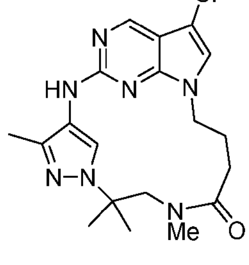
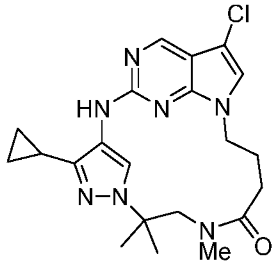
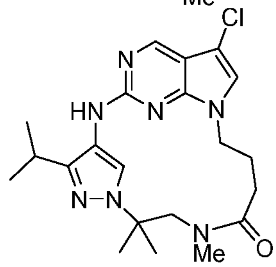
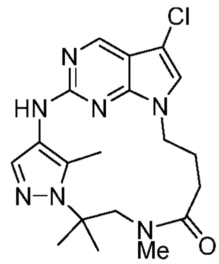

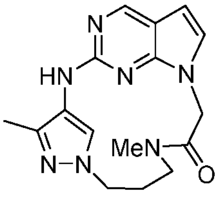
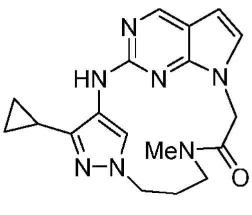
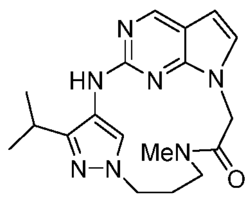
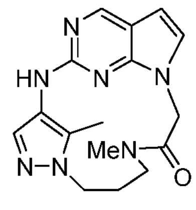

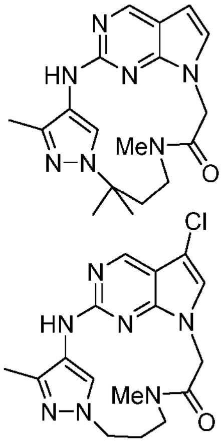 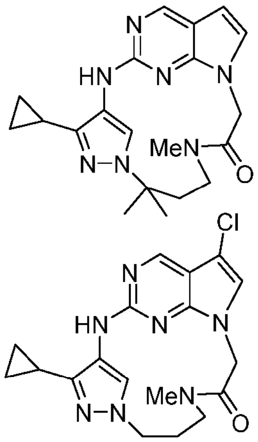 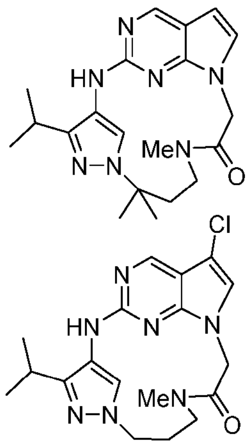 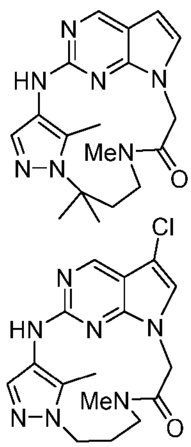

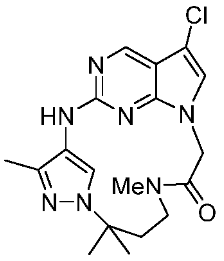 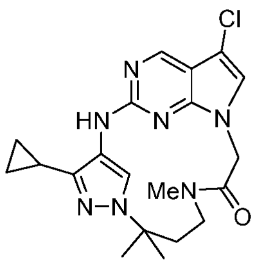 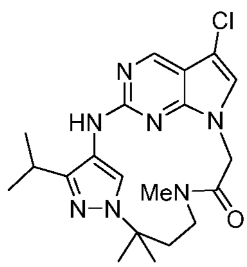 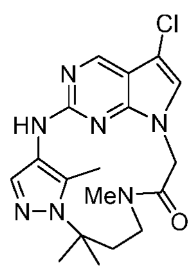

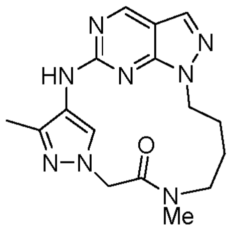 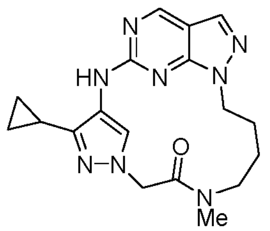 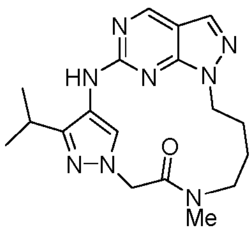 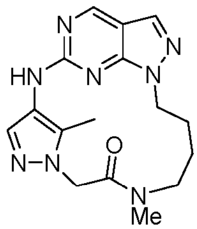

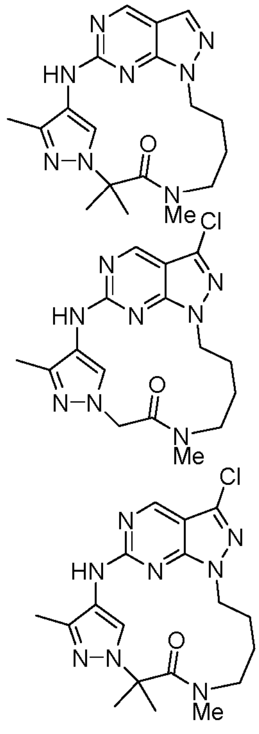
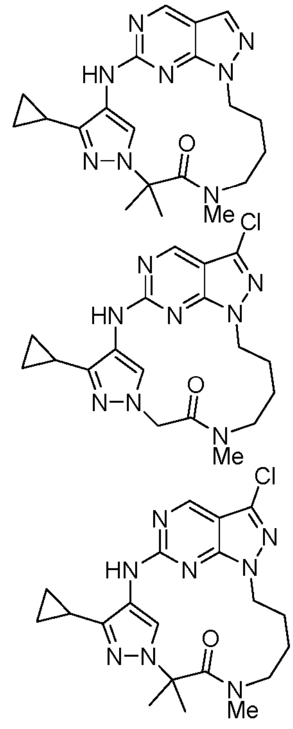
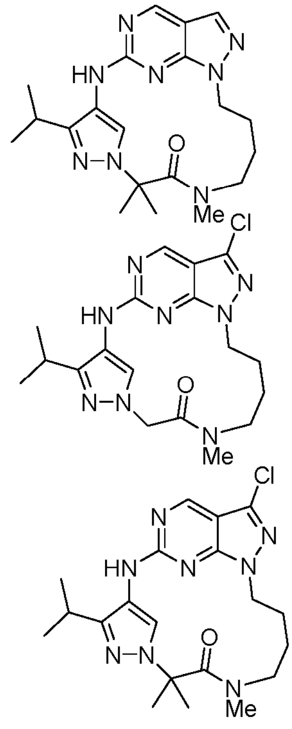
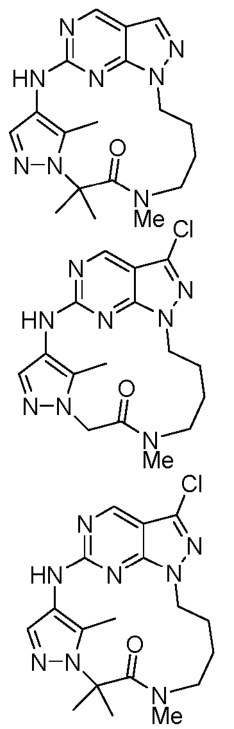

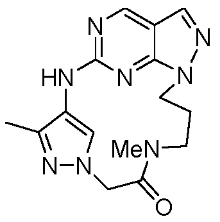
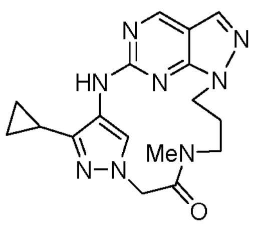
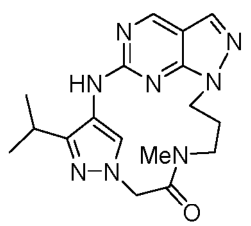
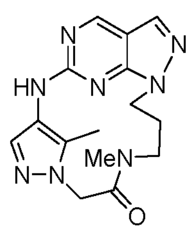

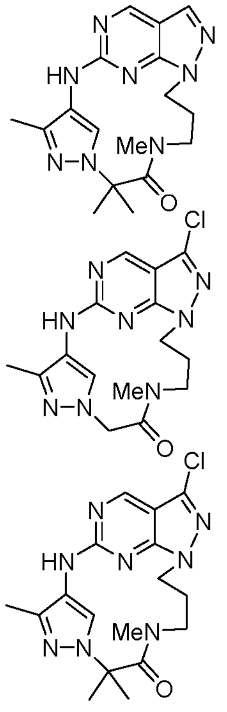
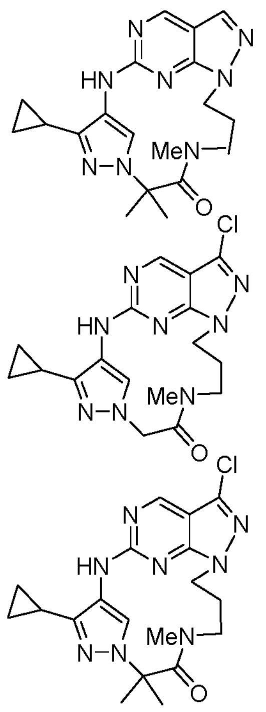
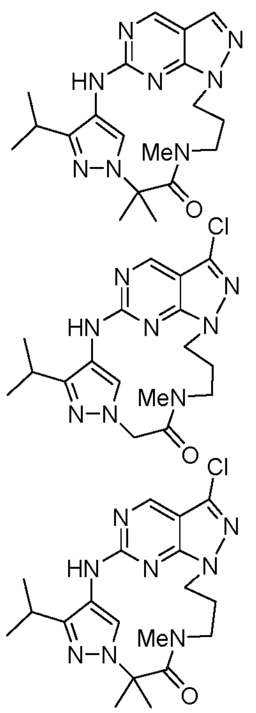
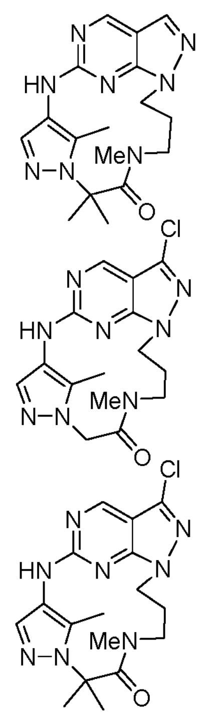

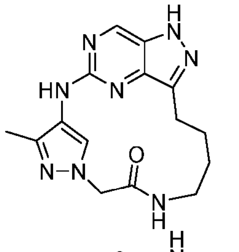 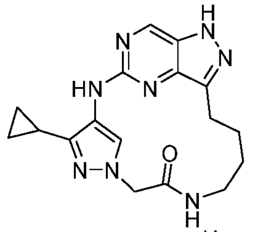 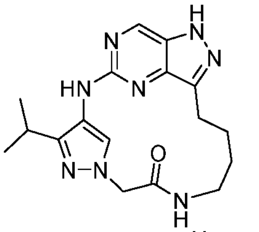 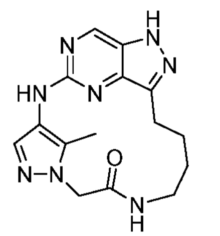

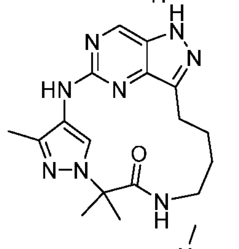 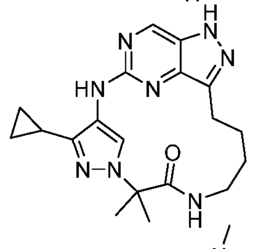 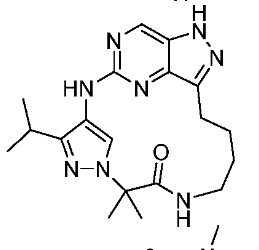 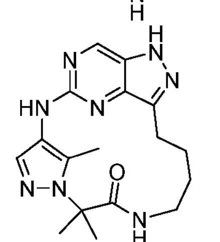

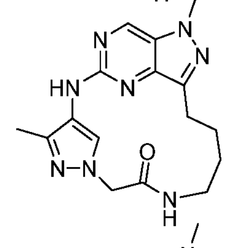 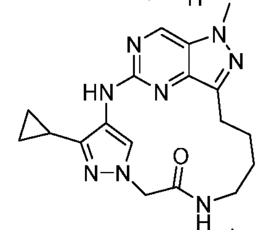 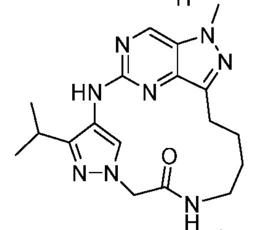 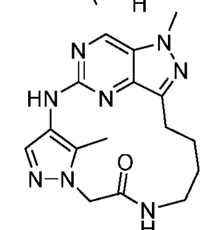

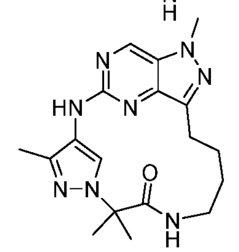 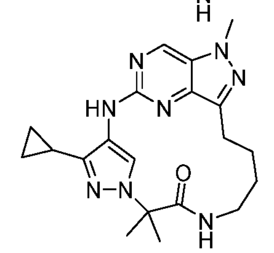 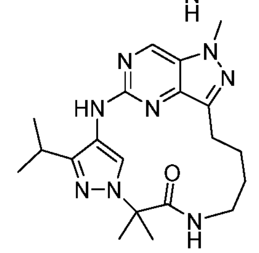 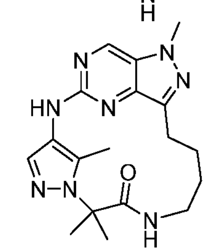

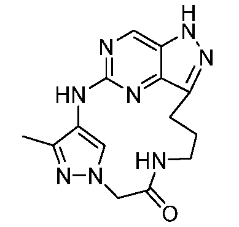 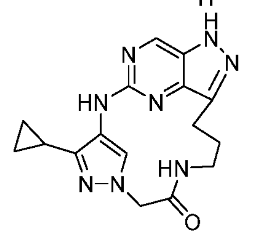 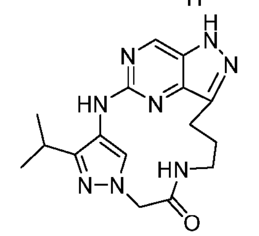 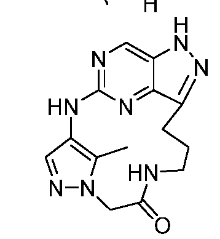

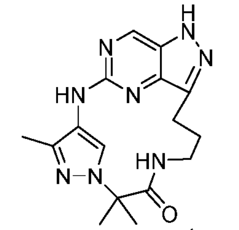 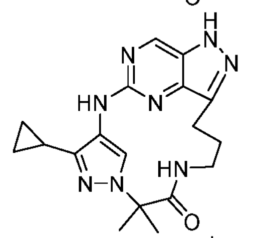 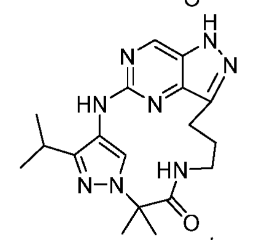 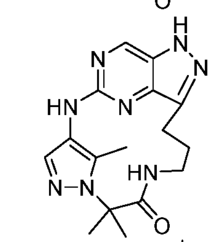

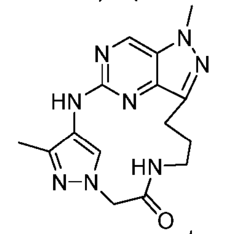 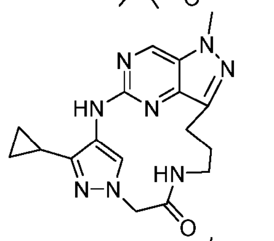 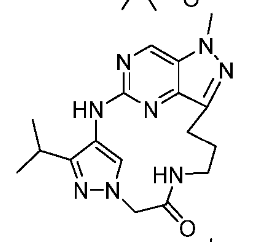 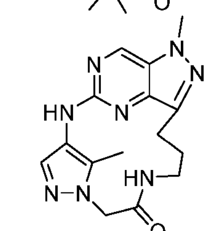

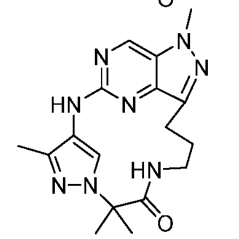 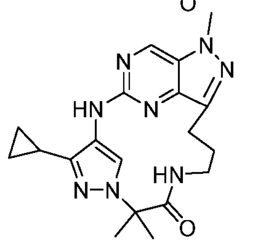 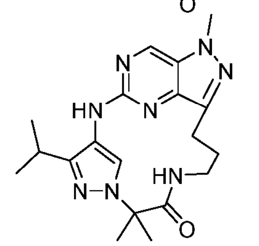 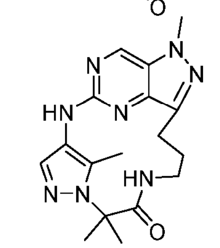

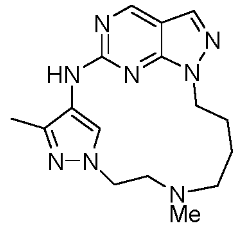
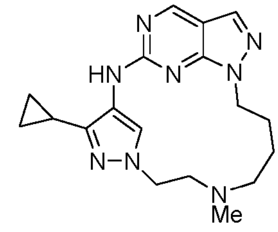
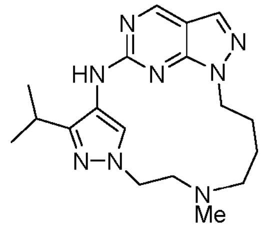
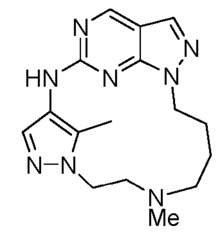

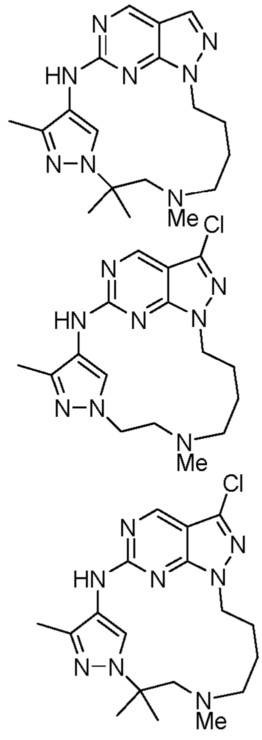
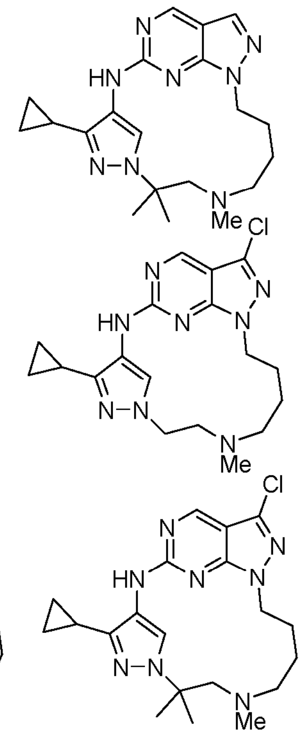
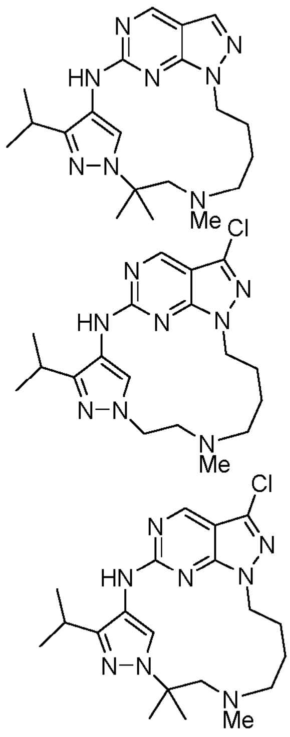
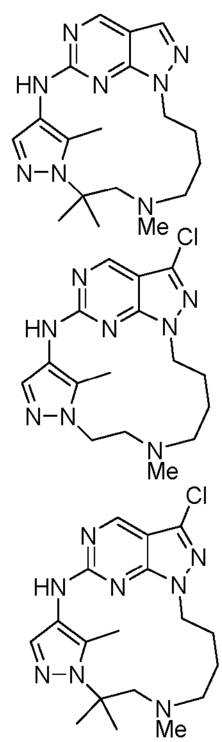

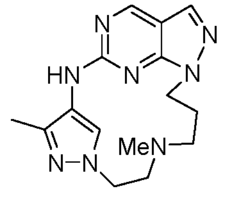
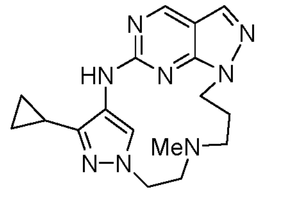
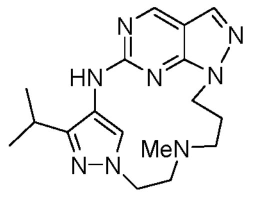
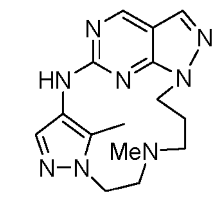

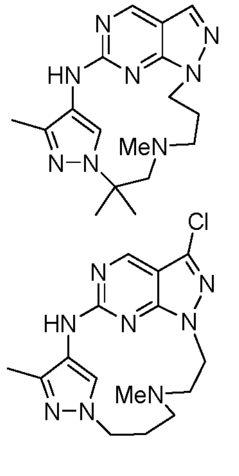
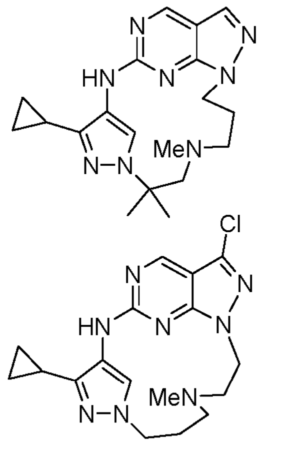
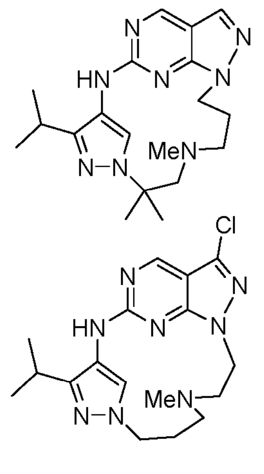
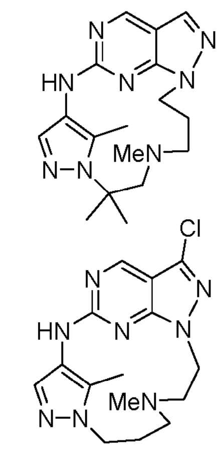

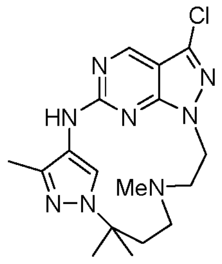 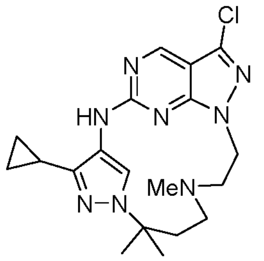 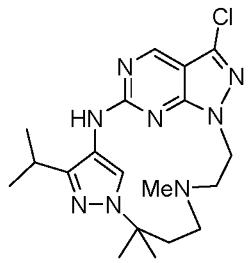 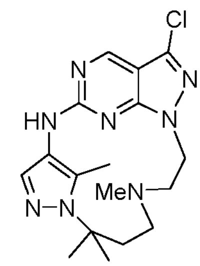

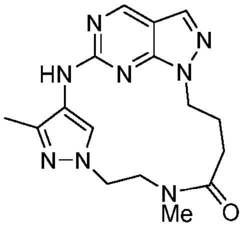 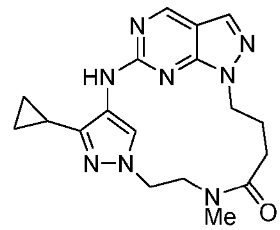 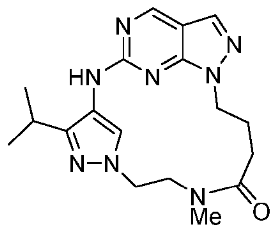 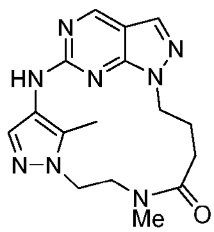

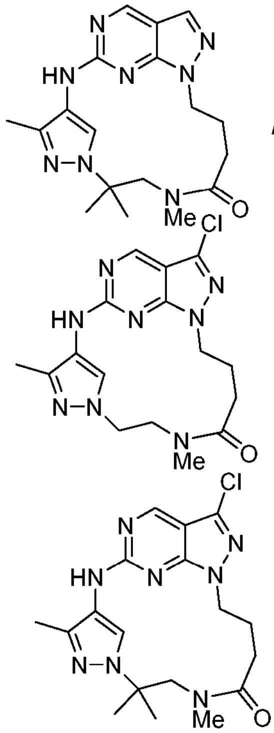 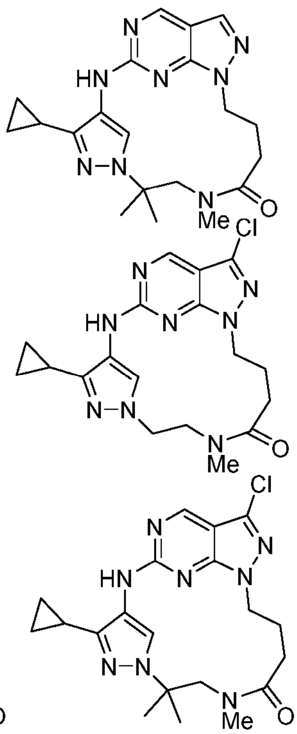 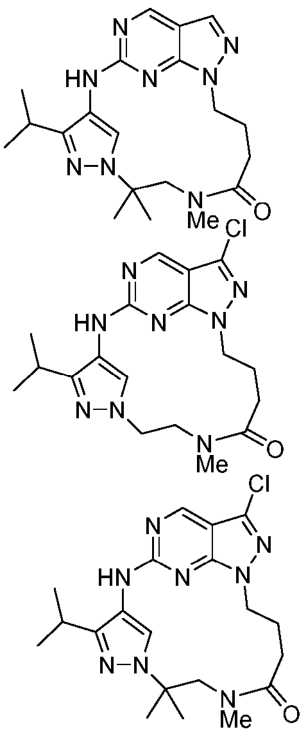 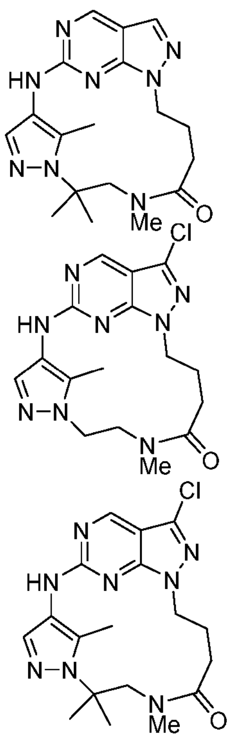

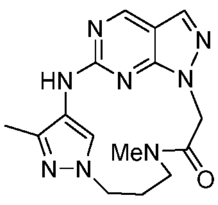 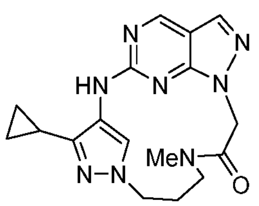 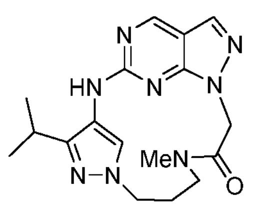 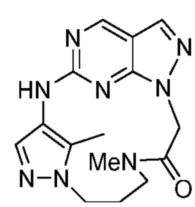

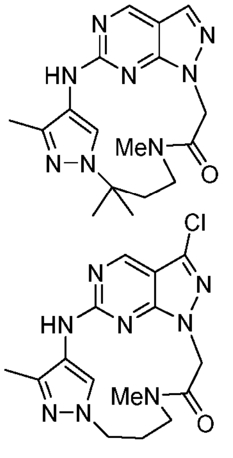
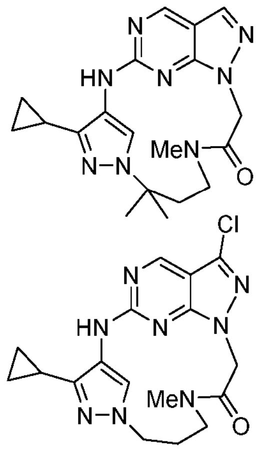
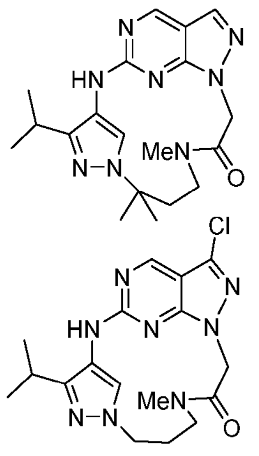
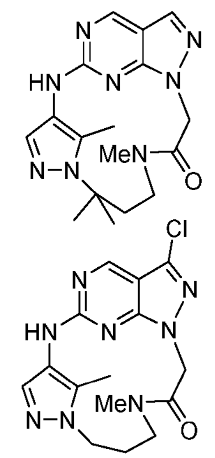
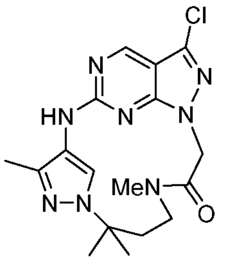
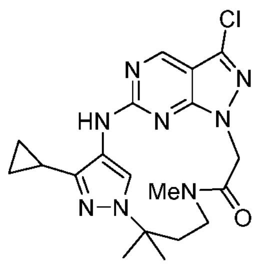
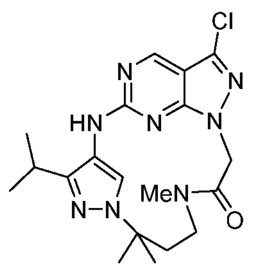
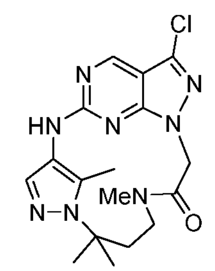
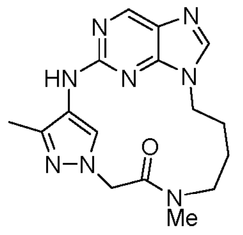
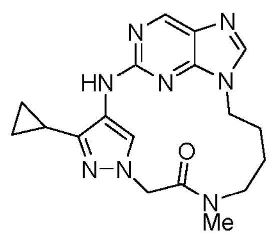
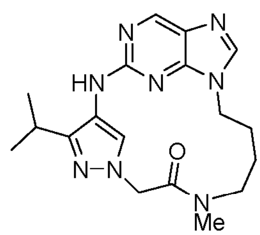
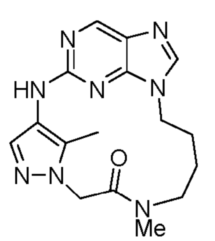
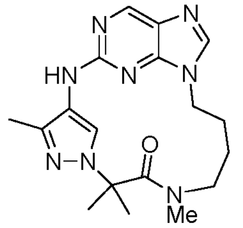
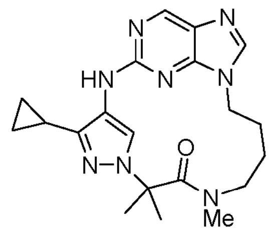
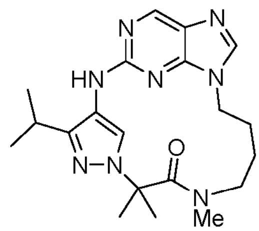
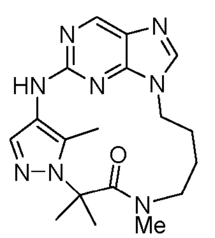
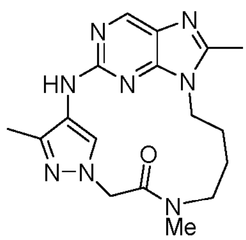
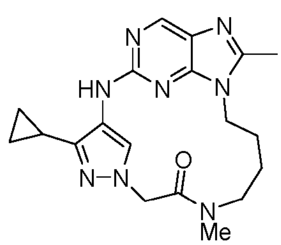
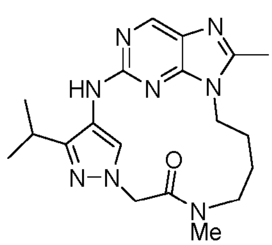
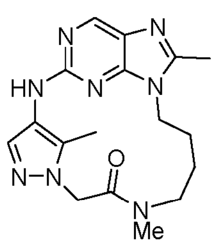

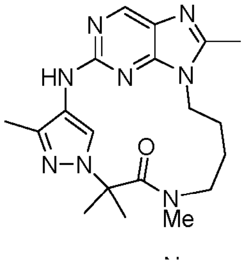
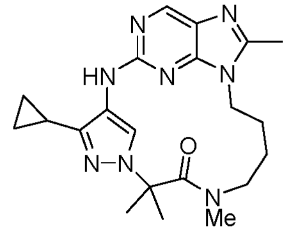
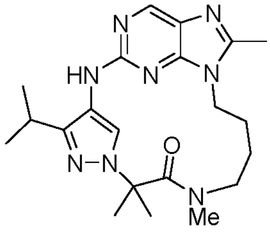
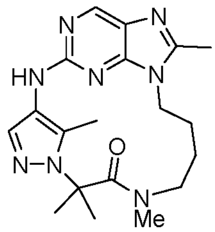

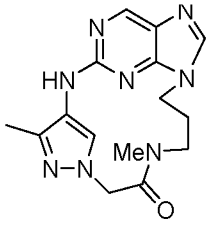
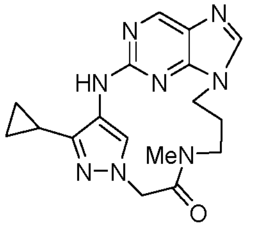
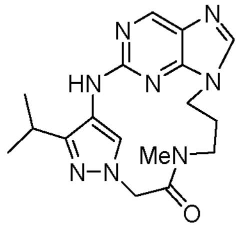
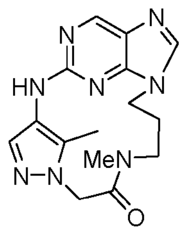

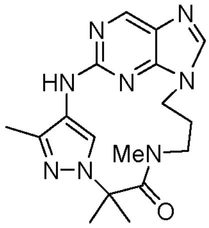
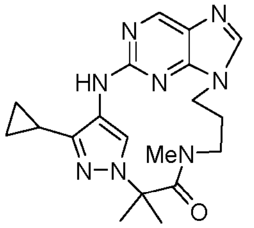
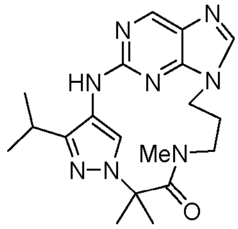
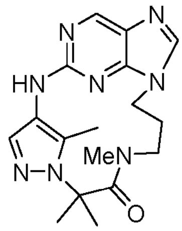

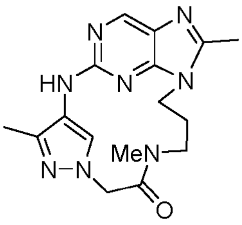
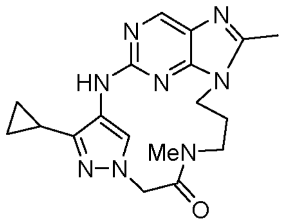
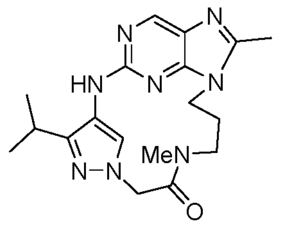
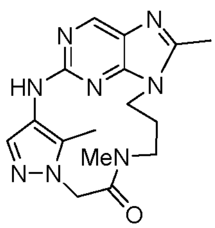

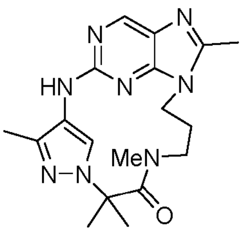
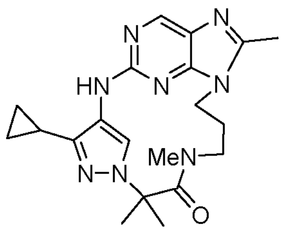
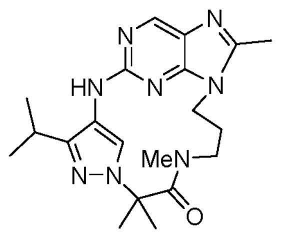
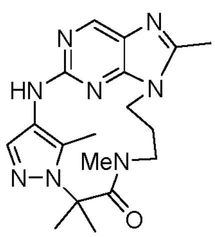

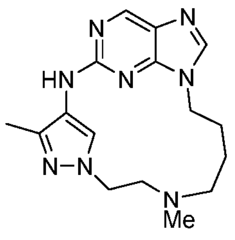
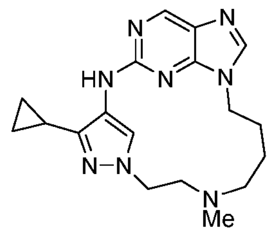
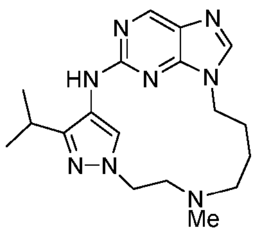
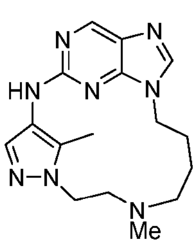

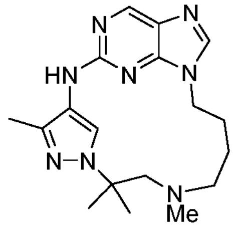 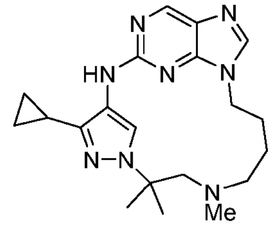 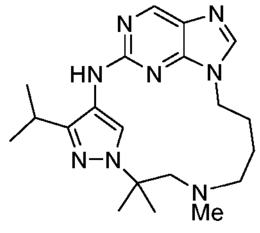 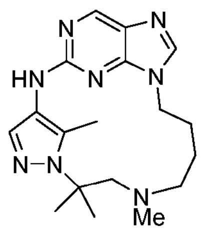

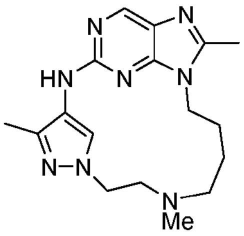 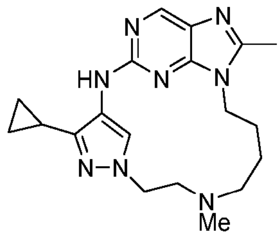 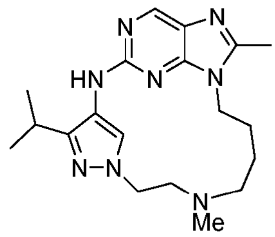 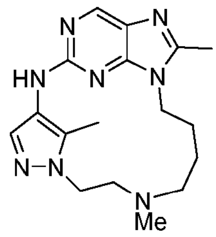

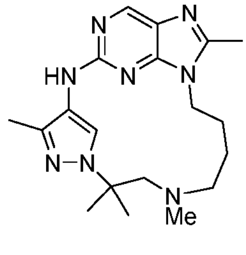 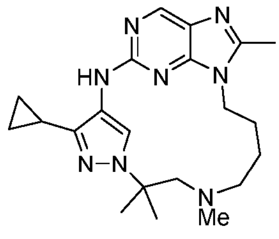 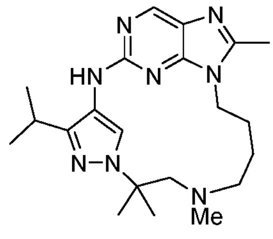 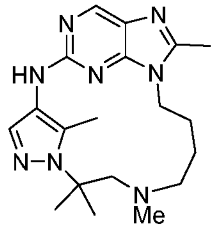

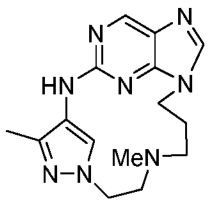 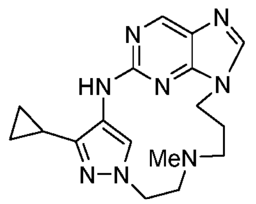 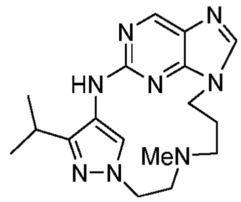 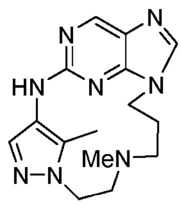

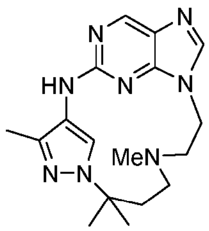 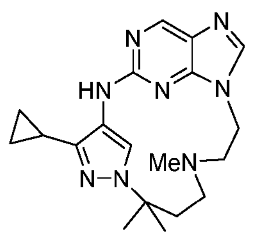 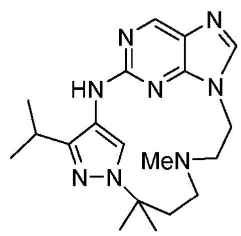 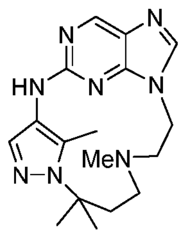

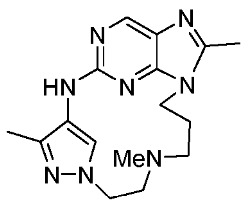 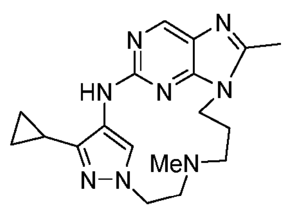 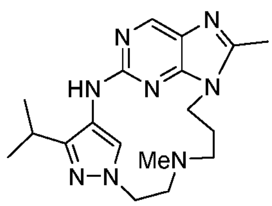 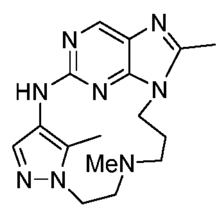

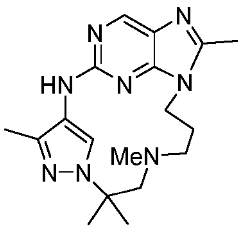
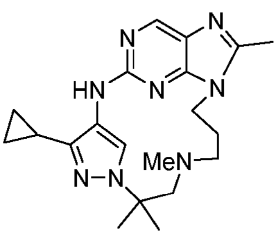
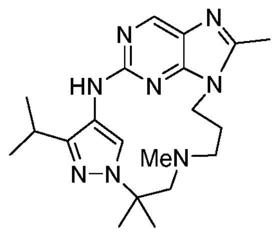
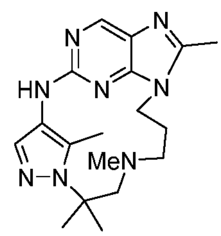

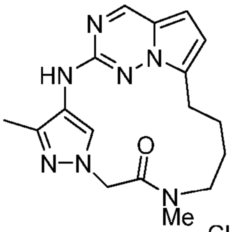
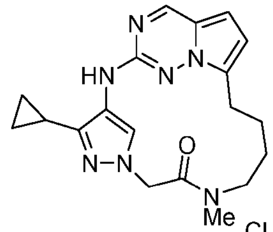
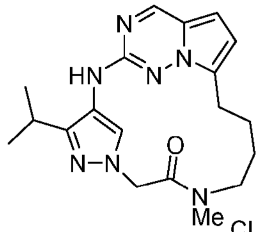
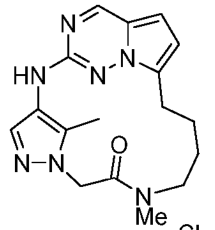

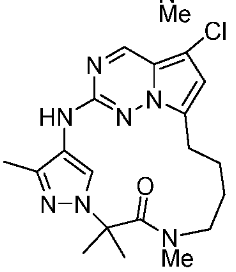
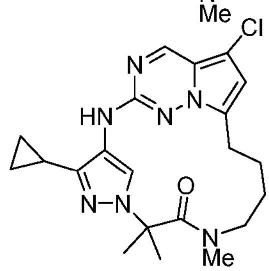
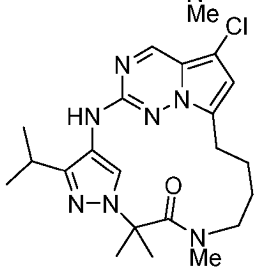
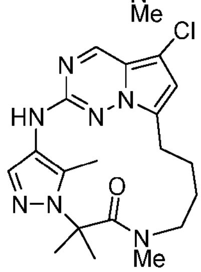

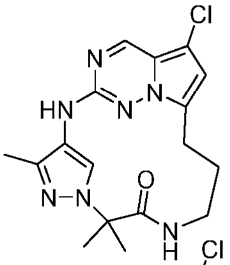
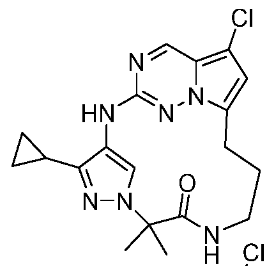
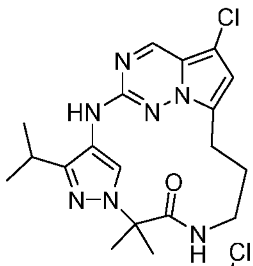
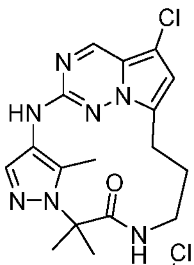

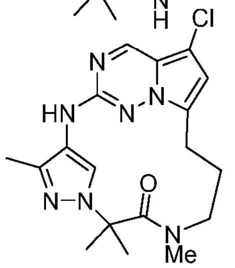
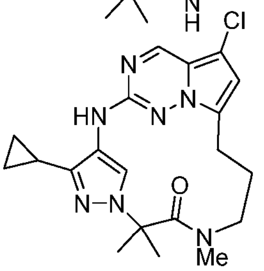
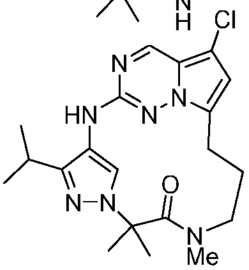
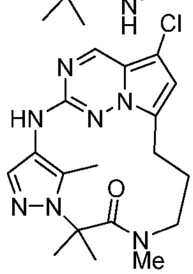

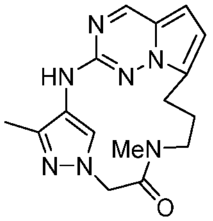
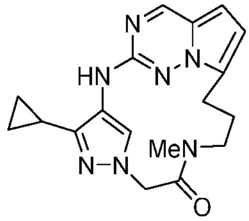
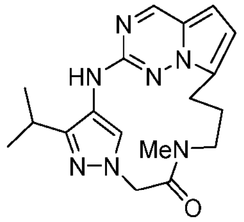
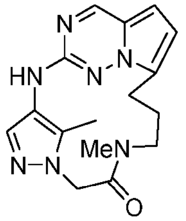

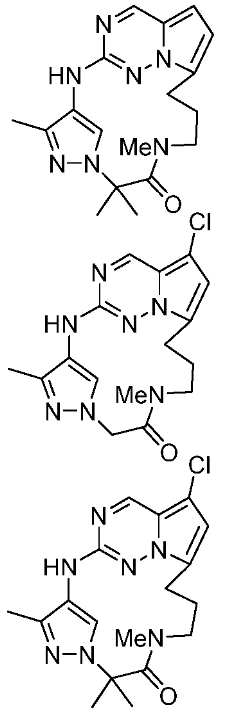
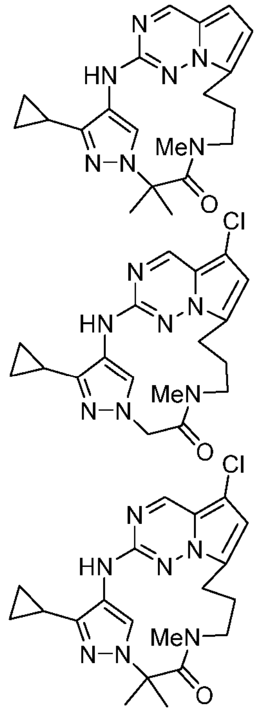
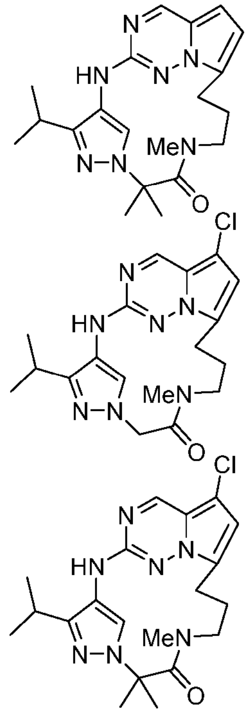
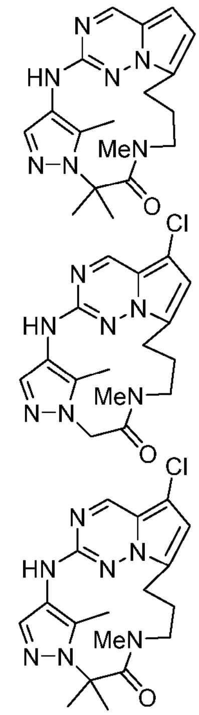
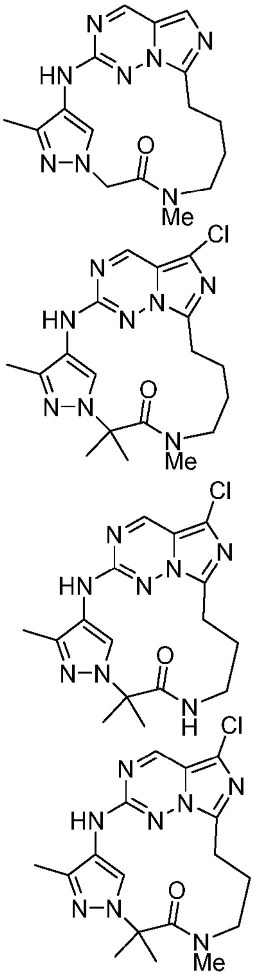
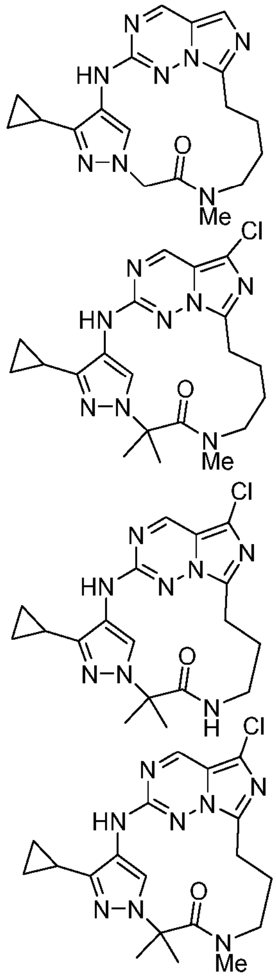
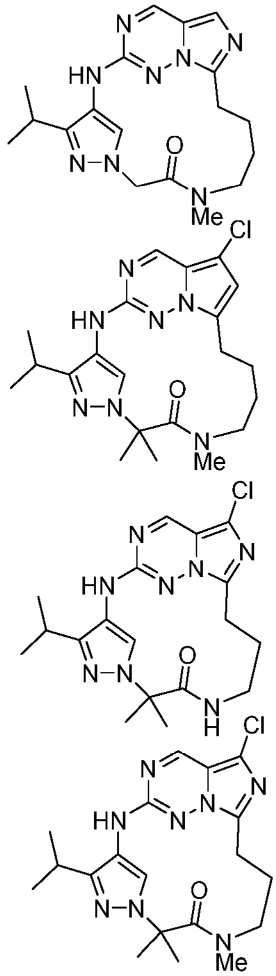
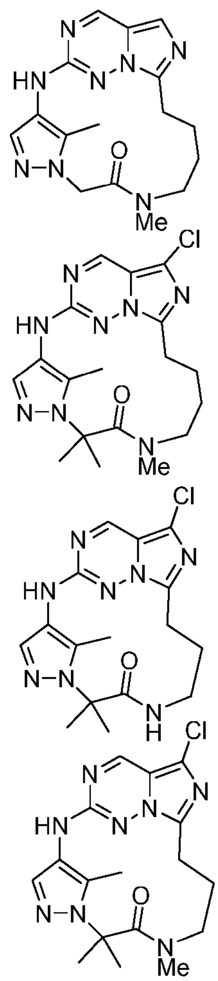

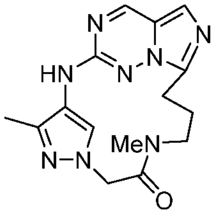
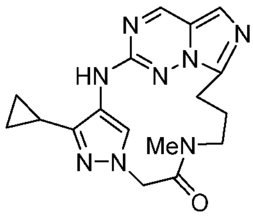
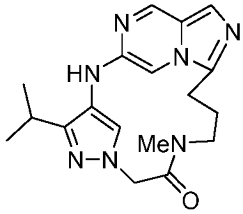
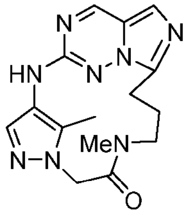

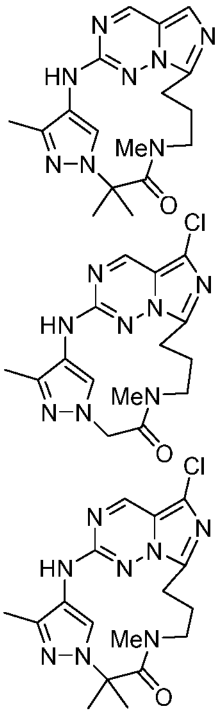
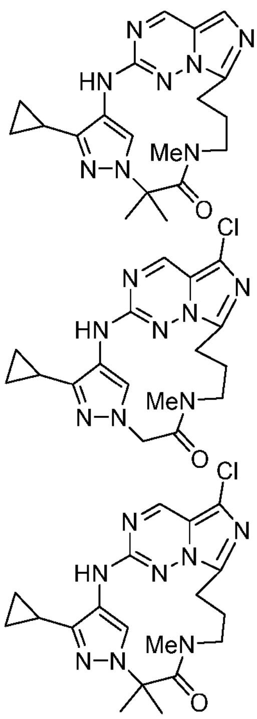
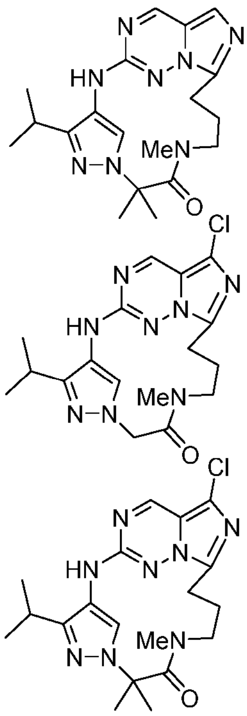
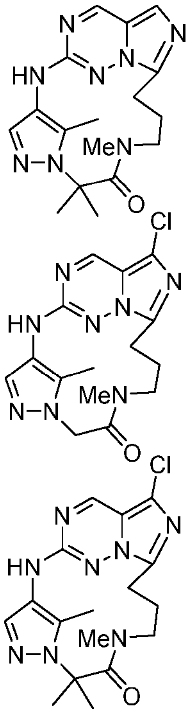

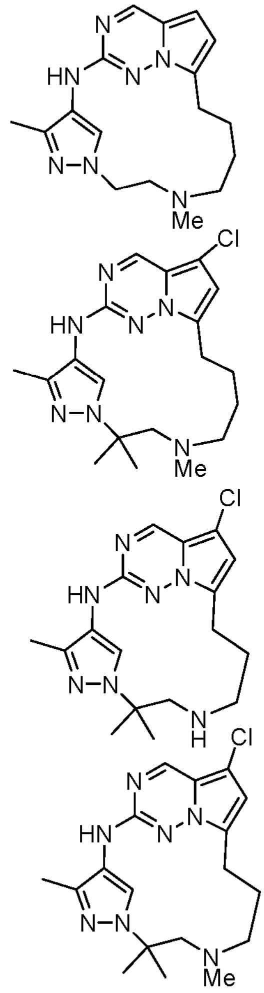
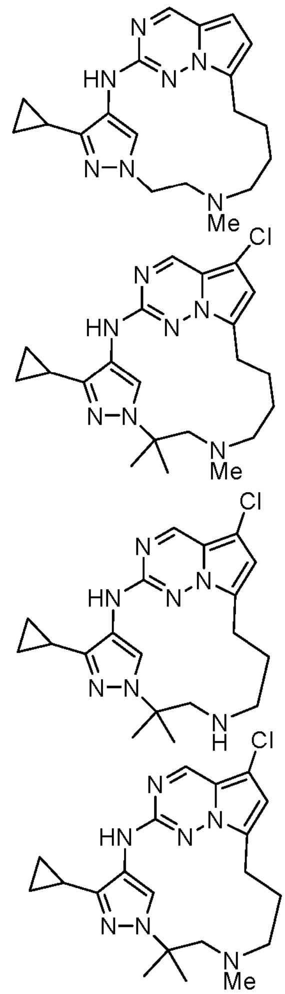
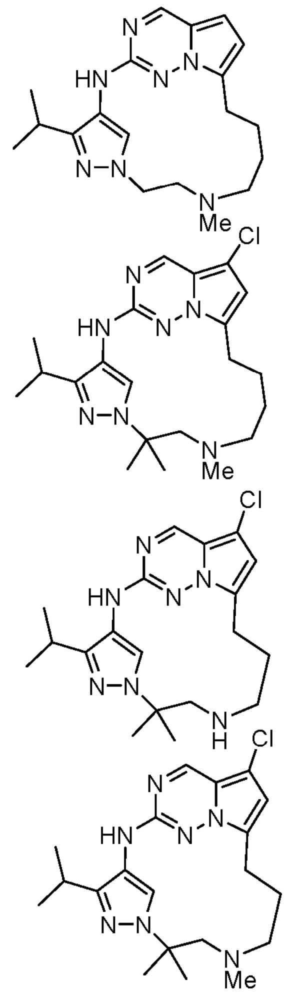
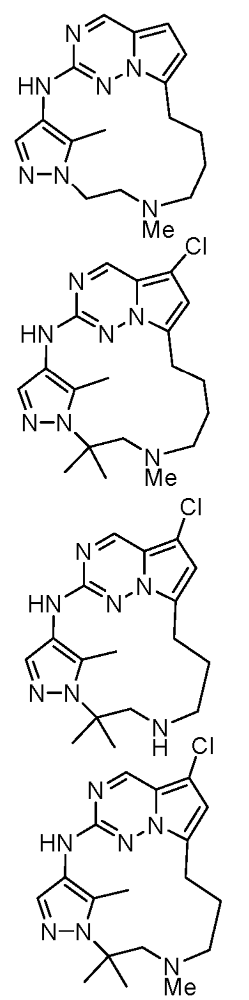

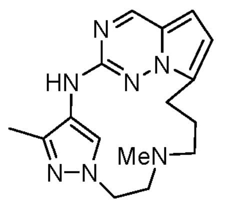
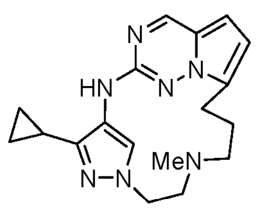
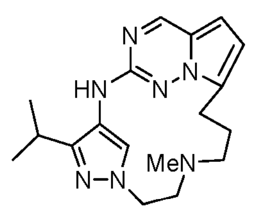
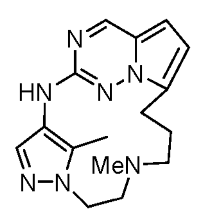

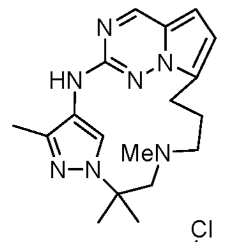
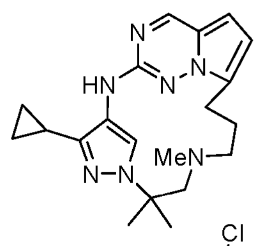
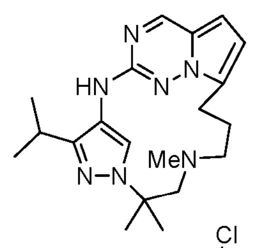
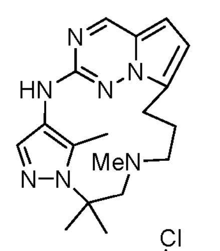

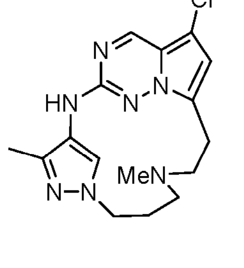
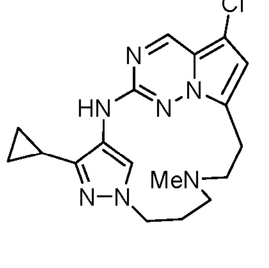
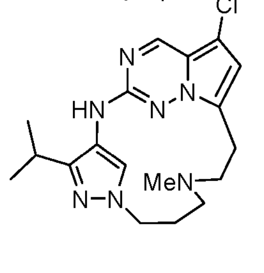
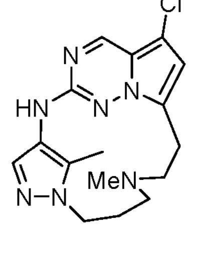

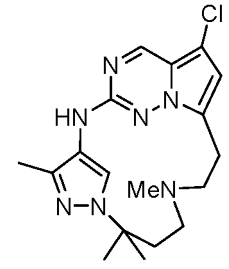
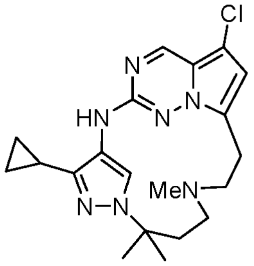
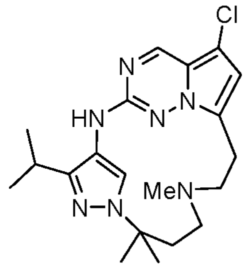
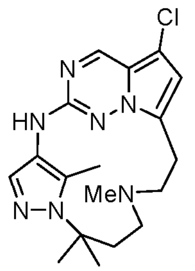

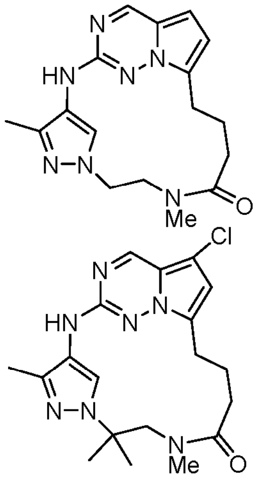
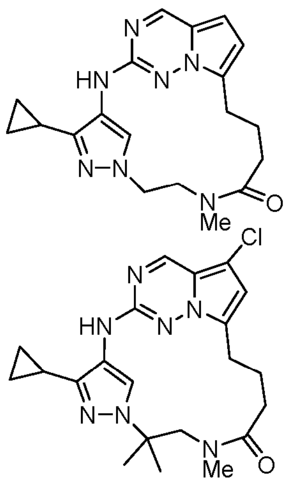
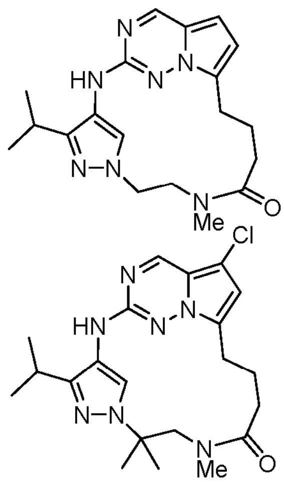
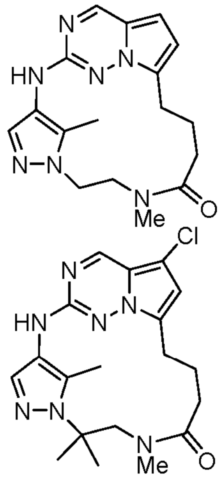

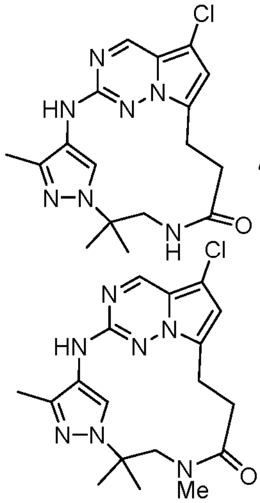
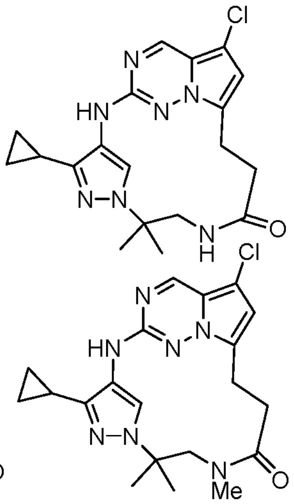
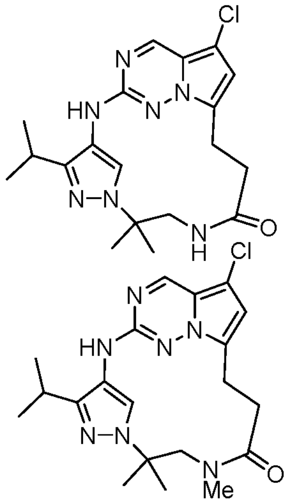
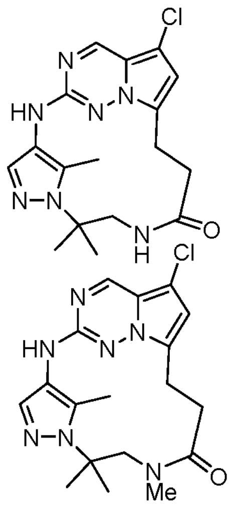

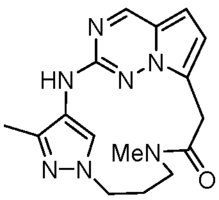
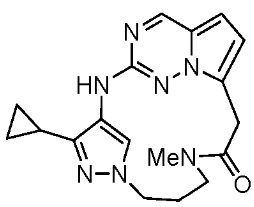
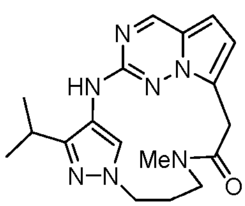
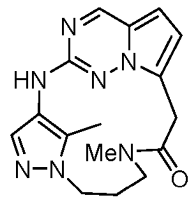

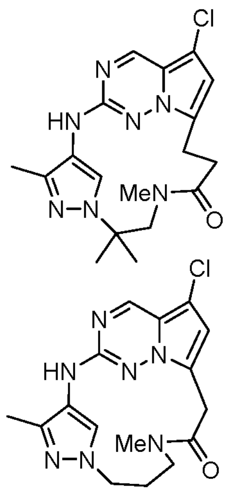
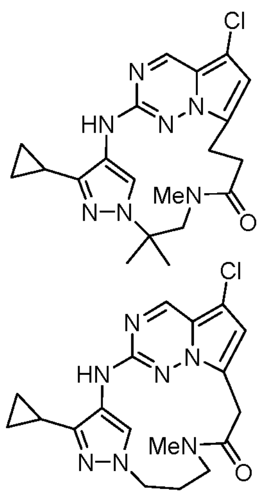
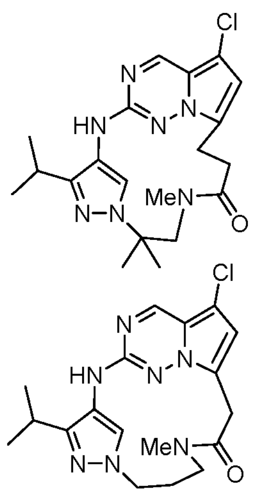
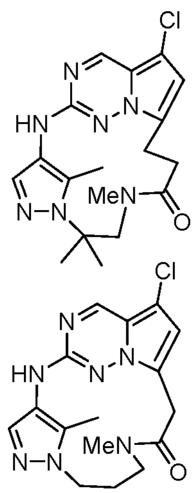

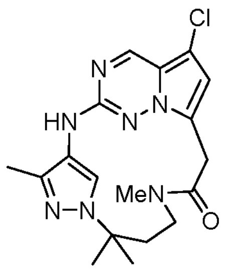
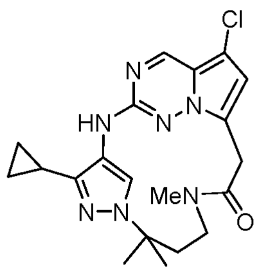
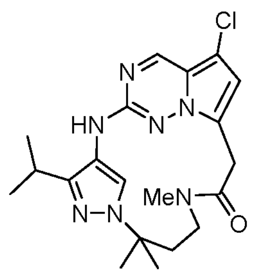
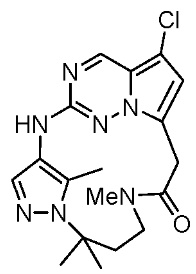

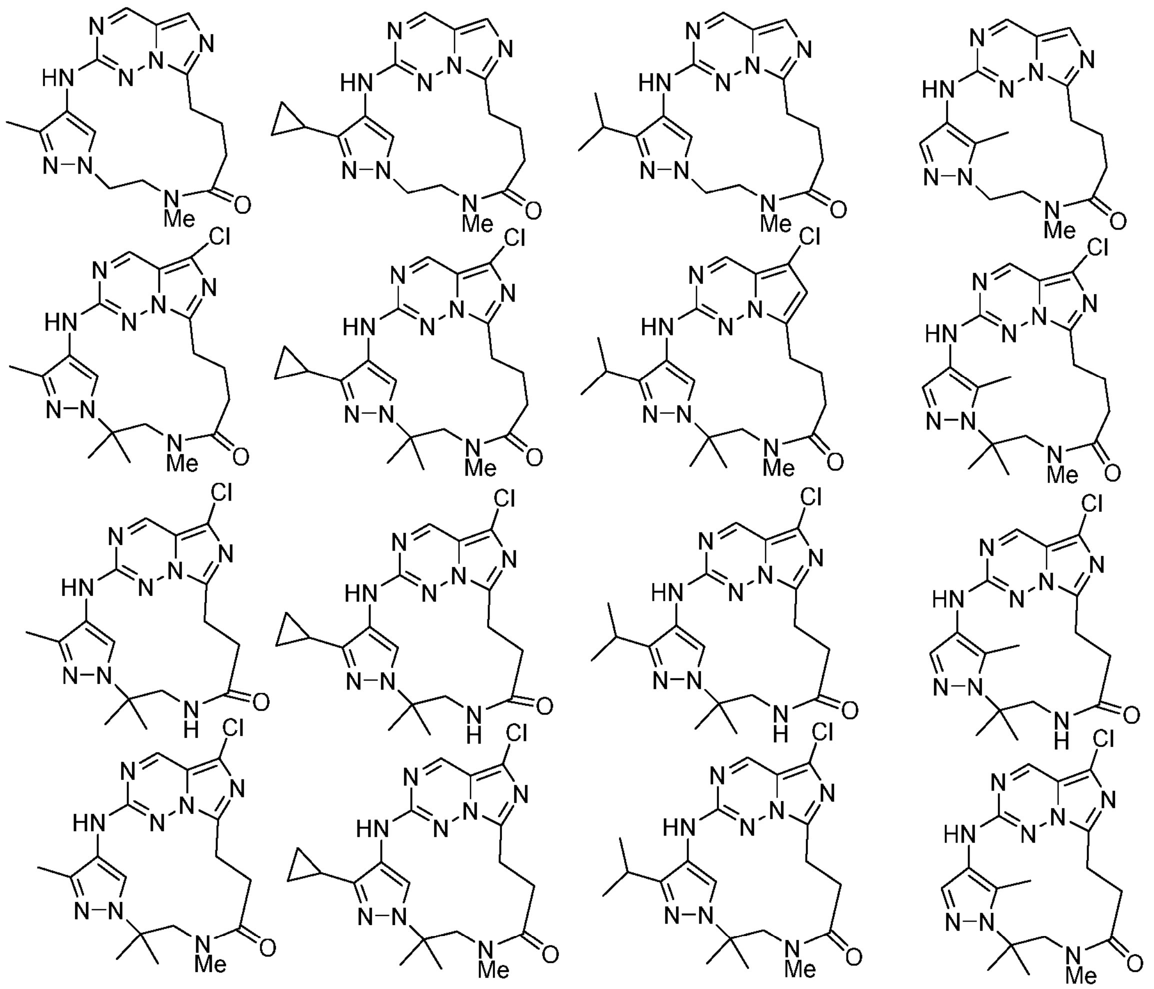

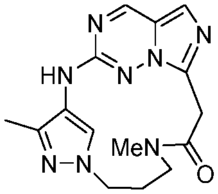

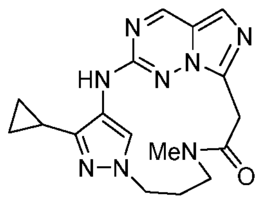

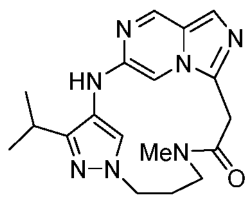

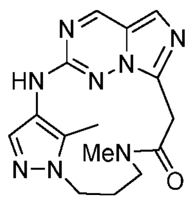

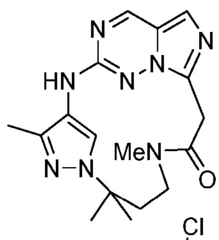

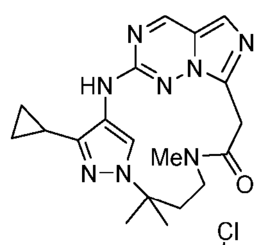

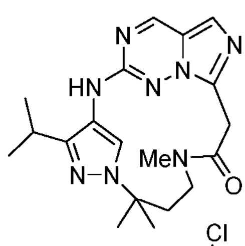

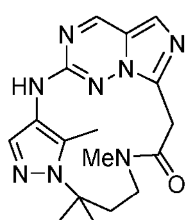

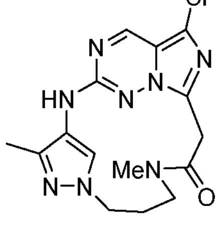

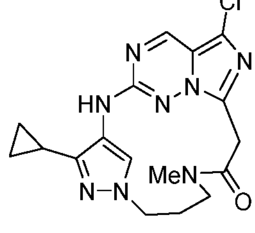

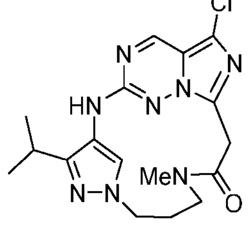

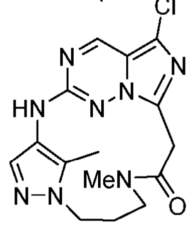

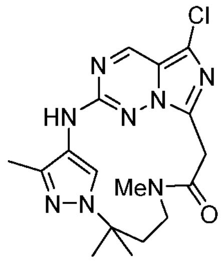
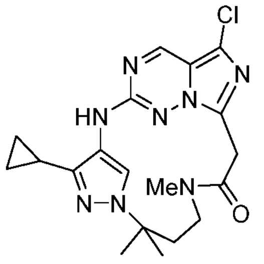
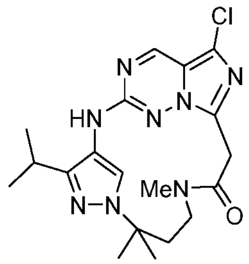
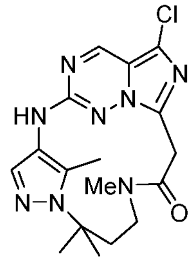

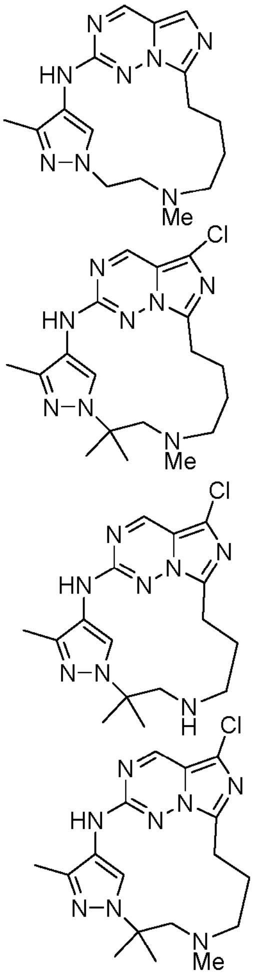
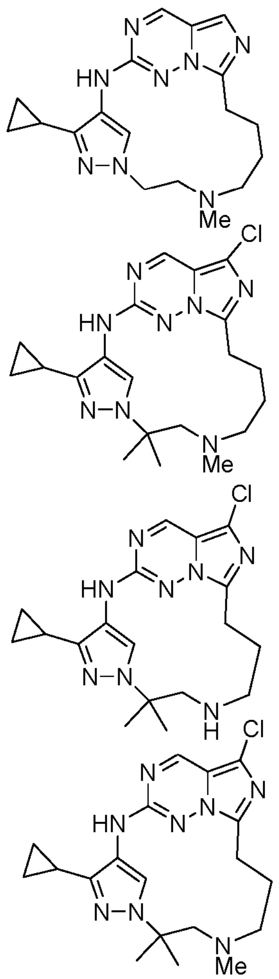
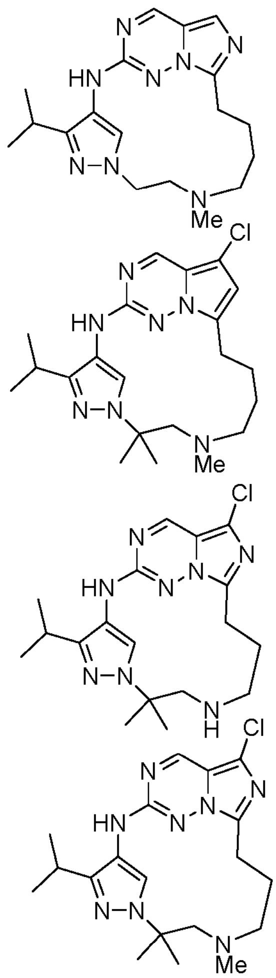
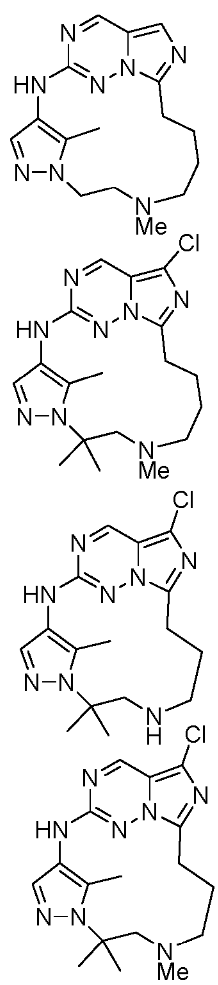

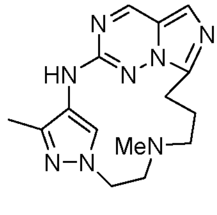
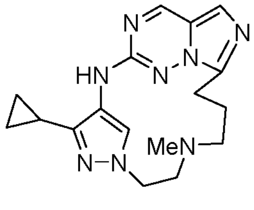
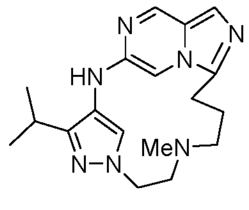
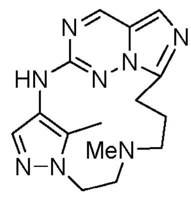

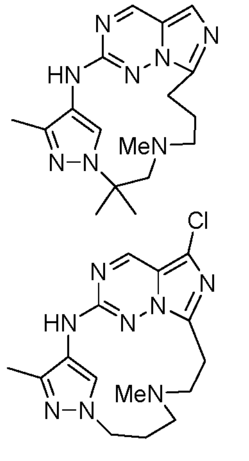 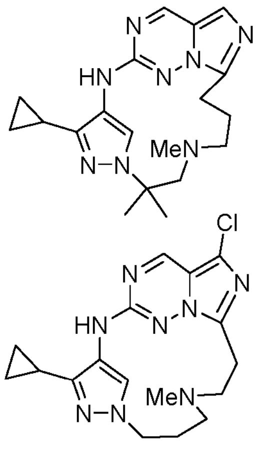 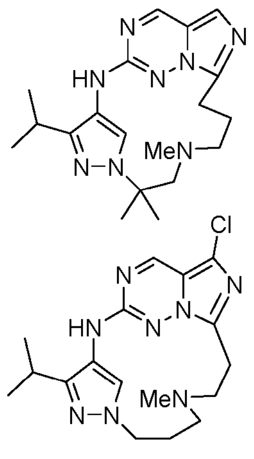 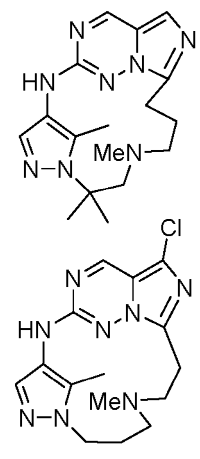

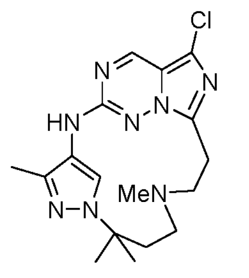 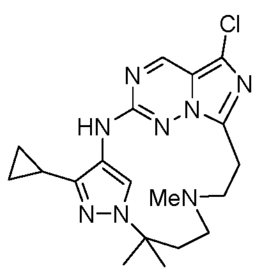 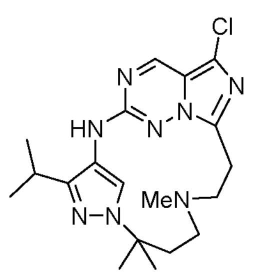 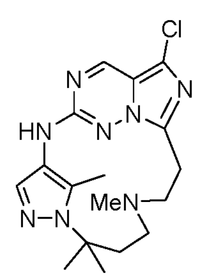

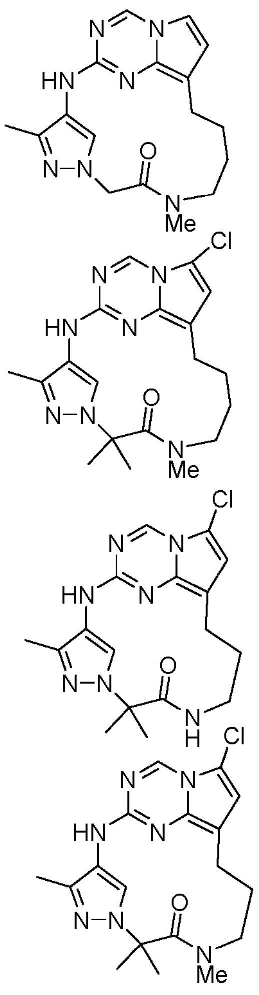 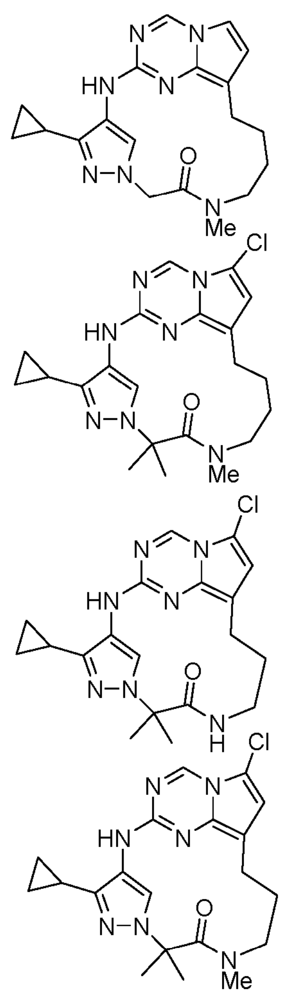 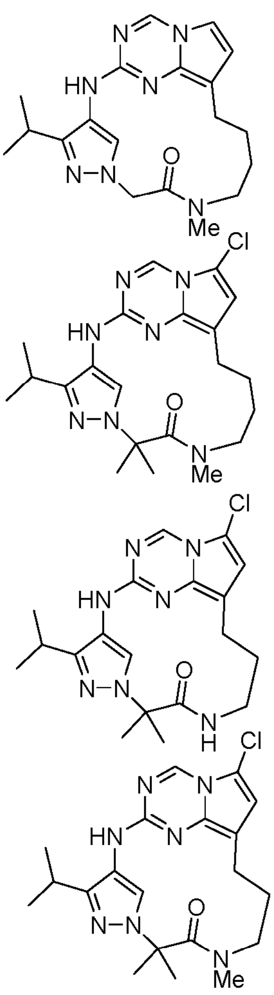 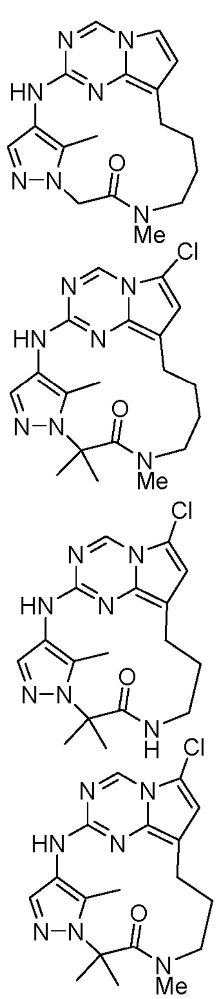

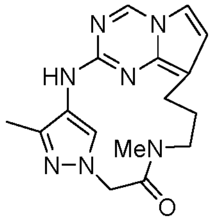
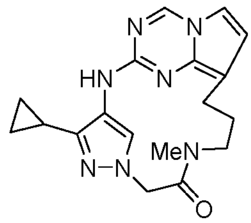
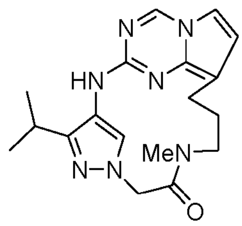
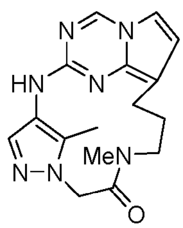

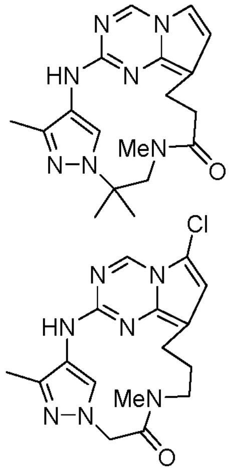
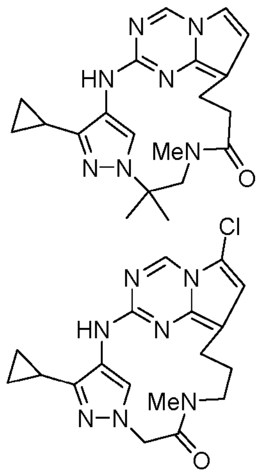
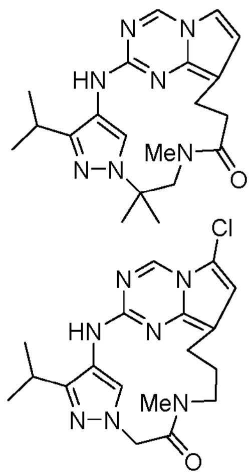
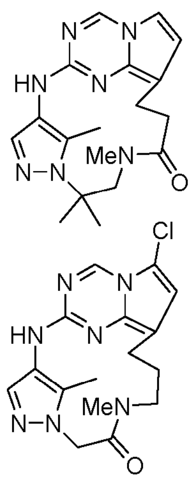

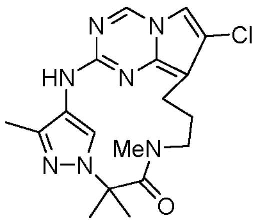
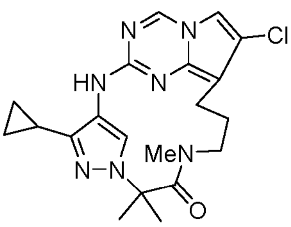
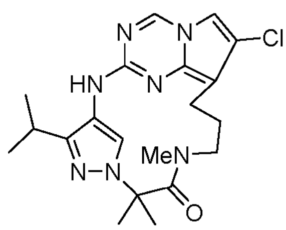
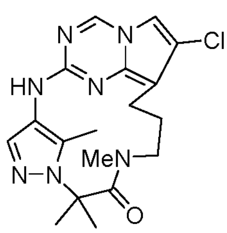

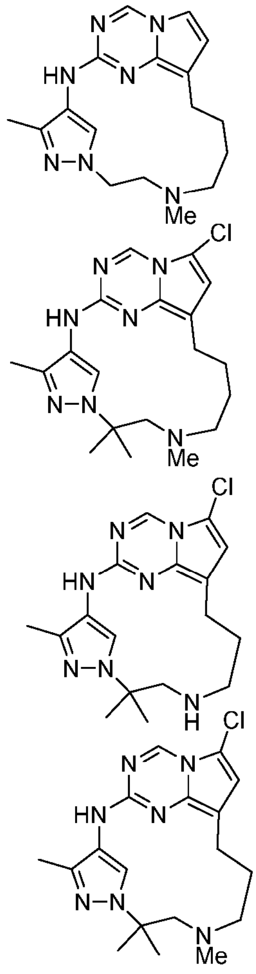
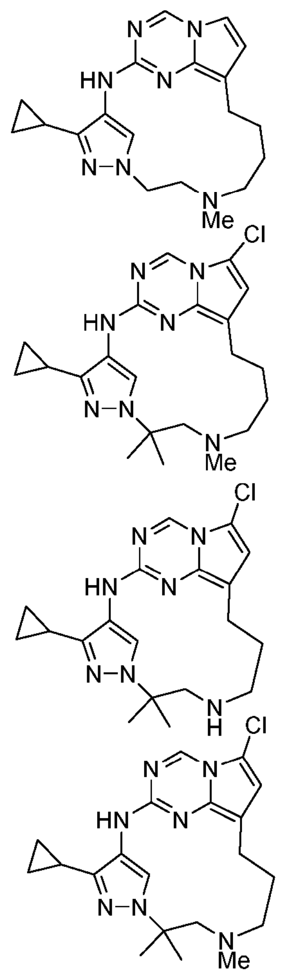
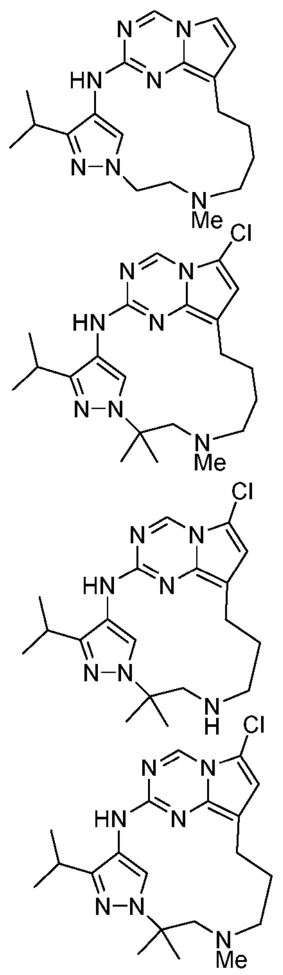
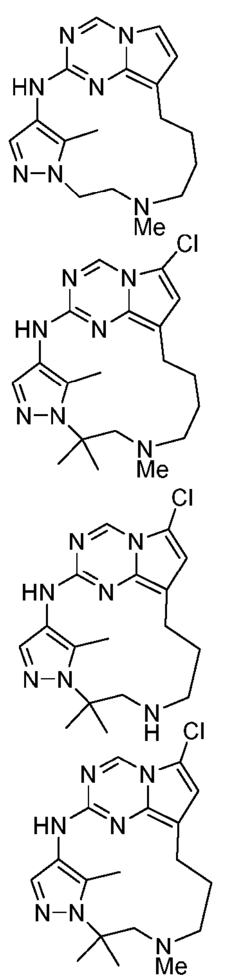

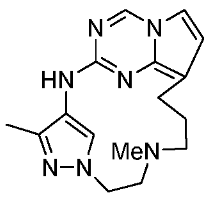
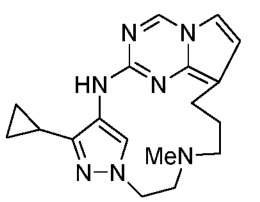
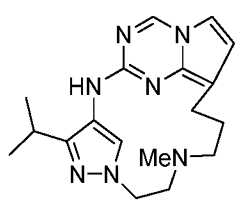
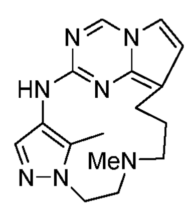

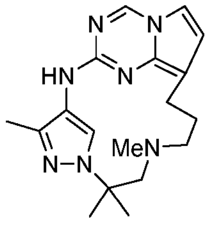
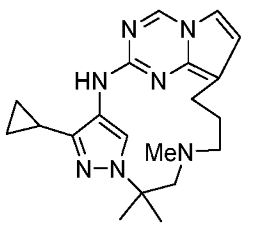
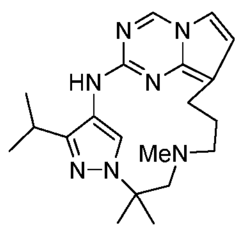
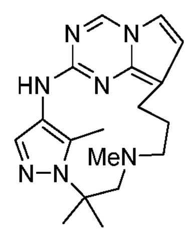

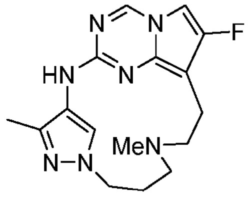
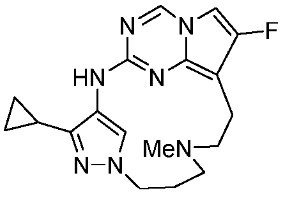
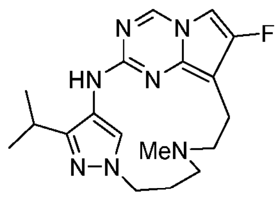
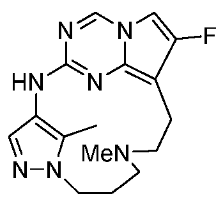

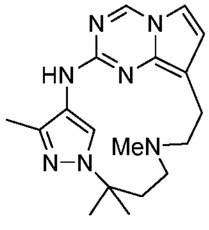 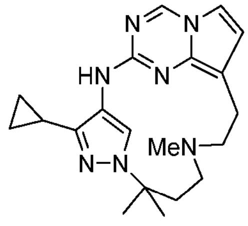 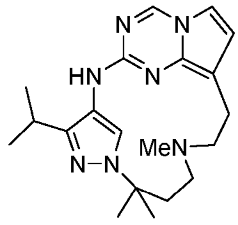 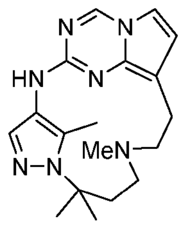

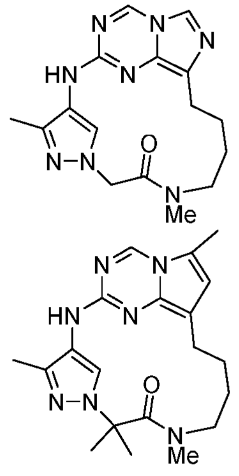 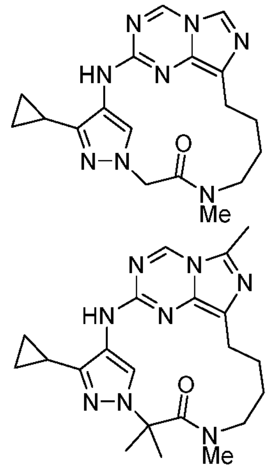 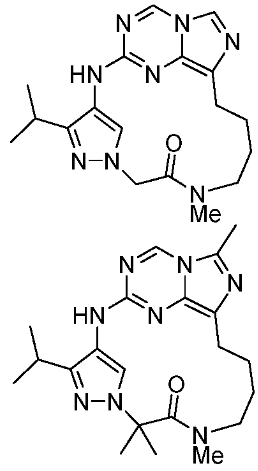 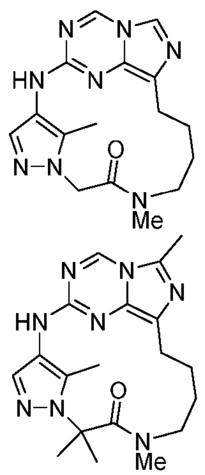

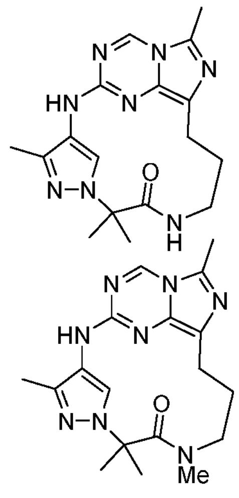 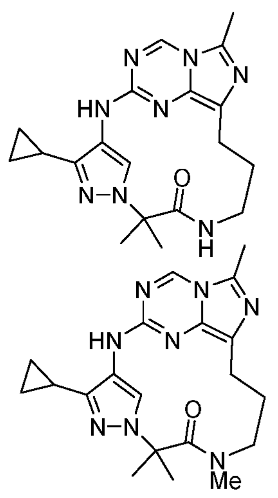 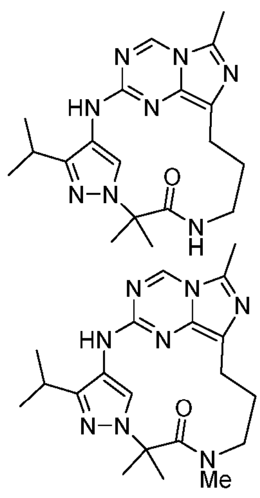 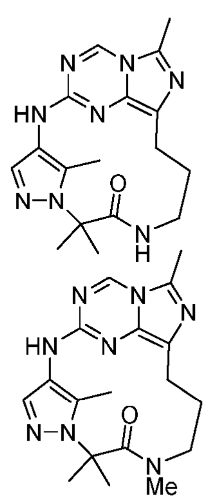

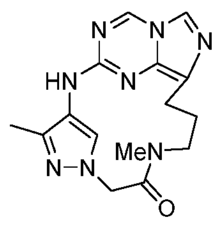 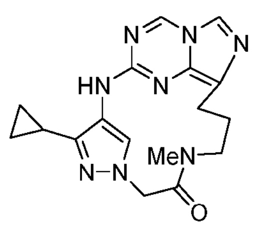 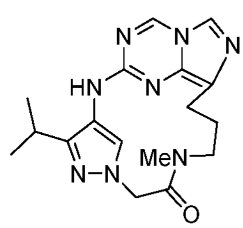 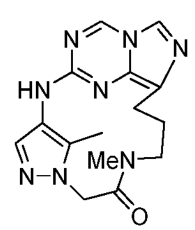

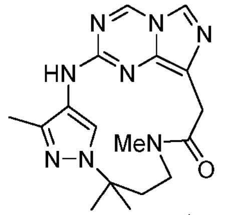
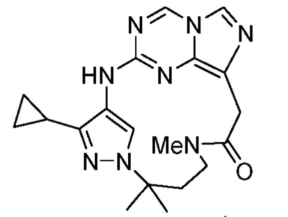
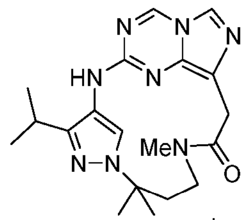
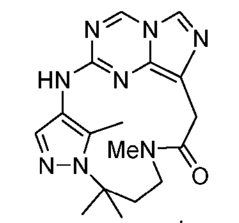
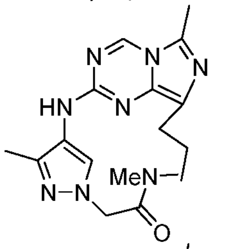
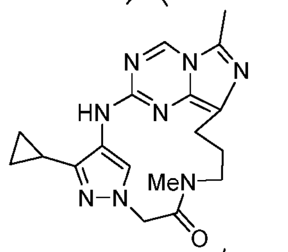
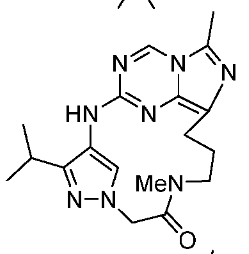
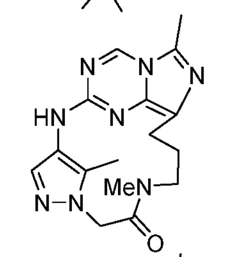
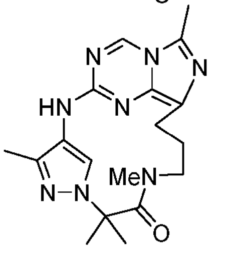
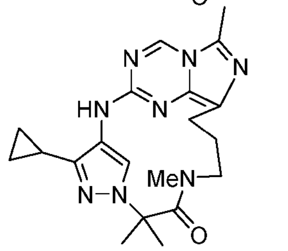
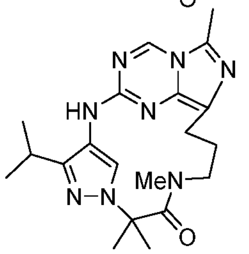
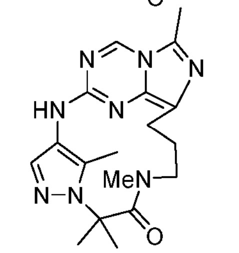
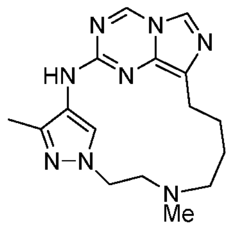
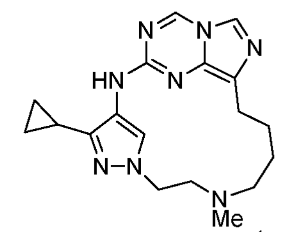
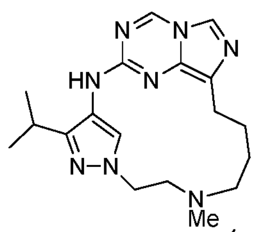
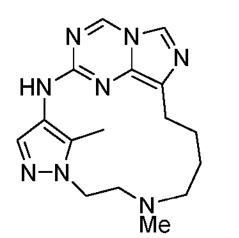
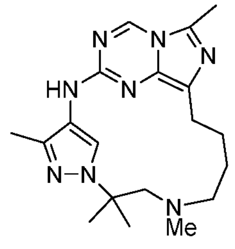
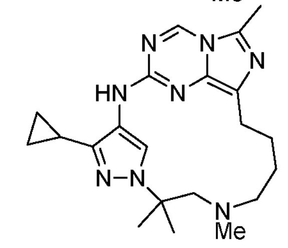
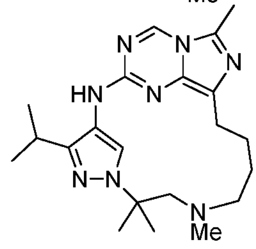
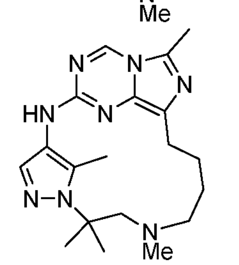
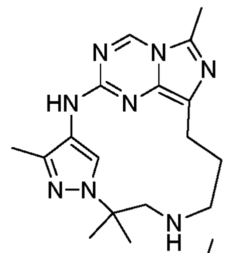
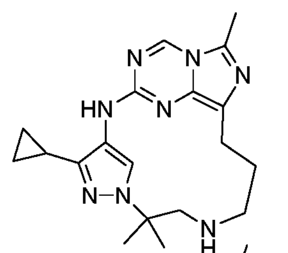
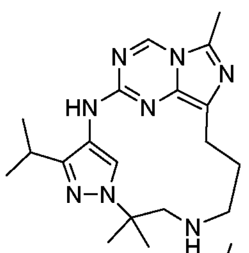
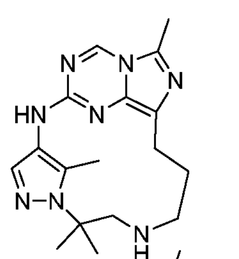
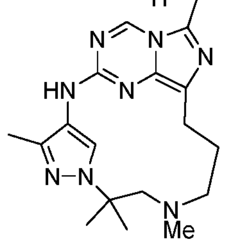
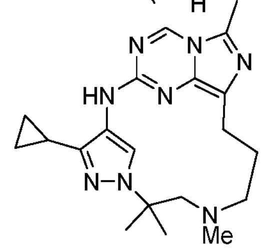
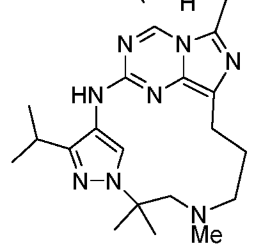
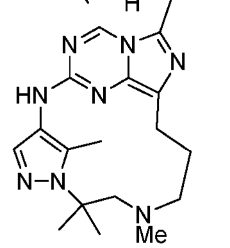

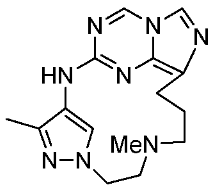
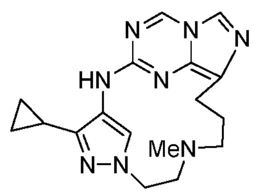
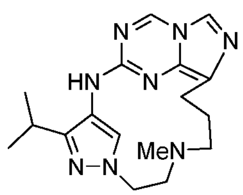
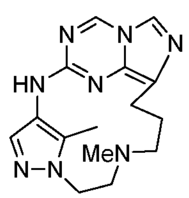
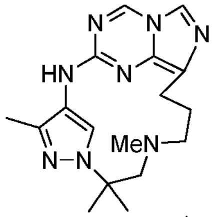
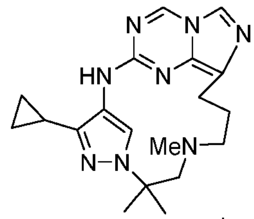
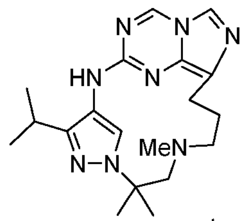
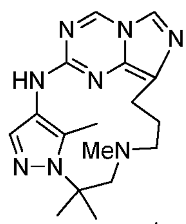
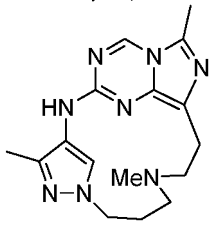
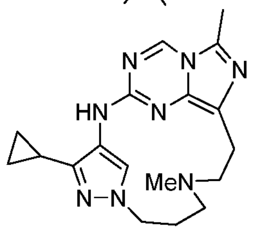
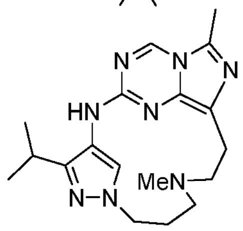
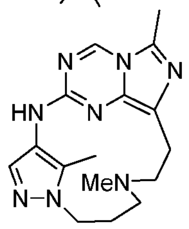
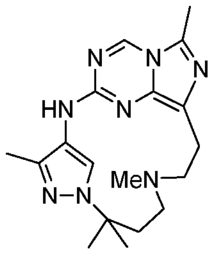
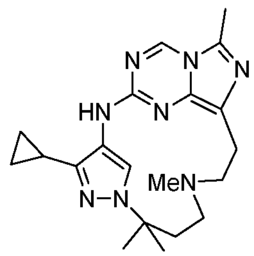
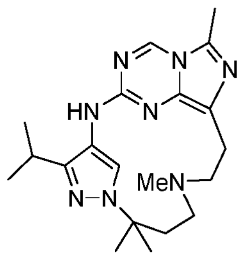
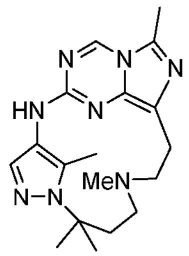
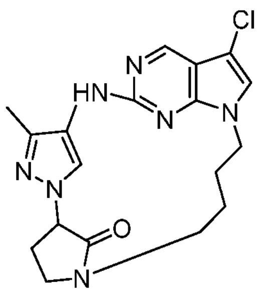
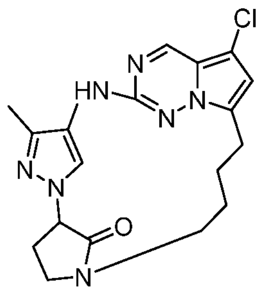
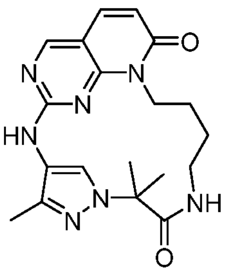
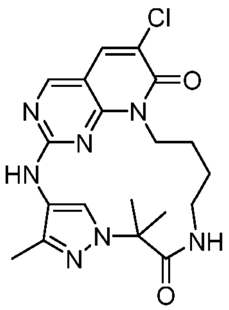
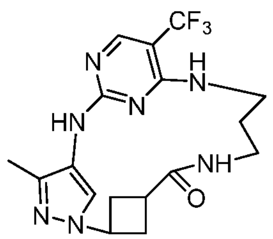
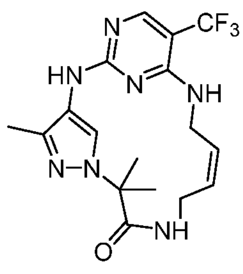

In another preferred embodiment, the compounds of general formula I to general formulaXXII include all stereoisomers.

In another preferred embodiment, thecompounds of general formula I to general formula XXII include all atropisomers.

In another preferred embodiment, thestereoisomers of the compounds of general formula I to general formula XXII are cis-trans isomers.

In another preferred embodiment, the compounds of general formula I to general formula XXII are optically pure isomers.

In another preferred embodiment, the compounds of general formula I to general formula XXII are racemates.

In another preferred embodiment, the compounds of general formula I to general formula XXII are enantiomers.

In another preferred embodiment, any one or more hydrogen atoms in the compounds of general formula I to general formula XXII may be substituted with deuterium atoms.

In another preferred embodiment, the compounds of general formula I to general formula XXII include a prodrug thereof.

In another preferred embodiment, thepharmaceutically acceptable salts of the compounds of general formula I to general formulaXXII are selected from the group consisting of: hydrochloride, hydrobromide, sulfate, phosphate, methanesulfonate, trifluoromethanesulfonate, benzenesulfonate, p-toluenesulfonate (tosylate), 1-naphthalenesulfonate, 2-naphthalenesulfonate, acetate, trifluoroacetate, malate, tartrate, citrate, lactate, oxalate, succinate, fumarate, maleate, benzoate, salicylate, phenylacetate, and mandelate.

**Definitions**

The term "alkyl" refers to a monovalent saturated aliphatic hydrocarbon group having 1 to 10 carbon atoms, including straight-chain and branched-chain hydrocarbon groups, such as methyl (i.e., $CH_3$-), ethyl (i.e., $CH_3CH_2$-), n-propyl (i.e., $CH_3CH_2CH_2$-), isopropyl (i.e., $(CH_3)_2CH$-), n-butyl (i.e., $CH_3CH_2CH_2CH_2$-), isobutyl (i.e., $(CH_3)_2CHCH_2$-), sec-butyl (i.e., $(CH_3)(CH_3CH_2)CH$-), tert-butyl (i.e., $(CH_3)_3C$-), n-pentyl (i.e., $CH_3CH_2CH_2CH_2CH_2$-), and neopentyl (i.e., $(CH_3)_3CCH_2$-).

As used herein, the term "aryl" refers to a monovalent aromatic carbocyclic group having 6 to 20 (preferably 6-14) carbon atoms, which has monocyclic (e.g., phenyl) or polycyclic (e.g., naphthyl or anthryl). If the attachment point is on an aromatic carbon atom, the polycyclic system may be non-aromatic (e.g., 2-benzoxazolone, 2H-1,4-benzoxazin-3(4H)-one-7-yl, etc.). Preferably, an aryl group includes phenyl and naphthyl.

As used herein, the term "alkenyl" refers to an alkenyl group having 2 to 10 (e.g., 2 to 6 or 2 to 4) carbon atoms and at least one (e.g., 1 to 2) unsaturated olefinic bond (>C = C<). Examples of such groups include vinyl, allyl, and but-3-enyl. As used herein, the term "cycloalkyl" refers to a cyclic alkyl group having 3 to 10 carbon atoms and a monocyclic or polycyclic structure (including fused systems, bridged ring systems, and spiro ring systems). In the fused ring systems, one or more rings may be cycloalkyl, heterocyclic, aryl, or heteroaryl, provided that the attachment point is through the cycloalkyl ring. Examples of suitable cycloalkyl include, for instance, adamantyl, cyclopropyl, cyclobutyl, cyclopentyl, and cyclooctyl.

As used herein, the term "halo" or "halogen" refers to fluorine, chlorine, bromine, and iodine.

As used herein, the term "heteroaryl" refers to an aromatic group containing 1 to 10 carbon atoms and 1 to 4 heteroatoms selected from oxygen, nitrogen, and sulfur in the ring. Such heteroaryl groups may be monocyclic (e.g., pyridyl or furyl) or fused rings (e.g., indolizinyl or benzothienyl), wherein the fused ring may be non-aromatic and/or contain one heteroatom, provided that the attachment point is through an atom of the aromatic heteroaryl. In one embodiment, the ring atoms such as nitrogen and/or sulfur of the heteroaryl group are optionally oxidized to N-oxide (N-O), sulfinyl, or sulfonyl. Preferably, the heteroaryl group includes pyridyl, pyrrolyl, indolyl, thienyl, and furyl.

As used herein, the term "substituted heteroaryl" refers to a heteroaryl group substituted with 1 to 5, preferably 1 to 3, more preferably 1 to 2 substituents selected from the same substituents as those defined for the substituted aryl.

As used herein, the terms "heterocycle", or "heterocyclic", or "heterocycloalkyl", or "heterocyclyl" refer to saturated, partially saturated, or unsaturated (but not aromatic) groups having a monocyclic or fused rings (including bridged ring systems and spiro ring systems) and containing 1 to 10 carbon atoms and 1 to 4 heteroatoms selected from nitrogen, sulfur, or oxygen within the ring. In the fused ring systems, one or more rings may be cycloalkyl, aryl, or heteroaryl, provided that the attachment point is through a non-aromatic ring. In one embodiment, the nitrogen and/or sulfur atoms of the heterocyclic group are optionally oxidized to provide N-oxide, sulfinyl, or sulfonyl moieties.

As used herein, the terms "substituted heterocyclic", "substituted heterocycloalkyl" or "substituted heterocyclyl" refer to a heterocyclic group substituted with 1 to 5 (e.g., 1 to 3) substituents, which are the same as those defined for the substituted cycloalkyl.

Unless otherwise specially indicated, each cycloalkyl is $C_{3-6}$ cycloalkyl, each heterocyclyl is a 3-10 membered heterocyclyl, each aryl is $C_{6-10}$ aryl, and each heteroaryl is a 5-10 membered heteroaryl.

The substituents are selected from, but are not limited to, the following chemical groups: halogen, - $C_{1-6}$ alkyl, -$C_{3-8}$ cycloalkyl, -$C_{1-6}$ haloalkyl, -$C_{3-8}$ halocycloalkyl, -$C_{1-6}$ alkoxy, -$C_{3-8}$ cycloalkoxy, - $C_{1-6}$ alkylthio, -$C_{0-6}$ alkylene-OH, nitro, formyl, -$SF_5$, -$C_{0-6}$ alkylene-$NR^aR^b$, -$C_{0-6}$ alkylene-alkyl-carboxyl, -$C_{0-6}$ alkylene-$COR^a$, -$C_{0-6}$ alkylene-$CO_2R^a$, -$C_{0-6}$ alkylene-$CONR^dR^e$, -$C_{0-6}$ alkylene-$SO_2R^a$, -$C_{0-6}$ alkylene-$SO_2NR^dR^e$, carbonyl, -$C_{0-6}$ alkylene-CN, -$C_{3-8}$ cycloalkyl-OH,

$-C_{2-6}$ alkenyl, $C_{2-6}$ alkynyl, $-C_{0-6}$ alkylene-S(O)(NH)$C_{1-6}$alkyl, $-C_{0-6}$ alkylene-S(O)(NCN)$C_{1-6}$alkyl, $-C_{0-6}$ alkylene-$NRCS(O)_2R^b$, $-C_{0-6}$ alkylene-$NR^cS(O)_2NRCR^b$, $-C_{0-6}$ alkylene-$NR^cC(O)NH_2$, $-C_{0-6}$ alkylene-$NR^cC(O)R^b$, $-C_{0-6}$ alkylene-$NR^cC(O)NR^dR^e$, $-C_{0-6}$ alkylene-$NR^cC(O)OR^b$, $-C_{0-6}$ alkylene-$NRSO_2R^bC(O)-R^b$, $-C_{0-6}$ alkylene-$P(O)R^cR^b$, $-C_{0-6}$ alkylene-$P(O)(OR^c)(OR^b)$, $-C_{0-6}$ alkylene-C(O)$C_{1-6}$ alkyleneamino, $C_{1-6}$ heteroalkyl, $C_{5-10}$ carbocycle, $C_{5-10}$ aryl, $C_{2-10}$heterocyclyl, and $C_{2-10}$ heteroaryl.

In this application, the $C_{0-6}$ alkylene refers to the absence of alkylene or the presence of $C_{1-6}$ alkylene.

As used herein, the term "stereoisomer" refers to compounds that differ in chirality at one or more stereocenters. Stereoisomers include enantiomers and diastereomers.

As used herein, the term "tautomer" refers to alternative forms of a compound differing in proton position, such as enol-keto and imine-enamine tautomers, or tautomeric forms of a heteroaryl group containing a ring atom connected to both the -NH- and =N- moieties of the ring, such as pyrazole, imidazole, benzimidazole, triazole, and tetrazole.

The term "prodrug" refers to any derivative of the exemplified compounds that, when administered to a subject, is capable of directly or indirectly providing the exemplified compound or active metabolites or residues thereof. Particularly preferred derivatives and prodrugs are those that, when administered to a subject, enhance the bioavailability of the exemplified compound (e.g., making orally administered compounds more readily absorbed into the bloodstream) or improve delivery of the parent compounds to biological compartments (e.g., brain or lymphatic system) relative to the parent species. Prodrugs include ester forms of the compounds of the present disclosure.

Where there are stereoisomers of the compounds according to the present disclosure, the present disclosure encompasses all stereoisomers of the compounds.

Where there are tautomers of the compounds according to the present disclosure, the present disclosure encompasses all tautomers of the compounds.

Where there are tautomers of the compounds according to the present disclosure, the present disclosure encompasses all atropisomers of the compounds.

The present disclosure also includes deuterated compounds generated by replacing one or more hydrogen atoms in said compounds with their stable isotope deuterium.

The present disclosure further provides active ingredients in a safe and effective amount range of compounds of general formula I and general formula XXII, as well as a pharmaceutically acceptable carrier.

The "active ingredient" described in the present disclosure refers to the compounds of general formula I to general formula XXII described in the present disclosure.

The "active ingredient" and pharmaceutical compositions described in the present disclosure can be used for treating diseases such as neurodegenerative diseases (including Parkinson's disease) and cancers, etc.

"A safe and effective amount" refers to a quantity of the active ingredient sufficient to significantly ameliorate the condition without causing severe side effects. Typically, the pharmaceutical composition contains 1-2000 mg of active ingredient per dose, preferably 10-200 mg of active ingredient per dose. Preferably, the "dose" refers to one tablet or capsule.

The term "pharmaceutically acceptable carrier" refers to one or more compatible solid or liquid fillers or gel substances suitable for human use, which must be of sufficient purity and sufficiently low toxicity. The term "compatibility" herein means that the components in the composition can blend with the active ingredients of the present disclosure and with each other without significantly reducing the efficacy of the active ingredients.

Generally, the compounds of the preferred embodiments will be administered in a therapeutically effective amount via any acceptable mode used for agents with similar effects. The actual dosage of the compounds of the preferred embodiments (i.e., the active ingredients) is determined by multiple factors, such as the severity of the disease to be treated, the age and relative health condition of the patients, the potency of the compound used, the route and form of administration, as well as other factors. The drug may be administered multiple times a day, preferably once or twice a day. All these factors are within the consideration of the attending physicians.

For the purposes of the preferred embodiments, the therapeutically effective dose is generally a total daily dose administered to the patient either as a single dose or in divided doses, for example, from about 0.001 to about 1000 mg/kg body weight per day, and preferably from about 1.0 to about 30 mg/kg body weight per day. Dosage unit composition may contain their dose factors to form the daily dose. The choice of dosage form depends on various factors, such as the mode of administration and the bioavailability of the drug substance. Generally, the compounds of the preferred embodiments may be administered as a pharmaceutical composition via any of the following routes: oral, systemic (e.g., transdermal, intranasal, or via suppository), or parenteral (e.g., intramuscular, intravenous, or subcutaneous) administration. The preferred mode of administration is oral administration, which allows convenient daily dosage adjusted according to the degree of bitterness. The composition may take the form of tablets, pills, capsules, semi-solids, powders, sustained release preparations, solutions, suspensions, elixirs, aerosols, or any other suitable compositions. Another preferred mode of administering the compounds of the preferred embodiments is via inhalation. This is an effective method for delivering the therapeutic agents directly to the respiratory tract (see, for example, U.S. Patent No. 5,607,915).

Suitable pharmaceutically acceptable carriers or excipients include, for example, treatment agents and drug

delivery modifiers and enhancers, such as calcium phosphate, magnesium stearate, talc, monosaccharides, disaccharides, starch, gelatin, cellulose, sodium methylcellulose, carboxymethyl cellulose, glucose, hydroxypropyl-B-cyclodextrin, polyvinylpyrrolidone, low-melting-point wax, and ion exchange resins, etc., and any combination of two or more thereof. Liquid and semi-solid excipients may be selected from glycerol, propylene glycol, water, ethanol, and various oils, including petroleum, animal, vegetable, or synthetic oils, such as peanut oil, soybean oil, mineral oil, and sesame oil, etc. Preferred liquid carriers, particularly those for injectable solutions, include water, saline, aqueous solution of glucose, and ethylene glycol. Other suitable pharmaceutically acceptable excipients are described in Remington's Pharmaceutical Sciences, Mack Pub. Co., New Jersey (1991), which is incorporated herein by reference.

As used herein, the term "pharmaceutically acceptable salt" refers to non-toxic acid or alkaline earth metal salts of compounds of general formula I to general formula XXII. These salts may be prepared in situ during the final isolation and purification of compounds of general formula I to general formula XXII, or by separately reacting a suitable organic or inorganic acid or base with a basic or acidic functional group. Representative salts include, but are not limited to, acetate, adipate, alginate, citrate, aspartate, benzoate, benzenesulfonate, bisulfate, butyrate, camphorate, camphorsulfonate, digluconate, cyclopentanepropionate, dodecyl sulfate, ethanesulfonate, glucoheptonate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, pamoate, pectate, thiocyanate, 3-phenylpropionate, picrate, pivalate, propionate, succinate, sulfate, tartrate, thiocyanate, tosylate, and undecanoate. Furthermore, a nitrogen-containing basic group may be quaternized with the following reagents: alkyl halides such as methyl, ethyl, propyl, and butyl chlorides, bromides, and iodides; dialkyl sulfates such as dimethyl, diethyl, dibutyl, and dipentyl sulfates; long-chain halides such as decyl, lauryl, myristyl, and stearyl chlorides, bromides, and iodides; and aralkyl halides such as benzyl and phenethyl bromides, thereby producing water-soluble, or oil-soluble, or dispersible products. Examples of acids that may be used to form pharmaceutically acceptable acid addition salts include inorganic acids such as hydrochloric, sulfuric, and phosphoric acids, and organic acids such as oxalic, maleic, methanesulfonic, succinic, and citric acids. Base addition salts may be prepared in situ during the final isolation and purification of compounds of general formula I to general formula XXII, or by separately reacting a carboxylic acid moiety with a suitable base, such as hydroxides, carbonates, or bicarbonates of a pharmaceutically acceptable metal cation, or with ammonia or an organic primary, secondary, or tertiary amine. Pharmaceutically acceptable salts include, but are not limited to, those based on alkali and alkaline earth metal cations, such as sodium, lithium, potassium, calcium, magnesium, and aluminum, etc., as well as non-toxic ammonium, quaternary ammonium, and amine cations, including, but not limited to, ammonium, tetramethylammonium, tetraethylammonium, methylamine, dimethylamine, trimethylamine, triethylamine, and ethylamine, etc. Other representative organic amines for forming base addition salts include diethylamine, ethylenediamine, ethanolamine, diethanolamine, and piperazine, etc.

In a second aspect, the present disclosure provides a method for preparing the compounds of general formula I to general formula XXII according to the first aspect, wherein the method is selected from one of the following schemes.

Scheme 1:

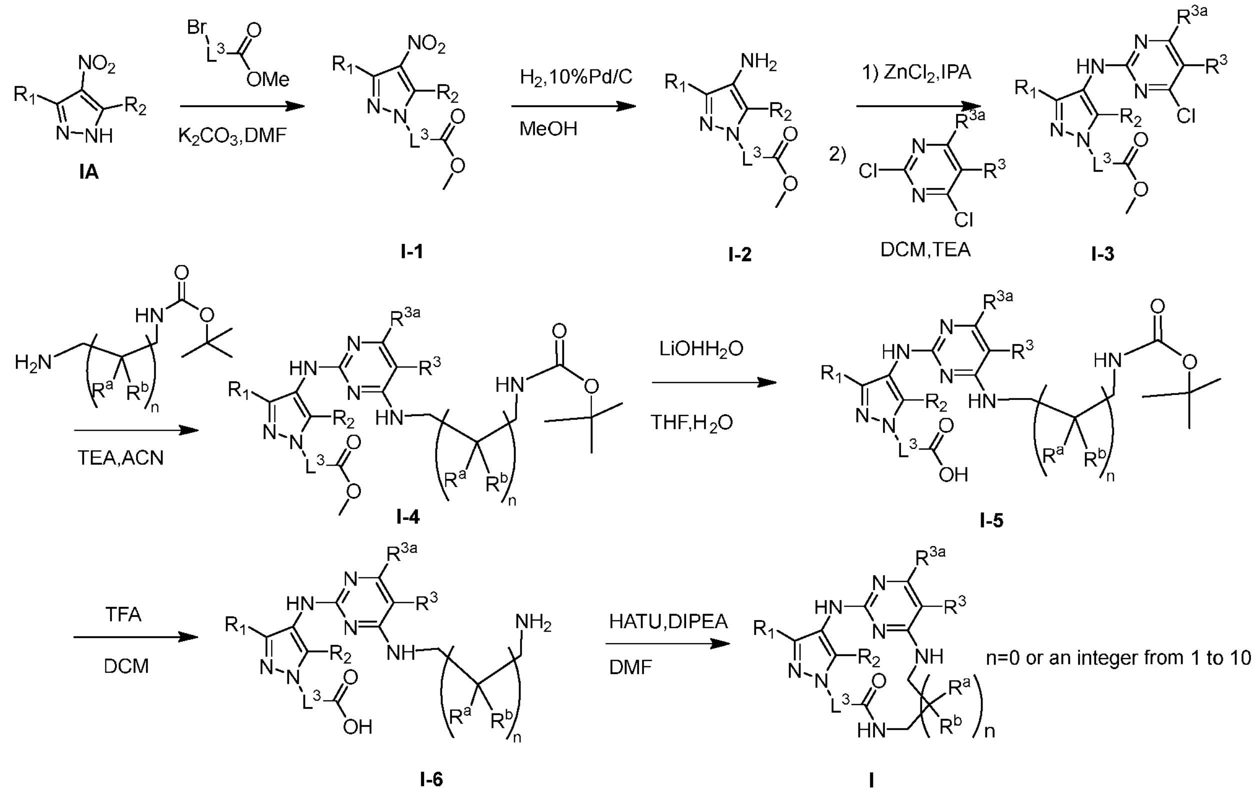


Compound IA is reacted with the corresponding bromide in the presence of a base to yield I-1, the nitro group is reduced to amine I-2 (under the reduction conditions such as, but not limited to, Zn/AcOH, Fe/$NH_4Cl$/EtOH, Zn/$NH_4Cl$/EtOH, and Pd/C/$H_2$/MeOH), the amine I-2 is then reacted with a suitable substituted 2,4-dichloropyrimidine under the catalysis of an acid (under the acid catalysis conditions such as, but not limited to, pTsOH, and $ZnCl_2$) to yield I-3, which is then reacted with a mono-Boc diamine under basic conditions (with the basic conditions such as, but not limited to, DIPEA, and TEA) to yield I-4, and the ester is hydrolyzed under basic conditions (with the basic conditions such as, but not limited to, KOH/$H_2O$, NaOH/$H_2O$, and LiOH/$H_2O$) to yield I-5, and then the Boc group is removed under acidic conditions (with the acidic conditions such as, but not limited to, TFA/DCM, and HCl/1,4-dioxane) to yield I-6, which is finally subjected to intramolecular condensation using a condensation reagent to yield the final product I (with the condensation reagent such as, but not limited to, HOAT, HOBT, HATU, EDCI, BOP, and TCFH/NMI).

## Scheme 2:

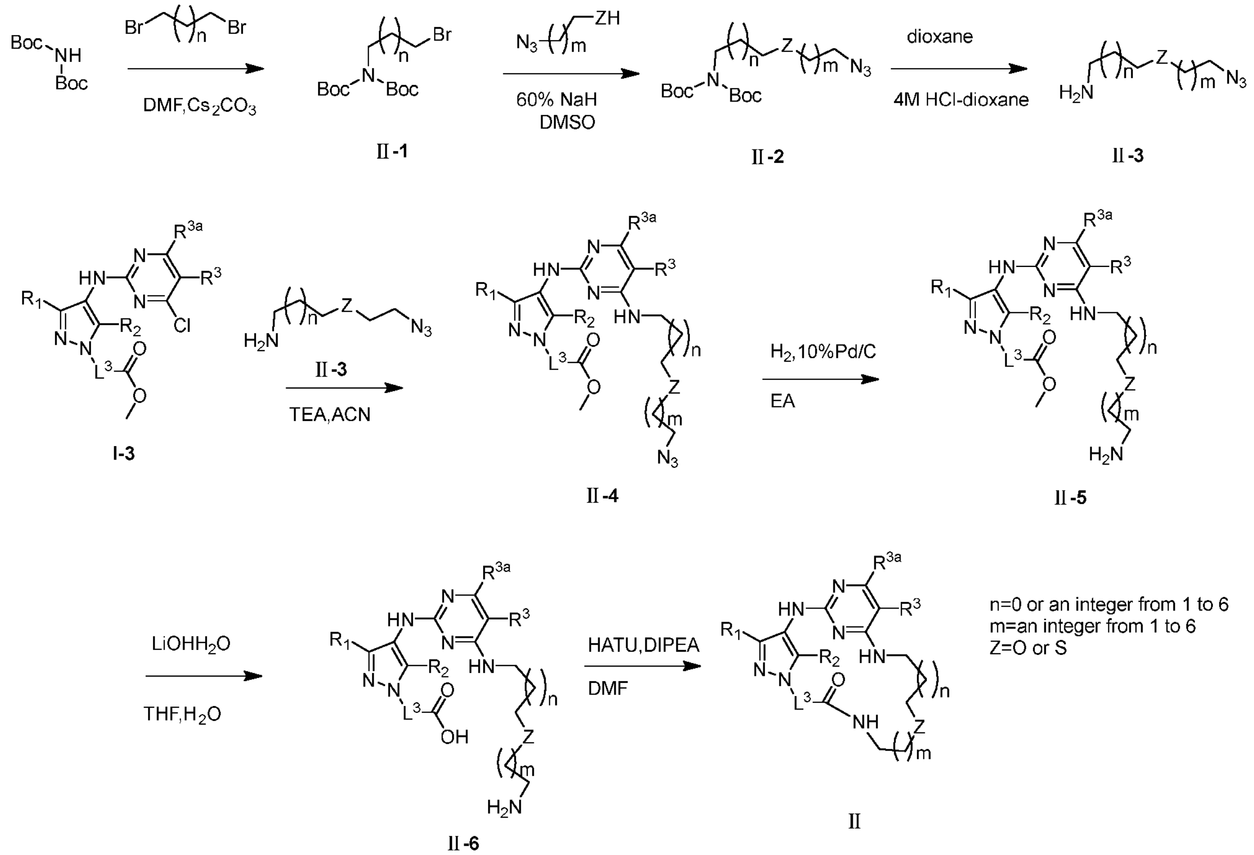


$(Boc)_2NH$ is reacted with the corresponding dibromoalkane under basic conditions (basic conditions including but not limited to $K_2CO_3$, and $Cs_2CO_3$) to yield II-1, which is then reacted with the corresponding azido alcohol or azidothiol under basic conditions (basic conditions including but not limited to nBuLi, and NaH) to yield II-2, which is then reacted under acidic conditions (acid conditions including but not limited to TFA/DCM, and HCl/1,4-dioxane) to yield II-3. Compound II-3 is reacted with intermediate I-3 under basic conditions (basic conditions including but not limited to DIPEA, and TEA) to yield II-4, then the azido group is reduced to amine II-5 (reduction conditions including but not limited to Pd/C/$H_2$/EA, $PtO_2$/MeOH, and Raney-Ni/$N_2H_4$ $H_2O$/TEA/MeOH), the ester is then hydrolyzed under basic conditions (acid conditions including but not limited to KOH/$H_2O$, NaOH/$H_2O$, and LiOH/$H_2O$) to yield II-6, which is subjected to intramolecular condensation using a condensation reagent to yield the final product II (condensation reagents including but not limited to HOAT, HOBT, HATU, EDCI, BOP, and TCFH/NMI).

## Scheme 3:

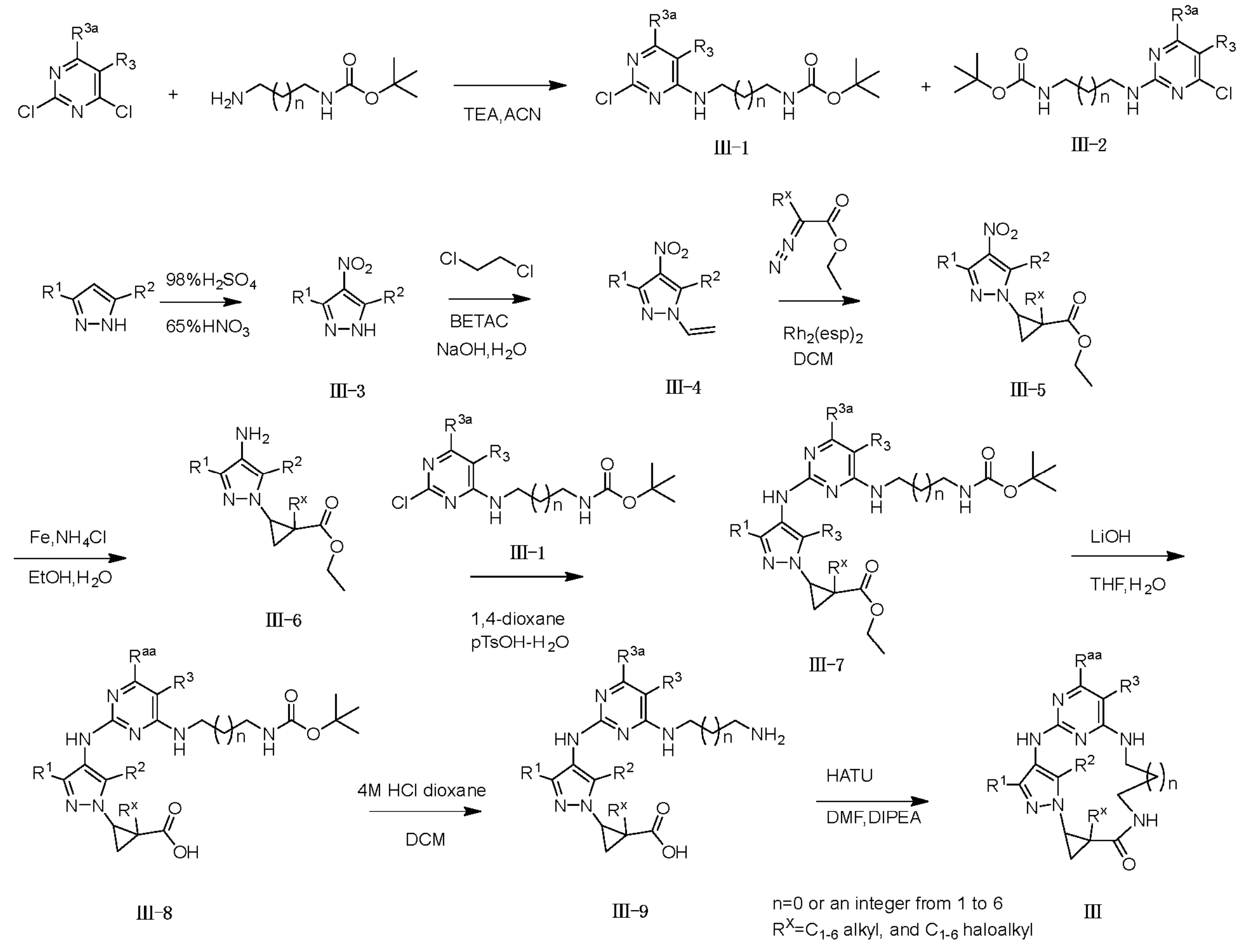


The corresponding mono-Boc diamine is reacted with a suitable substituted 2,4-dichloropyrimidine under basic conditions (basic conditions including but not limited to DIPEA, and TEA) to yield III-1 and III-2, then the suitable substituted 1H-pyrazole is subjected to nitration under acidic conditions (nitration conditions including but not limited to 98% $H_2SO_4$/68% $HNO_3$, 98% $H_2SO_4$/$KNO_3$, and $Ac_2O$/68% $HNO_3$) to yield III-3, which is reacted with 1,2-dichloroethane under basic conditions to yield intermediate III-4, which is then reacted with a suitable substituted ethyl diazoacetate to yield III-5, the nitro group is then reduced to amine III-6 (reduction conditions including but not limited to Zn/AcOH, Fe/$NH_4Cl$/EtOH, Zn/$NH_4Cl$/EtOH, and Pd/C/$H_2$/MeOH), amine III-6 is reacted with compound III-1 under the catalysis of an acid (acid catalysis conditions including but not limited to pTsOH, and $ZnCl_2$) to yield III-7, and the ester is hydrolyzed under basic conditions (basic conditions including but not limited to KOH/$H_2O$, NaOH/$H_2O$, and LiOH/$H_2O$) to yield III-8, which is then reacted under acidic conditions (acidic conditions including but not limited to TFA/DCM, and HCl/1,4-dioxane) to yield III-9, which is subjected to intramolecular condensation using a condensation reagent to yield the final product III (condensation reagents including but not limited to HOAT, HOBT, HATU, EDCI, BOP, and TCFH/NMI).

## Scheme 4

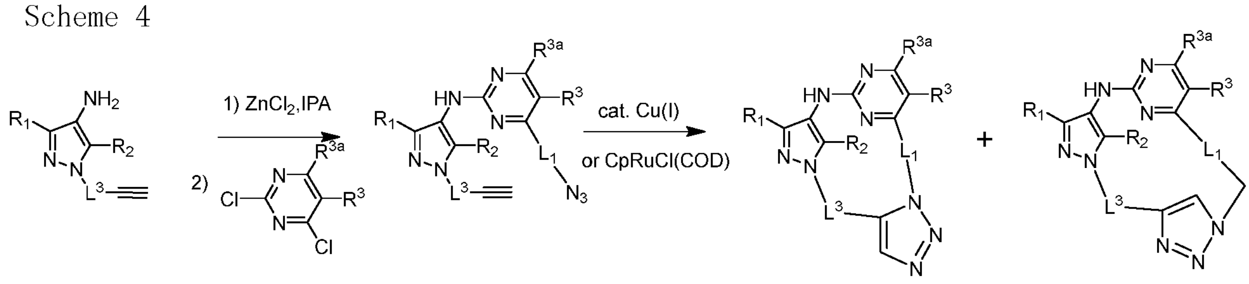

Scheme 5:

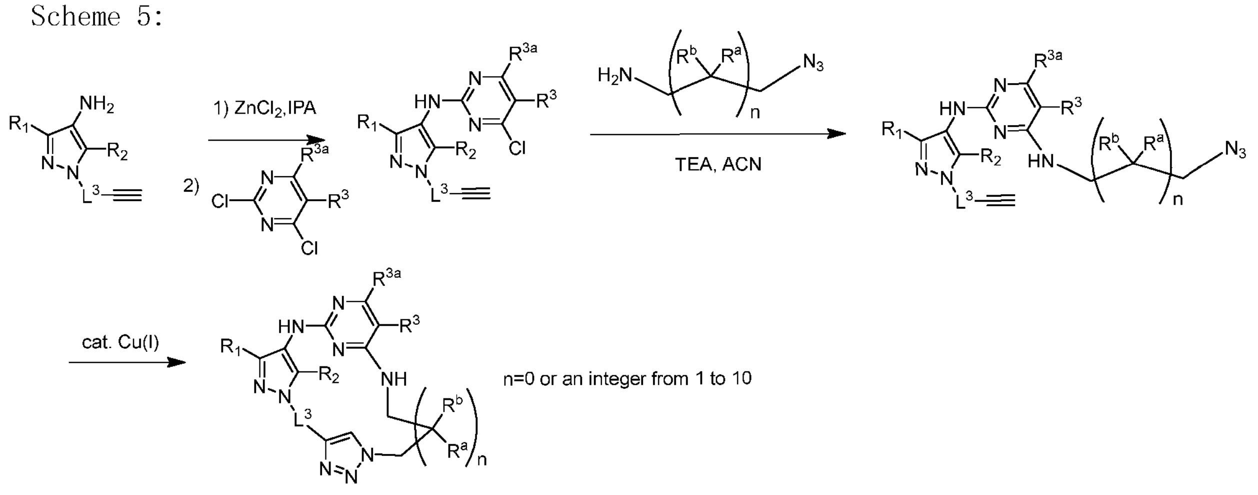


## Scheme 6

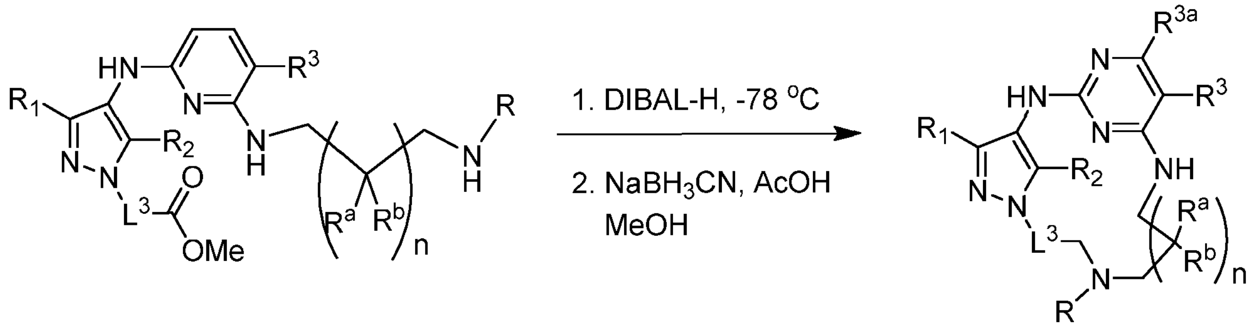


n=0 or an integer from 1 to 10

R=H, $C_{1-6}$ alkyl, and $C_{3-6}$ cycloalkyl

Scheme 7:

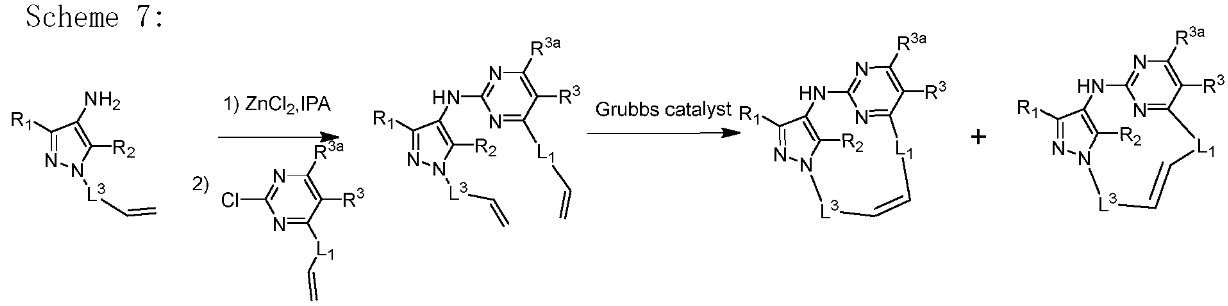

**Scheme 8:**

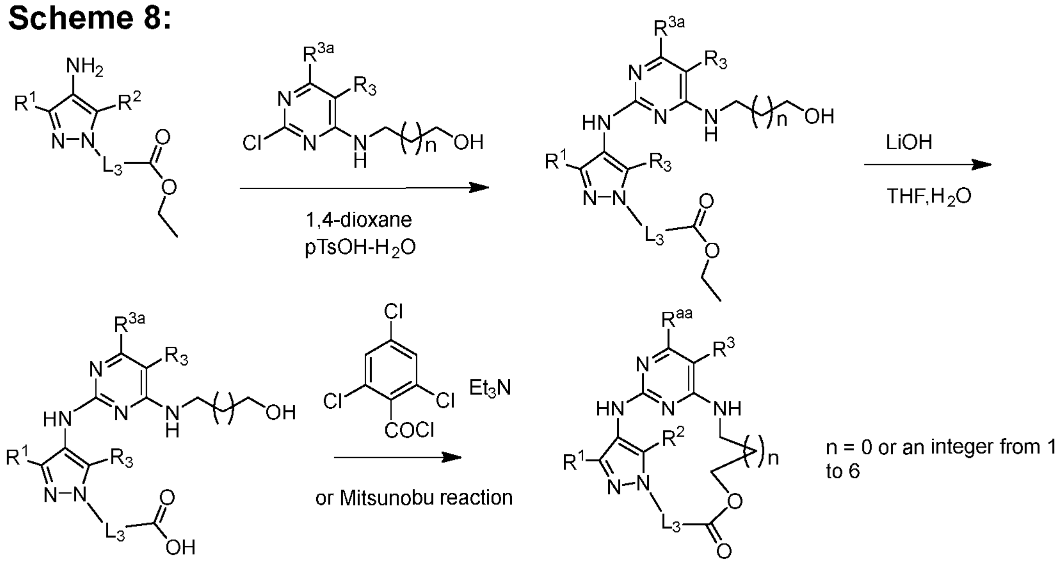


Scheme 9:

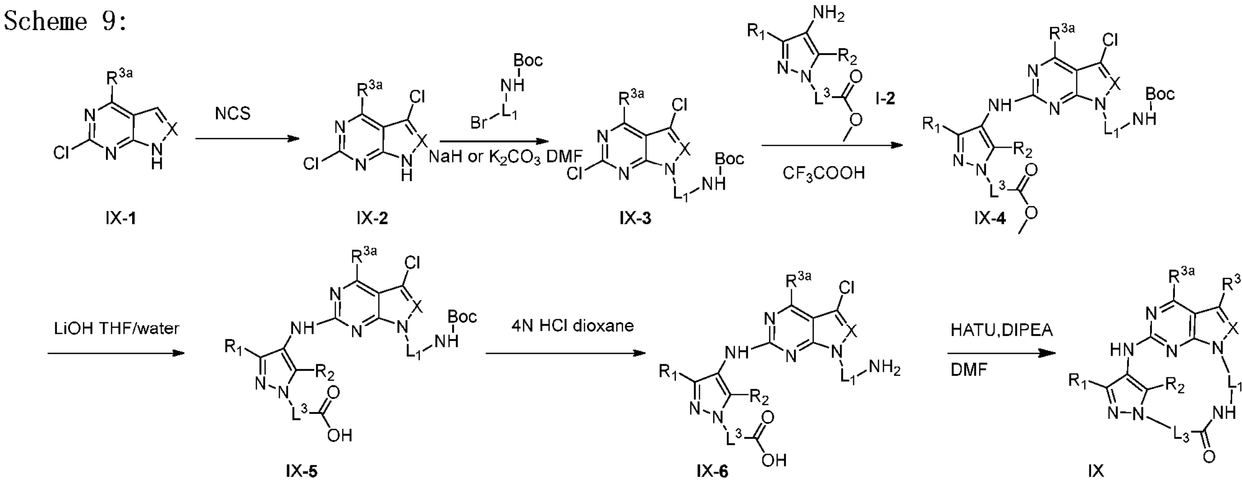


IX-1 IX-2 IX-3 IX-4 IX-5 IX-6 IX

IX-1 is used as the starting material to carry out the chlorination with a chlorination reagent such as NCS, etc., to yield product IX-2, which is then subjected to a substitution reaction with a halogenated amino compound under basic conditions to afford product IX-3. IX-3 undergoes substitution with I-2 under acidic conditions, such as catalysis by $CF_3COOH$, to yield IX-4, which is then subjected to esterolysis and removal of the BOC protecting group under acidic conditions, followed by cyclization reaction using an amide condensation reagent such as HATU to afford the final target product IX.

Scheme 10:

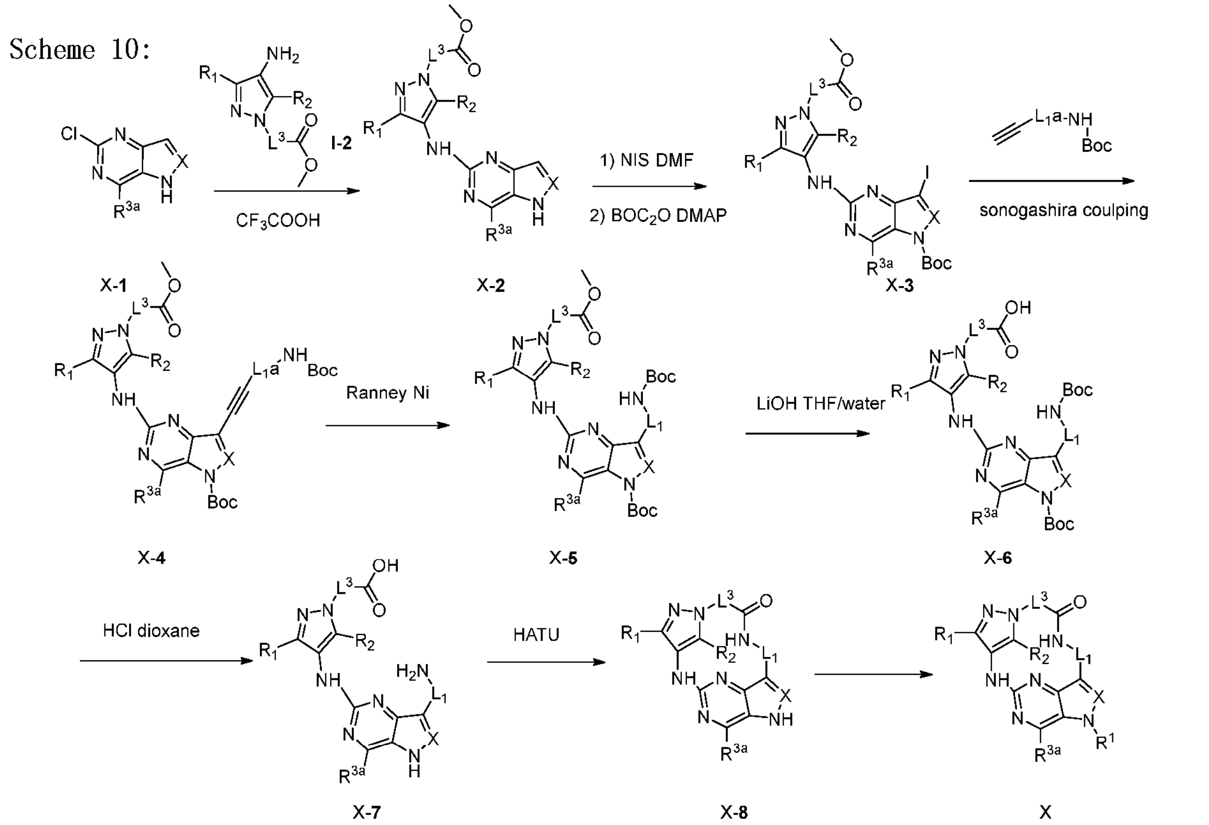


X-1 is used as the starting material and reacted with I-2 under acid catalysis to yield X-2, which is then subjected to halogenation using a suitable halogenation reagent such as NIS or NBS, etc., followed by protection with an appropriate protecting group such as BOC or Ts, etc., to afford X-3. X-3 is subjected to Sonogashira reaction with a suitable alkynylamine to yield X-4, which is then reduced using Raney Ni to afford X-5. X-5 is subjected to esterolysis and removal of protecting group under catalysis of an acid, followed by cyclization reaction using an amide condensation reagent to yield X-8. Substitution is carried out on X-8 under suitable conditions to yield the final product X.

Scheme 11:

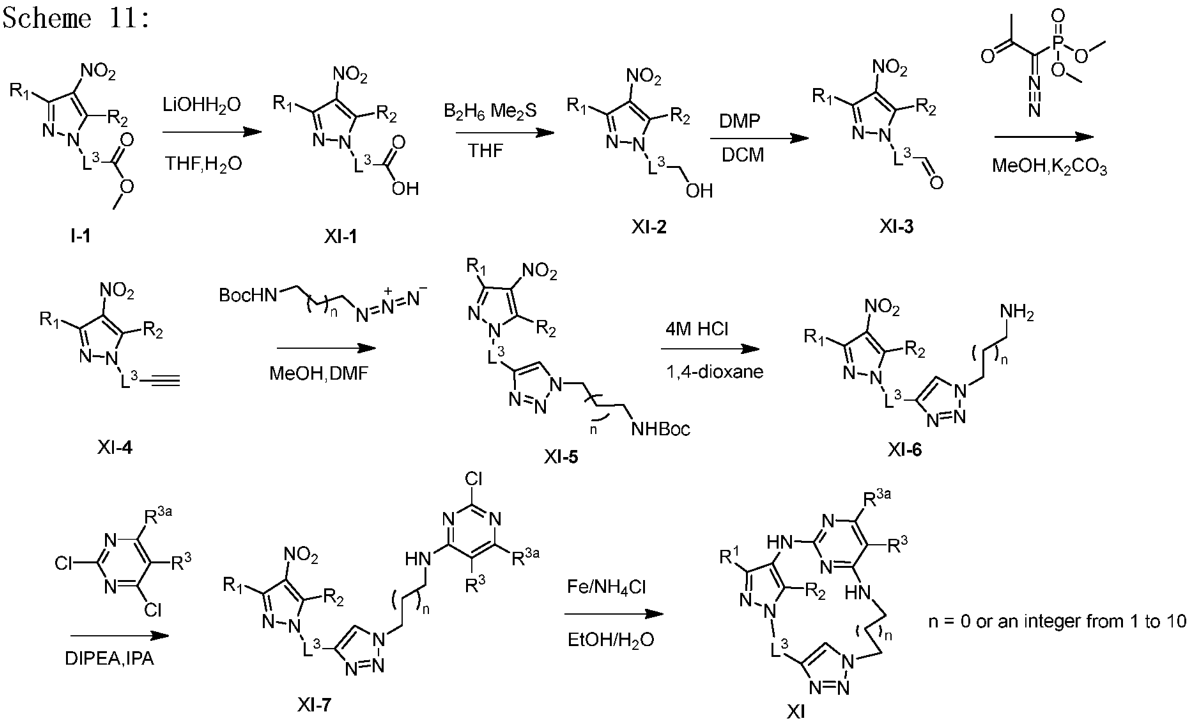

Intermediate I-1 is used as the starting material, and the ester is hydrolyzed under basic conditions (basic conditions including but not limited to $KOH/H_2O$, $NaOH/H_2O$, and $LiOH/H_2O$) to yield XI-1, then the carboxyl group is reduced to an alcohol using a reducing agent such as borane-dimethyl sulfide to yield XI-2, the alcohol is subsequently oxidized using a suitable oxidizing agent (oxidizing condition including but not limited to DMP, PCC, and PDC) to yield aldehyde XI-3. Intermediate XI-3 is alkynylated using a suitable reagent such as the Bestmann-Ohira reagent to yield XI-4, which is then subjected to Click reaction with a suitable azido-substituted Boc amine to afford intermediate XI-5. The Boc protecting group of XI-5 is removed under suitable conditions (including but not limited to TFA/DCM and HCl/1,4-dioxane) to yield XI-6. XI-6 is then reacted with a suitable substituted 2,4-dichloropyrimidine under basic conditions (basic conditions including but not limited to TEA and DIPEA) to yield intermediate XI-7; and finally, a reduction reaction is performed in the presence of a suitable metal (such as but not limited to Zn and Fe) to yield the final product XI through one-step cyclization reaction.

Scheme 12:

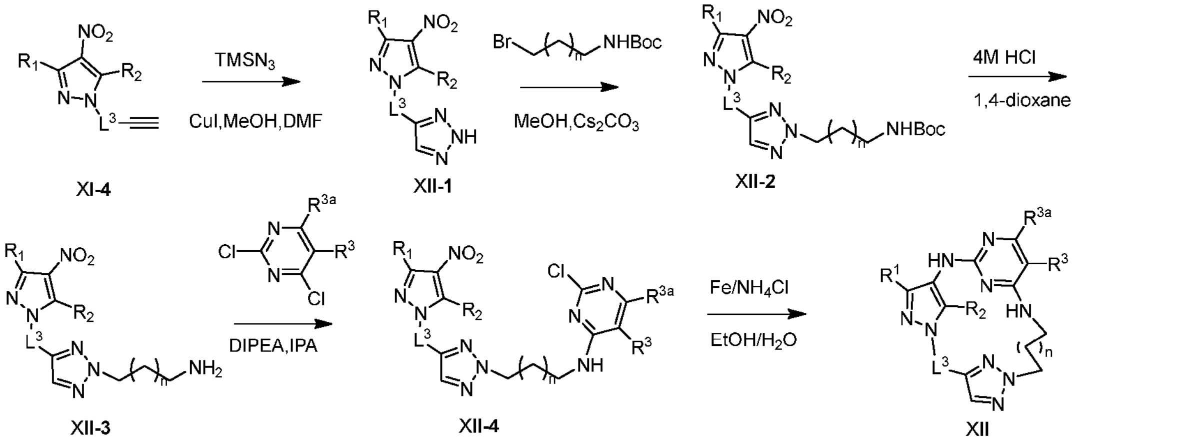


XI-4 XII-1 XII-2 XII-3 XII-4 XII

n = 0 or an integer from 1 to 10

Intermediate XI-4 is used as the starting material to carry out the Click reaction with $TMSN_3$ so as to yield the intermediate XII-1, which is then subjected to a substitution reaction with a suitable bromo-substituted Boc amine under basic conditions such as cesium carbonate to afford XII-2. The Boc protecting group of XII-2 is removed under suitable conditions (including but not limited to TFA/DCM and HCl/1,4-dioxane) to yield XII-3. XII-3 is then reacted with a suitable substituted 2,4-dichloropyrimidine under basic conditions (basic conditions including but not limited to TEA and DIPEA) to yield intermediate XII-4; and finally, a reduction reaction is performed in the presence of a suitable metal (including but not limited to Zn and Fe) to yield the final product XII through one-step cyclization reaction.

Scheme 13:

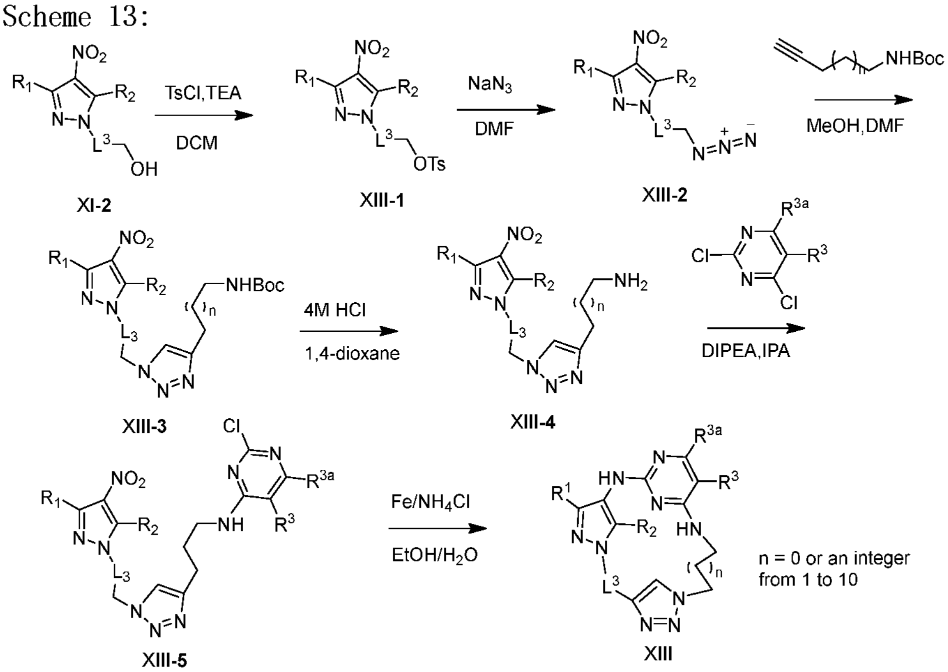


Intermediate XI-2 is used as the starting material to react with TsCl under basic conditions such as TEA to yield XIII-1, which is then reacted with sodium azide to yield intermediate XIII-2. Intermediate XIII-2 is subjected to Click reaction with a suitable Boc-protected aminoalkyne to afford intermediate XIII-3, then the Boc protecting group is removed under suitable conditions (including but not limited to TFA/DCM, and HCl/1,4-dioxane) to yield XIII-4. Intermediate XIII-4 is then reacted with a suitable substituted 2,4-dichloropyrimidine under basic conditions (basic conditions including but not limited to TEA and DIPEA) to yield intermediate XIII-5; and finally, a reduction reaction is performed in the presence of a suitable metal (including but not limited to Zn and Fe) to yield the final product XIII through one-step cyclization reaction.

Scheme 14

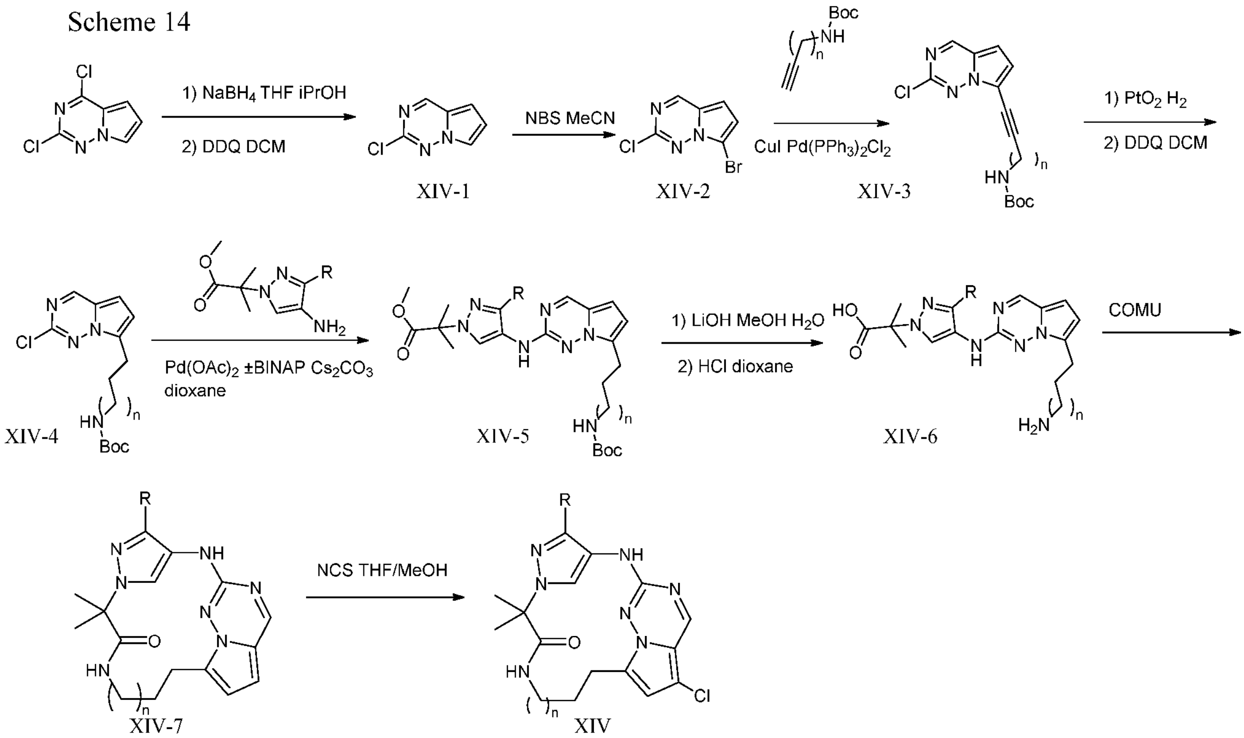


2,4-dichloropyrrolo[2,1-f][1,2,4]triazine is used as the starting material, one halogen is removed by reducing with $NaBH_4$, then halogenation is carried out with a halogenation reagent such as NBS, etc., to yield XIV-2. XIV-2 is subjected to Sonogashira coupling reaction with alkynylamine, and the alkynyl group is then reduced to an alkyl group using $PtO_2$ to

afford XIV-4. XIV-4 is subjected to Buchwald coupling with aminopyrazole to yield XIV-5, which is then subjected to esterolysis and removal of Boc to afford XIV-6. XIV-6 is subjected to condensation using an amide condensation reagent such as COMU, etc., then chlorination is carried out with a halogenation reagent such as NCS, etc., to yield XIV.

In a third aspect, the present disclosure provides use of the compounds of general formula I to general formula XXII described in the first aspect in the:

(i) preparation of a multi-kinase inhibitor for treating neurodegenerative diseases, inflammatory diseases (e.g., IBD), and cancers;
(ii) preparation of a drug for preventing and/or treating LRRK2-mediated diseases, particularly Parkinson's disease;
(iii) preparation of a combination drug for treating tumors, comprising compounds of general formula I to general formula XXII and other antitumor drugs such as PD-1 antibodies, CTLA-4 antibodies, PD-L1 antibodies, PD-L2 antibodies, adoptive cell transfer therapy, cancer vaccines, IDO (indoleamine 2,3-dioxygenase) inhibitors, TDO (tryptophan 2,3-dioxygenase) inhibitors, IDO/TDO dual inhibitors, EP4 antagonists, HDAC (histone deacetylase) inhibitors, STING (stimulator of interferon genes) activators, kinase inhibitors, any other chemotherapeutic or targeted therapeutic drugs, or radiotherapeutic drugs.
(iv) prevention and/or treatment diseases mediated by FGFR, VEGFR, RET, DYRK2, and TYRO3, etc., such as cancers and dyschondroplasia.

It should be understood that, within the scope of the present disclosure, the aforementioned technical features of the present disclosure and the technical features specifically described hereinafter (e.g., in the examples) can be combined with each other to constitute new or preferred technical solutions. Due to space limitations, they will not be elaborately enumerated here.

The following abbreviations have the indicated meanings: BETAC: Benzyltriethylammonium chloride; EA: Ethyl acetate; DBU: 1,8-diazabicyclo[5.4.0]undec-7-ene; DCM: Dichloromethane; DIBAL: Diisobutylaluminum hydride; DIPEA: Diisopropylethylamine; DMAP: N,N-Dimethylaminopyridine; DME: 1,2-Dimethoxyethane; DMF: N,N-Dimethylformamide; DMPE: 1,2-Bis(dimethylphosphino)ethane; DMSO: Dimethyl sulfoxide; DPPB: 1,4-Bis(diphenylphosphino)butane; DPPE: 1,2-Bis(diphenylphosphino)ethane; DPPF: 1,1'-Bis(diphenylphosphino)ferrocene; DPPM: 1,1'-Bis(diphenylphosphino)methane; DIAD: Diisopropyl azodicarboxylate; EDCI: 1-(3-dimethylaminopropyl)-3-ethylcarbodiimide; HATU: O-(7-azabenzotriazol-1-yl)-1,1,3,3-tetramethyluronium hexafluorophosphate; HMPA: Hexamethylphosphoramide; HOAT: N-hydroxy-7-azabenzotriazole; IPA: Isopropyl alcohol; LDA: Lithium diisopropylamide; LHMDS: Lithium Hexamethyldisilazide; LAH: Lithium aluminum hydride; NCS: N-chlorosuccinimide; PE: Petroleum ether; PyBOP: Benzotriazol-1-yl-oxytripyrrolidinophosphonium hexafluorophosphate; TDA: Tris[2-(2-methoxyethoxy)ethyl]amine; DCM: Dichloromethane; TEA: Triethylamine; TFA: Trifluoroacetic acid; THF: Tetrahydrofuran; NCS: N-chlorosuccinimide; NMM: N-methylmorpholine; NMP: N-methylpyrrolidone; $PPh_3$: triphenylphosphine; RT: Room temperature; T3P: Propylphosphonic anhydride; TCFH: N,N,N',N'-tetramethylchloroformamidinium hexafluorophosphate.

Reverse-phase HPLC purification conditions: HPLC-MS analysis was performed on a Waters HPLC 2790 using a Waters micromass ZQ 4000 (Model MAA050) as the mass detector and a Waters 2487 UV as the detector. The chromatographic column used was a Phenomenex OOB-4605-E0 (5U-XB-C18-100A, 50×4.6 mm). The mobile phase consisted of eluent A (water, 0.05% TFA) and eluent B ($CH_3CN$, 0.05% TFA), with an elution rate of 1 mL/min. The initial conditions were 90% A for 1 min, followed by a linear decrease from 90% A to 10% A over 5 min, and then a rise from 10% A back to 90% A over 1 min, with a total running time of 7 min. The gradient of mobile phase and the running time may be adjusted appropriately based on the properties of the compound.

Chiral HPLC separation conditions: Agilent Technologies 1200 Infinity liquid chromatography system was used, and Daicel CHIRALPAK IG (Catalog No.: IG00EE-AT002) was used as the chiral chromatographic column. The mobile phase consisted of eluent A (n-hexane) and eluent B (ethanol), with an elution rate of 3 mL/min, and the elution conditions adopted a constant proportion of ethanol. The type of chiral chromatographic column, proportion of ethanol in the mobile phase, and running time may be adjusted appropriately based on the properties of the compound.

## DETAILED DESCRIPTION OF THE INVENTION

It is easier to understand the content of the present disclosure with reference to the following examples, which are provided to illustrate the present disclosure rather than limiting its scope. Unless otherwise specified, percentages and parts are calculated by weight, with the unit being parts by weight. Unless otherwise specified, the materials and reagents used in the examples of the present disclosure are commercially available products.

Unless otherwise defined, all technical and scientific terms as used herein have the same meanings as commonly understood by those skilled in the art. Furthermore, any methods and materials similar or equivalent to those described herein may be used in the methods of the present disclosure. The preferred implementation methods and materials described herein are for illustrative purposes only.

**Example 1: T-1**

(E)-$3^3$,4,4-Trimethyl-15-(trifluoromethyl)-$3^1$H-2,6,10-triaza-1(2,4)-pyrimidina-3(4,1)-pyrazolacyclodecan-5-one

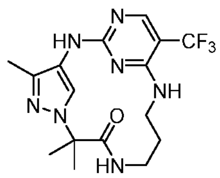

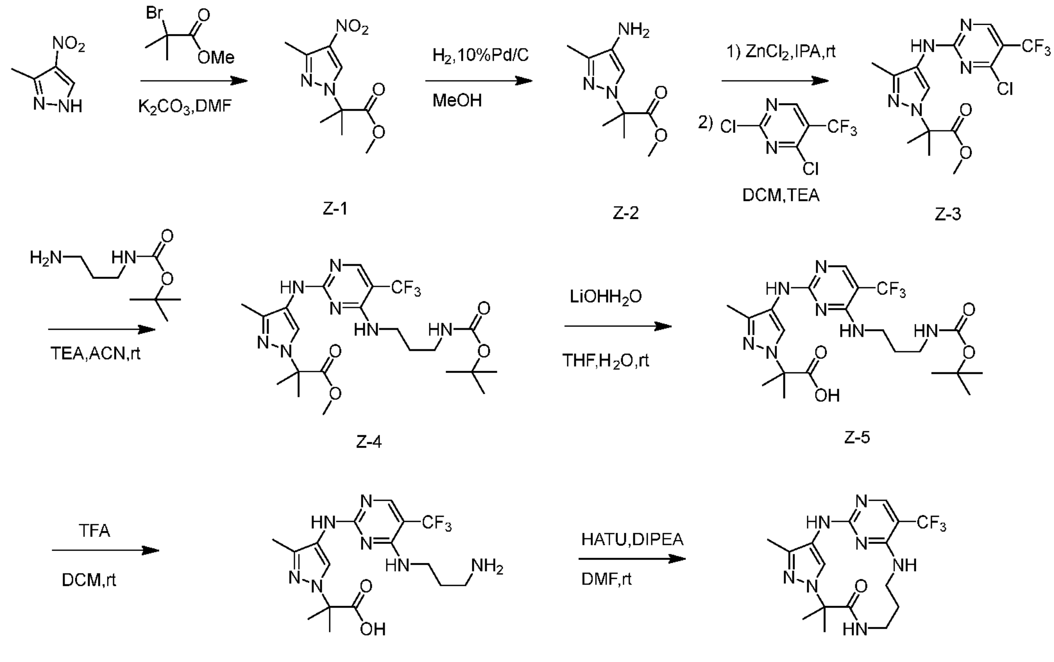


Z-1 Z-2 Z-3 Z-4 Z-5 Z-6 T-1

Step 1: Preparation of Z-1

3-Methyl-4-nitropyrazole (7.34 g, 1.0 eq) was dissolved in DMF, the mixture was held in an ice bath and stirred. Potassium carbonate (12 g, 1.5 eq) was added in portions, and stirred at room temperature for 10 min, methyl 2-bromoisobutyrate (15.68 g, 1.5 eq) was added dropwise in an ice bath, and then the resulting mixture was allowed to react at room temperature for 15-20 h. Upon completion of the reaction, the mixture was quenched with water and extracted with ethyl acetate. The organic phase was washed with saturated brine, dried over anhydrous sodium sulfate, concentrated, separated and purified by flash column chromatography (100% PE - 10% EA:PE) to afford intermediate Z-1 as a colorless oil (12.77 g).

MS ESI: m/z=228.1, $[M+H]^+$.

Step 2: Preparation of Z-2

Intermediate Z-1 (4.62 g) was dissolved in methanol. The system was stirred at room temperature and purged with nitrogen, then 10% Pd/C (454 mg) was added. The system was purged with hydrogen, and allowed to react at room temperature for 18 h. Upon completion of the reaction, the system was purged with nitrogen, and the mixture was filtered through diatomite. The filtrate was concentrated, separated and purified by flash column chromatography (50% EA:PE - 100% EA) to afford intermediate Z-2 as a brown oil (3.92 g).

MS ESI: m/z=198.1, $[M+H]^+$.

Step 3: Preparation of Z-3

Intermediate Z-2 (1.97 g, 1.0 eq) was dissolved in isopropanol (30 mL), and the mixture was stirred at room

temperature, then zinc chloride (1.64 g, 1.2 eq) was added, and the resulting mixture was allowed to react at room temperature for 2 h. 2,4-dichloro-5-(trifluoromethyl)pyrimidine (2.39 g, 1.1 eq) was dissolved in dichloromethane (64 mL), and the mixture was held in an ice bath and stirred. Then, Z-7 system was added dropwise. After the addition was completed, the mixture was reacted in an ice bath for 1 h. Subsequently, triethylamine (1.1 g, 1.1 eq) was added dropwise in an ice bath, and the reaction was held at that temperature and stirred for 1 h. Upon completion of the reaction, the mixture was quenched with 5% sodium bicarbonate solution (48 mL), the system was diluted with dichloromethane, and filtered through diatomite, and then subjected to liquid seperation. The aqueous phase was extracted twice with dichloromethane. The organic phases were combined, dried over anhydrous sodium sulfate, concentrated, separated and purified by flash column chromatography (100% PE - 20% EA:PE) to afford intermediate Z-3 as a cream-colored solid (3.6 g).

MS ESI: m/z=378.1, $[M+H]^+$.

Step 4: Preparation of Z-4

Intermediate Z-3 (1.1 g, 1.0 eq) was dissolved in acetonitrile (20 mL), the mixture was stirred at room temperature, then N-Boc-1,3-propanediamine (608 mg, 1.2 eq) and triethylamine (587 mg, 2 eq) were added, and the resulting mixture was allowed to react at room temperature for 16 h. Upon completion of the reaction, the system was concentrated, separated and purified by flash column chromatography (100% PE - 50% EA:PE) to afford intermediate Z-4 (1.2 g).

MS ESI: m/z=516.1, $[M+H]^+$.

Step 5: Preparation of Z-5

Intermediate Z-4 (270 mg, 1.0 eq) was dissolved in tetrahydrofuran (2 mL), and the mixture was stirred at room temperature, then water (0.5 mL) and lithium hydroxide monohydrate (26.4 mg, 1.2 eq) were added, and the resulting mixture was allowed to react at room temperature for 2 h. Upon completion of the reaction, water and ethyl acetate were added. The pH value of the system was adjusted to 4 with 1M hydrochloric acid, then extracted twice with ethyl acetate, the organic phases were combined, the system was concentrated, separated and purified by flash column chromatography (100% DCM to 20% MeOH:DCM) to afford intermediate Z-5.

Step 6: Preparation of Z-6

Intermediate Z-5 (50 mg, 1.0 eq) was dissolved in dichloromethane (1 mL, 0.1 M), and the mixture was stirred at room temperature, then TFA (205 mg, 18.0 eq) was added, and the resulting mixture was allowed to react at room temperature for 1 h. Upon completion of the reaction, the system was concentrated, and toluene (2 mL) was added and concentrated to afford crude product Z-6, which was directly used in the next cyclization reaction.

Step 7: Preparation of T-1

The crude product Z-6 from Step 7 was dissolved in ultradry DMF (2.5 mL, 0.04 M), and the mixture was stirred at room temperature, then HATU (42 mg, 1.1 eq) and DIPEA (65 mg, 5.0 eq) were added, and the resulting mixture was allowed to react at room temperature for 1 h. Upon completion of the reaction, water and ethyl acetate were added. The system was extracted with ethyl acetate, the organic phase was washed five times with saturated brine, dried over anhydrous sodium sulfate, concentrated, separated and purified by flash column chromatography (100% DCM - 5% MeOH:DCM) to afford T-1 as a white solid (30 mg).

MS ESI: m/z=384.2, $[M+H]^+$.

$^1$H NMR (400 MHz, CDCl3) δ 8.11 (s, 1H), 7.74 (s, 1H), 6.76 (s, 1H), 5.40 (s, 1H), 4.68 (s, 1H), 3.30 (q, J = 7.4 Hz, 2H), 3.16 (q, J = 5.7, 5.3 Hz, 2H), 2.24 (s, 3H), 1.88 (s, 6H), 1.52 - 1.42 (m, 2H).

**Example 2: T-2**

**(E)-3$^3$,4,4-Trimethyl-1$^5$-(trifluoromethyl)-3$^1$H-9-oxa-2,6,13-triaza-1(2,4)-pyrimidina-3(4,1)-pyrazolacyclotridecan-5-one**

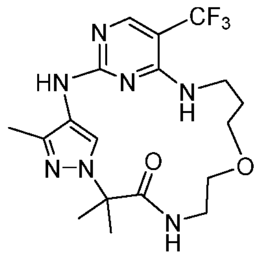

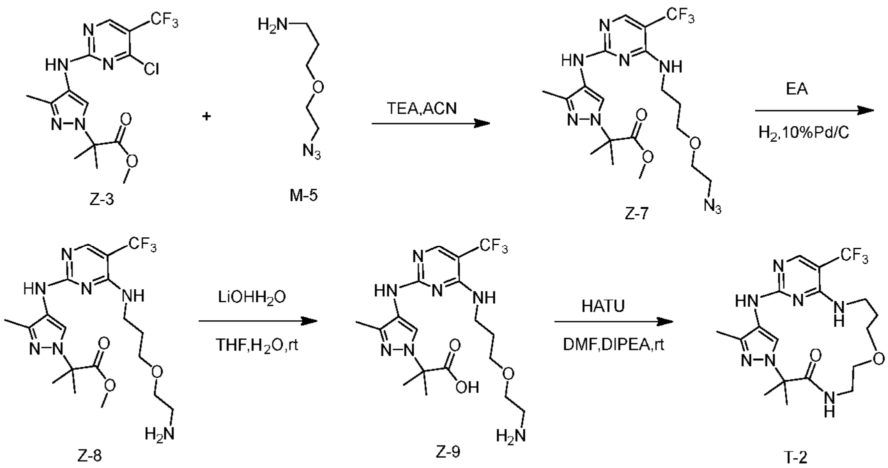


Step 1: Preparation of Z-7

Using M-5 (21 mg, 1.0 eq) and Z-3 (66 mg, 1.2 eq) as raw materials, intermediate Z-7 (52.6 mg) was obtained as a colorless oil under the same conditions as those for the preparation of Z-4 in Step 4 of Example 1.
MS ESI: m/z=486.2, $[M+H]^+$.

Step 2: Preparation of Z-8

Intermediate Z-7 (31 mg) was dissolved in ethyl acetate (1.5 mL). The system was stirred at room temperature and purged with nitrogen, then 10% Pd/C (5 mg) was added. The system was purged with hydrogen, and allowed to react at room temperature for 2 h. Upon completion, the reaction mixture was filtered through diatomite, washed with methanol. The filtrate was concentrated to afford crude product Z-8 (28 mg).
MS ESI: m/z=460.2, $[M+H]^+$.

Step 3: Preparation of Z-9

Using Z-8 (14.4 mg) as raw material, intermediate Z-9 was obtained under the same conditions as those for the preparation of Z-5 in Step 5 of Example 1.
MS ESI: m/z=446.2, $[M+H]^+$.

Step 4: Preparation of T-2

Using crude product Z-9 as raw material, 10 mg of T-2 was obtained as a white solid under the same conditions as those in Step 7 of Example 1.
MS ESI: m/z=428.2, $[M+H]^+$.
$^1$H NMR (400 MHz, $CDCl_3$) δ 8.12 - 8.09 (m, 1H), 7.74 (s, 1H), 6.42 (s, 1H), 5.87 (s, 1H), 5.10 (s, 1H), 3.59 (dt, J = 10.9, 6.1 Hz, 2H), 3.42 (t, J = 5.2 Hz, 4H), 3.37 (t, J = 5.2 Hz, 2H), 2.22 (s, 3H), 1.84 (s, 6H), 1.76 (dq, J = 10.6, 5.5 Hz, 2H).

**Example 3: T-3**

**(±)-($1^{1}$R,$1^{2}$S,$2^{1}$R,E)-$2^{3}$-Cyclopropyl-$4^{5}$-(trifluoromethyl)-$2^{1}$H-3,5,9-triaza-4(2,4)-pyrimidina-2(1,4)-pyrazola-1(1,2)-cyclopropacyclodecan-10-one**

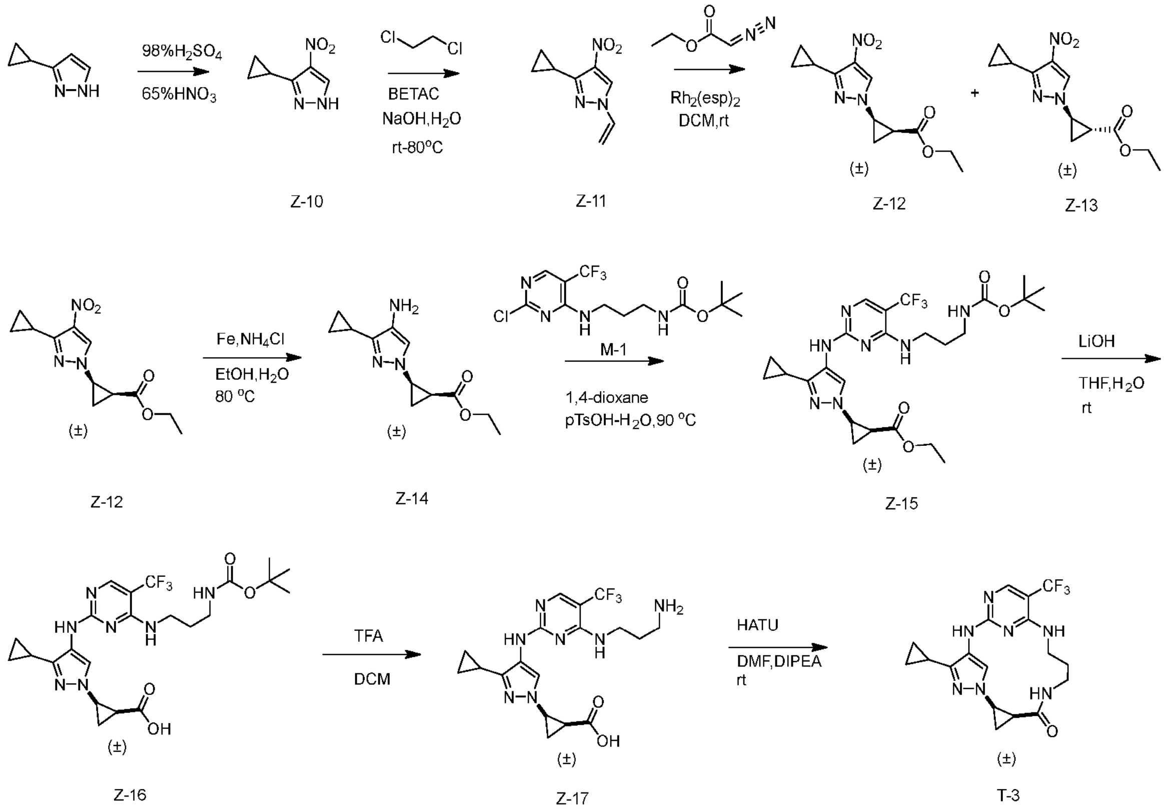


Z-10 Z-11 Z-12 Z-13

Z-12 Z-14 Z-15

Z-16 Z-17 T-3

Step 1: Preparation of Z-10

Concentrated sulfuric acid (29 mL) was placed in a 250 mL single-necked flask, held in an ice bath and stirred. 3-Cyclopropyl-pyrazole (5 g, 1.0 eq) was slowly added dropwise. After completion of addition, the mixture was stirred in the ice bath for 5 min, followed by dropwise addition of concentrated nitric acid (3.5 mL, 1.1 eq). The reaction was maintained in the ice bath for 1 h. Upon completion of the reaction, the system was poured into 300 mL of ice water and extracted three times with ethyl acetate. The organic phases were combined, washed with saturated sodium bicarbonate solution until neutral, then washed with saturated sodium chloride solution, dried over anhydrous sodium sulfate, and concentrated to afford crude product Z-10 as a brown-red solid (5.6 g).

MS ESI: m/z=154.2, $[M+H]^+$.

Step 2: Preparation of Z-11

Z-10 (5.6 g, 1.0 eq) was dissolved in 1,2-dichloroethane (50 mL), and stirred at room temperature. Benzyltriethylammonium chloride (833.7 mg, 0.1 eq) was added, and the mixture was held in an ice bath and stirred. Aqueous NaOH solution (7.3 g, 5.0 eq, 10 mL) was added. The system was purged with nitrogen and allowed to react at 80°C for 8 h, then cooled to room temperature. The reaction mixture was filtered. The filtrate was concentrated, separated and purified by flash column chromatography (100% PE - 20% EA:PE) to afford Z-11 as a white solid (2.6 g).

MS ESI: m/z=180.1, $[M+H]^+$.

Step 3: Preparation of Z-12 and Z-13

Z-11 (2.59 g, 1.0 eq) and bis[(α,α,α',α'-tetramethyl-1,3-phenylenedipropionic acid)rhodium] (110 mg, 0.01 eq) were dissolved in dichloromethane (55 mL), and stirred at room temperature. A solution of ethyl diazoacetate (9.9 g, 6.0 eq) in dichloromethane (16.5 mL) was slowly added dropwise (over about 2 h) under ice bath cooling. After addition, the system was allowed to react at room temperature for 12 h. The reaction mixture was directly concentrated and purified by

flash column chromatography (100% PE - 5% EA:PE - 20% EA:PE) to afford Z-13 as a blue solid (1.53 g, Rf: 0.5, 20% EA:PE) and Z-12 as a brown solid (1.13 g, Rf: 0.3, 20% EA:PE).

MS ESI: m/z=266.1, $[M+H]^+$.

Step 4: Preparation of Z-14

Z-12 (265.3 mg, 1.0 eq) was dissolved in ethanol (5.5 mL), then water (1.5 mL) was added. The mixture was stirred at room temperature, and ammonium chloride (230 mg, 4.3 eq) and reduced iron powder (240 mg, 4.3 eq) were added. The system was allowed to react at 80°C for 2 h, then cooled to room temperature. Upon completion of the reaction, the system was filtered through diatomite and washed with ethanol. The filtrate was concentrated and purified by flash column chromatography (100% DCM - 10% MeOH:DCM) to afford Z-14 (150 mg, as brown oil).

MS ESI: m/z=236.1, $[M+H]^+$.

Step 5: Preparation of Z-15

Z-14 (118 mg, 1.0 eq) was dissolved in 1,4-dioxane (5 mL) and stirred at room temperature, then M-1 (177 mg, 1.0 eq) and p-toluenesulfonic acid monohydrate (28.8 mg, 0.3 eq) were added. The system was purged with nitrogen and allowed to react at 90°C for 2 h, then cooled to room temperature. Upon completion of the reaction, water was added to the system, and the mixture was extracted twice with ethyl acetate. The organic phases were combined and washed with saturated brine, dried over anhydrous sodium sulfate, concentrated, separated and purified by flash column chromatography (100% PE - 70% EA:PE) to afford Z-15 as a pink solid (100 mg).

MS ESI: m/z=554.3, $[M+H]^+$.

Step 6: Preparation of Z-16

Using Z-15 (56 mg) as raw material, intermediate Z-16 was obtained under the same conditions as those for the preparation of Z-5 in Step 5 of Example 1.

MS ESI: m/z=526.2, $[M+H]^+$.

Step 7: Preparation of Z-17

Using Z-16 as raw material, intermediate Z-17 was obtained under the same conditions as those for the preparation of Z-6 in Step 6 of Example 1.

MS ESI: m/z=426.2, $[M+H]^+$.

Step 8: Preparation of T-3

Using Z-17 as raw material, 21 mg of T-3 was obtained as a white solid under the same conditions as those for the preparation of T-1 in Step 7 of Example 1.

MS ESI: m/z=408.2, $[M+H]^+$.

$^1$H NMR (400 MHz, DMSO-d6) δ 9.05 (s, 1H), 8.56 (s, 1H), 8.09 (s, 1H), 7.81 (s, 1H), 7.22 (s, 1H), 3.78 (s, 1H), 3.58 (d, J = 5.0 Hz, 2H), 3.03 (s, 1H), 2.66 (d, J = 12.7 Hz, 1H), 2.15 (s, 2H), 2.06 (q, J = 7.8 Hz, 1H), 1.59 (d, J = 6.4 Hz, 1H), 1.25 (d, J = 5.1 Hz, 1H), 1.12 (s, 1H), 0.85 - 0.61 (m, 4H).

**Examples 4 and 5 (T4 and T5)**

Example 3 (13 mg) was subjected to chiral resolution under the following conditions:

Chiral column: CHIRALPAK IG (5μm; 10 mm ×250 mm);
Mobile phase: 25%EtOH/75% n-hexane;
Flow rate: 2 mL/min;

Example 4:Retention time = 11.1 min (6 mg).

Example 5: Retention time = 15.2 min (4 mg).

**Example 6: T-6**

**(*E*)-$1^5$-Chloro-$3^3$,4,4-trimethyl-$1^7$*H*,$3^1$*H*-2,6-diaza-1(2,7)-pyrrolo[2,3-d]pyrimidine-3(4,1)-pyrazolacyclononan-5-one**

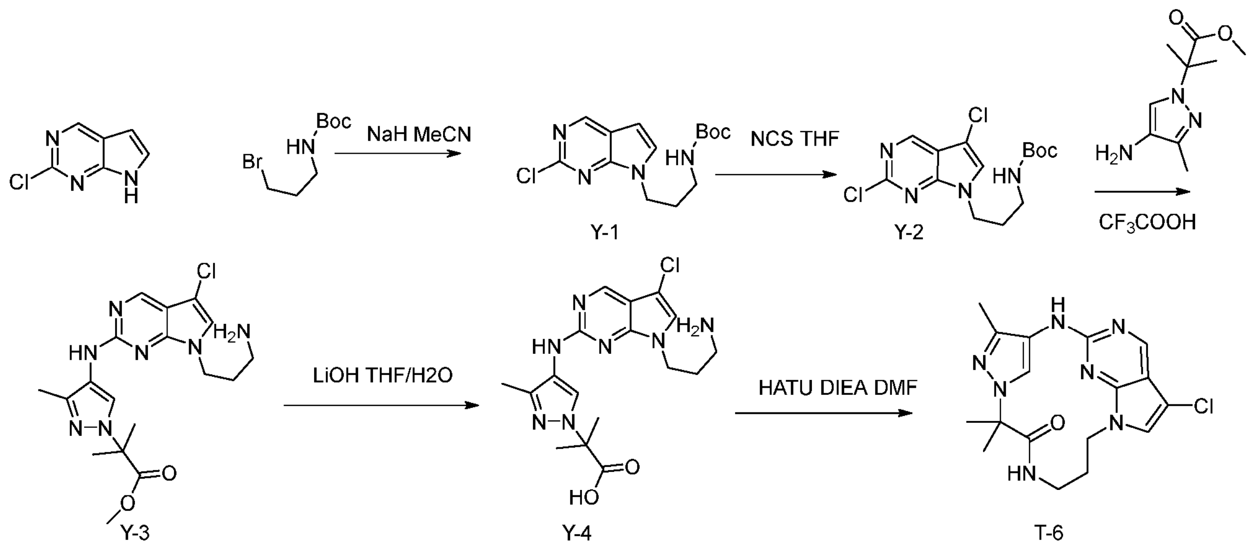


Y-1 Y-2 Y-3 Y-4 T-6

Step 1: Preparation of Y-1

2-Chloro-7H-pyrrolo[2,3-d]pyrimidine (5 g) was dissolved in MeCN (70 mL). NaH (1.56 g, 1.2 eq, 60% in petroleum ether) was added, and the mixture was stirred for 5 min. Tert-butyl (3-bromopropyl)carbamate (7.98 g, 1.02 eq) was then added. After stirring overnight, the reaction was quenched by addition of saturated aqueous $NH_4Cl$ solution. The mixture was extracted with EA, and washed twice with saturated NaCl solution. The organic phase was evaporated using a rotary evaporator to afford the product Y-1 in quantitative yield.

MS ESI: m/z=311, $[M+1]^+$.

Step 2: Preparation of Y-2

Y-1 (6 g) was dissolved in THF (50 mL), then NCS (2.83 g, 1.1 eq) was added. After the mixture was stirred at room temperature for 4 h, the reaction was quenched with saturated $NH_4Cl$ solution, extracted with EA, and then washed three times with saturated NaCl solution. The organic phase was evaporated using a rotary evaporator to afford 4.45 g of Y-2.

MS ESI: m/z=345, $[M+1]^+$.

Step 3: Preparation of Y-3

Y-2 (500 mg) was dissolved in NMP (7 mL), then methyl 2-(4-amino-3-methyl-1H-pyrazol-1-yl)-2-methylpropanoate (342.6 mg, 1.2 eq) and $CF_3COOH$ (0.55 mL, 5 eq) were added. The reaction flask was purged with nitrogen to displace air and heated at 100°C overnight. The reaction was quenched with saturated $NaHCO_3$ solution and extracted with EA. The organic phase was evaporated using a rotary evaporator, and the residue was purified by flash silica gel column chromatography to afford 406 mg of Y-3 as a pale yellow crude product.

MS ESI: m/z=406, $[M+1]^+$.

Step 4: Preparation of Y-4

100 mg of crude product Y-3 was dissolved in THF (2 mL) and water (0.2 mL), then lithium hydroxide monohydrate (54.7 mg, 5 eq) was added.After stirring at room temperature overnight, the reaction mixture was acidified to pH <7 with 2N HCl, evaporated using a rotary evaporator directly and then used for the next step.

MS ESI: m/z=391, $[M+1]^+$.

Step 5: Preparation of T-6

Y-4 obtained from the previous step was dissolved in DMF (3 mL), then HATU (103 mg, 1.1 eq) and DIPEA (0.21 mL, 5 eq) were added. The reaction mixture was stirred at room temperature for 2 h, then quenched with saturated $NH_4Cl$ solution and extracted with EA. The organic phase was evaporated using a rotary evaporator, and the residue was purified by flash silica gel column chromatography and reversed-phase C18 preparative chromatography to afford 12 mg of T-6 as a white solid.
MS ESI: m/z=374, $[M+1]^+$.
$^1H$ NMR (400 MHz, $CDCl_3$) δ 8.49 (s, 1H), 8.42 - 8.20 (bs, 1H), 7.83 (s, 1H), 6.96 (s, 1H), 5.20 (s, 1H), 3.95 - 3.90 (m, 2H), 3.19 (m, 2H), 2.24 (s, 3H), 1.92 (s, 6H), 1.82 (m, 2H).

**Example 7: T-7**

**(*E*)-1$^5$-Chloro-3$^3$,4,4,6-tetramethyl-1$^7$*H*,3$^1$*H*-2,6-diaza-1(2,7)-pyrrolo[2,3-d]pyrimidine-3(4,1)-pyrazolacyclononan-5-one**

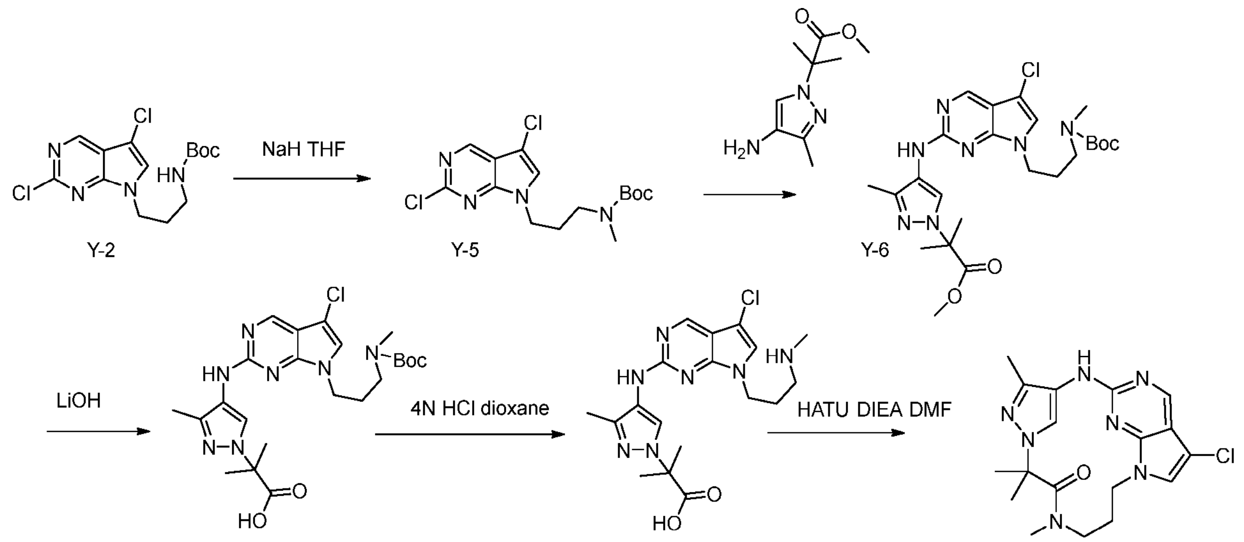


Y-2 Y-5 Y-6

Y-7 Y-8 T-7

Step 1: Preparation of Y-5

Y-2 (200 mg) was dissolved in THF (4 mL), then NaH (34.78 mg, 1.5 eq, 60%) was added. After stirring for several minutes, MeI (43 μL, 1.2 eq) was added. After stirring overnight, additional NaH (60 mg, 60%) and MeI (50 μL) were added. The reaction mixture was stirred for 6 h, then quenched with saturated $NH_4Cl$ solution and extracted with EA. The organic phase was evaporated using a rotary evaporator, and the residue was purified by flash silica gel column chromatography to afford 153 mg of Y-5 as a colorless oil.
MS ESI: m/z=359, $[M+1]^+$.

Step 2: Preparation of Y-6

Y-5 (153 mg) was dissolved in n-BuOH (3 mL), then methyl 2-(4-amino-3-methyl-1H-pyrazol-1-yl)-2-methylpropanoate (100 mg, 1.2 eq) and $CF_3COOH$ (0.16 mL, 5eq) were added. The reaction flask was purged with nitrogen to displace air and heated at 100°C for 4 h. The reaction was quenched with saturated $NaHCO_3$ solution and extracted with EA.The organic phase was evaporated using a rotary evaporator, and the residue was purified by flash silica gel column chromatography to afford 34mg of Y-6.
MS ESI: m/z=520, $[M+1]^+$.

Step 3: Preparation of Y-7

34 mg of Y-6 was dissolved in THF (2 mL) and water (0.2 mL), then lithium hydroxide monohydrate (13.7 mg, 5 eq) was added. After stirring at room temperature overnight, the reaction mixture was diluted with a small amount of saturated NaCl solution, then acidified to pH <7 with 2N HCl, and extracted with EA. The organic phase was evaporated using a rotary evaporator to afford a solid, which was used directly in the next step.
MS ESI: m/z=506, $[M+1]^+$.

Step 4: Preparation of Y-8

To Y-7 obtained from the previous step, HCl/dioxane solution (2 mL, 4N) was added. The mixture was stirred at room temperature for 2 h., then evaporated using a rotary evaporator. The resulting solid was directly used in the next step.
MS ESI: m/z=406, $[M+1]^+$.

Step 5: Preparation of T-7

Using Y-8 obtained from the previous step as raw material, T-7 (15 mg) was obtained as a white solid under the same conditions as those in Step 5 of Example 6.
MS ESI: m/z=388, $[M+1]^+$.
$^1H$ NMR (400 MHz, $CDCl_3$) δ 8.47 (s, 1H), 8.40 (bs, 1H), 8.13 (s, 1H), 6.96 (s, 1H), 4.65 - 4.52 (m, 1H), 4.23 (m, 1H), 3.94 (m, 1H), 2.55 (m, 1H), 2.36 (s, 3H), 2.33 (s, 3H), 2.00 (m, 1H), 1.98 (s, 3H), 1.80 (s, 3H), 1.73 (m, 1H).

**Example 8: T-8**

**(*E*)-1$^3$-Chloro-3$^3$,4,4-trimethyl-1$^1$*H*,3$^1$*H*-2,6-diaza-1(6,1)pyrazolo[3,4-d]pyrimidine-3(4,1)-pyrazolacyclononan-5-one**

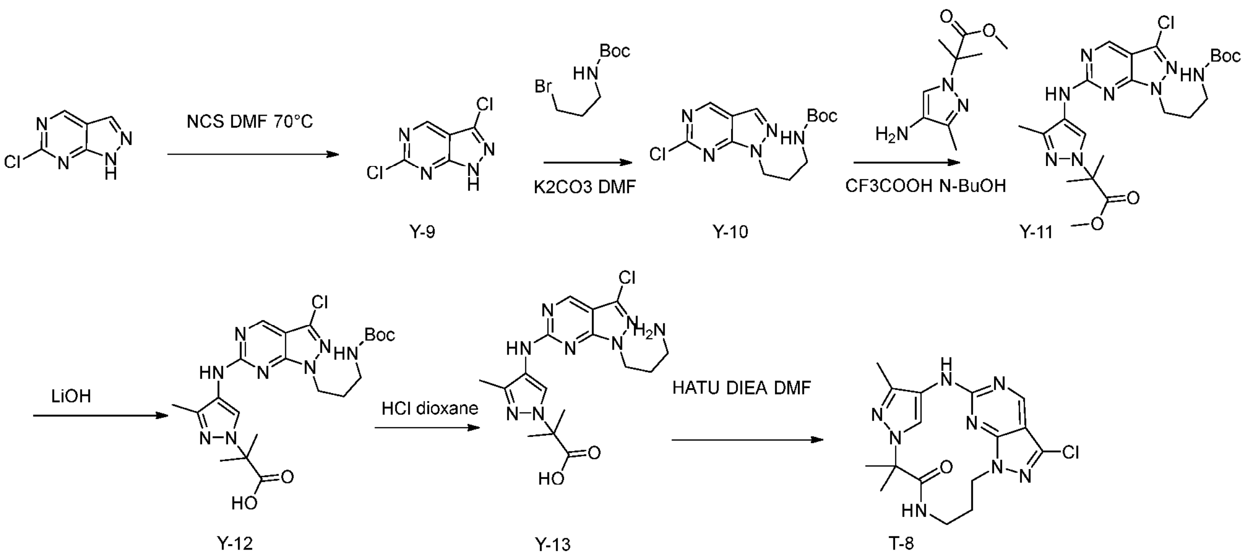

Y-9
Y-10
Y-11
Y-12
Y-13
T-8

Step 1: Preparation of Y-9

6-Chloro-1H-pyrazolo[3,4-d]pyrimidine (2 g) was dissolved in DMF (30 mL), then NCS (1.91 g, 1.1 eq) was added. The reaction flask was purged with nitrogen to displace air and heated at 70°C overnight. The reaction was quenched with saturated $NH_4Cl$ solution and extracted with EA. The organic phase was washed twice with saturated NaCl solution, then evaporated using a rotary evaporator, and the residue was purified by flash silica gel column chromatography to afford 1.37g of Y-9.
MS ESI: m/z=189, $[M+1]^+$.

Step 2: Preparation of Y-10

Y-9 (1.37 g) was dissolved in DMF (20 mL), then $K_2CO_3$ (3.34 mg, 2 eq) and tert-butyl (3-bromopropyl)carbamate (2.07 g, 1.2 eq) were added. After stirring at room temperature overnight, the reaction was quenched with saturated $NH_4Cl$ solution and extracted with EA.The organic phase was washed twice with saturated NaCl solution, then evaporated using a rotary evaporator, and the residue was purified by flash silica gel column chromatography to afford 1.26g of Y-10 as a white solid.

MS ESI: m/z=346, $[M+1]^+$.

Step 3: Preparation of Y-11

Y-10 (200 mg) was dissolved in n-BuOH (4 mL), then methyl 2-(4-amino-3-methyl-1H-pyrazol-1-yl)-2-methylpropanoate (160 mg, 1.4 eq) and $CF_3COOH$ (0.22 mL, 5 eq) were added. The reaction flask was purged with nitrogen to displace air, heated at 100°C overnight. The mixture was adjusted to basic pH with saturated $NaHCO_3$ solution, then THF (4 mL) and $BOC_2O$ (1.2 eq, 0.16 mL) were added. After stirring for 3 h, the mixture was extracted with EA. The organic phase was evaporated using a rotary evaporator, and the residue was purified by flash silica gel column chromatography to afford 270 mg of Y-11 as a light yellow solid.

MS ESI: m/z=507, $[M+1]^+$.

Step 4: Preparation of Y-12

Using Y-11 (270 mg) as raw material, Y-12 was obtained under the same conditions as those in Step 3 of Example 7.

MS ESI: m/z=493, $[M+1]^+$.

Step 5: Preparation of Y-13

Using Y-12 obtained from the previous step as raw material, Y-13 was obtained under the same conditions as those in Step 4 of Example 7.

MS ESI: m/z=393, $[M+1]^+$.

Step 6: Preparation of T-8

Using Y-13 obtained from the previous step as raw material, 23mg of T-8 was obtained as a white solid under the same conditions as those in Step 5 of Example 6.

MS ESI: m/z=375, $[M+1]^+$.

$^1H$ NMR (400 MHz, $CDCl_3$) δ 8.74 (s, 1H), 7.89 (s, 1H), 7.13 (s, 1H), 5.55 (s, 1H), 4.15 (t, J = 7.1 Hz, 2H), 3.16 (m, 2H), 2.23 (s, 3H), 1.93 (s, 6H), 1.91 (m, 2H).

**Example 9: T-9**

**(*E*)-$3^3$,4,4-Trimethyl-$1^1H$,$3^1H$-2,6-diaza-1(5,3)pyrazolo[4,3-d]pyrimidine-3(4,1)-pyrazolacyclononan-5-one**

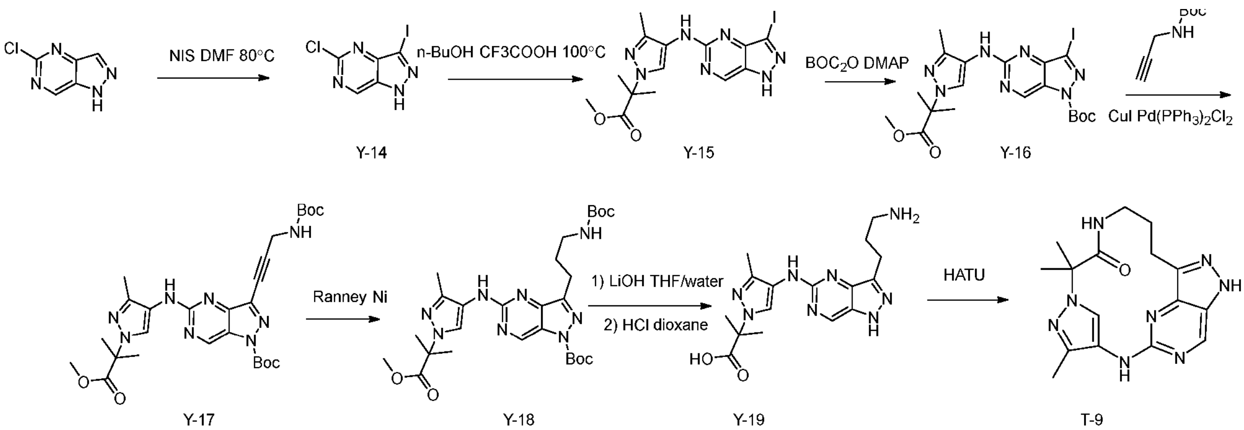


Y-14 Y-15 Y-16 Y-17 Y-18 Y-19 T-9

Step 1: Preparation of Y-14

Using 5-chloro-1H-pyrazolo[4,3-d]pyrimidine (1.5 g) and NIS (4.38 mg, 2 eq) as raw materials, the reaction temperature was set to 80°C, and 2.97 g of Y-14 was obtained as a light yellow crude product under the same procedure as those in Step 2 of Example 8.

MS ESI: m/z=281, $[M+1]^+$.

Step 2: Preparation of Y-15

Using Y-14 (300 mg) as raw material, 300 mg of Y-15 was obtained as a yellow solid under the same conditions as those in Step 3 of Example 8.

MS ESI: m/z=442, $[M+1]^+$.

Step 3: Preparation of Y-16

Y-15 (300 mg) was dissolved in THF (3 mL), then $BOC_2O$ (0.17 mL, 1.1 eq), DMAP (8.3 mg, 0.1 eq), and $NEt_3$ (0.14 mL, 1.5 eq) were added. After stirring at room temperature for 20 min, the reaction mixture was evaporated using a rotary evaporator and used directly in the next step.

MS ESI: m/z=542, $[M+1]^+$.

Step 4: Preparation of Y-17

The crude product (320 mg) obtained from the previous step was dissolved in DMF (3 mL) and $NEt_3$ (3 mL), then N-Boc-aminopropyne (110 mg, 1.2 eq), $Pd(PPh_3)_2Cl_2$ (41.5 mg, 0.1 eq), and CuI (22.5 mg, 0.2 eq) were added. The reaction flask was purged with nitrogen to displace air and heated at 80°C for 1 h. The reaction was quenched with saturated $NH_4Cl$ solution and extracted with EA. The organic phase was evaporated using a rotary evaporator, and the residue was purified by flash silica gel column chromatography to afford 267 mg of Y-17 as a yellow crude product.

MS ESI: m/z=569, $[M+1]^+$.

Step 5: Preparation of Y-18

Y-17 (267 mg) was dissolved in a mixed solution of methanol (9 mL) and EA (5 mL), then an appropriate amount of Ranney Ni was added. The mixture was reduced with hydrogen at standard atmospheric pressure for 16 h, then filtered, evaporated using a rotary evaporator, and purified by flash silica gel column chromatography to afford 118 mg of Y-18 as a yellow solid.

MS ESI: m/z=573, $[M+1]^+$.

Step 6: Preparation of Y-19

Using Y-18 (118 mg) as raw material, Y-19 was obtained under the same conditions as those in Steps 3 and 4 of Example 7.

MS ESI: m/z=359, $[M+1]^+$.

Step 7: Preparation of T-9

Y-19 obtained from the previous step was dissolved in DMF (5 mL), then HATU (121.8 mg, 1.5 eq) and DIPEA (0.18 mL, 5 eq) were added. The mixture was stirred at room temperature for 2 h, then treated with 4 mL of 2N NaOH solution and stirred for 1 h. After dilution with $NaHCO_3$ solution, the mixture was extracted with EA. The organic phase was evaporated using a rotary evaporator, and the residue was purified by flash silica gel column chromatography and reverse-phase C18 preparative chromatography to afford 28 mg of T-9 as a light yellow solid.
MS ESI: m/z=341, $[M+1]^+$.
$^1H$ NMR (400 MHz, $CDCl_3$) δ 8.85 (s, 1H), 7.93 (s, 1H), 6.71 (s, 1H), 5.88 (s, 1H), 3.20 (m, 2H), 2.91 (t, J = 7.1 Hz, 2H), 2.22 (s, 3H), 1.95 (s, 6H), 1.81 (m, 2H).

**Example 10: T-10**

**($1^1$R,$1^2$S,$2^1$R,E)-$2^3$-Cyclopropyl-9-methyl-$4^5$-(trifluoromethyl)-$2^1$*H*-3,5,9-triaza-4(2,4)-pyrimidina-2(1,4)-pyrazola-1(1,2)-cyclopropacyclodecan-10-one**

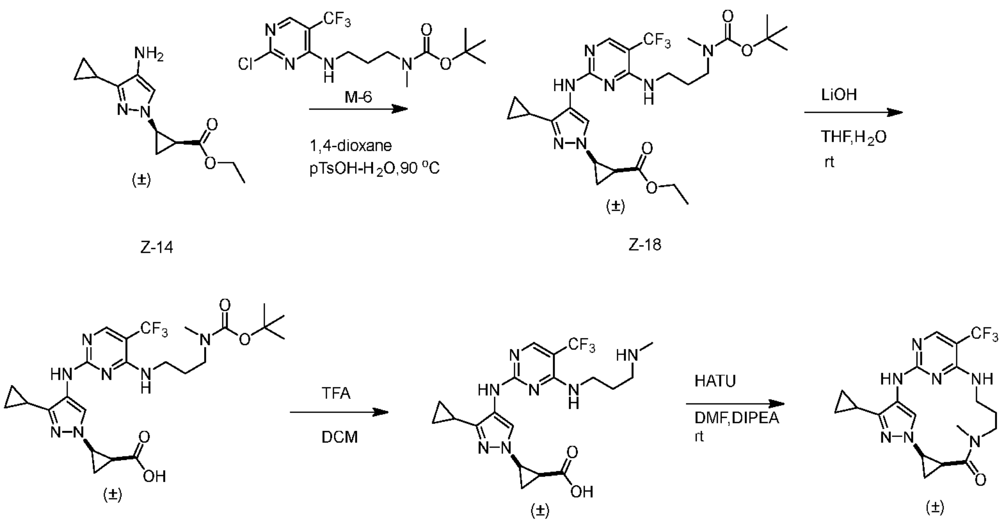


(±) Z-14 (±) Z-18 (±) Z-19 (±) Z-20 (±) T-10

Step 1: Preparation of Z-18

Using Z-14 (100 mg, 1 eq) and M-6 (160 mg, 1 eq) as starting materials, intermediate Z-18 (70 mg) was obtained under the same conditions as those for the preparation of Z-15 in Step 5 of Example 3. MS ESI: m/z=568.2, $[M+H]^+$.

Step 2: Preparation of Z-19

Using Z-18 (70 mg) as raw material, intermediate Z-19 was obtained under the same conditions as those for the preparation of Z-5 in Step 5 of Example 1.
MS ESI: m/z=540.2, $[M+H]^+$.

Step 3: Preparation of Z-20

Using Z-19 as raw material, intermediate Z-20 was obtained under the same conditions as those for the preparation of Z-6 in Step 6 of Example 1.
MS ESI: m/z=440.2, $[M+H]^+$.

Step 4: Preparation of T-10

Using Z-20 as raw material, 15 mg of T-10 was obtained as a white solid under the same conditions as those for the preparation of T-1 in Step 7 of Example 1.

MS ESI: m/z=422.2, $[M+H]^+$.

$^1H$ NMR (400 MHz, Chloroform-d) δ 8.08 (s, 1H), 7.54 (s, 1H), 7.13 (s, 1H), 5.51 (s, 1H), 4.29 - 4.19 (m, 1H), 3.87 (td, J = 7.8, 5.2 Hz, 1H), 3.79 (dt, J = 13.2, 7.3 Hz, 1H), 3.47 (m, J = 7.1, 3.3 Hz, 1H), 3.31 (s, 3H), 2.74 - 2.62 (m, 1H), 2.45 - 2.34 (m, 1H), 2.23 (q, J = 7.9 Hz, 1H), 2.02 (q, J = 5.9 Hz, 1H), 1.70 (ddd, J = 10.3, 8.0, 5.5 Hz, 1H), 1.58 - 1.42 (m, 2H), 0.90 (dd, J = 7.4, 3.4 Hz, 3H), 0.82 - 0.74 (m, 1H).

### Example 11: T-11

**($1^1$R,$1^2$R,$2^1$S,E)-$2^3$-Cyclopropyl-$4^5$-(trifluoromethyl)-$2^1$*H*-3,5,9-triaza-4(2,4)-pyrimidina-2(1,4)-pyrazola-1(1,2)-cyclopropacyclodecan-10-one**

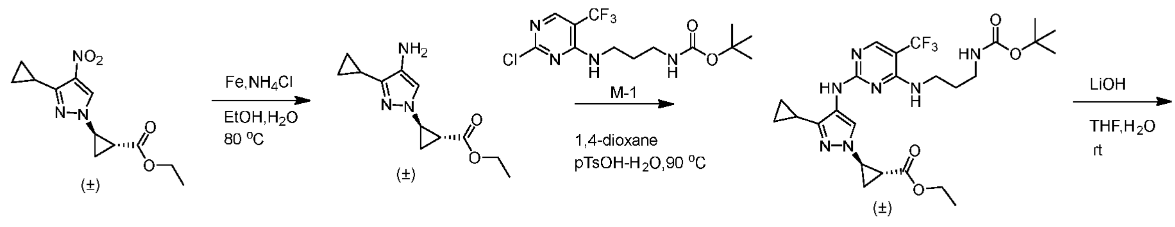


Z-13 Z-21 Z-22

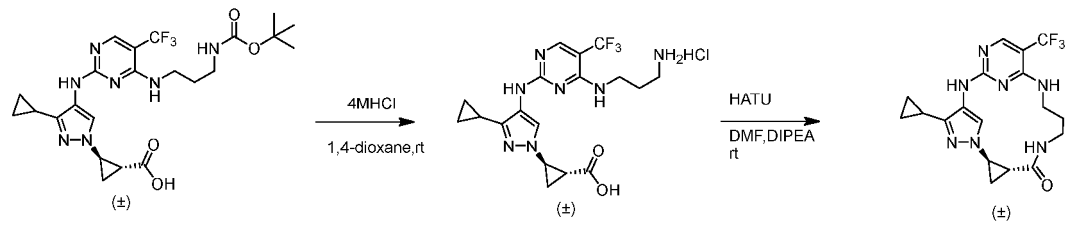


Z-23 Z-24 T-11

Step 1: Preparation of Z-21

Using Z-13 (265 mg) as starting material, intermediate Z-21 (170 mg) was obtained under the same conditions as those for the preparation of Z-14 in Step 4 of Example 3.

MS ESI: m/z=236.1, $[M+H]^+$.

Step 2: Preparation of Z-22

Using Z-21 (47 mg, 1 eq) and M-1 (70.8 mg, 1 eq) as starting materials, intermediate Z-22 (71 mg) was obtained under the same conditions as those for the preparation of Z-15 in Step 5 of Example 3. MS ESI: m/z=554.3, $[M+H]^+$.

Step 3: Preparation of Z-23

Using Z-22 (55 mg) as raw material, intermediate Z-23 was obtained under the same conditions as those for the preparation of Z-5 in Step 5 of Example 1.

MS ESI: m/z=526.2, $[M+H]^+$.

Step 4: Preparation of Z-24

Using Z-23 (0.1 mmol) as raw material, 4M hydrogen chloride in dioxane solution (2 mL) was added, and the mixture was stirred at room temperature for 2 h. Upon completion of the reaction, the system was directly evaporated using a rotary evaporator to afford intermediate Z-24, which was directly used in the next cyclization reaction.

MS ESI: m/z=426.2, $[M+H]^+$.

Step 5: Preparation of T-11

Using Z-24 (0.1 mmol) as raw material, 10 mg of T-11 was obtained as a white solid under the same conditions as

those for the preparation of T-1 in Step 7 of Example 1, followed by purification via reverse-phase C18 preparative chromatography.

MS ESI: m/z=408.2, $[M+H]^+$.

$^1H$ NMR (400 MHz, DMSO-d6) δ 9.31 (s, 0.20H), 9.03 (s, 0.66H), 8.34 (s, 0.18H), 8.14 (s, 0.22H), 8.10 (d, J = 5.6 Hz, 1.28H), 7.86 (d, J = 9.1 Hz, 0.21H), 7.67 - 7.58 (m, 0.76H), 7.14 (t, J = 5.8 Hz, 0.72H), 6.93 (d, J = 8.2 Hz, 0.21H), 3.61 (dt, J = 7.7, 3.8 Hz, 0.96H), 3.51 (s, 0.96H), 2.89 (qd, J = 12.5, 6.7 Hz, 2H), 2.06 (ddd, J = 9.4, 6.0, 2.8 Hz, 1.06H), 1.98 (m, J = 12.9, 7.6, 6.1 Hz, 1.81H), 1.83 - 1.59 (m, 2H), 1.55 - 1.46 (m, 1.13H), 1.24 (s, 0.98H), 0.86 - 0.66 (m,3.54H).

**Example 12: T-12**

**(E)-$3^3$,4,4-Trimethyl-$1^5$-(trifluoromethyl)-$3^1$*H*-2,6,9-triaza-1(2,4)-pyrimidina-3(4,1)-pyrazolacyclononan-5-one**

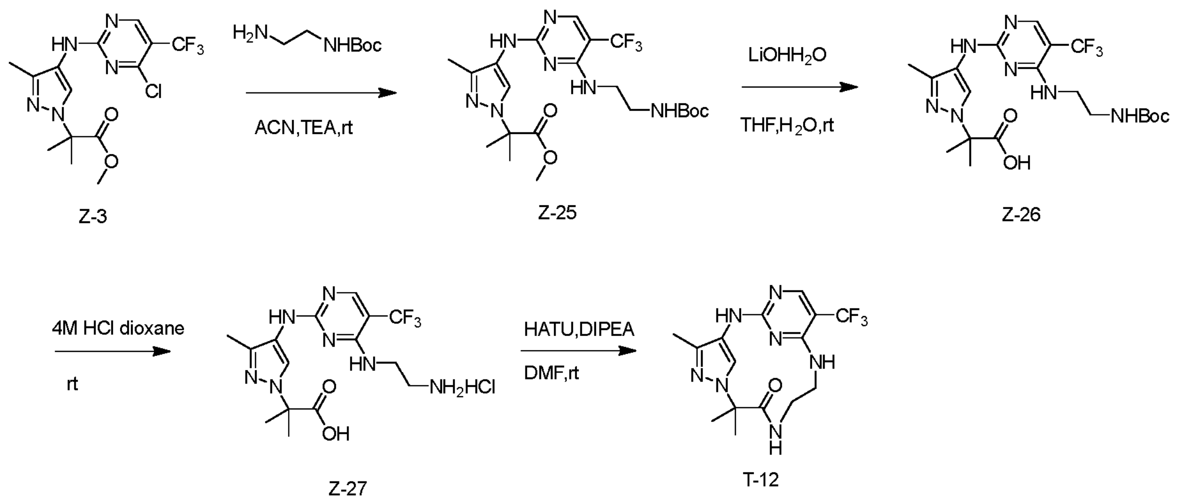


Z-3 Z-25 Z-26 Z-27 T-12

Step 1: Preparation of Z-25

Using Z-3 (189 mg, 1 eq) and mono-Boc ethylenediamine (120 mg, 1.5 eq) as raw materials, intermediate Z-25 (244 mg) was obtained as a pale yellow oil under the same conditions as those for the preparation of Z-4 in Step 4 of Example 1.

MS ESI: m/z=502.2, $[M+H]^+$.

Step 2: Preparation of Z-26

Using Z-25 (200 mg, 1 eq) as raw material, intermediate Z-26 (189 mg) was obtained as a white solid under the same conditions as those for the preparation of Z-5 in Step 5 of Example 1.

MS ESI: m/z=488.2, $[M+H]^+$.

Step 3: Preparation of Z-27

Using Z-26 (0.38 mmol) as raw material, intermediate Z-27 was obtained under the same conditions as those for the preparation of Z-24 in Step 4 of Example 11.

MS ESI: m/z=388.2, $[M+H]^+$.

Step 4: Preparation of T-12

Using Z-27 (0.38 mmol) as raw material, 24 mg of T-12 was obtained as a white solid under the same conditions as those for the preparation of T-1 in Step 7 of Example 1, followed by purification via reverse-phase C18 preparative chromatography.

MS ESI: m/z=370.2, $[M+H]^+$.

$^1H$ NMR (400 MHz, Chloroform-d) δ 8.14 (s, 1H), 7.71 (s, 1H), 6.55 (s, 1H), 6.14 (s, 1H), 5.43 (s, 1H), 3.33 (s, 4H), 2.25 (s, 3H), 1.89 (s, 6H).

**Example 13: T-13**

**(E)-3$^3$,4,4-Trimethyl-1$^5$-(trifluoromethyl)-3$^1$*H*-2,6,11-triaza-1(2,4)-pyrimidina-3(4,1)-pyrazolacycloundecan-5-one**

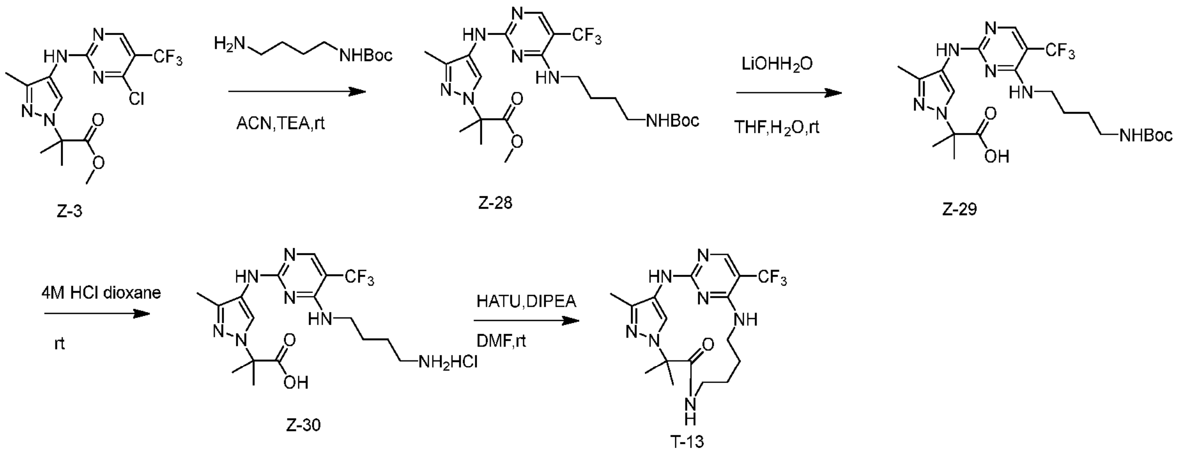


Z-3 Z-28 Z-29 Z-30 T-13

Step 1: Preparation of Z-28

Using Z-3 (189 mg, 1 eq) and 1,4-Boc diaminobutane (141 mg, 1.5 eq) as raw materials, intermediate Z-28 (263 mg) was obtained as a pale yellow oil under the same conditions as those for the preparation of Z-4 in Step 4 of Example 1.

MS ESI: m/z=530.2, $[M+H]^+$.

Step 2: Preparation of Z-29

Using Z-28 (212 mg, 1 eq) as raw material, intermediate Z-29 (208 mg) was obtained as a white solid under the same conditions as those for the preparation of Z-5 in Step 5 of Example 1.

MS ESI: m/z=516.2, $[M+H]^+$.

Step 3: Preparation of Z-30

Using Z-29 (0.38 mmol) as raw material, intermediate Z-30 was obtained under the same conditions as those for the preparation of Z-24 in Step 4 of Example 11.

MS ESI: m/z=416.2, $[M+H]^+$.

Step 4: Preparation of T-13

Using Z-30 (0.38 mmol) as raw material, 27.5 mg of T-13 was obtained as a white solid under the same conditions as those for the preparation of T-1 in Step 7 of Example 1, followed by purification via reverse-phase C18 preparative chromatography.

MS ESI: m/z=398.2, $[M+H]^+$.

$^1$H NMR (400 MHz, Chloroform-*d*) δ 8.09 (s, 1H), 7.64 (s, 1H), 6.51 (s, 1H), 5.38 (s, 1H), 5.33 (s, 1H), 3.14 (h, *J* = 5.3 Hz, 4H), 2.24 (s, 2H), 1.85 (s, 5H), 1.58 (t, *J* = 8.5 Hz, 2H), 1.47 (t, *J* = 6.1 Hz, 2H).

**Example 14: T-14**

**(E)-3$^3$-Methyl-1$^5$-(trifluoromethyl)-3$^1$H-2,6,10-triaza-1(2,4)-pyrimidina-3(4,1)-pyrazolacyclodecan-5-one**

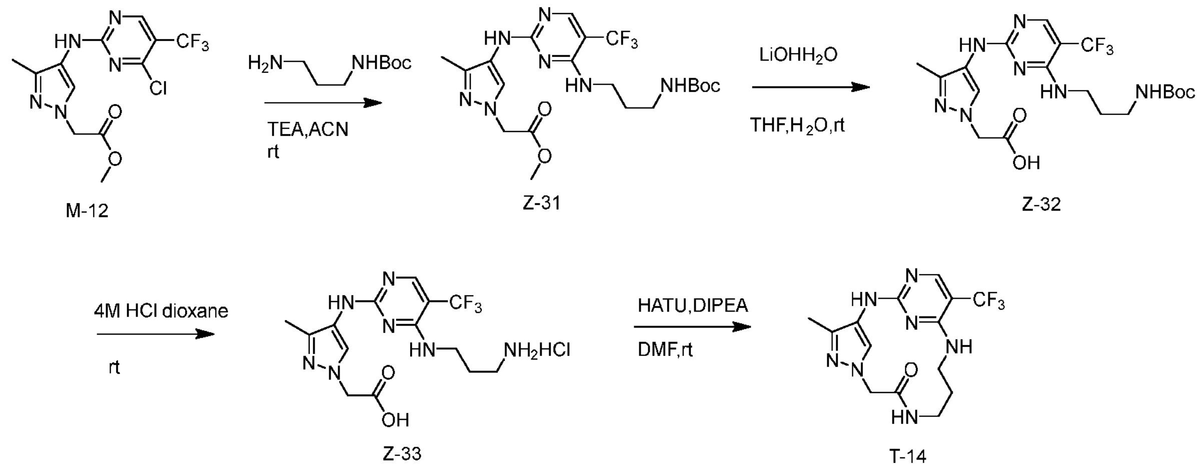


M-12 Z-31 Z-32 Z-33 T-14

Step 1: Preparation of Z-31

Using intermediate M-12 (105 mg, 1 eq) and 1,3-mono-Boc-diaminopropane (78.4 mg, 1.5 eq) as raw materials, intermediate Z-31 was obtained under the same conditions as those for the preparation of Z-4 in Step 4 of Example 1.
MS ESI: m/z=488.2, $[M+H]^+$.

Step 2: Preparation of Z-32

Using Z-31 (0.3 mmol) as raw material, intermediate Z-32 was obtained as a white solid under the same conditions as those for the preparation of Z-5 in Step 5 of Example 1.
MS ESI: m/z=474.2, $[M+H]^+$.

Step 3: Preparation of Z-33

Using Z-32 (0.3 mmol) as raw material, intermediate Z-33 was obtained under the same conditions as those for the preparation of Z-24 in Step 4 of Example 11.
MS ESI: m/z=374.2, $[M+H]^+$.

Step 4: Preparation of T-14

Using Z-33 (0.3 mmol) as raw material, 16.4 mg of T-14 was obtained as a white solid under the same conditions as those for the preparation of T-1 in Step 7 of Example 1.
MS ESI: m/z=356.1, $[M+H]^+$.
$^1$H NMR (400 MHz, Chloroform-d) δ 8.14 - 8.09 (m, 1H), 7.60 (s, 1H), 6.56 (s, 1H), 5.41 (s, 1H), 4.81 (s, 3H), 3.33 (q, J = 7.4 Hz, 2H), 3.21 (q, J = 5.1 Hz, 2H), 2.23 (s, 3H), 1.25 (s, 2H).

**Example 15: T-15**

**(E)-3$^3$-Methyl-1$^5$-(trifluoromethyl)-3$^1$H-2,6,9-triaza-1(2,4)-pyrimidina-3(4,1)-pyrazolacyclononan-5-one**

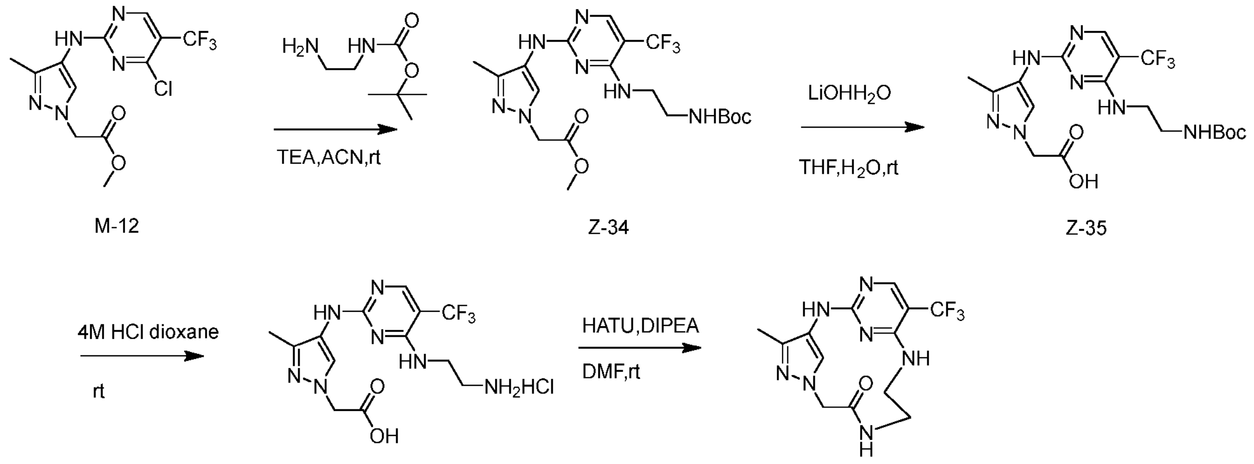


M-12 Z-34 Z-35

Z-36 T-15

Step 1: Preparation of Z-34

Using intermediate M-12 (105 mg, 1 eq) and mono-Boc-ethylenediamine (72.2 mg, 1.5 eq) as raw materials, intermediate Z-34 was obtained under the same conditions as those for the preparation of Z-4 in Step 4 of Example 1.
MS ESI: m/z=474.2, $[M+H]^+$.

Step 2: Preparation of Z-35

Using Z-34 (0.3 mmol) as raw material, intermediate Z-35 was obtained as a white solid under the same conditions as those for the preparation of Z-5 in Step 5 of Example 1.
MS ESI: m/z=460.2, $[M+H]^+$.

Step 3: Preparation of Z-36

Using Z-35 (0.3 mmol) as raw material, intermediate Z-36 was obtained under the same conditions as those for the preparation of Z-24 in Step 4 of Example 11.
MS ESI: m/z=360.2, $[M+H]^+$.

Step 4: Preparation of T-15

Using Z-36 (0.3 mmol) as raw material, 5.4 mg of T-15 was obtained as a white solid under the same conditions as those for the preparation of T-1 in Step 7 of Example 1.
MS ESI: m/z=342.1, $[M+H]^+$.
$^1$H NMR (400 MHz, Chloroform-d) δ 8.15 (s, 1H), 7.54 (s, 1H), 6.53 (s, 1H), 6.48 (s, 1H), 5.46 (s, 1H), 4.77 (d, *J* = 23.7 Hz, 2H), 3.63 (s, 1H), 3.35 (s, 2H), 2.97 (s, 1H), 2.25 (s, 3H).

**Example 16: T-16**

**($3^4$E,$5^4$Z)-$3^3$,4,4-Trimethyl-$1^5$-(trifluoromethyl)-$3^1$H,$5^1$H-2,9-diaza-1(2,4)-pyrimidina-5(4,1)-triazole-3(4,1)-pyrazolacyclononane**

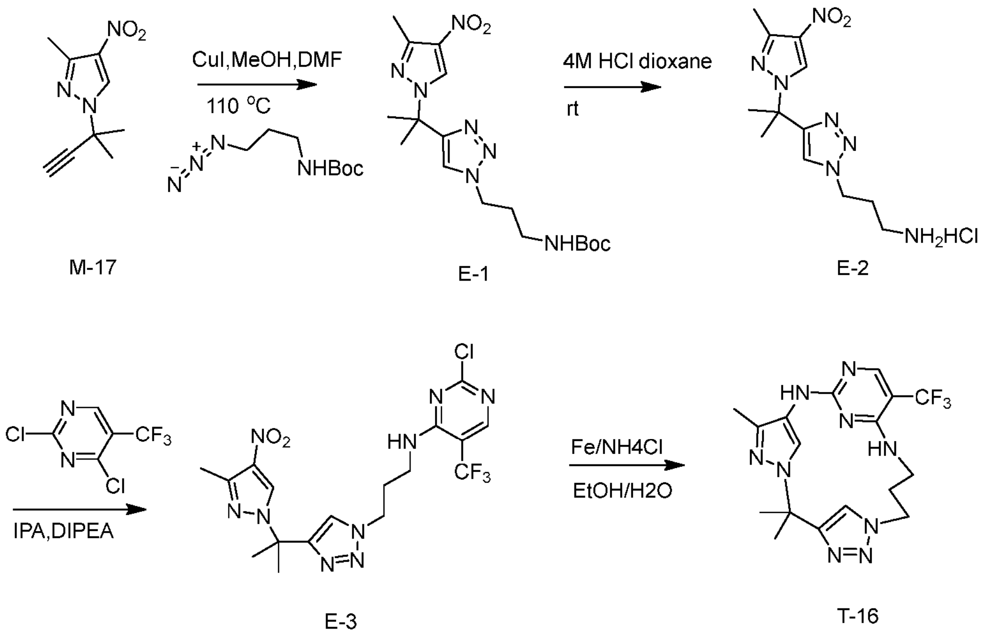


M-17 E-1 E-2 E-3 T-16

Step 1: Preparation of E-1

Intermediate M-17 (193 mg, 1.0 eq) was dissolved in MeOH (0.5 mL) and DMF (4 mL), then N-BOC-3-azidopropylamine (300 mg, 1.5 eq) and CuI (19 mg, 0.12 eq) were added sequentially. The system was purged with nitrogen and allowed to react at 110°C for 16 h. Upon completion of the reaction, the mixture was cooled to room temperature, then treated with sodium chloride solution and ethyl acetate, and dried over anhydrous sodium sulfate. The organic phase was concentrated, then separated and purified by flash column chromatography (100% PE-50% EA/PE) to afford E-1 (350 mg) as a yellow oil.

MS ESI: m/z=394.1, $[M+H]^+$.

Step 2: Preparation of E-2

Using intermediate E-1 (0.657 mmol) as raw material, intermediate E-2 was obtained as a white solid under the same conditions as those for the preparation of Z-24 in Step 4 of Example 11.

MS ESI: m/z=294.1, $[M+H]^+$.

Step 3: Preparation of E-3

Intermediate E-2 (0.657 mmol, 1.0 eq) was dissolved in isopropanol (5 mL), then DIPEA (340 mg, 4 eq) was added. The mixture was stirred at room temperature until a clear solution was obtained. Under ice-bath cooling, a solution of 2,4-dichloro-5-(trifluoromethyl)pyrimidine (157 mg, 1.1 equiv) in isopropanol (2 mL) was added dropwise to the system. After addition, the system was allowed to react at room temperature for 1-2 h. Upon completion of the reaction, the system was concentrated, then separated and purified by flash column chromatography (100% PE-50% EA/PE) to afford intermediate E-3 (140 mg) as a pale yellow oil.

MS ESI: m/z=474.1, $[M+H]^+$.

Step 4:Preparation of T-16

Intermediate E-3 (70 mg, 1.0 eq) was dissolved in ethanol (4 mL), then water (1.5 mL) was added. The mixture was stirred at room temperature, and ammonium chloride (144 mg, 18 eq) and reduced iron powder (75 mg, 9 eq) were added. The system was purged with nitrogen and allowed to react at 90°C for 12 h, then cooled to room temperature. Upon completion of the reaction, the system was filtered through diatomite and washed with ethyl acetate. Sodium chloride solution and sodium bicarbonate solution were added, and the aqueous phase was extracted twice with ethyl acetate, then dried over anhydrous sodium sulfate. The organic phase was concentrated, then separated and purified by flash column chromatography (100% PE-100% EA) to afford T-16 (40 mg) as a white solid.

MS ESI: m/z=408.1, $[M+H]^+$.

$^1$H NMR (400 MHz, Chloroform-*d*) δ 8.08 (s, 1H), 7.34 (s, 1H), 7.17 (s, 1H), 6.58 (s, 1H), 5.20 (s, 1H), 4.58 - 4.48 (m, 2H), 3.33 - 3.20 (m, 2H), 2.26 (s, 3H), 2.07 (s, 6H), 2.05 (s, 2H).

**Example 17: T-17**

**($3^4$E,$5^3$Z)-$3^3$,4,4-Trimethyl-$1^5$-(trifluoromethyl)-$3^1$*H*,$5^2$*H*-2,9-diaza-1(2,4)-pyrimidina-5(4,2)-triazole-3(4,1)-pyrazolacyclononane**

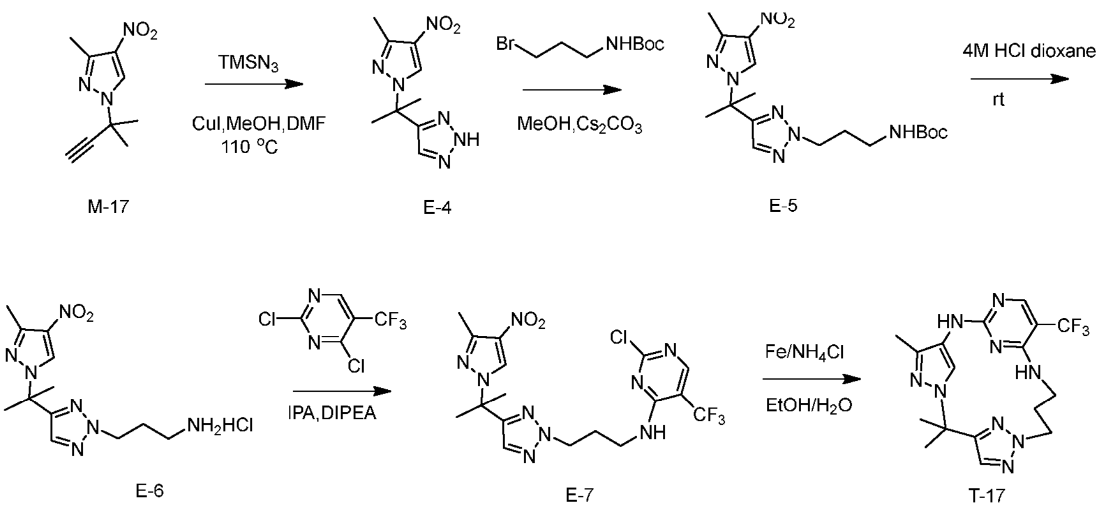


M-17 E-4 E-5

E-6 E-7 T-17

Step 1: Preparation of E-4

TMSN$_3$ (172.8 mg, 1.5 eq) was dissolved in MeOH (0.5 mL) and DMF (4 mL), then intermediate M-17 (193 mg, 1.0 eq) and CuI (19 mg, 0.12 eq) were added sequentially. The system was purged with nitrogen and allowed to react at 110°C for 16 h. Upon completion of the reaction, the system was cooled to room temperature and poured into 20 g of ice. Ethyl acetate was added. The mixture was filtered through diatomite, and the aqueous phase was extracted twice with EA. The organic phase was washed with saturated sodium chloride and dried over anhydrous sodium sulfate. The organic phase was concentrated, then separated and purified by flash column chromatography (100% PE-50% EA/PE) to afford E-4 (159 mg) as a yellow oil.
MS ESI: m/z=237.1, [M+H]$^+$.

Step 2: Preparation of E-5

Intermediate E-4 (159 mg, 1.0 eq) was dissolved in methanol (3.5 mL), the mixture was stirred at room temperature, then N-Boc-3-aminopropylbromide (797.6 mg, 5.0 eq) and Cs$_2$CO$_3$ (873 mg, 4.0 eq) were added. The system was purged with nitrogenand allowed to react at 50°C for 36 h. Upon completion of the reaction, the mixture was cooled to room temperature and filtered through diatomite, the organic phase was concentrated, and separated and purified by flash column chromatography (100% PE - 50% EA/PE) to afford E-5 (160 mg).
MS ESI: m/z=394.2, [M+H]$^+$.

Step 3: Preparation of E-6

Using intermediate E-5 (0.4 mmol) as raw material, intermediate E-6 was obtained as a yellow oil under the same conditions as those for the preparation of Z-24 in Step 4 of Example 11.
MS ESI: m/z=294.1, [M+H]$^+$.

Step 4: Preparation of E-7

Using intermediate E-6 (0.4 mmol) as raw material, intermediate E-7 (75 mg) was obtained as a pale yellow oil under the same conditions as those for the preparation of E-3 in Step 3 of Example 16.
MS ESI: m/z=474.1, [M+H]$^+$.

Step 5: Preparation of T-17

Using Z-7 (0.07 mmol) as raw material, T-17 (10 mg) was obtained as a white solid under the same conditions as those for the preparation of T-16 in Step 4 of Example 16, followed by purification via reverse-phase C18 preparative chromatography.
MS ESI: m/z=408.1, [M+H]+. $^1$H NMR (400 MHz, Chloroform-d) δ 8.06 (s, 1H), 7.60 (s, 1H), 7.57 (s, 1H), 6.80 (s, 1H), 5.26 (s, 1H), 4.65 - 4.57 (m, 2H), 3.41 (dd, *J* = 17.3, 6.6 Hz, 2H), 2.26 (s, 3H), 2.24 - 2.16 (m, 2H), 2.02 (s, 6H).

**Example 18: T-18**

**(*E*)-3$^3$,4,4-Trimethyl-1$^5$H,3$^1$H-2,6-diaza-1(2,7)-pyrrolo[3,2-*d*]pyrimidine-3(4,1)-pyrazolacyclononan-5-one**

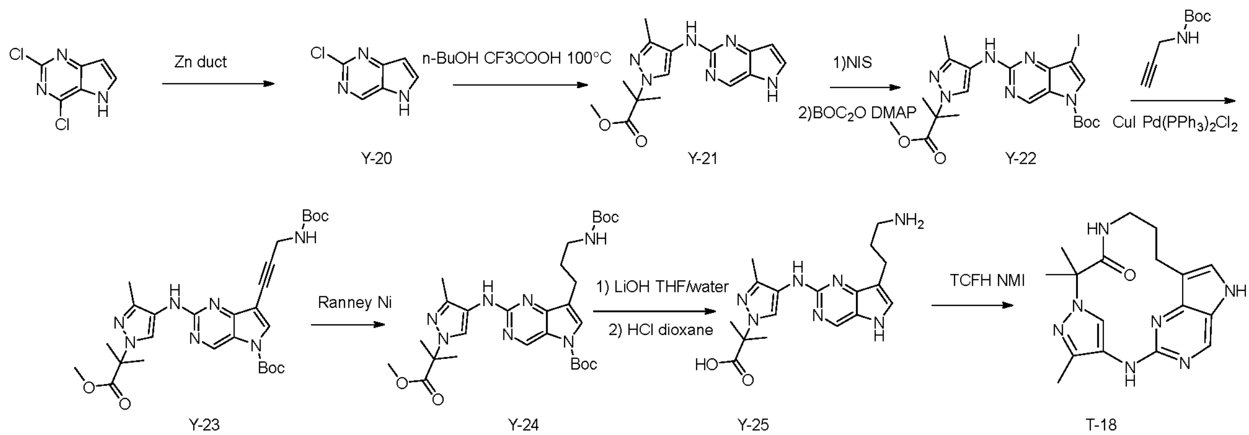

Y-20 Y-21 Y-22
Y-23 Y-24 Y-25 T-18

Step 1: Preparation of Y-20

2,4-Dichloropyrrolo[3,2-D]pyrimidine (6.7 g) was dissolved in MeOH (100 mL), then zinc powder (9.32 g) and $CH_3COOH$ (13.45 mL) were added. The reaction was heated at 70°C for approximately 5 h until completion. The reaction mixture was evaporated using a rotary evaporator, and the residue was purified by flash silica gel column chromatography to afford 3.37g of Y-20 as a white solid.
MS ESI: m/z=154, [M+H]+.

Step 2: Preparation of Y-21

Using Y-20 (1 g) as raw material, 1.17 g of Y-21 was obtained as a crude product under the same conditions as those in Step 2 of Example 9.
MS ESI: m/z=315, [M+H]+.

Step 3: Preparation of Y-22

Crude product Y-21 (1.17 g) was dissolved in DMF (30 mL), then NIS (1617.6 mg) was added. After stirring for 30 min, the reaction was completed. The reaction solution was quenched with saturated aqueous $Na_2S_2O_4$ solution and then extracted with EA. The organic phase was washed with saturated NaCl solution, then evaporated using a rotary evaporator, and the residue was purified by flash silica gel column chromatography to afford the intermediate compound. The intermediate was dissolved in THF (15 mL), then $Boc_2O$ (1.03 mL), DMAP (45.47 mg), and $NEt_3$ (0.77 mL) were added. After stirring for approximately 30 min, the reaction mixture was evaporated using a rotary evaporator and then purified by silica gel column chromatography to afford 1.27 g of Y-22.
MS ESI: m/z=541, [M+H]+.

Step 4: Preparation of Y-23

Using Y-22 (1.27 g) as raw material, 1.57 g of Y-23 was obtained under the same conditions as those in Step 4 of Example 9.

MS ESI: m/z=567, $[M+H]^+$.

Step 5: Preparation of Y-24

Using Y-23 (1.57g) as raw material, 720 mg of Y-24 was obtained under the same conditions as those in Step 5 of Example 9.

MS ESI: m/z=572, $[M+H]^+$.

Step 6: Preparation of Y-25

Using Y-24 (720 mg) as raw material, 450 mg of Y-25 was obtained under the same conditions as those in Step 6 of Example 9.

MS ESI: m/z=358, $[M+H]^+$.

Step 7: Preparation of T-18

Y-25 (720 mg) was dissolved in MeCN (20 mL), then TCFH (424 mg) and NMI (0.5 mL) were added. After stirring at room temperature for 2 h, the reaction was completed. The reaction mixture was quenched with saturated aqueous $NH_4Cl$ solution and then extracted with EA. The organic phase was evaporated using a rotary evaporator, and the residue was purified by flash silica gel column chromatography to afford 100mg of T-18.

MS ESI: m/z=340, $[M+H]^+$.

$^1H$ NMR (400 MHz, $CDCl_3$) δ 8.95 (s, 1H), 8.64 (s, 1H), 7.94 (s, 1H), 7.39 (s, 1H), 7.01 (s, 1H), 5.78 (s, 1H), 3.14 (m, 2H), 2.63 (m, 2H), 2.23 (s, 3H), 1.94 (s, 6H), 1.66 (m, 2H).

**Example 19: T-19 and T-20**

**(*E*)-$1^1$,$3^3$,4,4-Tetramethyl-$1^1$*H*,$3^1$*H*-2,6-diaza-1(5,3)-pyrazolo[4,3-d]pyrimidine-3(4,1)-pyrazinacyclononan-5-one (T-19)**

**($1^3$*E*,$1^4$*Z*,$3^4$*E*)-$1^2$,$3^3$,4,4-Tetramethyl-$1^2$H,$3^1$H-2,6-diaza-1(5,3)-pyrazolo[4,3-d]pyrimidine-3(4,1)-pyrazinacyclononan-5-one (T-20)**

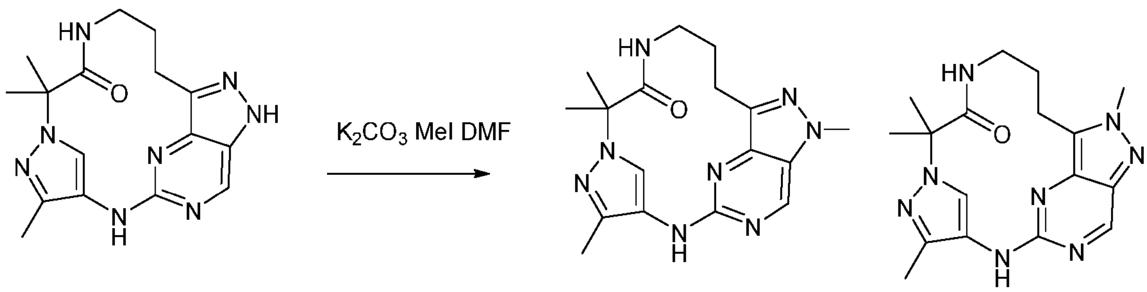


T-9 T-19 T-20

T-9 (30 mg) was dissolved in DMF (2 mL) under an ice bath, then $K_2CO_3$ (14.6 mg) and MeI (8 μL) were added. After stirring at room temperature overnight, the reaction mixture was quenched with saturated aqueous $NH_4Cl$ solution, and extracted with EA. The organic phase was evaporated using a rotary evaporator, and the residue was purified by flash silica gel column chromatography and reversed-phase C18 preparative chromatography to afford 5.2 mg of T-19 and 6.7 mg of T-20.

MS ESI: m/z=355, $[M+H]^+$.

$^1H$ NMR (400 MHz, $CDCl_3$) δ 8.73 (s, 1H), 7.92 (s, 1H), 6.55 (s, 1H), 5.95 (s, 1H), 4.03 (s, 3H), 3.17 (m, 2H), 2.87 (m, 2H), 2.21 (s, 3H), 1.84 (s, 6H), 1.79 (m, 2H). (T-19)

$^1H$ NMR (400 MHz, $CDCl_3$) δ 9.07 (s, 1H), 7.95 (s, 1H), 6.46 (s, 1H), 6.35 (s, 1H), 4.08 (s, 2H), 3.06 (m, 2H), 2.92 (m, 2H), 2.21 (s, 3H), 1.94 (s, 6H), 1.83 (m, 2H). (T-20)

**Example 20: T-21**

**(*E*)-1$^5$,3$^3$,4,4-Tetramethyl-1$^5$*H*,3$^1$*H*-2,6-diaza-1(2,7)-pyrrolo[3,2-d]pyrimidine-3(4,1)-pyrazolacyclononan-5-one**

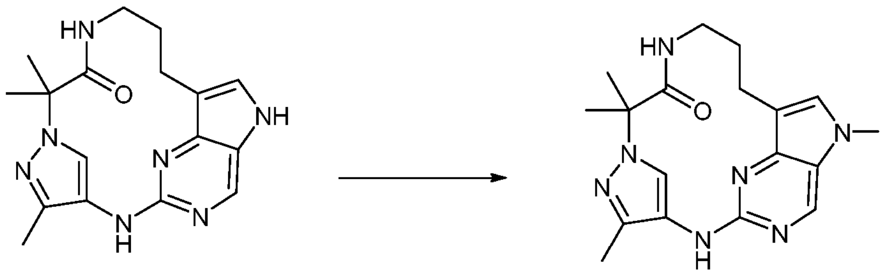

T-18

T-21

T-18 (30 mg) was dissolved in DMF (2 mL), then KOH (20 mg) and MeI (8 μL) were added. After stirring at room temperature overnight, the reaction mixture was quenched with saturated aqueous $NH_4Cl$ solution, and extracted with EA. The organic phase was evaporated using a rotary evaporator, and the residue was purified by flash silica gel column chromatography and reverse-phase C18 preparative chromatography to afford 10.7 mg of T-21.

MS ESI: m/z=355, $[M+H]^+$.

$^1$H NMR (400 MHz, $CDCl_3$) δ 8.43 (s, 1H), 7.93 (s, 1H), 7.08 (s, 1H), 6.70 (s, 1H), 5.91 (s, 1H), 3.77 (s, 3H), 3.12 (m, 2H), 2.59 (m, 2H), 2.21 (s, 3H), 1.93 (s, 6H), 1.65 (m, 2H).

**Example 21: T-22**

**(*E*)-1$^5$-Chloro-3$^3$,4,4-trimethyl-1$^7$*H*,3$^1$*H*-2,6-diaza-1(2,7)-pyrrolo[2,3-d]pyrimidine-3(4,1)-pyrazolacyclodecan-5-one**

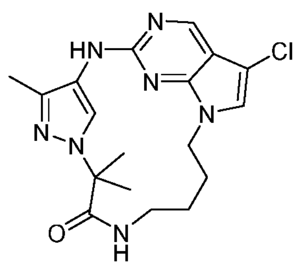

T-22

Compound T-22 was prepared under similar conditions to those in Example 6.

MS ESI: m/z=391, $[M+H]^+$.

$^1$H NMR (400 MHz, $CDCl_3$) δ 8.57 (s, 1H), 7.79 (s, 1H), 6.75 (s, 1H), 6.55 (s, 1H), 5.55 (s, 1H), 3.88 - 3.67 (m, 2H), 3.29 (dd, *J* = 11.2, 5.7 Hz, 2H), 2.26 (s, 3H), 2.10 - 1.91 (m, 2H), 1.86 (s, 5H), 1.62 (dd, *J* = 11.9, 6.5 Hz, 3H).

**Example 22: T-23**

**(*E*)-1$^5$-Chloro-3$^3$,4,4,6-tetramethyl-1$^7$*H*,3$^1$*H*-2,6-diaza-1(2,7)-pyrrolo[2,3-d]pyrimidine-3(4,1)-pyrazolacyclodecan-5-one**

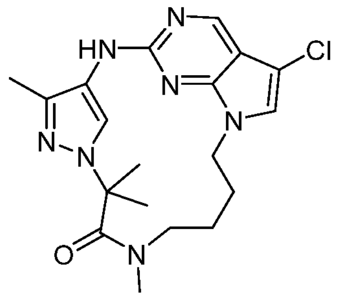

T-23

Compound T-23 was prepared under similar conditions to those in Example 7.
MS ESI: m/z=405, $[M+H]^+$.

**Example 23: T-24 and T-25**

**($1^1Z,3^4E$)-$3^3$,4,4-Trimethyl-$3^1H$-2,6-diaza-1(2,7)-pyrrolo[2,1-f][1,2,4]triazine-3(4,1)-pyrazolacyclononan-5-one (T-24)**

**($1^1Z,3^4E$)-$1^5$-Chloro-$3^3$,4,4-trimethyl-$3^1H$-2,6-diaza-1(2,7)-pyrrolo[2,1-f][1,2,4]triazine-3(4,1)-pyrazolacyclononan-5-one (T-25)**

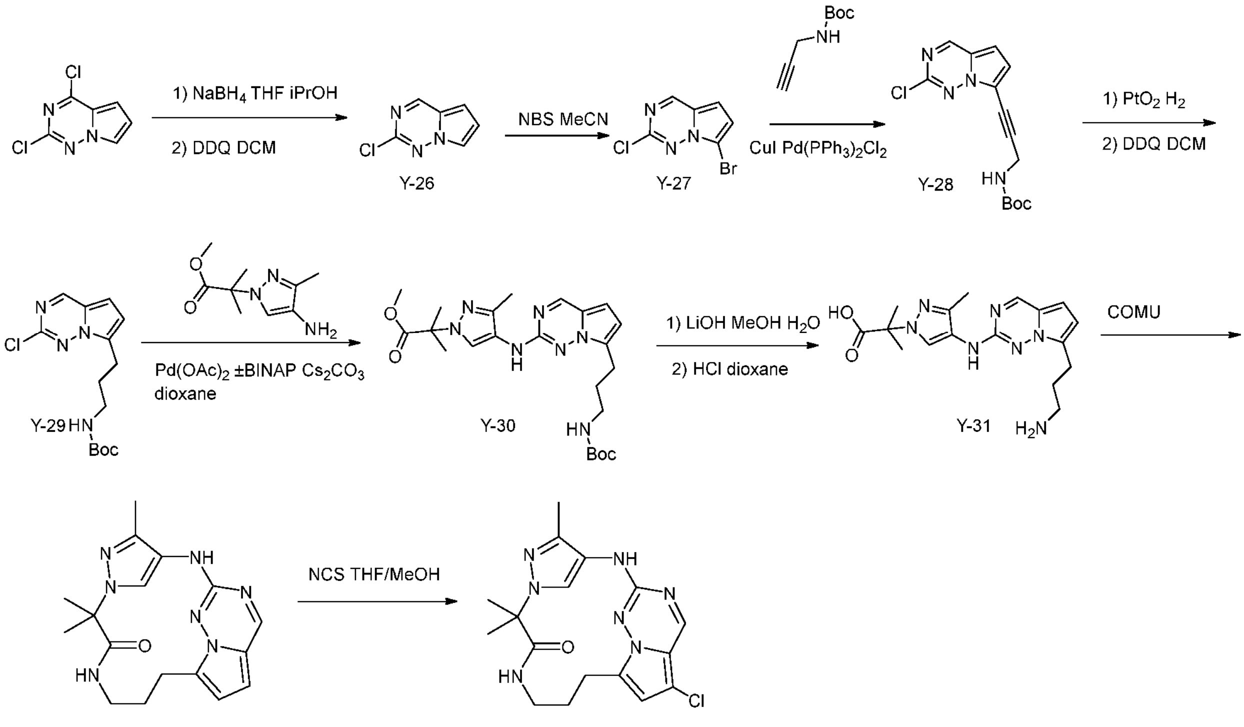


Step 1: Preparation of Y-26

2,4-Dichloropyrrolo[2,1-f][1,2,4]triazine (25 g) was dissolved in isopropanol (50 mL) and tetrahydrofuran (300 mL), then $NaBH_4$ (8.04 g) was added. After stirring at room temperature for 2 h, the mixture was filtered and evaporated using a rotary evaporator. The obtained solid was dissolved in DCM (350 mL), then DDQ (36.22 g) was added. After stirring for 1.5 h, the mixture was washed twice with saturated aqueous $NaHCO_3$ solution. The organic phase was evaporated using a rotary evaporator, dissolved with EA, then filtered through a silica gel pad. The organic phase was evaporated using a rotary evaporator to afford 20.38 g of yellow Y-26.

MS ESI: m/z=154, $[M+H]^+$.

Step 2: Preparation of Y-27

Y-26 (20.38 g) was dissolved in MeCN (500 mL) under an ice bath, then NBS (23.62 g) was added. After stirring at room temperature for 2 h, the reaction mixture was quenched with water. The solid product was collected by filtration, and the liquid phase was evaporated using a rotary evaporator to remove MeCN. The residue was extracted with EA, evaporated using a rotary evaporator, and combined with the obtained solid. The solid was purified via silica gel column chromatography to afford 28.69 g of crude product Y-27.

MS ESI: m/z=232, $[M+H]^+$.

Step 3: Preparation of Y-28

Y-27 (28.69 g) was dissolved in DMF (300 mL) and $NEt_3$ (300 mL), then N-Boc-aminopropyne (20.08 g), $Pd(PPh_3)_2Cl_2$ (4.33 g), and CuI (4.7 g) were added. The reaction flask was purged with nitrogen to displace air and heated at 80°C for 3 h, until the reaction was completed. The reaction was quenched with $NH_4Cl$ solution, extracted with EA. The organic phase was washed with saturated NaCl solution, then evaporated using a rotary evaporator, and purified by column chromatography to afford 29.6 g of crude product Y-28.

MS ESI: m/z=307, $[M+H]^+$.

Step 4: Preparation of Y-29

Y-28 (29.6 g) was dissolved in MeOH (400 mL), then $PtO_2$ (1.1 g) was added. The atmosphere was replaced with $H_2$, and the mixture was stirred at room temperature overnight. Additional $PtO_2$ (1.1 g) was added, and stirring was continued for approximately 2 d until the reaction ceased. The mixture was filtered, evaporated using a rotary evaporator, and purified by flash silica gel column chromatography to remove impurities. The obtained product was dissolved in MeOH (300 mL), then $PtO_2$ (500 mg) was added. The atmosphere was replaced with $H_2$, and the mixture was stirred overnight until the reaction was completed. After the mixture was filtered and evaporated using a rotary evaporator, 19.31 g of over-reduced product was obtained by flash silica gel column chromatography. The obtained over-reduced product was dissolved in DCM (400 mL), then DDQ (14.02 g, 1 eq) was added. After stirring at room temperature for 2 h, the reaction was quenched by washing twice with saturated aqueous $NaHCO_3$ solution. The organic phase was evaporated using a rotary evaporator and purified by flash silica gel column chromatography to afford 19.2 g of Y-29. MS ESI: m/z=311, $[M+H]^+$.

Step 5: Preparation of Y-30

Y-29 (19.2 g) was dissolved in 1,4-dioxane (300 mL), and methyl 2-(4-amino-3-methyl-1H-pyrazol-1-yl)-2-methylpropanoate (14.62 g), $Pd(OAc)_2$ (1.03 g), ±BINAP (3.85 g), and $Cs_2CO_3$ (40.26 g) were added. The atmosphere was replaced with nitrogen, and the mixture was heated at 100°C for 2 h. After completion of the reaction, the mixture was quenched with saturated $NH_4Cl$ solution and extracted with EA. The organic phase was evaporated using a rotary evaporator, and the residue was purified by flash silica gel column chromatography to afford 25.74g of Y-30.

MS ESI: m/z=472, $[M+H]^+$.

Step 6: Preparation of Y-31

Y-30 (25.74 g) was dissolved in MeOH (100 mL) and water (10 mL), then lithium hydroxide monohydrate (9.17 g) was added. After stirring at room temperature overnight, the mixture was evaporated using a rotary evaporator to remove MeOH. An appropriate amount of saturated aqueous NaCl solution was added, and the mixture was acidified with 2N HCl, then extracted with EA. The organic phase was evaporated using a rotary evaporator, then 100 mL of HCl dioxane (4N) was added. After stirring at room temperature for 2.5 h, the mixture was evaporated using a rotary evaporator. The obtained Y-31 exceeded the theoretical yield, and thus the subsequent reaction was carried out using the theoretical amount.

MS ESI: m/z=358, $[M+H]^+$.

Step 7: Preparation of T-24

Y-31 (19.5 g) was dissolved in DMF (800 mL), and $NEt_3$ (15.14 mL) was added, then COMU (28 g) in DMF (800 mL) was slowly added dropwise. After stirring at room temperature overnight, additional COMU (3 g) was added. After

stirring for 2 h, the reaction was quenched with $H_2O$ and extracted with EA. The organic phase was evaporated using a rotary evaporator, and the residue was purified by flash silica gel column chromatography to afford 9.17 g of T-24.

MS ESI: m/z=340, $[M+H]^+$.

$^1H$ NMR (400 MHz, $CDCl_3$) δ 8.61 (s, 1H), 7.91 (s, 1H), 6.67 (d, J = 4.5 Hz, 1H), 6.49 (d, J = 4.5 Hz, 1H), 6.08 (s, 1H), 5.05 (s, 1H), 3.22 (m, 2H), 2.81 - 2.71 (m, 8H), 2.25 (s, 11H), 1.92 (s, 22H), 1.62 - 1.52 (m, 8H).

Step 8: Preparation of T-25

T-24 was dissolved in THF (90 mL) and MeOH (30 mL), then NCS (3.176 g) was added. After stirring at room temperature for 1 d, additional NCS (600 mg) was added. After stirring overnight, the reaction mixture was quenched with saturated aqueous $Na_2S_2O_3$ solution, then extracted with EA. The organic phase was washed with saturated aqueous NaCl solution. The organic phase was evaporated using a rotary evaporator, and the residue was purified by flash silica gel column chromatography to afford 7.2g of T-25.

MS ESI: m/z=374, $[M+H]^+$.

$^1H$ NMR (400 MHz, $CDCl_3$) δ 8.64 (s, 1H), 7.89 (s, 1H), 6.43 (s, 1H), 6.16 (s, 1H), 4.96 (s, 1H), 3.21 (m, 2H), 2.78 - 2.65 (m, 2H), 2.25 (s, 3H), 1.89 (s, 6H), 1.57 (m, 2H).

**Example 24: T-26**

**($1^1$*Z*,$3^4$*E*)-$1^5$-Chloro-$3^3$,4,4-trimethyl-$3^1$*H*-2,6-diaza-1(2,7)-pyrrolo[2,1-f][1,2,4]triazine-3(4,1)-pyrazolacyclodecan-5-one**

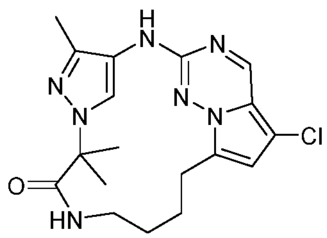

T-26

Using Y-27 as raw material, T-26 was prepared under the same conditions as those in Example 23, except that N-Boc-aminopropyne was replaced with N-Boc-3-butyn-1-amine.

MS ESI: m/z=391, $[M+H]^+$.

$^1H$ NMR (400 MHz, $CDCl_3$) δ 8.61 (s, 1H), 7.76 (s, 1H), 6.39 (s, 1H), 6.19 (s, 1H), 5.45 (s, 1H), 3.27 (dd, *J* = 10.6, 5.4 Hz, 2H), 2.65 (dt, *J* = 11.7, 6.2 Hz, 2H), 2.27 (s, 3H), 1.85 (s, 6H), 1.77 - 1.67 (m, 2H), 1.65 - 1.48 (m, 2H).

**Example 25: T-27**

**($1^1$*Z*,$3^4$*E*)-$1^5$$3^3$-Dichloro-4,4-dimethyl-$3^1$*H*-2,6-diaza-1(2,7)-pyrrolo[2,1-f][1,2,4]triazine-3(4,1)-pyrazolacyclononan-5-one**

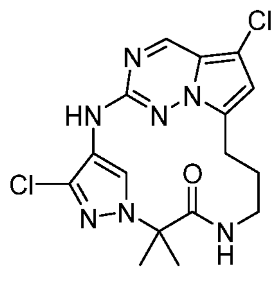

T-27

Using Y-29 as raw material, T-27 was prepared under the same conditions as those in Example 23, except that methyl 2-(4-amino-3-methyl-1H-pyrazol-1-yl)-2-methylpropionate was replaced with methyl 2-(4-amino-3-chloro-1H-pyrazol-1-yl)-2-methylpropionate.

MS ESI: m/z=394, $[M+H]^+$.

$^1H$ NMR (400 MHz, $CDCl_3$) δ 8.66 (s, 1H), 7.99 (s, 1H), 6.45 (s, 1H), 6.24 (s, 1H), 5.02 (s, 1H), 3.38 - 3.16 (m, 2H), 2.85 - 2.67 (m, 2H), 1.91 (s, 6H), 1.59 (m, 2H).

**Example 26: T-28**

**($1^1Z,3^4E$)-$1^5$-Chloro-$3^3$-cyclopropyl-4,4-dimethyl-$3^1H$-2,6-diaza-1(2,7)-pyrrolo[2,1-f][1,2,4]triazine-3(4,1)-pyrazolacyclononan-5-one**

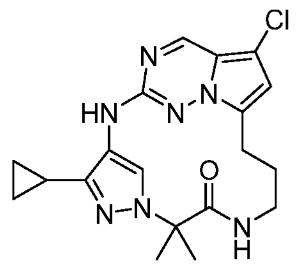

T-28

Using Y-29 as raw material, T-28 was prepared under the same conditions as those in Example 23, except that methyl 2-(4-amino-3-methyl-1H-pyrazol-1-yl)-2-methylpropionate was replaced with methyl 2-(4-amino-3-cyclopropyl-1H-pyrazol-1-yl)-2-methylpropionate.

MS ESI: m/z=403, $[M+H]^+$.

$^1H$ NMR (400 MHz, $CDCl_3$) δ 8.65 (s, 1H), 7.84 (s, 1H), 6.44 (s, 1H), 6.24 (s, 1H), 4.93 (s, 1H), 3.20 (d, $J$ = 5.2 Hz, 2H), 2.81 - 2.65 (m, 2H), 1.88 (s, 5H), 1.85 - 1.76 (m, 2H), 1.53 (d, $J$ = 39.7 Hz, 5H), 0.90 - 0.81 (m, 4H).

**Example 27: T-29**

**($1^1Z,3^4E$)-$3^3$,4,4-Trimethyl-$1^5$-(trifluoromethyl)-$3^1H$-2,6-diaza-1(2,7)-pyrrolo[2,1-f][1,2,4]triazine-3(4,1)-pyrazolacyclononan-5-one**

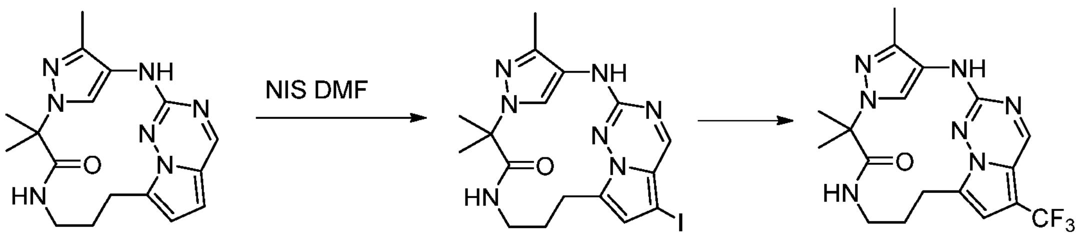


T-24 Y-32 T-29

Step 1: Preparation of Y-32

T-24 (100 mg) was dissolved in DMF (2 mL), then NIS (70 mg) was added. After stirring at room temperature for 2 h, the reaction was quenched with saturated aqueous $Na_2S_2O_3$ solution, and then extracted with EA. The organic phase was evaporated using a rotary evaporator, and the residue was purified by flash silica gel column chromatography to afford 135 mg of Y-32.

Step 2: Preparation of T-29

T-29 (40 mg) was dissolved in DMF (2 mL), then methyl fluorosulfonyldifluoroacetate (25 mg) and CuI (18 mg) were added. The atmosphere was replaced with nitrogen, and the mixture was heated at 80°C for about 2 h. Additional methyl fluorosulfonyldifluoroacetate (50 mg) was added. The mixture was heated at 80°C for about 15 h. The reaction was quenched with saturated aqueous $NH_4Cl$ solution and extracted with EA. The organic phase was evaporated using a rotary evaporator, and the residue was purified by flash silica gel column chromatography and C18 preparative chromatography to afford 9 mg of T-29.

MS ESI: m/z=408, $[M+H]^+$.

$^1H$ NMR (400 MHz, $CDCl_3$) δ 8.83 (s, 1H), 7.85 (s, 1H), 6.80 (s, 1H), 6.59 (s, 1H), 4.91 (s, 1H), 3.25 (m, 2H), 2.86 - 2.71 (m, 2H), 2.26 (s, 3H), 1.92 (s, 6H), 1.59 (m, 2H).

**Example 28: T-30**

**($1^1$*Z*,$3^4$*E*)-$3^3$,4,4-Trimethyl-5-oxo-$3^1$*H*-2,6-diaza-1(2,7)-pyrrolo[2,1-f][1,2,4]triazine-3(4,1)-pyrazolacyclononan-$1^5$-carbonitrile**

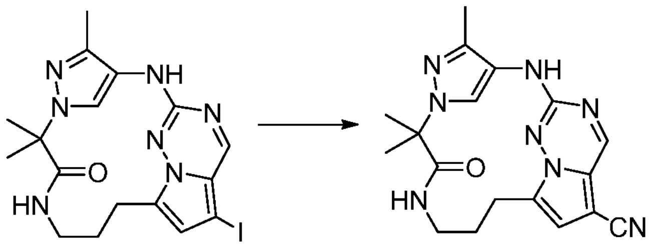

Y-32 T-30

Y-32 (30 mg) was dissolved in DMF (2 mL), then $Zn(CN)_2$ (15 mg), $Pd(dppf)_2Cl_2$ DCM (26 mg), and zinc powder (2 mg) were added. The atmosphere was replaced with nitrogen, and the mixture was heated at 100°C for about 3 h. The reaction was quenched with saturated $NH_4Cl$ solution and extracted with EA. The organic phase was evaporated using a rotary evaporator, and the residue was purified by flash silica gel column chromatography and C18 preparative chromatography to afford 18 mg of T-30.

MS ESI: m/z=365, $[M+H]^+$.

$^1H$ NMR (400 MHz, CDCl3) δ 8.89 (s, 1H), 7.91 (s, 1H), 6.84 (s, 1H), 6.37 (s, 1H), 4.93 (s, 1H), 3.25 (m, 2H), 2.85 - 2.72 (m, 2H), 2.28 (s, 3H), 1.93 (s, 6H), 1.58 (m, 2H).

**Example 29: T-31**

**(*E*)-$3^3$,4,4,6,10-Pentamethyl-$1^5$-(trifluoromethyl)-$3^1$H-2,6,10-triaza-1(2,4)-pyrimidina--3(4,1)-pyrazolacyclodecan-5-one**

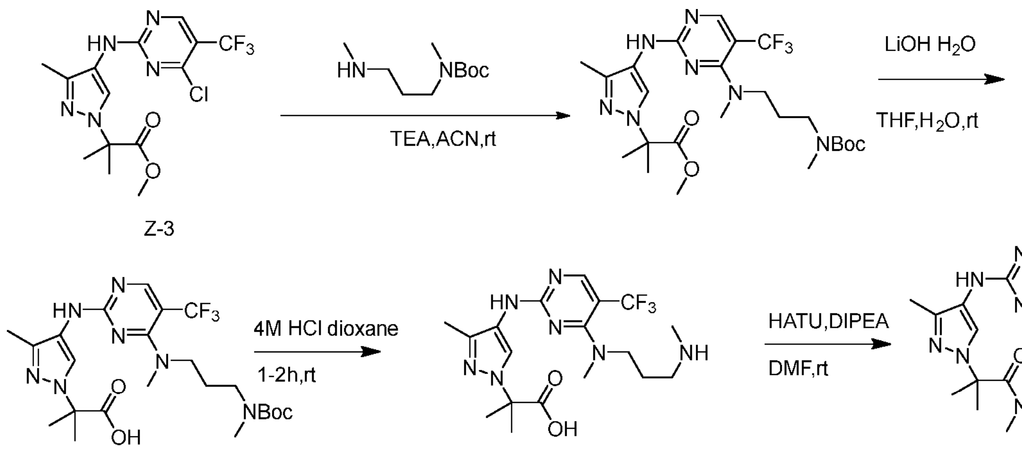


T-31

Using Z-3 and tert-butyl methyl(3-(methylamino)propyl)carbamate as raw materials, T-31 was prepared under the same conditions as those in Example 1.

MS ESI: m/z=412, $[M+H]^+$.

$^1H$ NMR (400 MHz, DMSO-$d_6$) δ 9.27 (s, 1H), 8.28 (s, 1H), 7.85 (s, 1H), 4.24 (s, 1H), 4.07 (s, 1H), 3.06 (s, 4H), 2.67 (s, 2H), 2.19 (d, *J* = 12.5 Hz, 5H), 1.81 (s, 4H), 1.59 (s, 4H).

**Example 30: T-32**

**(*E*)-3$^3$,4,4,10-Tetramethyl-1$^5$-(trifluoromethyl)-3$^1$H-2,6,10-triaza-1(2,4)-pyrimidina-3(4,1)-pyrazolacyclodecan-5-one**

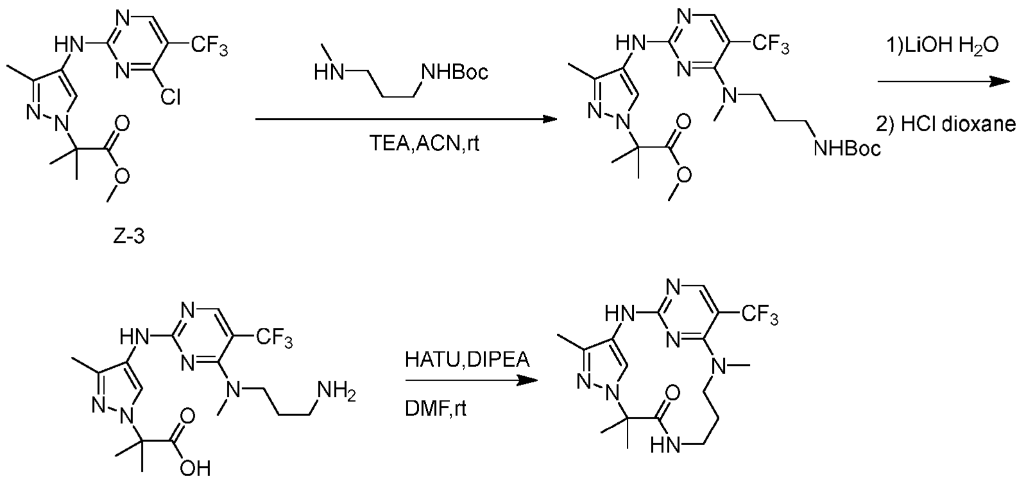


T-32

Using Z-3 and tert-butyl (2-(methylamino)propyl)carbamate as raw materials, T-32 was prepared under the same conditions as those in Example 1.

MS ESI: m/z=398, $[M+H]^+$.

$^1H$ NMR (400 MHz, Chloroform-d) δ 8.28 (s, 1H), 7.69 (s, 1H), 6.52 (s, 1H), 4.61 (s, 1H), 3.44 - 3.37 (m, 2H), 3.19 (d, *J* = 4.7 Hz, 2H), 3.09 (s, 3H), 2.24 (s, 3H), 1.87 (s, 6H), 1.55 (d, *J* = 7.4 Hz, 2H).

**Example 31: T-33**

**(*E*)-3$^3$,4,4,6-Tetramethyl-1$^5$-(trifluoromethyl)-3$^1$*H*-2,6,10-triaza-1(2,4)-pyrimidina-3(4,1)-pyrazolacyclodecan-5-one**

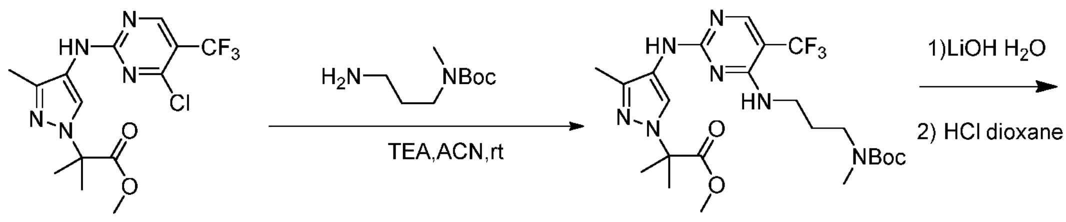


Z-3

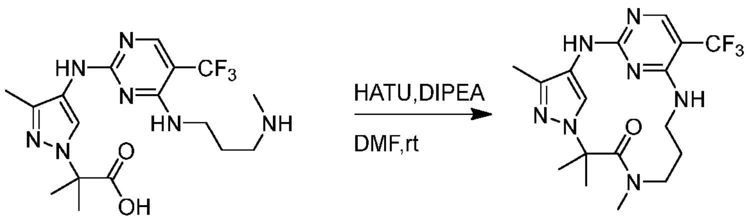


T-33

Using Z-3 and tert-butyl (3-aminopropyl)(methyl)carbamate as raw materials, T-33 was prepared under the same conditions as those in Example 1.

MS ESI: m/z=398, $[M+H]^+$.

$^1$H NMR (400 MHz, Chloroform-*d*) δ 8.10 (s, 1H), 7.99 (s, 1H), 7.52 (s, 1H), 5.62 (s, 1H), 4.48 (s, 1H), 3.84 (s, 1H), 3.21 (s, 1H), 2.53 (d, *J* = 14.2 Hz, 1H), 2.31 (s, 3H), 2.29 (s, 3H), 1.96 (s, 3H), 1.76 (s, 3H), 1.72 (s, 2H).

**Example 32: T-34**

**(*E*)-3³,4,4,6-Tetramethyl-1⁵-(trifluoromethyl)-3¹*H*-2,6,10-triaza-1(2,4)-pyrimidina-3(4,1)-pyrazolacyclodecane**

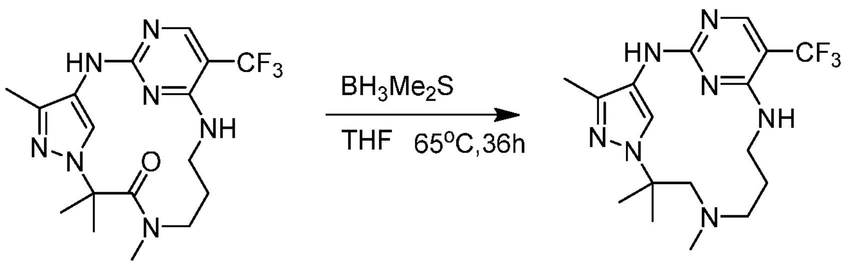


T-33 T-34

T-33 (80 mg) was dissolved in THF (2 mL), then 2M borane-dimethyl sulfide in THF (0.4 mL, 0.8 mmol, 4.0 eq) was added. The system was allowed to react at 65°C for 36 h, then cooled to room temperature. To the system was added methanol (2 mL), and stirred until it became clear. The system was directly evaporated using a rotary evaporator, and the residue was purified by flash column chromatography (100% PE - 30% EA/PE) to afford 6 mg of the solid compound T-34. MS ESI: m/z=384, $[M+H]^+$.

$^1$H NMR (400 MHz, Chloroform-*d*) δ 8.17 (s, 1H), 7.52 (s, 2H), 5.78 (s, 1H), 3.02 (s, 2H), 2.56 (s, 2H), 2.26 (s, 3H), 2.19 (s, 3H), 1.61 (s, 2H), 1.54 (s, 6H), 1.26 (s, 2H).

**Example 33: T-35**

**(3⁴*E*,6⁴*Z*)-3³,4,4-Trimethyl-1⁵-(trifluoromethyl)-3¹*H*,6¹*H*-2,9-diaza-1(2,4)-pyrimidina-6(4,1)-triazole-3(4,1)-pyrazolacyclononane**

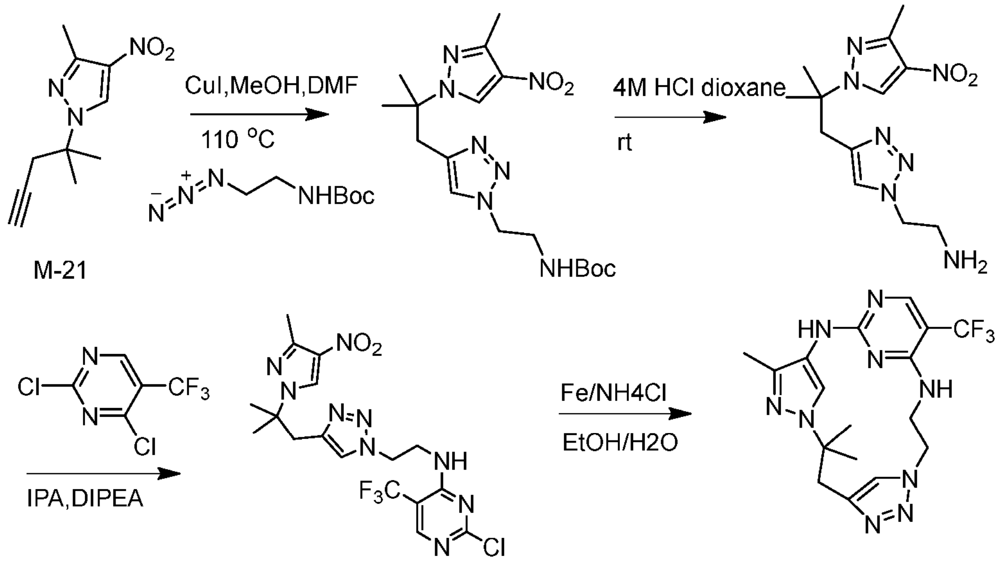


M-21

T-35

Using M-21 and N-Boc-2-azidoethylamine as raw materials, T-35 was prepared under the same conditions as those in Example 16.

MS ESI: m/z=408, $[M+H]^+$.

$^1H$ NMR (400 MHz, Chloroform-*d*) δ 8.16 (s, 1H), 7.16 (s, 1H), 6.79 (s, 1H), 5.88 (s, 1H), 5.66 (s, 1H), 4.32 (s, 2H), 3.67 (q, *J* = 6.8 Hz, 2H), 3.27 (s, 2H), 2.29 (s, 3H), 1.55 (s, 6H).

**Example 34: T-36**

**($3^4E$,$6^3Z$)-$3^3$,4,4-Trimethyl-$1^5$-(trifluoromethyl)-$3^1H$,$6^2H$-2,9-diaza-1(2,4)-pyrimidina-6(4,2)-triazole-3(4,1)-pyrazolacyclononane**

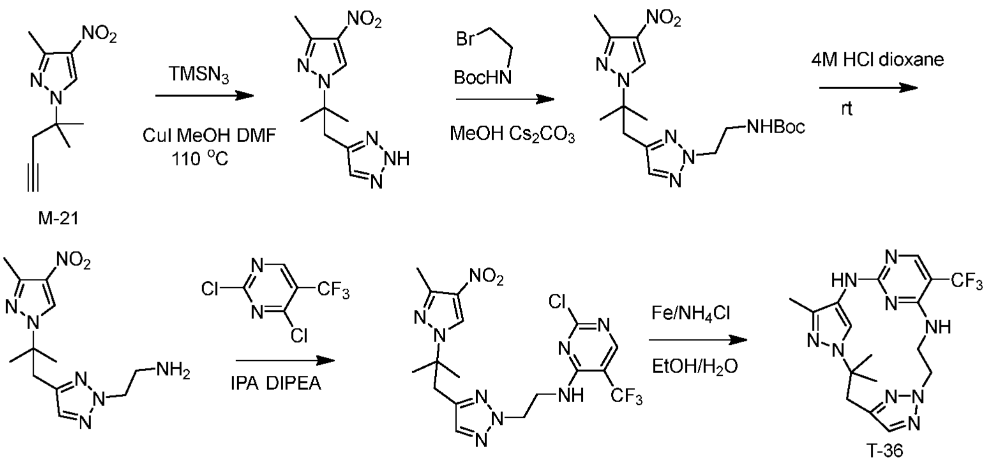


M-21

T-36

Using M-21 and N-Boc-bromoethylamine as raw materials, T-36 was prepared under the same conditions as those in Example 17.

MS ESI: m/z=408, $[M+H]^+$.

$^1H$ NMR (400 MHz, Chloroform-d) δ 8.09 (s, 1H), 7.18 (s, 1H), 7.14 (s, 1H), 7.05 (s, 1H), 5.62 (s, 1H), 4.45 (t, J = 6.2 Hz, 2H), 3.88 (q, J = 6.3 Hz, 2H), 3.08 (s, 2H), 2.23 (s, 3H), 1.68 (s, 6H).

**Example 35: T-37**

**(*E*)-3³,4,4-Trimethyl-1⁵-(trifluoromethyl)-3¹*H*-6-oxa-2,10-diaza-1(2,4)-pyrimidina-3(4,1)-pyrazolacyclodecan-5-one**

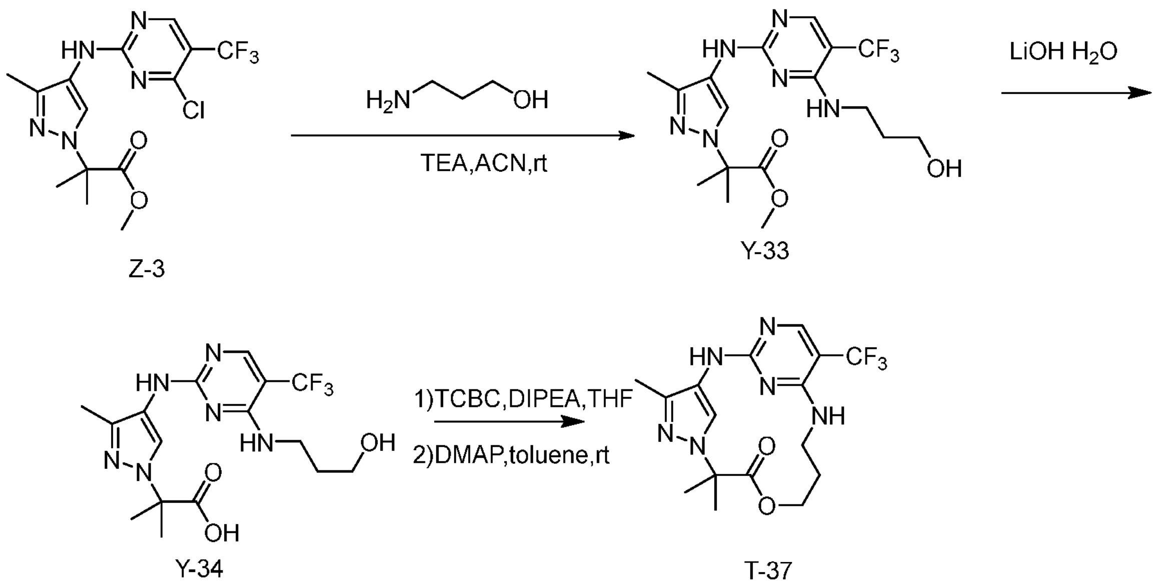


Z-3 Y-33 Y-34 T-37

Step 1: Preparation of Y-33

Using Z-3 (75.6 mg) and 3-amino-1-propanol as raw materials, 77.5 mg of Y-33 was obtained under the same conditions as those in Step 4 of Example 1.

MS ESI: m/z=417, $[M+H]^+$.

Step 2: Preparation of Y-34

Using Y-33 (70 mg) as raw material, 60 mg of Y-34 was obtained under the same conditions as those in Step 5 of Example 1.

MS ESI: m/z=403, $[M+H]^+$.

Step 3: Preparation of T-37

Y-33 (40 mg) was dissolved in 2 mL of ultradry THF, then a solution of DIPEA (155 mg) and TCBC (49 mg) in THF (2 mL) was added. The reaction mixture was stirred at room temperature for 19 h, then additional TCBC (25 mg) in THF (0.5 mL) was added to the system. After stirring at room temperature for 10 h, 30 mL of dry toluene was added to the system, and a solution of DMAP (73 mg, 0.6 mmol, 6 eq) in toluene (10 mL) was added dropwise at room temperature with stirring. The reaction mixture was stirred at room temperature for 18 h, then the system was directly evaporated using a rotary evaporator, and purified by flash silica gel column chromatography and C18 preparative chromatography to afford 19 mg of T-37 as a white solid.

MS ESI: m/z=385, $[M+H]^+$.

$^1H$ NMR (400 MHz, Chloroform-*d*) δ 8.12 (s, 1H), 7.91 (s, 1H), 6.53 (s, 1H), 5.37 (s, 1H), 4.14 - 4.08 (m, 2H), 3.49 (s, 2H), 2.20 (s, 3H), 1.88 (s, 6H), 1.82 - 1.73 (m, 2H).

**Example 36: T-38**

**(*E*)-3³,4,4,7-Tetramethyl-1⁵-(trifluoromethyl)-3¹*H*-2,6,10-triaza-1(2,4)-pyrimidina-3(4,1)-pyrazolacyclodecan-5-one**

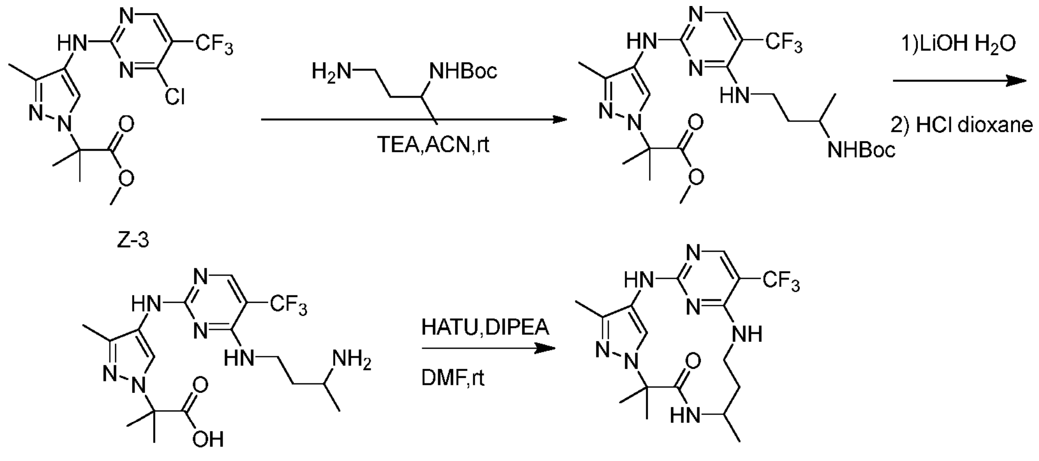


Z-3

T-38

Using Z-3 and 3-BOC-aminobutylamine as raw materials, T-38 was prepared under the same conditions as those in Example 1.

MS ESI: m/z=398, $[M+H]^+$.

$^1H$ NMR (400 MHz, DMSO-$d_6$) δ 9.00 (s, 1H), 8.11 (d, *J* = 8.4 Hz, 2H), 7.22 (d, *J* = 6.0 Hz, 1H), 5.71 (d, *J* = 7.9 Hz, 1H), 3.67 (d, *J* = 8.3 Hz, 1H), 3.46 (s, 1H), 3.14 - 3.03 (m, 1H), 2.14 (s, 3H), 1.74 (s, 3H), 1.63 (s, 3H), 1.57 - 1.45 (m, 1H), 1.28 (q, *J* = 11.4 Hz, 1H), 0.97 (d, *J* = 6.6 Hz, 3H).

**Example 37: T-39**

**(*E*)-3[3],4,4,7,7-Pentamethyl-1[5]-(trifluoromethyl)-3[1]*H*-2,6,10-triaza-1(2,4)-pyrimidina-3(4,1)-pyrazolacyclodecan-5-one**

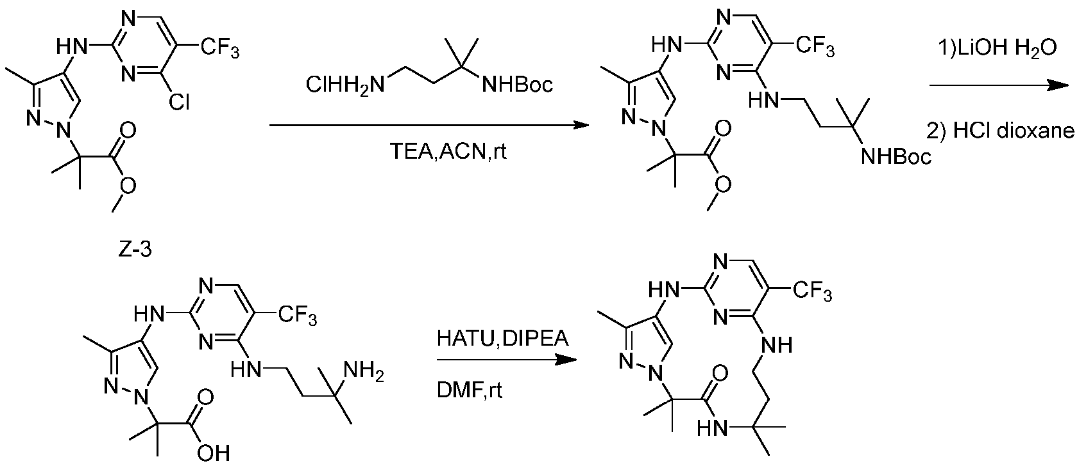


Z-3

T-39

Using Z-3 and tert-butyl (4-amino-2-methylbutan-2-yl)carbamate hydrochloride as raw materials, T-39 was prepared under the same conditions as those in Example 1.

MS ESI: m/z=412, $[M+H]^+$.

$^1H$ NMR (400 MHz, CDCl3) δ 8.10 (s, 1H), 7.76 (s, 1H), 6.97 (s, 1H), 5.38 (s, 1H), 4.85 (s, 1H), 3.35 (dd, J = 13.2, 9.3 Hz, 2H), 2.20 (s, 3H), 1.87 (s, 6H), 1.50 - 1.41 (m, 2H), 1.30 (s, 6H).

**Example 38: T-40**

**($3^4E$,$8E$)-$3^3$,4,4-Trimethyl-$1^5$-(trifluoromethyl)-$3^1H$-2,6,11-triaza-1(2,4)-pyrimidina-3(4,1)-pyrazolylundecapentaene-5-one**

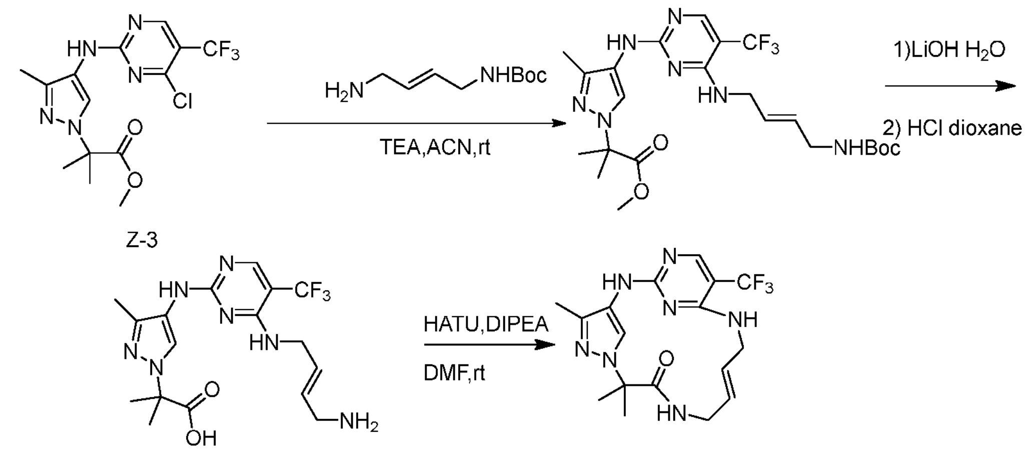

Z-3

T-40

Using Z-3 and tert-butyl (E)-(4-aminobut-2-en-1-yl)carbamate as raw materials, T-40 was prepared under the same conditions as those in Example 1.
MS ESI: m/z=396, $[M+H]^+$.
$^1H$ NMR (400 MHz, DMSO-$d_6$) δ 8.52 (s, 1H), 8.01 (s, 1H), 7.67 (s, 1H), 7.33 (s, 1H), 7.12 (s, 1H), 5.52 (d, *J* = 15.4 Hz, 1H), 5.37 (d, *J* = 15.7 Hz, 1H), 3.51 (s, 4H), 2.01 (d, *J* = 3.9 Hz, 3H), 1.62 (s, 6H).

**Example 39: T-41**

**($5^1$s,$5^4$s,*E*)-$1^3$,8,8-Trimethyl-$3^5$-(trifluoromethyl)-$1^1H$-2,4,6-triaza-3(2,4)-pyrimidina-1(4,1)-pyrazola-5(1,4)-cyclohexacyclooctan-7-one**

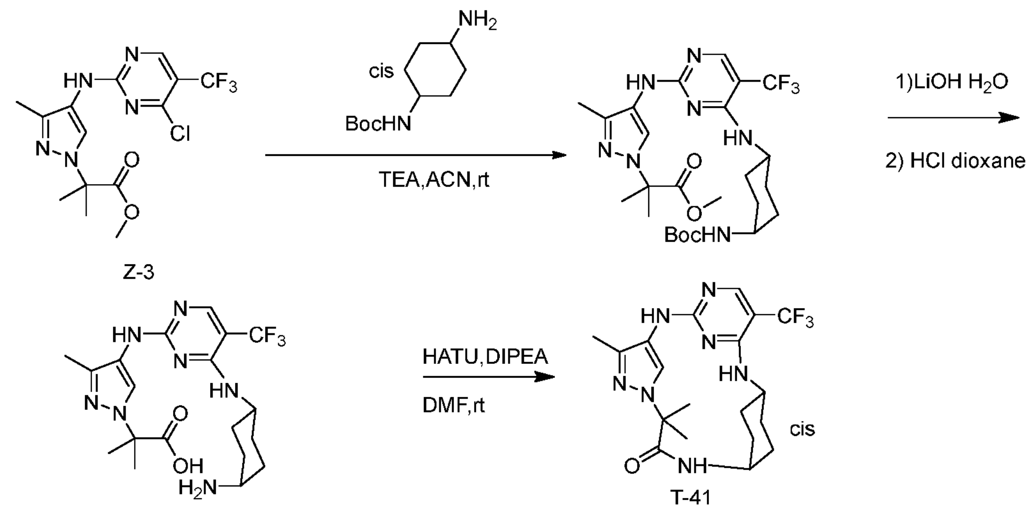

Z-3

T-41

Using Z-3 and 1-N-Boc-cis-1,4-cyclohexanediamine as raw materials, T-41 was prepared under the same conditions as those in Example 1.
MS ESI: m/z=424, $[M+H]^+$.
$^1H$ NMR (400 MHz, Chloroform-*d*) δ 8.09 (s, 1H), 7.67 (s, 1H), 6.29 (s, 1H), 5.55 (s, 1H), 5.10 (s, 1H), 3.93 (s, 1H), 3.04 (s, 1H), 2.37 - 2.23 (m, 2H), 2.19 (s, 2H), 1.88 (s, 4H), 1.77 (d, *J* = 13.5 Hz, 2H), 1.52 - 1.23 (m, 7H).

**Example 40: T-42**

**($5^3R$,*E*)-$1^3$,8,8-Trimethyl-$3^5$-(trifluoromethyl)-$1^1H$-2,4,6-triaza-3(2,4)-pyrimidina-1(4,1)-pyrazola-5(1,3)-cyclohexacyclooctan-7-one**

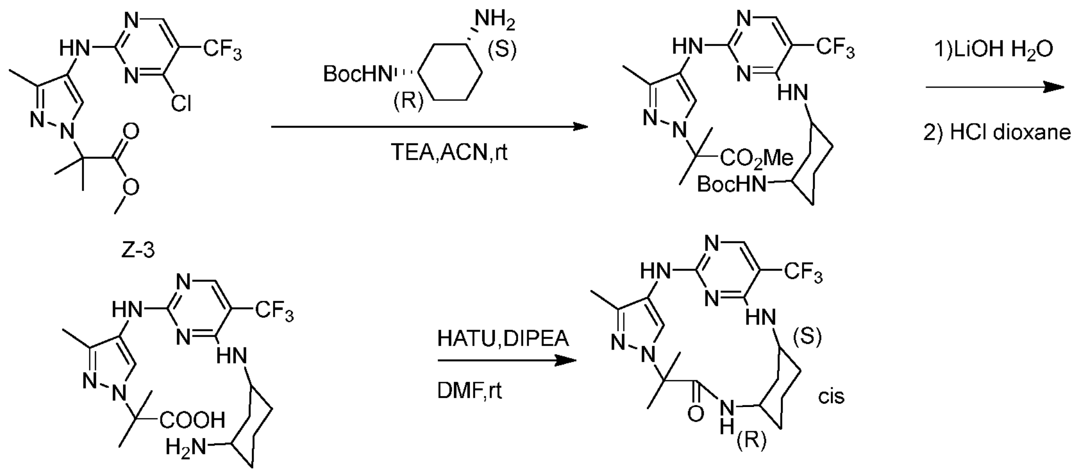


Z-3

T-42

Using Z-3 and tert-butyl ((1S,3R)-3-aminocyclohexyl)carbamate as raw materials, T-42 was prepared under the same conditions as those in Example 1.

MS ESI: m/z=424, $[M+H]^+$.

$^1$H NMR (400 MHz, Chloroform-*d*) δ 8.08 (s, 1H), 7.63 (s, 1H), 6.62 (d, *J* = 84.3 Hz, 1H), 5.36 (d, *J* = 64.0 Hz, 1H), 4.98 (s, 1H), 3.91 (s, 1H), 3.38 - 2.97 (m, 2H), 2.54 (s, 1H), 2.18 (s, 3H), 2.01 (s, 2H), 1.97 - 1.79 (m, 8H).

**Example 41: T-43**

**(*E*)-8,8-Difluoro-$3^3$,4,4-trimethyl-$1^5$-(trifluoromethyl)-$3^1H$-6-oxa-2,10-diaza-1(2,4)-pyrimidina-3(4,1)-pyrazolacyclodecan-5-one**

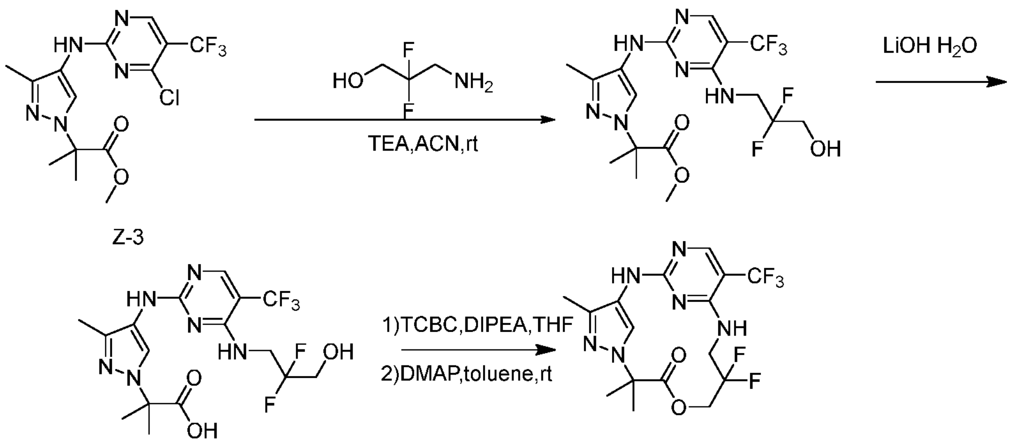


Z-3

T-43

Using Z-3 and 3-amino-2,2-difluoropropan-1-ol as raw materials, T-43 was prepared under the same conditions as those in Example 35.

MS ESI: m/z=421, $[M+H]^+$.

$^1$H NMR (400 MHz, Chloroform-*d*) δ 8.18 (s, 1H), 7.64 (s, 1H), 6.51 (s, 1H), 5.38 (s, 1H), 4.32 (t, *J* = 10.1 Hz, 2H), 3.99 (t, *J* = 14.4 Hz, 2H), 2.19 (s, 3H), 1.89 (s, 6H).

**Example 42: T-44**

**(*E*)-3',4',4'-Trimethyl-5'-(trifluoromethyl)spiro[cyclopropane-8'-2,6,10-triaza-1(2,4)-pyrimidina-3(4,1)-pyrazolacyclodecan]-5'-one**

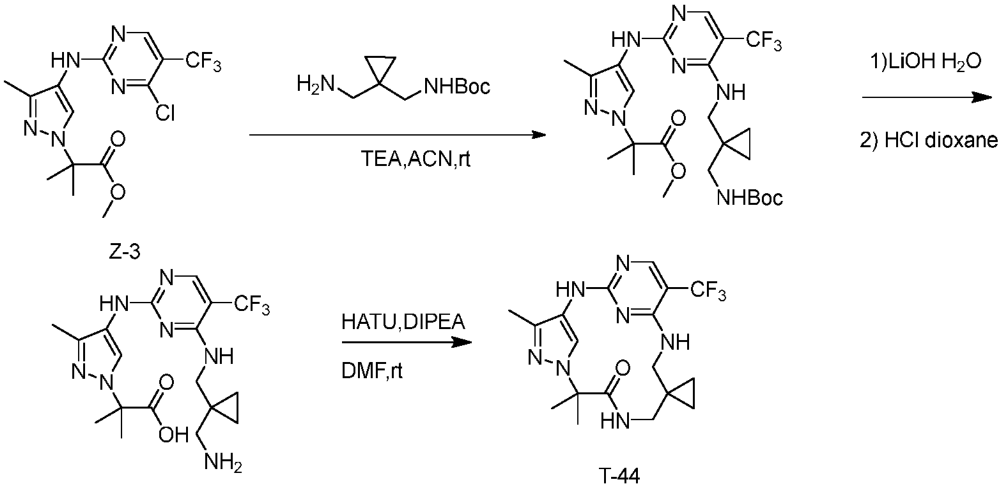


Z-3

T-44

Using Z-3 and tert-butyl (1-(aminomethyl)cyclopropyl)carbamate as raw materials, T-44 was prepared under the same conditions as those in Example 1.

MS ESI: m/z=410, $[M+H]^+$.

$^1H$ NMR (400 MHz, Chloroform-*d*) δ 8.11 (s, 1H), 7.74 (s, 1H), 6.86 (s, 1H), 5.13 (s, 1H), 4.98 (s, 1H), 3.50 (s, 2H), 3.01 (s, 2H), 2.23 (s, 3H), 1.89 (s, 6H), 0.23 (d, *J* = 22.4 Hz, 4H).

**Example 43: T-45**

**(*E*)-3[3],4,4,9,9-Pentamethyl-1[5]-(trifluoromethyl)-3[1]*H*-2,6,10-triaza-1(2,4)-pyrimidina-3(4,1)-pyrazolacyclodecan-5-one**

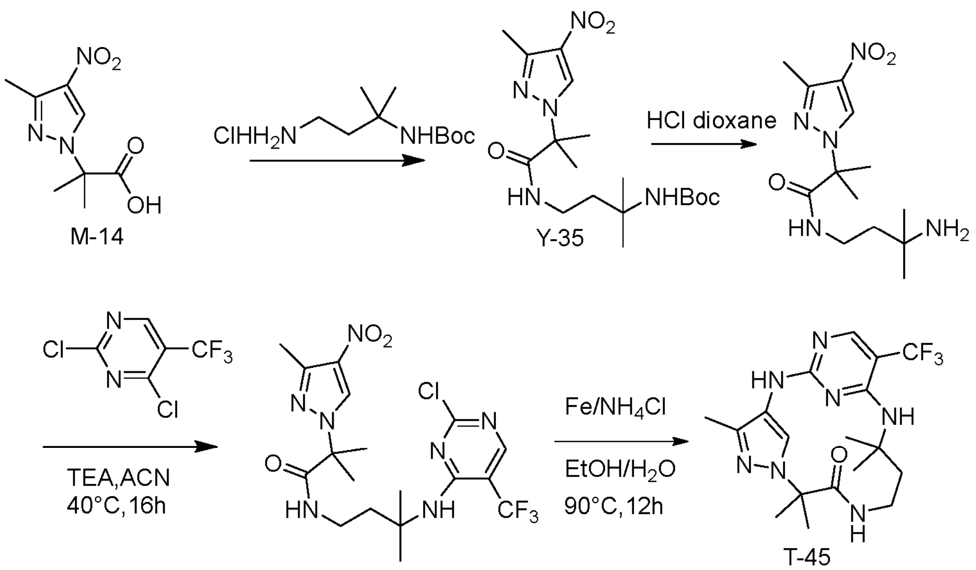


M-14

Y-35

T-45

Step 1: Preparation of Y-35

Using M-14 and tert-butyl (4-amino-2-methylbutan-2-yl)carbamate hydrochloride as raw materials, Y-35 was prepared under the same conditions as those in Step 7 of Example 1.

Step 2: Preparation of T-45

Using Y-35 as raw material, T-45 was prepared under the same conditions as those in Example 16. MS ESI: m/z=412, $[M+H]^+$.

$^1H$ NMR (400 MHz, Chloroform-*d*) δ 8.06 (s, 1H), 7.65 (s, 1H), 7.42 (s, 1H), 5.25 (s, 1H), 4.63 (s, 1H), 3.22 - 3.09 (m, 2H), 2.22 (s, 3H), 1.86 (s, 8H), 1.31 (s, 6H).

**Example 44: T-46**

**(*E*)-3$^3$,4,4,9-Tetramethyl-1$^5$-(trifluoromethyl)-3$^1$*H*-2,6,10-triaza-1(2,4)-pyrimidina-3(4,1)-pyrazolacyclodecan-5-one**

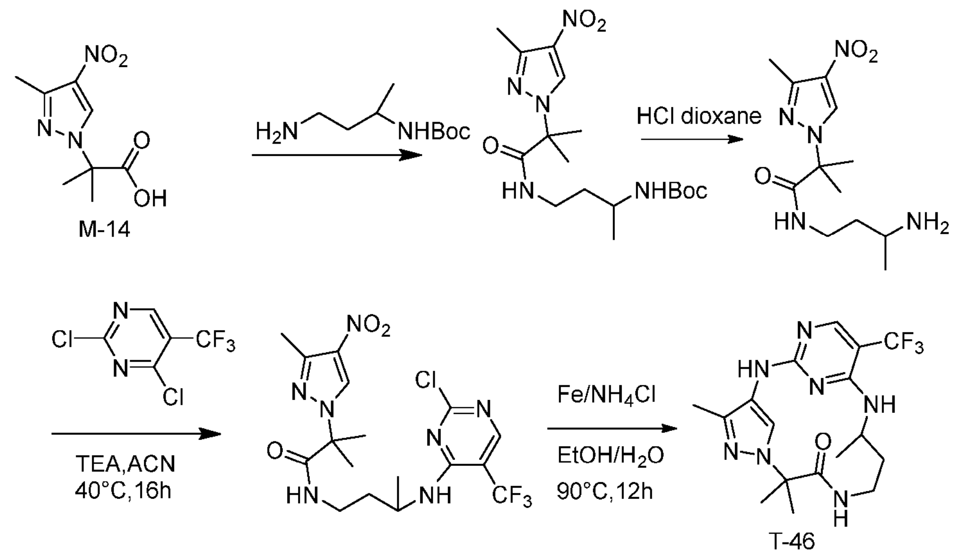

M-14
T-46

Using M-14 and 3-BOC-aminobutylamine as raw materials, compound T-46 was prepared under the same conditions as those in Example 43.

MS ESI: m/z=398, $[M+H]^+$.

$^1H$ NMR (400 MHz, Chloroform-*d*) δ 8.09 (s, 1H), 7.73 (s, 1H), 6.94 (s, 1H), 4.98 (d, *J* = 6.0 Hz, 1H), 4.64 (s, 1H), 3.85 (dt, *J* = 11.4, 6.7 Hz, 1H), 3.37 (td, *J* = 13.5, 7.4 Hz, 1H), 3.03 (d, *J* = 11.3 Hz, 1H), 2.23 (s, 3H), 1.89 (s, 3H), 1.86 (s, 3H), 1.61 (t, *J* = 13.6 Hz, 1H), 1.36 (t, *J* = 12.3 Hz, 1H), 1.25 (d, *J* = 6.6 Hz, 3H).

**Preparation of Intermediate M-1**

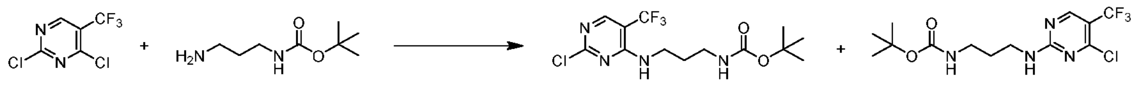
M-1 M-2

2,4-Dichloro-5-trifluoromethylpyrimidine (1.4 g) was dissolved in 24 mL of acetonitrile, N-Boc-1,3-propanediamine (1.24 mg) and TEA (1.3 g) were added at room temperature. The system was allowed to react at room temperature for 18 h. The reaction mixture was concentrated and purified via column chromatography (100% PE - 10% EA:PE) to afford intermediates M-1 (0.99 g, Rf: 0.4, 20% EA:PE) and M-2 (1.1 g, Rf: 0.3, 20% EA:PE).

MS ESI: m/z=355.2, $[M+H]^+$.

**Preparation of Intermediate M-5**

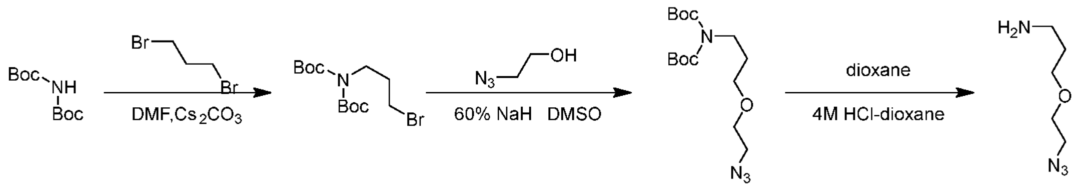


M-3 M-4 M-5

Step 1: Preparation of M-3

Bis-Boc amine (3.3 g) was dissolved in 165 mL of DMF, and cesium carbonate (9.9 g) was added in portions at room temperature. The system was allowed to react at room temperature for 1 h. Then, 1,3-dibromopropane was added dropwise. The mixture was stirred and allowed to react at room temperature for 12 h. The reaction solution was filtered, the filtrate was concentrated, and purified via column chromatography (100% PE - 10% EA:PE) to afford intermediate Z-3 (4.39 g).

Step 2: Preparation of M-4

Azidoethanol (87.1 mg) was dissolved in ultradry DMSO (3 mL), the mixture was held in an ice bath and stirred, 60% NaH was added under protection of nitrogen, and subsequently a solution of M-3 (338.2 mg) in DMSO (6 mL) was added dropwise in the ice bath. After addition, the system was allowed to react at room temperature for 2 h. Upon completion of the reaction, the system was quenched with 2 mL of saturated ammonium chloride solution, and extracted with ethyl acetate. The organic phase was washed with saturated brine, dried over anhydrous sodium sulfate, and concentrated to afford crude product M-4 as a pale yellow oil (243 mg).

Step 3: Preparation of M-5

Intermediate M-4 (240 mg, 1 eq) was dissolved in 1,4-dioxane (2.5 mL, 0.3 M), the mixture was held in an ice bath, and 4 M hydrogen chloride in dioxane (1 mL, 5.7 eq) was added dropwise. After addition, the mixture was stirred at room temperature for 16 h. The reaction mixture was directly concentrated, separated and purified by flash column chromatography (100% DCM - 10% MeOH:DCM) to afford intermediate M-5 as a colorless oil (21 mg).

MS ESI: m/z=145.2, $[M+H]^+$.

**Preparation of Intermediate M-6**

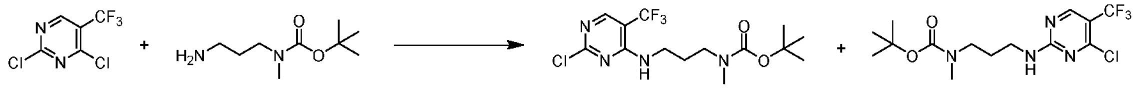

M-6 M-7

Using 2,4-dichloro-5-trifluoromethylpyrimidine and N-methyl-N-Boc-1,3-propanediamine as raw materials, intermediate M-6 was obtained under the same conditions as those for the preparation of intermediate M-1 in Example 6.

MS ESI: m/z=369.2, $[M+H]^+$.

**Preparation of Intermediates M-12 and M-13**

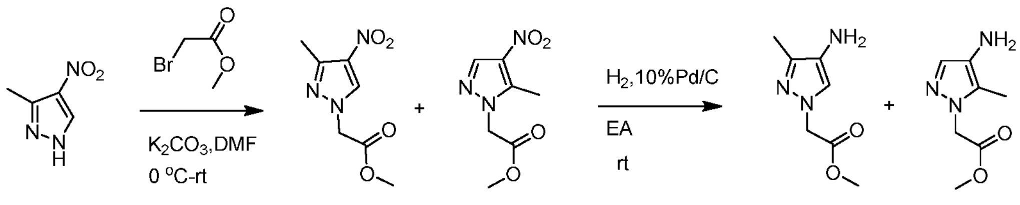


M-8 M-9 M-10 M-11

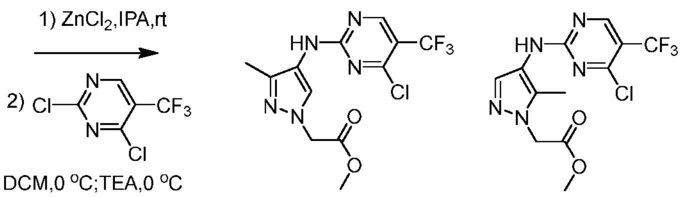


M-12 M-13

Step 1: Preparation of M-8 and M-9

Using 3-methyl-4-nitropyrazole (2.54 g, 1.0 eq) and methyl 2-bromoacetate (4.59 g, 1.5 eq) as raw materials, a mixture of intermediates M-8 and M-9 (3.7 g) as pale yellow oil was obtained under the same conditions as those for the preparation of Z-1 in Step 1 of Example 1.
MS ESI: m/z=200.1, $[M+H]^+$.

Step 2: Preparation of M-10 and M-11

Using the mixture of M-8 and M-9 (1.99 g) as starting material, a mixture of intermediates M-10 and M-11 (1.21 g) as brown oil was obtained under the same conditions as those for the preparation of Z-2 in Step 2 of Example 1.

Step 3: Preparation of M-12 and M-13

Using the mixture of M-10 and M-11 (1.11 g) as starting material, intermediate M-12 as a pink-white solid (850 mg, Rf: 0.15, 20% EA/PE) and M-13 (Rf: 0.3, 20% EA/PE) were obtained under the same conditions as those for the preparation of Z-3 in Step 3 of Example 1.
MS ESI: m/z=350.2, $[M+H]^+$.

**Preparation of Intermediate M-17**

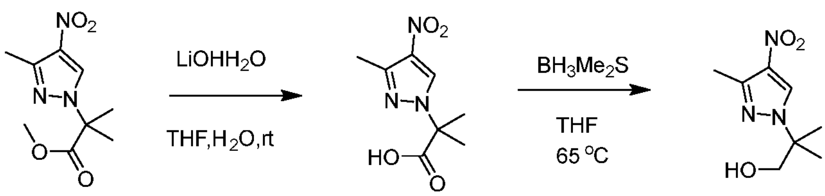


Z-1 M-14 M-15

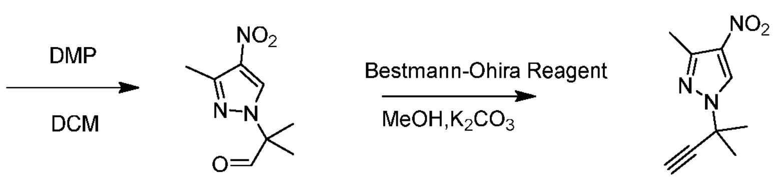


M-16 M-17

Step 1: Preparation of M-14

Using Z-1 (4g, 1.0eq) as starting material, intermediate M-14 (3.4 g) was obtained as a white solid under the same conditions as those for the preparation of Z-5 in Step 5 of Example 1.
MS ESI: m/z=212.2, $[M-H]^+$.

Step 2: Preparation of M-15

Intermediate M-14 (2.2 g, 1.0 eq) was dissolved in THF (40 mL) in a 100 mL sealed tube, and the mixture was stirred at room temperature, then 2M borane-dimethyl sulfide in THF (10 mL, 2.0 eq) was added. The system was allowed to react at 65°C for 48 h, then cooled to room temperature. Upon completion of the reaction, methanol and ammonium chloride solution were added to the system to quench the reaction. Most of the THF was removed by rotary evaporation, and the residue was diluted with ethyl acetate. Saturated brine was added, and the organic phase was dried over anhydrous sodium sulfate and concentrated, then separated and purified by flash column chromatography (100% PE - 30% EA/PE) to afford M-15 as a solid (2 g).
MS ESI: m/z=200.1, $[M+H]^+$.

Step 3: Preparation of M-16

Intermediate M-15 (1.59 g, 1.0 eq) was dissolved in DCM (110 mL), the mixture was held in an ice bath and stirred, then a suspension of Dess-Martin periodinane (3.8 g, 1.1 eq) in DCM (15 mL) was added dropwise. After addition, the system was allowed to react at room temperature for 12 h. Upon completion of the reaction, the mixture was treated with saturated aqueous sodium bicarbonate and sodium thiosulfate solutions, and stirred until the upper layer became clear. The organic phase was dried over anhydrous sodium sulfate, concentrated, then separated and purified by flash column chromatography (100% PE - 30% EA/PE) to afford M-16 as a white solid (1.34 g).

Step 3: Preparation of M-17

Intermediate M-16 (985 mg, 1.0 eq) was dissolved in MeOH (30 mL), the mixture was stirred at room temperature, and then dimethyl (1-diazo-2-oxopropyl)phosphonate (1.92 g, 2.0 eq) and potassium carbonate (1.38 g, 2.0 eq) were added in one portion. The mixture was stirred at room temperature for 14 h. Upon completion of the reaction, the system was poured into 30 g of ice and extracted three times with ethyl acetate, then dried over anhydrous sodium sulfate. The organic phase was concentrated, then separated and purified by flash column chromatography (100% PE - 10% EA/PE) to afford M-17 as a pale yellow solid (854 mg).
MS ESI: m/z=194.1, $[M+H]^+$.

**Preparation of Intermediate M-19**

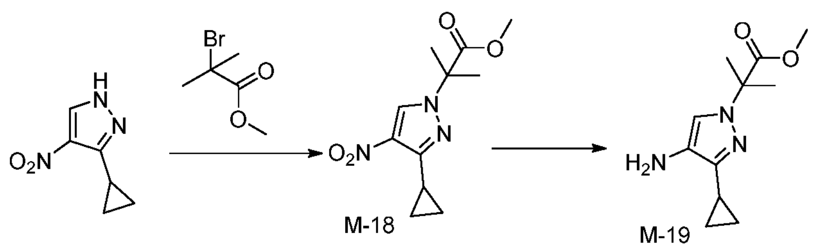

Step 1: Preparation of M-18

Using 3-cyclopropyl-4-nitro-1H-pyrazole as starting material, M-18 was prepared under the same conditions as those in Step 1 of Example 1.

Step 2: Preparation of M-19

M-19 (500 mg) was dissolved in MeOH (10 mL), then iron powder (553.4 mg) and $NH_4Cl$ (1057 mg) were added. The atmosphere was replaced with $N_2$, and the mixture was stirred at 80°C for 4 h, then filtered and evaporated using a

rotary evaporator to afford M-19.

MS ESI: m/z=224, $[M+H]^+$.

**Preparation of Intermediate M-21**

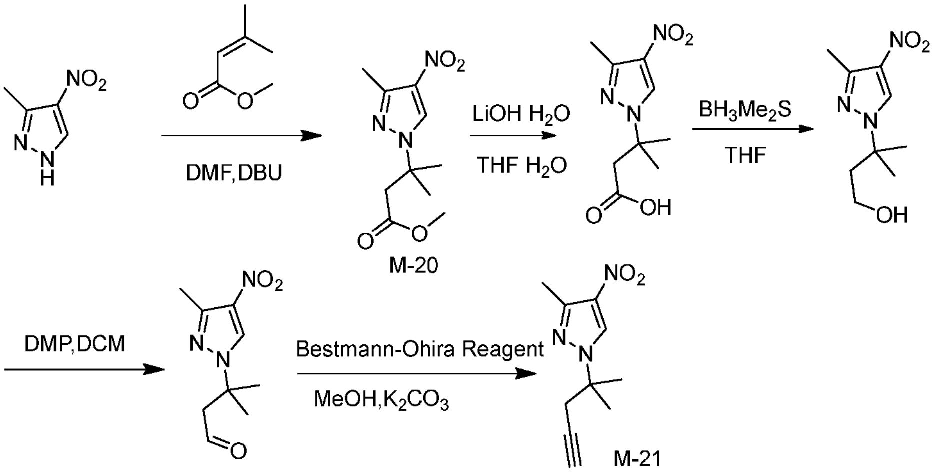


M-20

M-21

Step 1: Preparation of M-20

3-Methyl-4-nitropyrazole (20 g) was dissolved in DMF (100 mL), then methyl 3,3-dimethylacrylate (53.8 g) and DBU (62 g) were sequentially added at room temperature. The system was allowed to react at room temperature for 20 h. The system was treated with water and ethyl acetate, then stirred and partitioned. The organic phase was washed with saturated brine, dried over anhydrous sodium sulfate, concentrated, separated and purified by flash column chromatography to afford intermediate M-20 as a pale yellow oil (10.69 g).

Step 2: Preparation of M-21

Using M-20 as raw material, M-21 was prepared using the same preparation method as described for intermediate M-17.

**Kinase Activity Inhibition Assay**

The kinase activity inhibition assay was performed by ICE Bioscience (Beijing) using ADP-Glo and HTRF methods.

1. Kinase Reaction Reagent Formulation

1.1. Kinase Reaction Buffer (1X)

Table 1.1-1 ADP-Glo Buffer

| Name | Stock Concentration | Working Concentration |
|---|---|---|
| Hepes | 1M | 50 mM |
| $MgCl_2$ | 1M | 10 mM |
| Brij35 | 30% | 0.01% |
| DTT | 1M | 2 mM |
| $H_2O$ | N/A | N/A |

(continued)

| Name | Stock Concentration | Working Concentration |
|---|---|---|
| EGTA | N/A | 1mM |
| 2 mM DTT, prepared fresh before use. | | |

Table 1.1-2 HTRF Buffer

| Reagent | Work Conc. |
|---|---|
| 5× Buffer | 1× |
| $MgCl_2$ | 5 mM |
| SEB | 12.5 nM |
| $MnCl_2$ | 1 mM |
| DTT | 1 mM |

1.2. Kinase Formulation

Table 1.2-1 ADP-Glo Kinase and Substrate Formulation

| Kinase | Kinase | | Substrate | | ATP |
|---|---|---|---|---|---|
| | Store Conc.(nM) | Work Conc.(nM) | Name | Work Conc.(mg/mL) | Work Conc.(μM) |
| LRRK2 | 1074 | 5 | LRRK tide | 0.1 | 20 |
| LRRK2 G2019S | 781 | 4.88 | LRRK2 | 0.1 | 100 |
| DYRK2 | 1053 | 0.53 | DYRK | 0.1 | 5 |
| FGFR4 V550L | 1538 | 15.38 | Poly | 0.1 | 20 |
| FLT1 | 2222 | 2.00 | IGFIR | 0.1 | 50 |

Table 1.2-2 HTRF Kinase and Substrate Formulation

| Kinase | Kinase | | Substrate | | ATP |
|---|---|---|---|---|---|
| | Store Conc.(nM) | Work Conc.(nM) | Name | Work Conc.(μM) | Work Conc.(μM) |
| FGFR1 | 2667 | 0.10 | TK | 1 | 40 |
| FGFR2 | 2667 | 0.08 | TK | 1 | 20 |
| FGFR3 | 2941 | 0.30 | TK | 1 | 50 |
| FGFR4 | 8293 | 10.00 | TK | 1 | 50 |
| FGFR2 V5641 | 2667 | 0.03 | TK | 1 | 20 |
| FGFR3 K650M | 1370 | 0.10 | TK | 1 | 12 |
| FGFR3 V555L | 2941 | 0.02 | TK | 1 | 20 |
| RET V804L | 1351 | 1.35 | TK | 1 | 5 |
| FGFR4 N535K | 1538 | 1.00 | TK | 1 | 100 |
| TYRO3 | 2632 | 0.07 | TK | 1 | 0.5 |
| FGFR1 V561M | 1333 | 3.00 | TK | 1 | 20 |
| FGFR4 V550M | 1538 | 6.00 | TK | 1 | 60 |
| FGFR2 V564F | 1351 | 0.02 | TK | 1 | 15 |
| FGFR3 V555M | 1370 | 0.55 | TK | 1 | 20 |

(continued)

| Kinase | Kinase | | Substrate | | ATP |
|---|---|---|---|---|---|
| | Store Conc.(nM) | Work Conc.(nM) | Name | Work Conc.(μM) | Work Conc.(μM) |
| FGFR2 N549H | 1389 | 0.02 | TK | 1 | 9 |
| FLT4 | 2410 | 0.12 | TK | 1 | 32 |
| PDGFRα | 2247 | 0.12 | TK | 1 | 0.5 |
| KDR | 5900 | 0.07 | TK | 1 | 4 |
| FLT3 | 2597 | 0.02 | TK | 1 | 2 |

2. Experimental Procedure

**ADP-Glo &&HTRF METHODS**

1) 2×ATP/substrate solution and 2×kinase/metal ion solution were prepared using kinase reaction buffer;
2) 20 nL (ADP-GLo) or 40 nL (HTRF) of compound dilution was transferred using Echo 655 onto a 384-well assay plate; after centrifugation, 2 μL of 2×kinase and metal ion solution were added to the 384-well assay plate, centrifuged at 1000 rpm for 1 min, and incubated at 25°C for 10 min;
3) 2 μL of 2×substrate and ATP solution were added to the 384-well assay plate, centrifuged at 1000 rpm for 1 min, and incubated at 25°C for 60 min.
4) 4 μL of ADP-Glo was transferred to the 384-well assay plate, entrifuged at 1000 rpm for 1 min, and incubated at 25°C for 40 min.
5) 8 μL of detection solution was transferred to the 384-well assay plate, centrifuged at 1000 rpm for 1 min, and incubated at 25°C for 40 min.
6) Luminescence signals was read using a multifunctional microplate reader.

3. Data Processing Method

The reading of the negative control was set as 0% inhibition rate and the reading of the positive control was set as 100% inhibition rate, then the inhibition rate of each test solution was calculated.

$$\%\text{ Compound inhibition rate}=\left[1-\frac{\text{Data}_{\text{compound}}-\overline{\text{Data}}_{\text{positive control}}}{\overline{\text{Data}}_{\text{negative control}}-\overline{\text{Data}}_{\text{positive control}}}\right]*100\%$$

$\text{Data}_{\text{positive control}}$:Average ratio of positive control wells
$\text{Data}_{\text{negative control}}$: Average ratio of negative control wells

The $IC_{50}$ (half-maximal inhibitory concentration) of compounds was calculated using the following nonlinear fitting formula:

$$Y=\text{Bottom} + (\text{Top}-\text{Bottom})/(1+10\text{^}((\text{LogIC}_{50}-X)*\text{HillSlope}))$$

X: Logarithm of compound concentration
Y: Compound inhibition rate (% inh)
**Z' factor calculation equation:**

$$Z'=1-3(\text{SDmin}+\text{SDmax})/(\text{AVEmax}-\text{AVEmin})$$

wherein:
min is the data value of positive control, and max is the data value of negative control DMSO. SD is the standard error, and AVE is the average value.

Table 2-1 LRRK2 Activity Inhibition Test Results

| Example | LRRK2 wt | LRRK2 G2019S |
|---|---|---|
| Example | $IC_{50}$ or inhibition rate at 100 nM | |
| DNL-201 | 1.27nM | 2.88nM |
| T-1 | 4.48nM | 6.73nM |
| T-2 | 55%@100 nM | |
| T-3 | 4.5nM | 6.7nM |
| T-4 | 1.11nM | 3.4nM |
| T-5 | 12.06 nM | |
| T-6 | 92%@100 nM | 88%@100 nM |
| T-7 | 7%@300 nM | 4.3%@100 nM |
| T-8 | 64%@100 nM | 52%@100 nM |
| T-9 | -0.5%@100 nM | |
| T-10 | 94%@100 nM | 92%@100 nM |
| T-11 | 89%@100 nM | 90%@100 nM |
| T-12 | 5.7%@100 nM | 0.34%@300 nM |
| T-13 | 96%@100 nM | 92%@100 nM |
| T-14 | 87%@100 nM | 84%@100 nM |
| T-15 | 5%@300 nM | -9.6%@100 nM |
| T-16 | 86.07%@100 nM | |
| T-17 | 96.88%@100 nM | |
| T-18 | 6.61%@100 nM | |
| T-19 | 11.59%@100 nM | |
| T-20 | 0.84%@100 nM | |
| T-21 | 60.09%@100 nM | |
| T-22 | 92.46%@100 nM | |
| T-23 | 19.15%@100 nM | |
| T-24 | 57.28%@100 nM | |
| T-25 | 4.14nM | 2.24nM |
| T-26 | 98.4%@100 nM | |
| T-27 | 88.03%@100 nM | |
| T-28 | 98.35%@100 nM | |
| T-29 | 95.63%@100 nM | |
| T-30 | 61.27%@100 nM | |
| T-31 | 8.85%@100 nM | |
| T-32 | 91.63%@100 nM | |
| T-33 | 2.45%@100 nM | |
| T-34 | -12.71%@100 nM | |
| T-35 | 9.41%@100 nM | |
| T-36 | 28.87% @100 nM | |
| T-37 | 83.43%@100 nM | |

(continued)

| Example | LRRK2 wt | LRRK2 G2019S |
|---|---|---|
| T-38 | 37.72%@100 nM | |
| T-39 | 28.9%@100 nM | |
| T-40 | 56.07%@100 nM | |
| T-41 | 20.76%@100 nM | |
| T-42 | 42.08%@100 nM | |
| T-43 | 64.61%@100 nM | |
| T-44 | 56.78%@100 nM | |
| T-45 | -1.97%@100 Nm | |
| T-46 | 95.14%@100 nM | |

Table 2-2 Other Kinase Activity Inhibition Test Results

| Assay ID | Compound ID | $IC_{50}$(nM) | Assay ID | Compound ID | $IC_{50}$(nM) |
|---|---|---|---|---|---|
| FGFR4 V550L | Staurosporine | 16.6 | TYRO3 | Staurosporine | 1.009 |
| | T-1 | 67.41 | | T-1 | 277.6 |
| | T-25 | 207.5 | | T-25 | 56.06 |
| DYRK2 | Staurosporine | 85.1 | FGFR1 V561M | Infigratinib | 31.29 |
| | T-1 | 248.1 | | T-1 | 26.75 |
| | T-25 | 27.67 | | T-25 | 13.78 |
| FGFR1 | Infigratinib | 0.1632 | FGFR4 V550M | Staurosporine | 4.412 |
| | T-1 | 134.8 | | T-1 | 126.4 |
| | T-25 | 180.3 | | T-25 | 104.5 |
| FGFR2 | Infigratinib | 0.1198 | FGFR2 V564F | Infigratinib | 480.6 |
| | T-1 | 21.81 | | T-1 | 8.224 |
| | T-25 | 31.85 | | T-25 | 1.728 |
| FGFR3 | Infigratinib | 0.2373 | FGFR3 V555M | Infigratinib | 153.4 |
| | T-1 | 50.21 | | T-1 | 12.74 |
| | T-25 | 67.49 | | T-25 | 5.067 |
| FGFR4 | BLU554 | 2.985 | FGFR2 N549H | Infigratinib | 0.7378 |
| | T-1 | 32.94 | | T-1 | 16.42 |
| | T-25 | 58.32 | | T-25 | 17.45 |
| FGFR2 V564I | Infigratinib | 43.67 | FLT1 | Nintedanib | 5.411 |
| | T-1 | 76.12 | | T-1 | 15.16 |
| | T-25 | 60.2 | | T-25 | 8.829 |
| FGFR3 K650M | Infigratinib | 1.635 | FLT4 | Nintedanib | 11.22 |
| | T-1 | 9.096 | | T-1 | 65.11 |
| | T-25 | 14.12 | | T-25 | 19.18 |
| FGFR3 V555L | Infigratinib | 497.8 | PDGFRα | Nintedanib | 2.839 |
| | T-1 | 5.09 | | T-25 | 67.04 |
| | T-25 | 8.814 | | R-1847 | 17.22 |
| RET V804L | Pralsetinib | 0.1947 | KDR | Nintedanib | 1.326 |
| | T-1 | 175.2 | | T-1 | 18.89 |
| | T-25 | 43.89 | | T-25 | 7.671 |
| | | | | R-1860 | 0.4353 |
| FGFR4 N535K | BLU554 | 42.51 | FLT3 | CCT241736 | 13.87 |
| | T-1 | 609.1 | | T-1 | 369.8 |
| | T-25 | 621.4 | | T-25 | 88.11 |

**Oral pharmacokinetic study in rats:**

Two male SD rats were used as experimental animals, administered via oral gavage at a dose of 5 mg/kg. Drug preparation: 1% MC was added to the compound, ground, and shaken uniformly. Samples were collected at 5 min, 15 min, 30 min, 1 h, 2 h, 4 h, and 8 h post-dose, followed by analysis using liquid chromatography-tandem mass spectrometry (LC-MS/MS).

Table 3 PK Data of Selected Compounds

| Compound Number | $T_{1/2}$ (h) | $T_{max}$ (h) | $C_{max}$(ng/mL) | $AUC_{0-8}$ (h*ng/mL) | B/P (1h) | Dose |
|---|---|---|---|---|---|---|
| T-1 | 2.3 | 1 | 754.5 | 3818.43 | 0.13 | 3mpk |
| T-25 | 1.73 | 1 | 629.5 | 2205.61 | 0.58 | 5mpk |

All documents mentioned in the present disclosure are incorporated herein by reference as if each document were individually incorporated by reference. Furthermore, it should be understood that after reading the above teachings of the present disclosure, those skilled in the art may make various modifications or alterations to the present disclosure, and such equivalent forms shall also fall within the scope defined by the appended claims of this application.

## Claims

1. A compound as shown in general formula I or a pharmaceutically acceptable salt, stereoisomer, tautomer, or prodrug thereof:

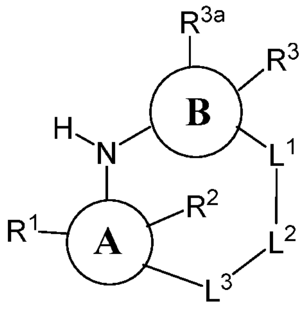

I

wherein:

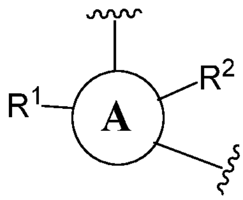

is selected from the following ring structures:

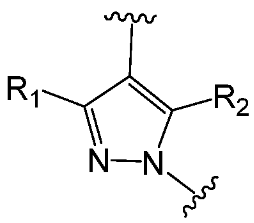 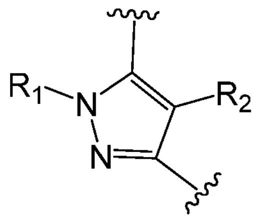 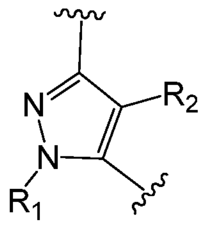 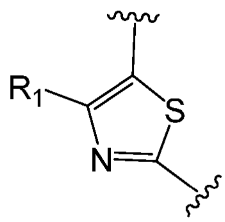

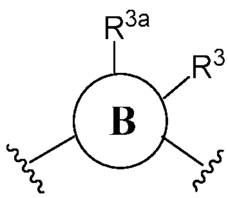

is selected from the following ring structures:

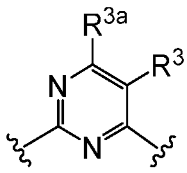
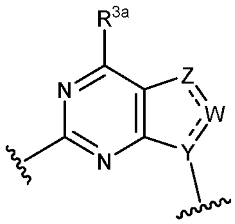
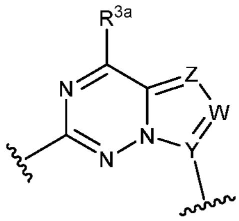
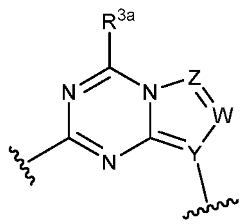

$R^1$ is selected from hydrogen, halo, cyano, $SF_5$, $C_{1-12}$ alkyl, $C_{2-12}$ alkenyl, $C_{2-12}$ alkynyl, $C_{1-12}$ alkoxy, $C_{3-10}$ cycloalkyl, 3-10 membered heterocyclyl, $C_{6-10}$ aryl, and 5-10 membered heteroaryl, $C(O)R^4$, $C(O)OR^4$, $-NR^4R^5$, $C(O)NR^4R^5$, $SO_2R^4$, $SO_2NR^4R^5$, wherein each alkyl, alkenyl, alkynyl, alkoxy, cycloalkyl, heterocyclyl, aryl, and heteroaryl of $R^1$ is unsubstituted or independently substituted with one or more groups selected from halogen, hydroxy, amino, cyano, $C_{1-6}$ alkoxy, and oxo (=O); $R^2$ is selected from hydrogen, halogen, cyano, $C_{1-12}$ alkyl,$C_{2-6}$ alkenyl, $C_{2-6}$ alkynyl, $C_{1-6}$ alkoxy, $C_{3-6}$ cycloalkyl, $C(O)R^4$, $C(O)OR^4$, $-NR^4R^5$, $C(O)NR^4R^5$, $SO_2R^4$, $SO_2NR^4R^5$, wherein each alkyl, alkenyl, alkynyl, alkoxy, and cycloalkyl of $R^2$ is unsubstituted or independently substituted with one or more groups selected from halo, hydroxy, amino, and cyano;

$R^{3a}$ is selected from hydrogen, halo, cyano, $C_{1-12}$ alkyl, $C_{2-6}$ alkenyl, $C_{2-6}$ alkynyl, $C_{1-6}$ alkoxy, $C_{3-6}$ cycloalkyl,$C(O)R^4$, $C(O)OR^4$, $-NR^4R^5$, $C(O)NR^4R^5$, $SO_2R^4$, $SO_2NR^4R^5$,wherein each alkyl, alkenyl, alkynyl, alkoxy, and cycloalkyl of $R^{3a}$ is unsubstituted or independently substituted with one or more groups selected from halo, hydroxy, amino, cyano;

$R^3$ is independently selected from hydrogen, halo, cyano, $SF_5$, $C_{1-12}$ alkyl,$C_{1-12}$ haloalkyl, $C_{2-6}$ alkenyl, $C_{2-6}$ alkynyl, $C_{3-10}$ cycloalkyl, $C_{1-6}$ alkoxy,$-NR^4R^5$, $-C(O)R^4$, $SO_2R^4$, $SO_2NR^4R^5$, $-C(O)OR^4$, and $-C(O)NR^4R^5$, wherein the alkyl of $R^3$ is unsubstituted or substituted with one or more halo or $C_{1-3}$ alkoxy; $R^3$ and $R^{3a}$ may join to form a 5-membered heterocyclic or heteroaromatic ring containing N, $NR^6$, $NC_{1-6}$ alkyl, O, or S; or two $R^3$ join together to form =O;

$R^4$ and $R^5$ are each independently selected from hydrogen, $C_{1-12}$ alkyl, $C_{2-12}$ alkenyl, $C_{2-12}$ alkynyl, $C_{1-12}$ alkoxy, $C_{3-10}$ cycloalkyl, 3-10 membered heterocyclyl, $C_{6-10}$ aryl, and 5-10 membered heteroaryl, wherein each alkyl, alkenyl, alkynyl, alkoxy, cycloalkyl, heterocyclyl, aryl, and heteroaryl of $R^4$ or $R^5$ is unsubstituted or independently substituted with one or more groups selected from halo, hydroxy, amino, nitro, cyano, $(C_{1-6})$ alkoxy, and oxo (=O); or $R^4$ and $R^5$ together with the atoms to which they are attached form a substituted or unsubstituted heterocyclyl;

Y is a carbon atom or nitrogen atom, provided that the requirements for the necessary covalent bonds are satisfied;

Z and W are independently CH, $CH_2$, $(CR^3{}_2)_t$, $(CR^3)_t$, NH, O, S, or N; or Z and W together with the substituents to which they are attached form a 3-7 membered ring, comprising a cycloalkane ring, cycloalkene ring, heterocyclic or heteroaryl ring containing 1-3 heteroatoms selected from N, O, or S; or when Z or W is $(CR^3{}_2)_t$, any two $R^3$ join to the carbon atom to form =O or a 3-6 membered ring, and the ring comprises a cycloalkane or cycloalkene ring;

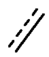

represents represents a single bond or double bond, preferably,

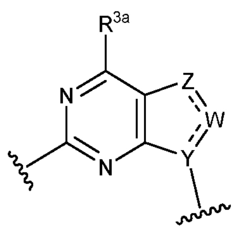

is an aromatic ring;

t is 1, 2, or 3;

Preferably, in the compound of general formula I,

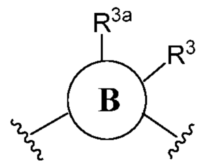

is selected from the following ring structures:

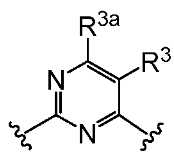 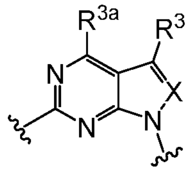 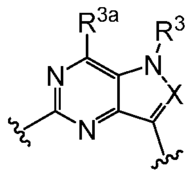 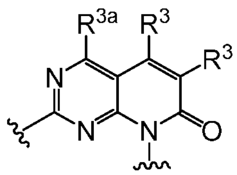 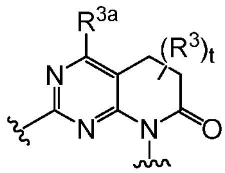

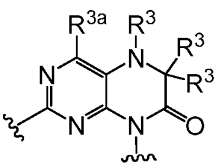 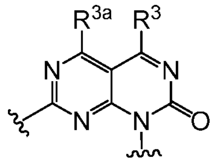 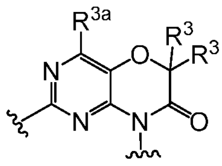 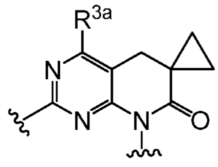 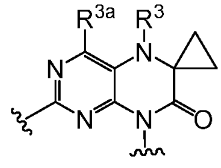

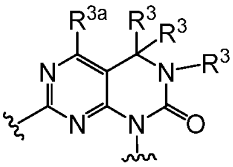 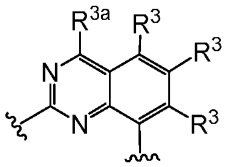 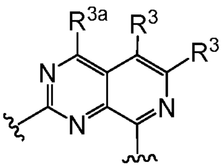 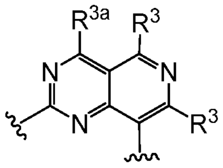 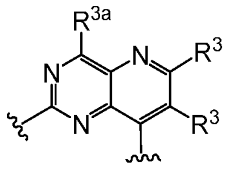

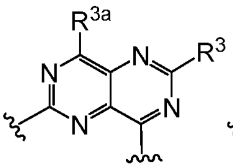 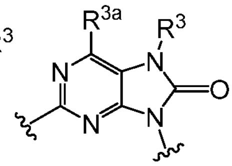 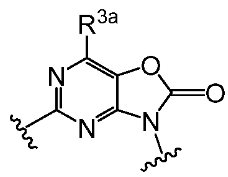 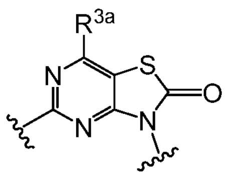 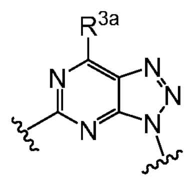

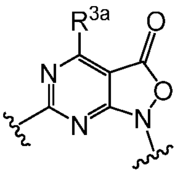 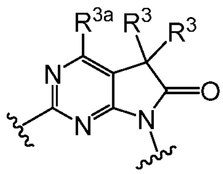 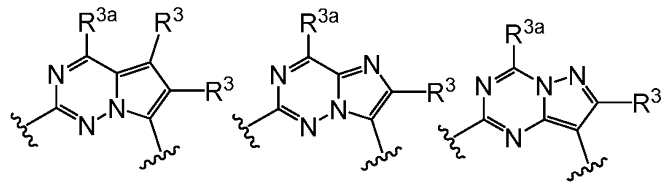

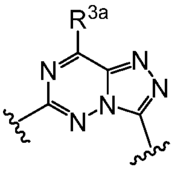 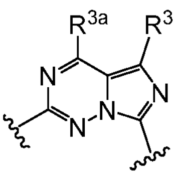 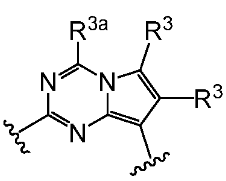 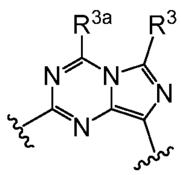 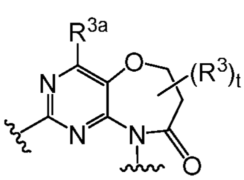

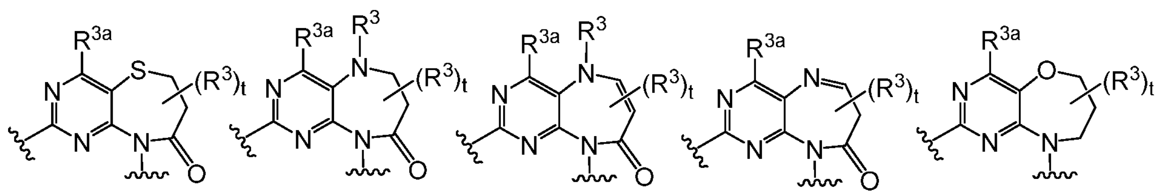

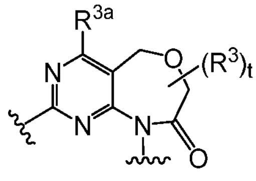 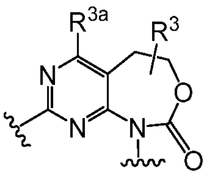 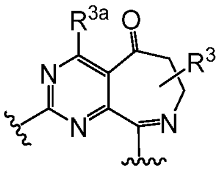 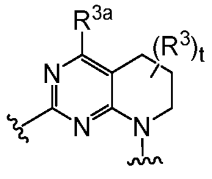 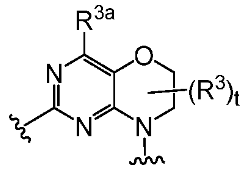

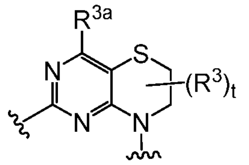 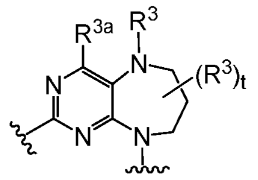 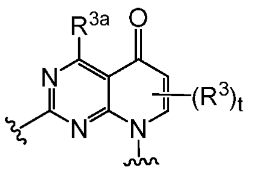 ;

wherein, two $R^3$ may form, together with the same carbon atom or different carbon atoms to which they are attached, a 3- to 10-membered carbocycle or heterocycle, wherein the heteroatom is selected from N, NH, $NC_{1-6}$ alkyl, O, or S;

$L^1$ is selected from a chemical bond, $-(CR^aR^b)_nNR^6(CR^aR^b)_m-$, $-O(CR^aR^b)_nNR^6(CR^aR^b)_m-$, $-(CR^aR^b)_nNR^6C(O)-$, $-O(CR^aR^b)_nNR^6C(O)-$, $-NR^6(CR^aR^b)_nNR^6C(O)-C_{3-6}$ cycloalkyl, $-(CR^aR^b)_nNR^6C(O)-(CR^aR^b)_m$, $-(CR^aR^b)_nC(O)NR^6a-$, $-O(CR^aR^b)_nC(O)NR^6-$, $-(CR^aR^b)_nNR^6(CR^aR^b)_mNR^6-$, $-(CR^aR^b)_nO(CR^aR^b)_m-$, $-NR^6(CR^aR^b)_nO(CR^aR^b)_m-$, $-(CR^aR^b)_nO(CR^aR^b)_mNR^6-$, $-(CR^aR^b)_nO(CR^aR^b)_mO-$, $-(CR^aR^b)_nS(CR^aR^b)_m-$, $-(CR^aR^b)_nS(CR^aR^b)_mNR^6-$, $-(CR^aR^b)_m-$, $-NR^6(CR^aR^b)_m-$, $-(CR^aR^b)_mNR^6-$, $-O(CR^aR^b)_m-$, $-(CR^aR^b)_mO-$, $-O(CR^aR^b)_mO-$, $-(CR^aR^b)_nNR^6C_{3-6}$ cycloalkyl-, $-(CR^aR^b)_nNR^6C_{3-6}$ cycloalkyl-$NR^6-$,$-O(CR^aR^b)_qCH=CH(CR^aR^b)_r-$,$-(CR^aR^b)_qCH=CH(CR^aR^b)_r-$, $-O(CR^aR^b)_qCH=CH(CR^aR^b)_rN-R^6-$,$-O(CR^aR^b)_qCH=CH(CR^aR^b)_rNR^6C(O)-$, $-O(CR^aR^b)_qCH=CH(CR^aR^b)_rC(O)NR^6-$,$-(CR^aR^b)_qCH=CH(CR^aR^b)_rNR^6-$,$-(CR^aR^b)_qCH=CH(CR^aR^b)_rNR^6CO-$, $-(CR^aR^b)_qCH=CH(CR^aR^b)_rC(O)NR^6-$, $-O(CR^aR^b)_nCH=CH(CR^aR^b)_mO-$, $-NR^6(CR^aR^b)_nCH=CH(CR^aR^b)_m-$, $-NR^6(CR^aR^b)_nCH=CH(CR^aR^b)_mO-$, $-NR^6(CR^aR^b)_nCH=CH(CR^aR^b)_mS-$, $-NR^6(CR^aR^b)_nCH=CH(CR^aR^b)_mNR^6-$, $-S(CR^aR^b)_nCH=CH(CR^aR^b)_m-$, $S(CR^aR^b)_nCH=CH(CR^aR^b)_mNR^6-$, $-NR^6(CR^aR^b)_nCH=CH(CR^aR^b)_mS-$, $-(R^aR^b)_nCH=CH(CR^aR^b)_mS-$, $-O(CR^aR^b)_nCH=CH(CR^aR^b)_mS-$, $-S(CR^aR^b)_nCH=CH(CR^aR^b)_mO-$, - phenyl-, -heterocyclyl-, -heteroaryl-, $NR^6(CR^aR^b)_nC\equiv C(CR^aR^b)_m-$;

n is selected from 0, 1, 2, 3, 4, 5, 6, 7, 8, 9, or 10;

m is selected from 1, 2, 3, 4, 5, 6, 7, 8, 9, or 10;

$L^2$ is selected from $-CR^aR^b-$, $-CR^{aa}=CR^{bb}-$, -C(O)-, -C(O)C(O)-, -C(S)-, $-S(O)_2-$, $-C_{3-6}$ cycloalkyl-, - phenyl-, -5- or 6-membered heterocyclyl-, -5- or 6-membered heteroaryl-;

$L^3$ is selected from a chemical bond, $-(CR^aR^b)_q-$, $-(CR^aR^b)_q-NR^6-$, $-NR^6(CR^aR^b)_q-$, $-O(CR^aR^b)_q-NR^6-$, $-NR^6(CR^aR^b)_qO-$, $-O(CR^aR^b)_q-$, $-(CR^aR^b)_qO-$, $-(CR^aR^b)_q-C_{3-6}$ cycloalkyl-, -5- or 6-membered heterocyclyl, $-(CR^aR^b)_q-CR^{aa}=CR^{bb}-$, $-C_{3-6}$ cycloalkyl-$NR^6-$, $-(CR^aR^b)_qNR^6-$, $-(CR^aR^b)_qNR^6C(O)-$, $-(CR^aR^b)_qNR^6C(O)O-$, $-(CR^aR^b)_q C(O)NR^6-$, or $(CR^aR^b)_q OC(O)NR^6-$;

q is selected from 0, 1, 2, 3, 4, 5, or 6;

r is 1, 2, 3, or 4;

each $R^6$ is each independently selected from the group consisting of hydrogen, $C_{1-6}$ alkyl, $C_{3-6}$ cycloalkyl, $-C(O)C_{1-6}$ alkyl, $-C(O)C_{1-6}$ aryl, $-C(O)C_{1-6}$ heteroaryl, $-C(O)C_{3-6}$ cycloalkyl, $-S(O)_2C_{1-6}$ alkyl, $-S(O)_2C_{1-6}$ aryl, $-S(O)_2C_{1-6}$ heteroaryl, $-S(O)_2C_{3-6}$ cycloalkyl, $-C(O)NR^7C_{1-6}$ aryl, $-C(O)NR^7C_{1-6}$ heteroaryl, $-C(O)NR^7C_{3-6}$ cycloalkyl, $-SO_2NR^7C_{1-6}$ alkyl, $-S(O)_2NR^7C_{1-6}$ aryl, $-S(O)_2NR^7C_{1-6}$ heteroaryl, $-S(O)_2NR^7C_{3-6}$ cycloalkyl, $-C(O)C(O)NR^7C_{1-6}$ aryl, $-C(O)C(O)NR^7C_{1-6}$ heteroaryl, or $-C(O)C(O)NR^7C_{3-6}$ cycloalkyl; wherein each alkyl, alkenyl, alkynyl, alkoxy, cycloalkyl, heterocyclyl, aryl, and heteroaryl of $R^6$ is unsubstituted or independently substituted with one or more groups selected from halogen, hydroxy, amino, cyano, $(C_{1-6})$alkoxy, and oxo (=O);

$R^7$ is selected from hydrogen, $C_{1-6}$ alkyl, $C_{3-6}$ cycloalkyl, heterocyclyl, aryl, and heteroaryl; wherein each alkyl, cycloalkyl, heterocyclyl, aryl, and heteroaryl of $R^7$ is unsubstituted or independently substituted with one or more groups selected from halo, hydroxy, amino, cyano, $(C_{1-6})$alkoxy, and oxo (=O);

$R^a$ and $R^b$ are independently selected from hydrogen, halogen, cyano, $C_{1-6}$ alkyl, $C_{1-6}$ haloalkyl, $C_{1-6}$ cycloalkyl, $C_{1-6}$ halocycloalkyl, $C_{1-6}$ alkoxy, $C_{1-6}$ haloalkoxy, hydroxy, $C(O)NH_2$, $C(O)NHC_{1-6}$ alkyl, $C(O)N(C_{1-6}$ alkyl$)_2$, $C_{1-6}$ alkylsulfonyl, $S(O)_2NH_2$, $S(O)_2NHC_{1-6}$ alkyl, $NHC(O)NH_2$, $NHC(O)NHC_{1-6}$ alkyl, $NHC(O)OC_{1-6}$ alkyl, $C(O)-C_{1-6}$ alkyl, $C_{1-6}$ heteroalkyl, heterocyclyl, or heterocyclylalkyl; or $R^a$ and $R^b$ together with the carbon atom to which they are attached may form a 3- to 6-membered carbocycle or heterocycle, wherein the heteroatom in the heterocycle is selected from $NR^6$, O, or S;

$R^{aa}$ and $R^{bb}$ are independently selected from hydrogen, halogen, cyano, $C_{1-6}$ alkyl, $C_{1-6}$ haloalkyl, $C_{1-6}$ cycloalkyl,

$C_{1-6}$ halocycloalkyl; or $R^{aa}$ and $R^{bb}$ together with the carbon atom to which they are attached may form a 3- to 6-membered carbocycle or heterocycle, wherein the heteroatom in the heterocycle is selected from $NR^6$, O, or S; Unless otherwise specially indicated, each cycloalkyl or cycloalkane ring is $C_{3-6}$ cycloalkyl, each cycloalkenyl or cycloalkene ring is $C_{3-6}$ cycloalkenyl, each heterocyclyl is a 3- to 10-membered heterocyclyl, each aryl is $C_{6-10}$ aryl, and each heteroaryl is a 5- to 10-membered heteroaryl.

**2.** The compound of claim 1, or a pharmaceutically acceptable salt, stereoisomer, tautomer or prodrug thereof, wherein the compound has a structure as shown in the general formula selected from the group consisting of,

| | |
|---|---|
| 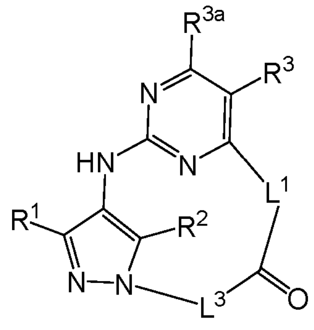 II | 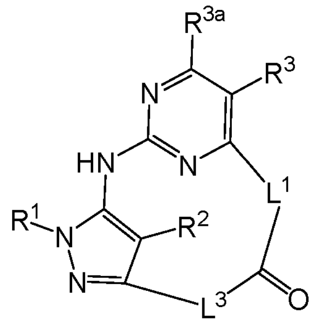 III |
| 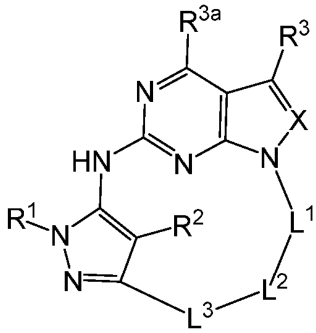 IV | 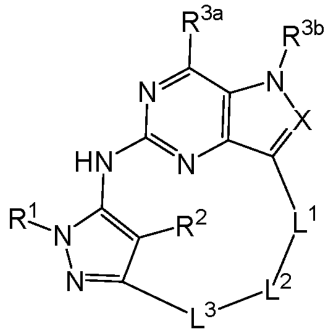 V |
| 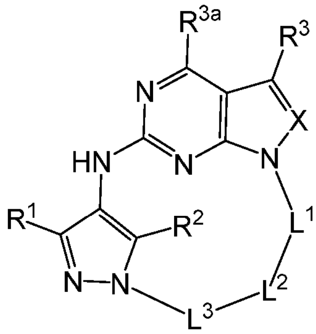 VI | 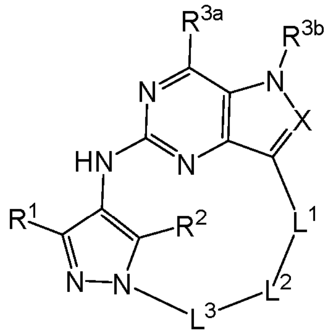 VII |

| | |
|---|---|
| 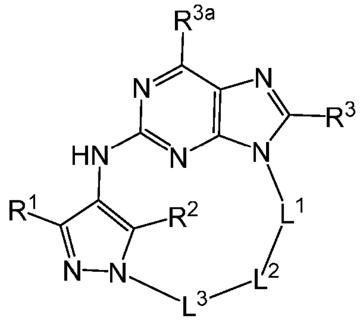<br>VIII | 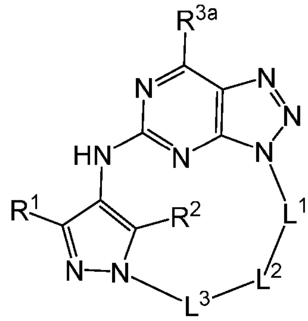<br>IX |
| 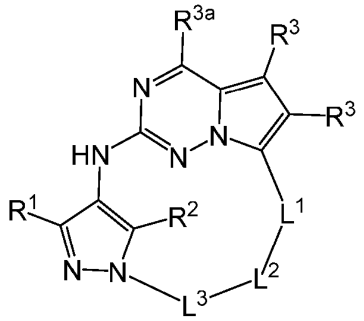<br>X | 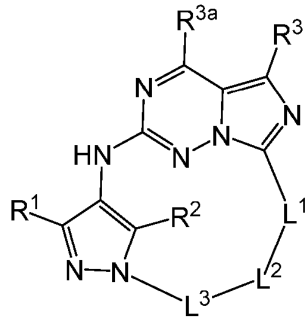<br>XI |
| 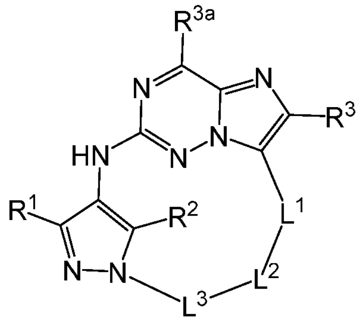<br>XII | 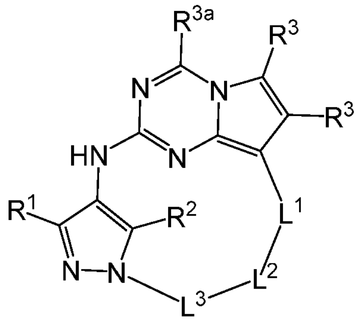<br>XIII |
| 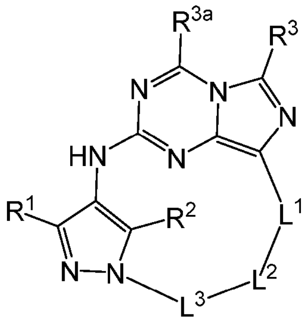<br>XIV | 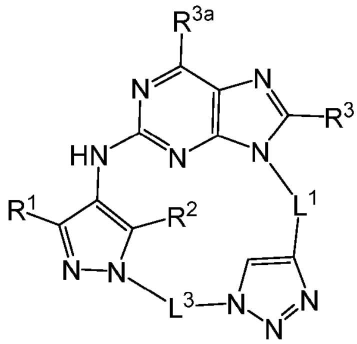<br>XV |

| | |
|---|---|
| 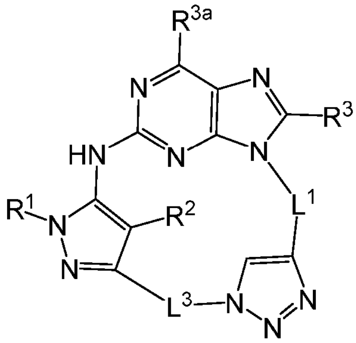<br>XVI | 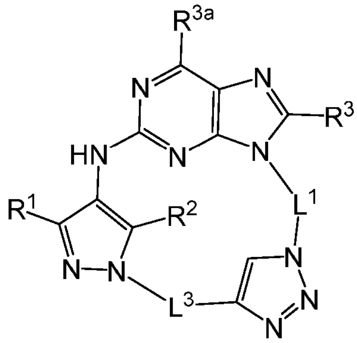<br>XVII |
| 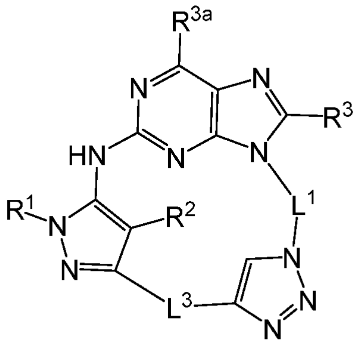<br>XVIII | 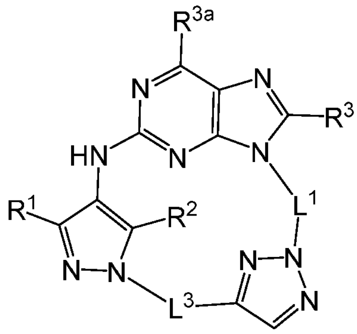<br>XIX |
| 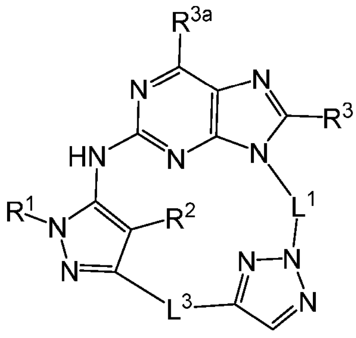<br>XX | 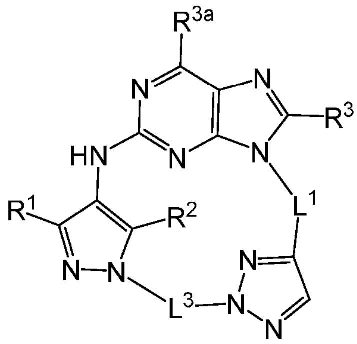<br>XXI |
| 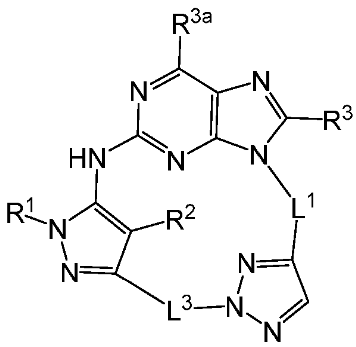<br>XXII | |

wherein each group is as defined in claim 1.

**3.** The compound of claim 1, or a pharmaceutically acceptable salt, stereoisomer, tautomer or prodrug thereof, wherein

the compound has a structure as shown in the formula below,

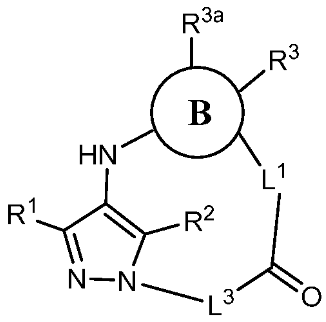

wherein
$R^1$, $R^2$, $R^3$, $R^{3a}$, $L^1$, $L^3$, and ring B are as defined in claim 1.

**4.** The compound of claim 1, or a pharmaceutically acceptable salt, stereoisomer, tautomer, or prodrug thereof, wherein the compound has a structure as shown in the formula below,

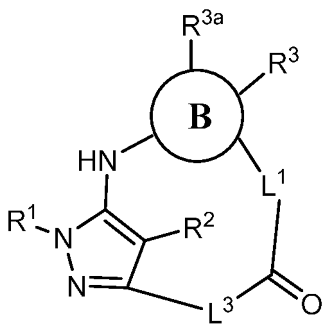

wherein
$R^1$, $R^2$, $R^3$, $R^{3a}$, $L^1$, $L^3$, and ring B are as defined in claim 1.

**5.** The compound of claim 1, or a pharmaceutically acceptable salt, stereoisomer, tautomer or prodrug thereof, wherein $R_1$ is selected from $C_{1-6}$ alkyl, $C_{1-6}$ haloalkyl, or $C_{3-6}$ cycloalkyl;

$R^2$ is selected from hydrogen, halogen, or methyl;
$R^3$ is selected from hydrogen, halogen, $CF_3$, $SF_5$, or CN.

**6.** The compound of claim 1, or a pharmaceutically acceptable salt, stereoisomer, tautomer or prodrug thereof, wherein $L^1$ is selected from: $-NR^6(CR^aR^b)_m-$, $-NR^6C(O)-$, $-NR^6(CR^aR^b)_mNR^6-$, $-NR^6(CR^aR^b)_nO(CR^aR^b)_m-$, $-NR^6C_{3-6}$ cycloalkyl-, $-NR^6C_{3-6}$ cycloalkyl-$NR^6$-, $NR^6(CR^aR^b)_nCH=CH(CR^aR^b)_m-$, $-NR^6(CR^aR^b)_nCH=CH(CR^aR^b)_mO-$, $-NR^6(CR^aR^b)_nCH=CH(CR^aR^b)_mS-$, $-NR^6(CR^aR^b)_nCH=CH(CR^aR^b)_mNR^6-$, $-NR^6(CR^aR^b)_nCH=CH(CR^aR^b)_mS-$, -phenyl-, -heterocyclyl-, -heteroaryl-, and $NR^6(CR^aR^b)_nC\equiv C(CR^aR^b)_m-$.

**7.** The compound of claim 1, or a pharmaceutically acceptable salt, stereoisomer, tautomer or prodrug thereof, wherein $L^3$ is selected from the group consisting of:

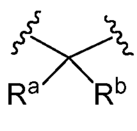 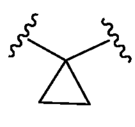 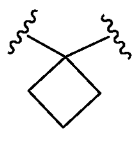 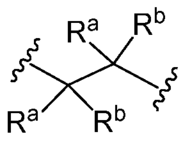 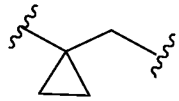 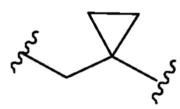

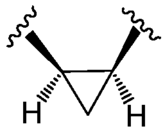 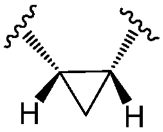 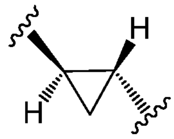 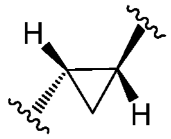 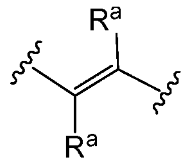 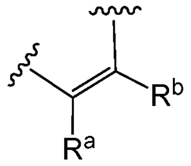

wherein $R^a$ and $R^b$ are each independently selected from hydrogen, halogen, cyano, $C_{1-6}$ alkyl, $C_{1-6}$ haloalkyl, $C_{1-6}$ cycloalkyl, $C_{1-6}$ halocycloalkyl, $C_{1-6}$ alkoxy, $C_{1-6}$ haloalkoxy, or hydroxyl; or $R^a$ and $R^b$ together with the carbon atom to which they are attached may form a 3- to 6-membered carbocycle or heterocycle, wherein the heteroatom in the heterocycle is selected from $NR^6$, O, or S; and $R^6$ is as defined above.

**8.** The compound of claim 1, or a pharmaceutically acceptable salt, stereoisomer, tautomer or prodrug thereof, wherein $L^2$ is selected from -C(O)- or the following heterocyclic structures:

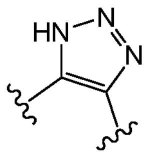 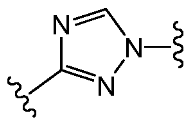 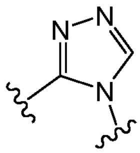 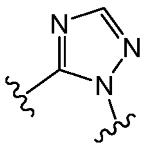 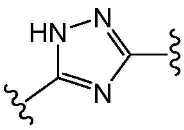 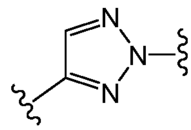

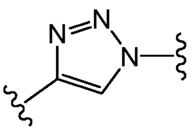 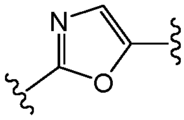 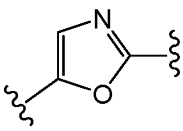 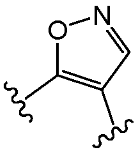 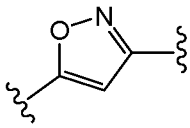 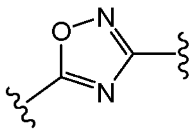

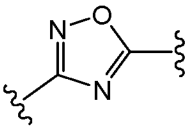 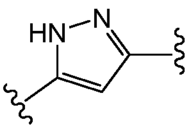 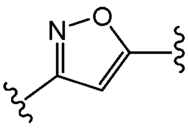 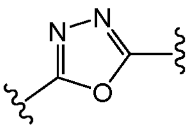 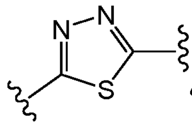 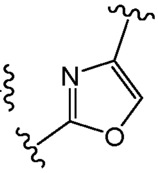

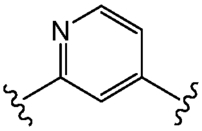 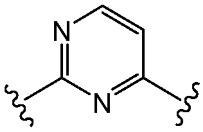 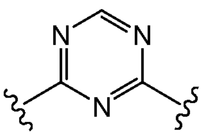 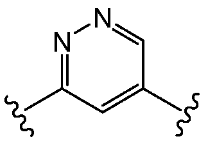 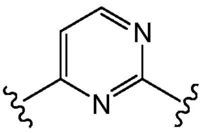

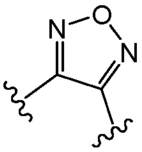 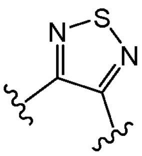 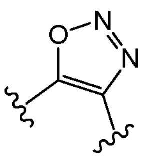 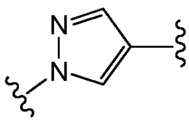 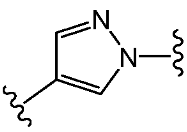

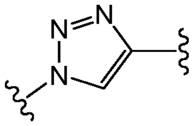 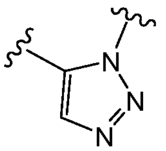 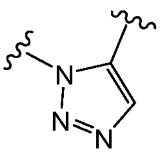

the above heterocyclic ring may be substituted with one or two of halogen, -CN, $C_1$-$C_6$ alkyl, or $C_1$-$C_6$ haloalkyl.

**9.** The compound of claim 1, wherein the compound is selected from all compounds described in the Examples and the Summary of the present disclosure.

**10.** The compound of claim 1, wherein the compound is selected from the group consisting of:

| | |
|---|---|
| 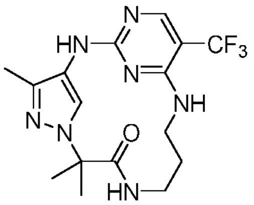 | 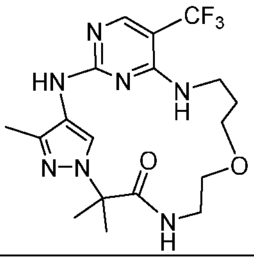 |
| 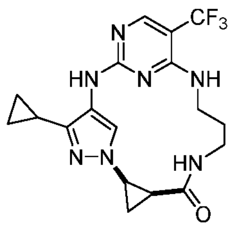<br>(±)<br>T-3 | 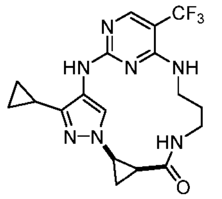 and 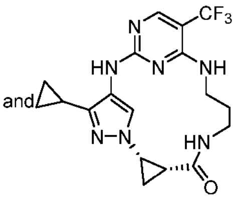<br>(T4 and T5, randomly assigned numbers). |
| 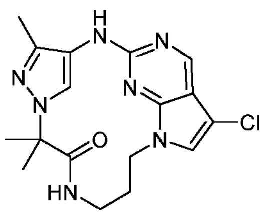<br>T-6 | 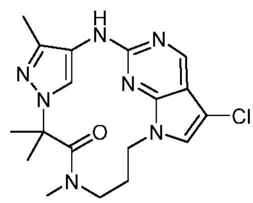<br>T-7 |
| 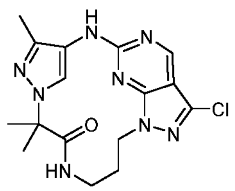<br>T-8 | 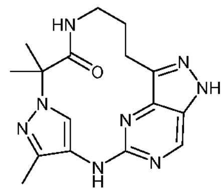<br>T-9 |
| 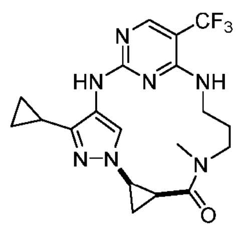<br>(±)<br>T-10 | 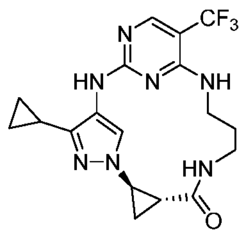<br>(±)<br>T-11 |
| 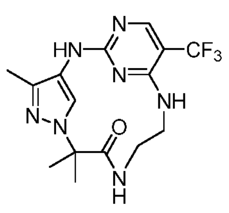<br>T-12 | 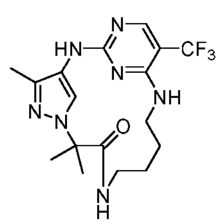<br>T-13 |

| | |
|---|---|
| 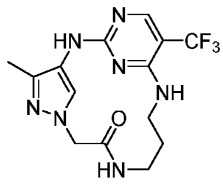<br>T-14 | 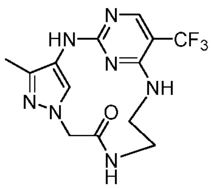<br>T-15 |
| 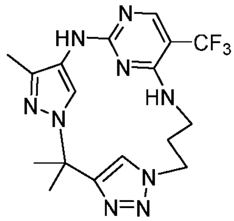<br>T-16 | 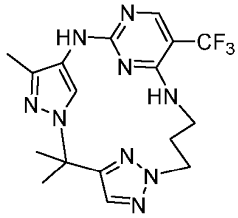<br>T-17 |
| 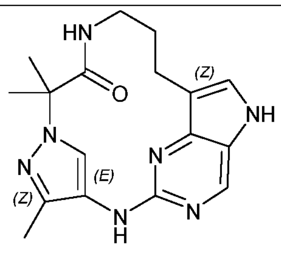<br>T-18 | 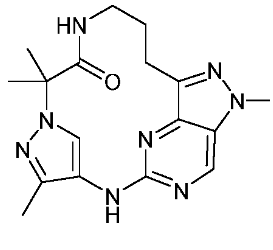<br>T-19 |
| 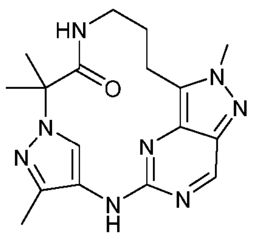<br>T-20 | 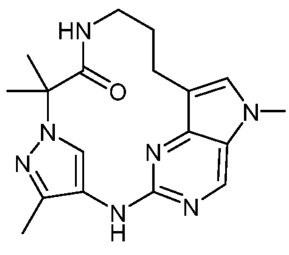<br>T-21 |
| 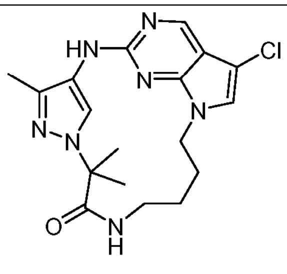<br>T-22 | 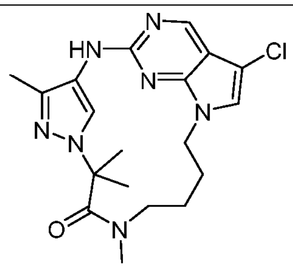<br>T-23 |
| 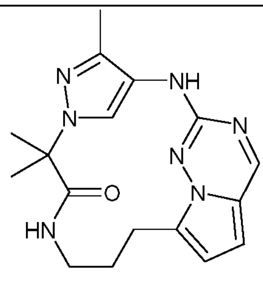<br>T-24 | 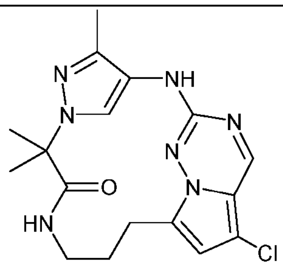<br>T-25 |

| | |
|---|---|
| 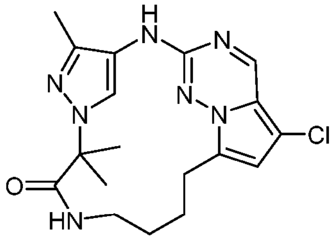<br>T-26 | 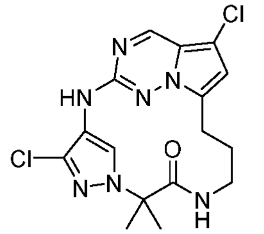<br>T-27 |
| 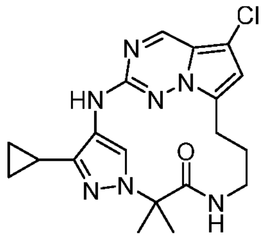<br>T-28 | 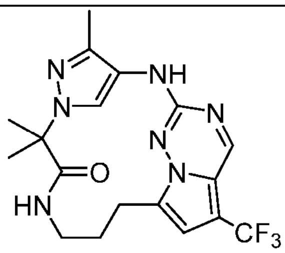<br>T-29 |
| 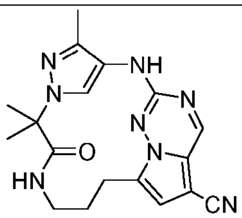<br>T-30 | 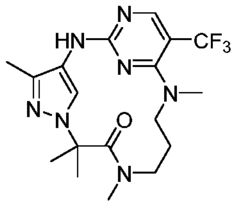<br>T-31 |
| 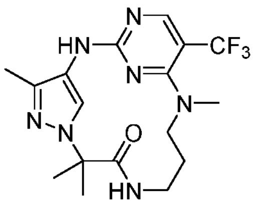<br>T-32 | 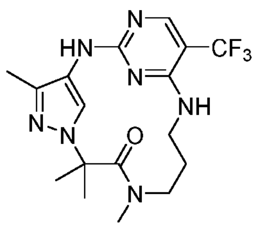<br>T-33 |
| 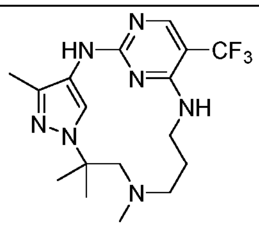<br>T-34 | 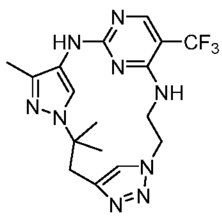<br>T-35 |
| 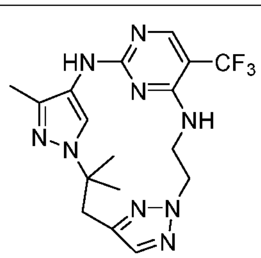<br>T-36 | 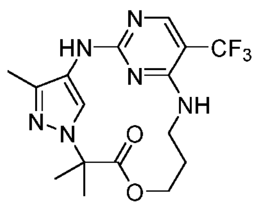<br>T-37 |

| | |
|---|---|
| 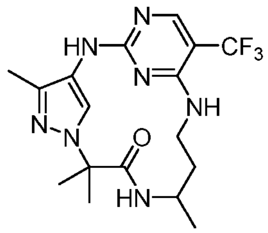 T-38 | 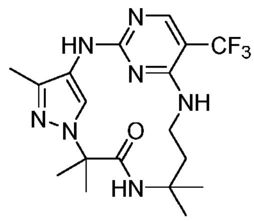 T-39 |
| 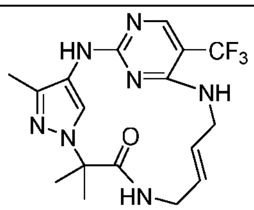 T-40 | 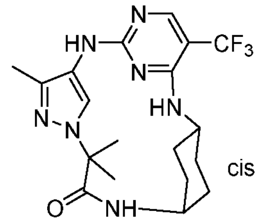 T-41 |
| 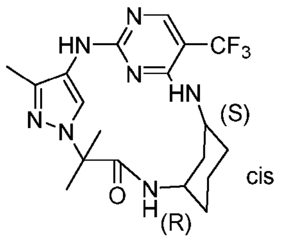 T-42 | 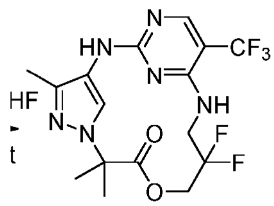 T-43 |
| 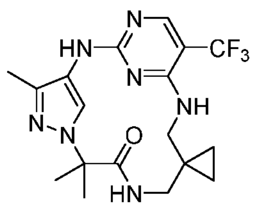 T-44 | 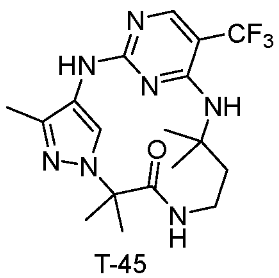 T-45 |
| 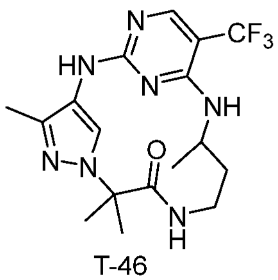 T-46 | |

**11.** Use of the compound of any one of claims 1-10, wherein the compound is used to:

(i) prepare a kinase inhibitor;
(ii) prepare a drug for treating kinase-mediated diseases;
(iii) prepare a drug for treating Parkinson's disease, cancers, and other neurodegenerative diseases;
(iv) treat cancers in combination with an immune checkpoint protein inhibitor or an antibody;
(v) prevention and/or treatment diseases mediated by FGFR, VEGFR, RET, DYRK2, and TYRO3, etc., such as cancers and dyschondroplasia.

**12.** The use of claim 11, wherein the kinase-mediated disease is selected from the group consisting of:cancers,

inflammatory bowel diseases(IBDs), tuberculosis, leprosy, ulcerative colitis, psoriasis, retinal detachment, retinitis pigmentosa, macular degeneration, pancreatitis, atopic dermatitis, rheumatoid arthritis, spondyloarthritis, gout, systemic lupus erythematosus, Sjögren's syndrome, systemic scleroderma, antiphospholipid syndrome, vasculitis, osteoarthritis, non-alcoholic steatohepatitis, autoimmune hepatitis, autoimmune hepatobiliary diseases, primary sclerosing cholangitis, nephritis, celiac disease, autoimmune ITP, transplant rejection, ischemia-reperfusion injury of solid organs, septicemia, systemic inflammatory response syndrome, cerebrovascular accident, myocardial infarction, Huntington's disease, Alzheimer's disease, Parkinson's disease, allergic diseases, asthma, multiple sclerosis, diabetes, Wegener's granulomatosis, pulmonary sarcoidosis, Behcet's disease, interleukin-I invertase-associated fever syndrome, chronic obstructive pulmonary disease, tumor necrosis factor receptor-associated periodic syndrome, periodontitis, achondroplasia, thanatophoric dysplasia, Crouzon syndrome-acanthosis nigricans syndrome, CATSHL syndrome, LADD syndrome, Muenke syndrome, and SADDAN syndrome, etc.

**13.** A pharmaceutical composition, wherein the pharmaceutical composition comprises a therapeutically effective amount of the compound of any one of claims 1-10, and a pharmaceutically acceptable carrier.

**INTERNATIONAL SEARCH REPORT**

International application No. **PCT/CN2023/132436**

**A. CLASSIFICATION OF SUBJECT MATTER**

C07D487/16(2006.01)i; C07D498/16(2006.01)i; C07D487/22(2006.01)i; A61K31/519(2006.01)i; A61K31/53(2006.01)i; A61K31/554(2006.01)i; A61P35/00(2006.01)i; A61P37/00(2006.01)i; A61P29/00(2006.01)i; A61P1/00(2006.01)i; A61P17/00(2006.01)i; A61P3/10(2006.01)i; A61P27/02(2006.01)i; A61P9/14(2006.01)i; A61P25/16(2006.01)i

According to International Patent Classification (IPC) or to both national classification and IPC

**B. FIELDS SEARCHED**

Minimum documentation searched (classification system followed by classification symbols)

IPC:
C07D487/16, C07D498/16, C07D487/22, A61K31/519, A61K31/53, A61K31/554, A61P35/-, A61P37/-, A61P29/-, A61P1/-,
A61P27/02, A61P9/14, A61P25/16, A61P25/28

Documentation searched other than minimum documentation to the extent that such documents are included in the fields searched

Electronic data base consulted during the international search (name of data base and, where practicable, search terms used)

CNTXT, DWPI, VEN, ENTXT, OETXT, CNKI, REGISTRY(STN), MARPAT(STN): 嘧啶, 并, 吡唑, 大环, 激酶, 抑制, macrocycle, pyrimidine, pyrazole, macrocycle, kinase, inhibit, 根据式I化合物进行的结构式检索, structure search according to the compound of formula I

**C. DOCUMENTS CONSIDERED TO BE RELEVANT**

| Category* | Citation of document, with indication, where appropriate, of the relevant passages | Relevant to claim No. |
|---|---|---|
| X | CN 105164136 A (MERCK PATENT GMBH) 16 December 2015 (2015-12-16) abstract, compound A1 of claim 7, and claims 1-3 and 9-16 | 1-13 |
| X | WO 2020085742 A1 (KOREA INSTITUTE OF SCIENCE AND TECHNOLOGY) 30 April 2020 (2020-04-30) abstract, description, paragraphs [266]-[267], and claims | 1-13 |
| PX | WO 2023101387 A1 (UNIV YONSEI IACF) 08 June 2023 (2023-06-08) abstract, description, paragraph [446], and claims | 1-13 |
| PX | WO 2023140629 A1 (DR I&B CO., LTD.) 27 July 2023 (2023-07-27) abstract, description, paragraph [1007], and claims | 1-13 |
| A | CN 110891954 A (GLAXOSMITHKLINE INTELLECTUAL PROPERTY DEVELOPMENT LIMITED) 17 March 2020 (2020-03-17) entire document | 1-13 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 February 2024** | **29 February 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/132436**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11**
because they relate to subject matter not required to be searched by this Authority, namely:
Although the use (iv) of claim 11 relates to a treatment method implemented on the human or animal body (PCT Rule 39.1(iv)), a search is still carried out on the basis of a pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/132436**

| Patent document cited in search report | | | Publication date (day/month/year) | Patent family member(s) | | | Publication date (day/month/year) |
|---|---|---|---|---|---|---|---|
| CN | 105164136 | A | 16 December 2015 | IL | 242347 | B | 30 August 2018 |
| | | | | US | 2016166574 | A1 | 16 June 2016 |
| | | | | US | 9861635 | B2 | 09 January 2018 |
| | | | | ES | 2637721 | T3 | 16 October 2017 |
| | | | | EP | 2994471 | A1 | 16 March 2016 |
| | | | | EP | 2994471 | B1 | 17 May 2017 |
| | | | | JP | 2016517894 | A | 20 June 2016 |
| | | | | JP | 6401247 | B2 | 10 October 2018 |
| | | | | CA | 2911259 | A1 | 13 November 2014 |
| | | | | WO | 2014180524 | A1 | 13 November 2014 |
| | | | | AR | 096149 | A1 | 09 December 2015 |
| | | | | AU | 2014264973 | A1 | 17 December 2015 |
| | | | | AU | 2014264973 | B2 | 18 January 2018 |
| WO | 2020085742 | A1 | 30 April 2020 | KR | 20200046952 | A | 07 May 2020 |
| | | | | KR | 102163494 | B1 | 08 October 2020 |
| WO | 2023101387 | A1 | 08 June 2023 | KR | 20230081667 | A | 07 June 2023 |
| WO | 2023140629 | A1 | 27 July 2023 | KR | 20230112554 | A | 27 July 2023 |
| CN | 110891954 | A | 17 March 2020 | BR | 112020000772 | A2 | 21 July 2020 |
| | | | | EP | 3652179 | A1 | 20 May 2020 |
| | | | | UY | 37808 | A | 28 February 2019 |
| | | | | US | 2020392158 | A1 | 17 December 2020 |
| | | | | CA | 3069554 | A1 | 17 January 2019 |
| | | | | AR | 112392 | A1 | 23 October 2019 |
| | | | | TW | 201920197 | A | 01 June 2019 |
| | | | | WO | 2019012093 | A1 | 17 January 2019 |
| | | | | JP | 2020526543 | A | 31 August 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5607915 A


### Non-patent literature cited in the description


- **MONFRINI, E** ; **DI FONZO, A.** Leucine-Rich Repeat Kinase (LRRK2) Genetics and Parkinson'sDisease.. *Adv. Neurobiol.*, 2017, vol. 14, 3-30
- **DI MAIO, R. et al.** LRRK2 activation in idiopathic Parkinson's disease. *Sci. Transl. Med.*, 2018, vol. 10
- **WALLINGS R.L et al.** *LRRK2 at the Interface Between Peripheral and Central Immune Function in Parkinson's*
- Remington's Pharmaceutical Sciences. Mack Pub. Co, 1991